# NEW EUROPEAN PATENT SPECIFICATION

(11) **EP 3 105 331 B2**
(45) Date of publication and mention of the opposition decision: **25.11.2026**
(45) Mention of the grant of the patent: 23.06.2021
(21) Application number: 15705888.4
(22) Date of filing: 11.02.2015
(51) Int. Cl.: C12N 15/113, A61K 31/713

(54) **KETOHEXOKINASE (KHK) IRNA COMPOSITIONS AND METHODS OF USE THEREOF**
KETOHEXOKINASE (KHK)-IRNA-ZUSAMMENSETZUNGEN UND VERFAHREN ZUR VERWENDUNG DAVON
COMPOSITIONS D'ARNI DE CÉTOHEXOKINASE (KHK) ET PROCÉDÉS POUR LES UTILISER

(30) Priority: 11.02.2014 US 201461938567 P
(43) Date of publication of application: 21.12.2016
(60) Divisional application: 21180120.4 / 3 960 860
(73) Proprietor: Alnylam Pharmaceuticals, Inc., Cambridge, MA 02142 (US)
(72) Inventor: FITZGERALD, Kevin, Cambridge, MA 02142 (US); BETTENCOURT, Brian, Cambridge, MA 02142 (US); HINKLE, Gregory, Cambridge, MA 02142 (US); WILLOUGHBY, Jennifer, Cambridge, MA 02142 (US)
(74) Representative: Vossius & Partner Patentanwälte Rechtsanwälte mbB
(86) International application number: PCT/US2015/015367
(87) International publication number: WO 2015/123264

(56) References cited:
- EP-A1- 1 752 536
- WO-A2-2012/019188
- DATABASE EMBL [online] 20 April 2011 (2011-04-20), "WO 2005116204-A/598150: Double strand polynucleotides generating RNA interference.", XP055418175, retrieved from EBI accession no. EM_PAT:FZ191625 Database accession no. FZ191625
- P. CIRILLO ET AL: "Ketohexokinase-Dependent Metabolism of Fructose Induces Proinflammatory Mediators in Proximal Tubular Cells", JOURNAL OF THE AMERICAN SOCIETY OF NEPHROLOGY, vol. 20, no. 3, 25 February 2009 (2009-02-25), pages 545 - 553, XP055187675, ISSN: 1046-6673, DOI: 10.1681/ASN.2008060576
- KAY LH WU ET AL: "An increase in adenosine-5'-triphosphate (ATP) content in rostral ventrolateral medulla is engaged in the high fructose diet-induced hyperten", JOURNAL OF BIOMEDICAL SCIENCE, KLUWER ACADEMIC PUBLISHERS, DO, vol. 21, no. 1, 27 January 2014 (2014-01-27), pages 8 - 14, XP021175336, ISSN: 1423-0127, DOI: 10.1186/1423-0127-21-8

### SEQUENCE LISTING

<110> ALNYLAM PHARMACEUTICALS, INC.
<120> KETOHEXOKINASE (KHK) IRNA COMPOSITIONS AND METHODS OF USE THEREOF
<130> 121301-01220
<140>
   <141>
<150> 61/938,567
   <151> 2014-02-11
<160> 326
<170> PatentIn version 3.5
<210> 1
   <211> 2433
   <212> DNA
   <213> Homo sapiens
<400> 1 
gggggcgggc cgccgcgacc gcgggcttca ggcagggctg cagatgcgag gcccagctgt 60

acctcgcgtg tcccgggtcg ggagtcggag acgcaggtgc aggagagtgc ggggcaagta 120

gcgcattttc tctttgcatt ctcgagatcg cttagccgcg ctttaaaaag gtttgcatca 180

gctgtgagtc catctgacaa gcgaggaaac taaggctgag aagtgggagg cgttgccatc 240

tgcaggccca ggcaacctgc tacgggaaga ccggggacca agacctctgg gttggctttc 300

ctagacccgc tcgggtcttc gggtgtcgcg aggaagggcc ctgctccttt cgttccctgc 360

acccctggcc gctgcaggtg gctccctgga ggaggagctc ccacgcggag gaggagccag 420

ggcagctggg agcggggaca ccatcctcct ggataagagg cagaggccgg gaggaacccc 480

gtcagccggg cgggcaggaa gctctgggag tagcctcatg gaagagaagc agatcctgtg 540

cgtggggcta gtggtgctgg acgtcatcag cctggtggac aagtacccta aggaggactc 600

ggagataagg tgtttgtccc agagatggca gcgcggaggc aacgcgtcca actcctgcac 660

cgttctctcc ctgctcggag ccccctgtgc cttcatgggc tcaatggctc ctggccatgt 720

tgctgacttc ctggtggccg acttcaggcg gcggggcgtg gacgtgtctc aggtggcctg 780

gcagagcaag ggggacaccc ccagctcctg ctgcatcatc aacaactcca atggcaaccg 840

taccattgtg ctccatgaca cgagcctgcc agatgtgtct gctacagact ttgagaaggt 900

tgatctgacc cagttcaagt ggatccacat tgagggccgg aacgcatcgg agcaggtgaa 960

gatgctgcag cggatagacg cacacaacac caggcagcct ccagagcaga agatccgggt 1020

gtccgtggag gtggagaagc cacgagagga gctcttccag ctgtttggct acggagacgt 1080

ggtgtttgtc agcaaagatg tggccaagca cttggggttc cagtcagcag aggaagcctt 1140
 
gaggggcttg tatggtcgtg tgaggaaagg ggctgtgctt gtctgtgcct gggctgagga 1200

gggcgccgac gccctgggcc ctgatggcaa attgctccac tcggatgctt tcccgccacc 1260

ccgcgtggtg gatacactgg gagctggaga caccttcaat gcctccgtca tcttcagcct 1320

ctcccagggg aggagcgtgc aggaagcact gagattcggg tgccaggtgg ccggcaagaa 1380

gtgtggcctg cagggctttg atggcatcgt gtgagagcag gtgccggctc ctcacacacc 1440

atggagacta ccattgcggc tgcatcgcct tctcccctcc atccagcctg gcgtccaggt 1500

tgccctgttc aggggacaga tgcaagctgt ggggaggact ctgcctgtgt cctgtgttcc 1560

ccacagggag aggctctggg gggatggctg ggggatgcag agcctcagag caaataaatc 1620

ttcctcagag ccagcttctc ctctcaatgt ctgaactgct ctggctgggc attcctgagg 1680

ctctgactct tcgatcctcc ctctttgtgt ccattcccca aattaacctc tccgcccagg 1740

cccagaggag gggctgcctg ggctagagca gcgagaagtg ccctgggctt gccaccagct 1800

ctgccctggc tggggaggac actcggtgcc ccacacccag tgaacctgcc aaagaaaccg 1860

tgagagctct tcggggccct gcgttgtgca gactctattc ccacagctca gaagctggga 1920

gtccacaccg ctgagctgaa ctgacaggcc agtggggggc aggggtgcgc ctcctctgcc 1980

ctgcccacca gcctgtgatt tgatggggtc ttcattgtcc agaaatacct cctcccgctg 2040

actgccccag agcctgaaag tctcaccctt ggagcccacc ttggaattaa gggcgtgcct 2100

cagccacaaa tgtgacccag gatacagagt gttgctgtcc tcagggaggt ccgatctgga 2160

acacatattg gaattggggc caactccaat atagggtggg taaggcctta taatgtaaag 2220

agcatataat gtaaagggct ttagagtgag acagacctgg attcaaatct gccatttaat 2280

tagctgcata tcaccttagg gtacagcact taacgcaatc tgcctcaatt tcttcatctg 2340

tcaaatggaa ccaattctgc ttggctacag aattattgtg aggataaaat catatataaa 2400

atgcccagca tgatgaaaaa aaaaaaaaaa aaa 2433
<210> 2
   <211> 2433
   <212> DNA
   <213> Homo sapiens
<400> 2 
tttttttttt ttttttttca tcatgctggg cattttatat atgattttat cctcacaata 60

attctgtagc caagcagaat tggttccatt tgacagatga agaaattgag gcagattgcg 120

ttaagtgctg taccctaagg tgatatgcag ctaattaaat ggcagatttg aatccaggtc 180

tgtctcactc taaagccctt tacattatat gctctttaca ttataaggcc ttacccaccc 240

tatattggag ttggccccaa ttccaatatg tgttccagat cggacctccc tgaggacagc 300

aacactctgt atcctgggtc acatttgtgg ctgaggcacg cccttaattc caaggtgggc 360
 
tccaagggtg agactttcag gctctggggc agtcagcggg aggaggtatt tctggacaat 420

gaagacccca tcaaatcaca ggctggtggg cagggcagag gaggcgcacc cctgcccccc 480

actggcctgt cagttcagct cagcggtgtg gactcccagc ttctgagctg tgggaataga 540

gtctgcacaa cgcagggccc cgaagagctc tcacggtttc tttggcaggt tcactgggtg 600

tggggcaccg agtgtcctcc ccagccaggg cagagctggt ggcaagccca gggcacttct 660

cgctgctcta gcccaggcag cccctcctct gggcctgggc ggagaggtta atttggggaa 720

tggacacaaa gagggaggat cgaagagtca gagcctcagg aatgcccagc cagagcagtt 780

cagacattga gaggagaagc tggctctgag gaagatttat ttgctctgag gctctgcatc 840

ccccagccat ccccccagag cctctccctg tggggaacac aggacacagg cagagtcctc 900

cccacagctt gcatctgtcc cctgaacagg gcaacctgga cgccaggctg gatggagggg 960

agaaggcgat gcagccgcaa tggtagtctc catggtgtgt gaggagccgg cacctgctct 1020

cacacgatgc catcaaagcc ctgcaggcca cacttcttgc cggccacctg gcacccgaat 1080

ctcagtgctt cctgcacgct cctcccctgg gagaggctga agatgacgga ggcattgaag 1140

gtgtctccag ctcccagtgt atccaccacg cggggtggcg ggaaagcatc cgagtggagc 1200

aatttgccat cagggcccag ggcgtcggcg ccctcctcag cccaggcaca gacaagcaca 1260

gcccctttcc tcacacgacc atacaagccc ctcaaggctt cctctgctga ctggaacccc 1320

aagtgcttgg ccacatcttt gctgacaaac accacgtctc cgtagccaaa cagctggaag 1380

agctcctctc gtggcttctc cacctccacg gacacccgga tcttctgctc tggaggctgc 1440

ctggtgttgt gtgcgtctat ccgctgcagc atcttcacct gctccgatgc gttccggccc 1500

tcaatgtgga tccacttgaa ctgggtcaga tcaaccttct caaagtctgt agcagacaca 1560

tctggcaggc tcgtgtcatg gagcacaatg gtacggttgc cattggagtt gttgatgatg 1620

cagcaggagc tgggggtgtc ccccttgctc tgccaggcca cctgagacac gtccacgccc 1680

cgccgcctga agtcggccac caggaagtca gcaacatggc caggagccat tgagcccatg 1740

aaggcacagg gggctccgag cagggagaga acggtgcagg agttggacgc gttgcctccg 1800

cgctgccatc tctgggacaa acaccttatc tccgagtcct ccttagggta cttgtccacc 1860

aggctgatga cgtccagcac cactagcccc acgcacagga tctgcttctc ttccatgagg 1920

ctactcccag agcttcctgc ccgcccggct gacggggttc ctcccggcct ctgcctctta 1980

tccaggagga tggtgtcccc gctcccagct gccctggctc ctcctccgcg tgggagctcc 2040

tcctccaggg agccacctgc agcggccagg ggtgcaggga acgaaaggag caggcccctt 2100

cctcgcgaca cccgaagacc cgagcgggtc taggaaagcc aacccagagg tcttggtccc 2160

cggtcttccc gtagcaggtt gcctgggcct gcagatggca acgcctccca cttctcagcc 2220

ttagtttcct cgcttgtcag atggactcac agctgatgca aaccttttta aagcgcggct 2280
 
aagcgatctc gagaatgcaa agagaaaatg cgctacttgc cccgcactct cctgcacctg 2340

cgtctccgac tcccgacccg ggacacgcga ggtacagctg ggcctcgcat ctgcagccct 2400

gcctgaagcc cgcggtcgcg gcggccccgc ccc 2433
<210> 3
   <211> 2433
   <212> DNA
   <213> Homo sapiens
<400> 3 
ggggcggggc cgccgcgacc gcgggcttca ggcagggctg cagatgcgag gcccagctgt 60

acctcgcgtg tcccgggtcg ggagtcggag acgcaggtgc aggagagtgc ggggcaagta 120

gcgcattttc tctttgcatt ctcgagatcg cttagccgcg ctttaaaaag gtttgcatca 180

gctgtgagtc catctgacaa gcgaggaaac taaggctgag aagtgggagg cgttgccatc 240

tgcaggccca ggcaacctgc tacgggaaga ccggggacca agacctctgg gttggctttc 300

ctagacccgc tcgggtcttc gggtgtcgcg aggaagggcc ctgctccttt cgttccctgc 360

acccctggcc gctgcaggtg gctccctgga ggaggagctc ccacgcggag gaggagccag 420

ggcagctggg agcggggaca ccatcctcct ggataagagg cagaggccgg gaggaacccc 480

gtcagccggg cgggcaggaa gctctgggag tagcctcatg gaagagaagc agatcctgtg 540

cgtggggcta gtggtgctgg acgtcatcag cctggtggac aagtacccta aggaggactc 600

ggagataagg tgtttgtccc agagatggca gcgcggaggc aacgcgtcca actcctgcac 660

cgttctctcc ctgctcggag ccccctgtgc cttcatgggc tcaatggctc ctggccatgt 720

tgctgatttt gtcctggatg acctccgccg ctattctgtg gacctacgct acacagtctt 780

tcagaccaca ggctccgtcc ccatcgccac ggtcatcatc aacgaggcca gtggtagccg 840

caccatccta tactatgaca ggagcctgcc agatgtgtct gctacagact ttgagaaggt 900

tgatctgacc cagttcaagt ggatccacat tgagggccgg aacgcatcgg agcaggtgaa 960

gatgctgcag cggatagacg cacacaacac caggcagcct ccagagcaga agatccgggt 1020

gtccgtggag gtggagaagc cacgagagga gctcttccag ctgtttggct acggagacgt 1080

ggtgtttgtc agcaaagatg tggccaagca cttggggttc cagtcagcag aggaagcctt 1140

gaggggcttg tatggtcgtg tgaggaaagg ggctgtgctt gtctgtgcct gggctgagga 1200

gggcgccgac gccctgggcc ctgatggcaa attgctccac tcggatgctt tcccgccacc 1260

ccgcgtggtg gatacactgg gagctggaga caccttcaat gcctccgtca tcttcagcct 1320

ctcccagggg aggagcgtgc aggaagcact gagattcggg tgccaggtgg ccggcaagaa 1380

gtgtggcctg cagggctttg atggcatcgt gtgagagcag gtgccggctc ctcacacacc 1440

atggagacta ccattgcggc tgcatcgcct tctcccctcc atccagcctg gcgtccaggt 1500
 
tgccctgttc aggggacaga tgcaagctgt ggggaggact ctgcctgtgt cctgtgttcc 1560

ccacagggag aggctctggg gggatggctg ggggatgcag agcctcagag caaataaatc 1620

ttcctcagag ccagcttctc ctctcaatgt ctgaactgct ctggctgggc attcctgagg 1680

ctctgactct tcgatcctcc ctctttgtgt ccattcccca aattaacctc tccgcccagg 1740

cccagaggag gggctgcctg ggctagagca gcgagaagtg ccctgggctt gccaccagct 1800

ctgccctggc tggggaggac actcggtgcc ccacacccag tgaacctgcc aaagaaaccg 1860

tgagagctct tcggggccct gcgttgtgca gactctattc ccacagctca gaagctggga 1920

gtccacaccg ctgagctgaa ctgacaggcc agtggggggc aggggtgcgc ctcctctgcc 1980

ctgcccacca gcctgtgatt tgatggggtc ttcattgtcc agaaatacct cctcccgctg 2040

actgccccag agcctgaaag tctcaccctt ggagcccacc ttggaattaa gggcgtgcct 2100

cagccacaaa tgtgacccag gatacagagt gttgctgtcc tcagggaggt ccgatctgga 2160

acacatattg gaattggggc caactccaat atagggtggg taaggcctta taatgtaaag 2220

agcatataat gtaaagggct ttagagtgag acagacctgg attcaaatct gccatttaat 2280

tagctgcata tcaccttagg gtacagcact taacgcaatc tgcctcaatt tcttcatctg 2340

tcaaatggaa ccaattctgc ttggctacag aattattgtg aggataaaat catatataaa 2400

atgcccagca tgatgaaaaa aaaaaaaaaa aaa 2433
<210> 4
   <211> 2433
   <212> DNA
   <213> Homo sapiens
<400> 4 
tttttttttt ttttttttca tcatgctggg cattttatat atgattttat cctcacaata 60

attctgtagc caagcagaat tggttccatt tgacagatga agaaattgag gcagattgcg 120

ttaagtgctg taccctaagg tgatatgcag ctaattaaat ggcagatttg aatccaggtc 180

tgtctcactc taaagccctt tacattatat gctctttaca ttataaggcc ttacccaccc 240

tatattggag ttggccccaa ttccaatatg tgttccagat cggacctccc tgaggacagc 300

aacactctgt atcctgggtc acatttgtgg ctgaggcacg cccttaattc caaggtgggc 360

tccaagggtg agactttcag gctctggggc agtcagcggg aggaggtatt tctggacaat 420

gaagacccca tcaaatcaca ggctggtggg cagggcagag gaggcgcacc cctgcccccc 480

actggcctgt cagttcagct cagcggtgtg gactcccagc ttctgagctg tgggaataga 540

gtctgcacaa cgcagggccc cgaagagctc tcacggtttc tttggcaggt tcactgggtg 600

tggggcaccg agtgtcctcc ccagccaggg cagagctggt ggcaagccca gggcacttct 660

cgctgctcta gcccaggcag cccctcctct gggcctgggc ggagaggtta atttggggaa 720
 
tggacacaaa gagggaggat cgaagagtca gagcctcagg aatgcccagc cagagcagtt 780

cagacattga gaggagaagc tggctctgag gaagatttat ttgctctgag gctctgcatc 840

ccccagccat ccccccagag cctctccctg tggggaacac aggacacagg cagagtcctc 900

cccacagctt gcatctgtcc cctgaacagg gcaacctgga cgccaggctg gatggagggg 960

agaaggcgat gcagccgcaa tggtagtctc catggtgtgt gaggagccgg cacctgctct 1020

cacacgatgc catcaaagcc ctgcaggcca cacttcttgc cggccacctg gcacccgaat 1080

ctcagtgctt cctgcacgct cctcccctgg gagaggctga agatgacgga ggcattgaag 1140

gtgtctccag ctcccagtgt atccaccacg cggggtggcg ggaaagcatc cgagtggagc 1200

aatttgccat cagggcccag ggcgtcggcg ccctcctcag cccaggcaca gacaagcaca 1260

gcccctttcc tcacacgacc atacaagccc ctcaaggctt cctctgctga ctggaacccc 1320

aagtgcttgg ccacatcttt gctgacaaac accacgtctc cgtagccaaa cagctggaag 1380

agctcctctc gtggcttctc cacctccacg gacacccgga tcttctgctc tggaggctgc 1440

ctggtgttgt gtgcgtctat ccgctgcagc atcttcacct gctccgatgc gttccggccc 1500

tcaatgtgga tccacttgaa ctgggtcaga tcaaccttct caaagtctgt agcagacaca 1560

tctggcaggc tcctgtcata gtataggatg gtgcggctac cactggcctc gttgatgatg 1620

accgtggcga tggggacgga gcctgtggtc tgaaagactg tgtagcgtag gtccacagaa 1680

tagcggcgga ggtcatccag gacaaaatca gcaacatggc caggagccat tgagcccatg 1740

aaggcacagg gggctccgag cagggagaga acggtgcagg agttggacgc gttgcctccg 1800

cgctgccatc tctgggacaa acaccttatc tccgagtcct ccttagggta cttgtccacc 1860

aggctgatga cgtccagcac cactagcccc acgcacagga tctgcttctc ttccatgagg 1920

ctactcccag agcttcctgc ccgcccggct gacggggttc ctcccggcct ctgcctctta 1980

tccaggagga tggtgtcccc gctcccagct gccctggctc ctcctccgcg tgggagctcc 2040

tcctccaggg agccacctgc agcggccagg ggtgcaggga acgaaaggag cagggccctt 2100

cctcgcgaca cccgaagacc cgagcgggtc taggaaagcc aacccagagg tcttggtccc 2160

cggtcttccc gtagcaggtt gcctgggcct gcagatggca acgcctccca cttctcagcc 2220

ttagtttcct cgcttgtcag atggactcac agctgatgca aaccttttta aagcgcggct 2280

aagcgatctc gagaatgcaa agagaaaatg cgctacttgc cccgcactct cctgcacctg 2340

cgtctccgac tcccgacccg ggacacgcga ggtacagctg ggcctcgcat ctgcagccct 2400

gcctgaagcc cgcggtcgcg gcggccccgc ccc 2433
<210> 5
   <211> 2146
   <212> DNA
   <213> Homo sapiens
<400> 5 
accgcgggct tcaggcaggg ctgcagatgc gaggcccagc tgtacctcgc gtgtcccggg 60

tcgggagtcg gagacgcagg tgcaggagag tgcggggcaa gtagcgcatt ttctctttgc 120

attctcgaga tcgcttagcc gcgctttaaa aaggtttgca tcagctgtga gtccatctga 180

caagcgagga aactaaggct gagaagtggg aggcgttgcc atctgcaggc ccaggcaacc 240

tgctacggga agaccgggga ccaagacctc tgggttggct ttcctagacc cgctcgggtc 300

ttcgggtgtc gcgaggaagg gccctgctcc tttcgttccc tgcacccctg gccgctgcag 360

gtggctccct ggaggaggag ctcccacgcg gaggaggagc cagggcagct gggagcgggg 420

acaccatcct cctggataag aggcagaggc cgggaggaac cccgtcagcc gggcgggcag 480

gaagctctgg gagtagcctc atggaagaga agcagatcct gtgcgtgggg ctagtggtgc 540

tggacgtcat cagcctggtg gacaagtacc ctaaggagga ctcggagata aggagcctgc 600

cagatgtgtc tgctacagac tttgagaagg ttgatctgac ccagttcaag tggatccaca 660

ttgagggccg gaacgcatcg gagcaggtga agatgctgca gcggatagac gcacacaaca 720

ccaggcagcc tccagagcag aagatccggg tgtccgtgga ggtggagaag ccacgagagg 780

agctcttcca gctgtttggc tacggagacg tggtgtttgt cagcaaagat gtggccaagc 840

acttggggtt ccagtcagca gaggaagcct tgagggcctt gtatggtcgt gtgaggaaag 900

gggctgtgct tgtctgtgcc tggctgaggg agggcgccga cgccctgggc cctgatggca 960

aattgctcca ctcggatgct ttcccgccac cccgcgtggt ggatacactg ggagctggag 1020

acaccttcaa tgcctccgtc atcttcagcc tctcccaggg gaggagcgtg caggaagcac 1080

tgagattcgg gtgccaggtg gccggcaaga agtgtggcct gcagggcttt gatggcatcg 1140

tgtgagagca ggtgccggct cctcacacac catggagact accattgcgg ctgcatcgcc 1200

ttctcccctc catccagcct ggcgtccagg ttgccctgtt caggggacag atgcaagctg 1260

tggggaggac tctgcctgtg tcctgtgttc cccacaggga gaggctctgg ggggatggct 1320

gggggatgca gagcctcaga gcaaataaat cttcctcaga gccagcttct cctctcaatg 1380

tctgaactgc tctggctggg cattcctgag gctctgactc ttcgatcctc cctctttgtg 1440

tccattcccc aaattaacct ctccgcccag gcccagagga ggggctgcct gggctagagc 1500

agcgagaagt gccctgggct tgccaccagc tctgccctgg ctggggagga cactcggtgc 1560

cccacaccca gtgaacctgc caaagaaacc gtgagagctc ttcggggccc tgcgttgtgc 1620

agactctatt cccacagctc agaagctggg agtccacacc gctgagctga actgacaggc 1680

cagtgggggg caggggtgcg cctcctctgc cctgcccacc agcctgtgat ttgatggggt 1740

cttcattgtc cagaaatacc tcctcccgct gactgcccca gagcctgaaa gtctcaccct 1800
 
tggagcccac cttggaatta agggcgtgcc tcagccacaa atgtgaccca ggatacagag 1860

tgttgctgtc ctcagggagg tccgatctgg aacacatatt ggaattgggg ccaactccaa 1920

tatagggtgg gtaaggcctt ataatgtaaa gagcatataa tgtaaagggc tttagagtga 1980

gacagacctg gattaaaatc tgccatttaa ttagctgcat atcaccttag ggtacagcac 2040

ttaacgcaat ctgcctcaat ttcttcatct gtcaaatgga accaattctg cttggctaca 2100

gaattattgt gaggataaaa tcatatataa aatgcccagc atgatg 2146
<210> 6
   <211> 2146
   <212> DNA
   <213> Homo sapiens
<400> 6 
catcatgctg ggcattttat atatgatttt atcctcacaa taattctgta gccaagcaga 60

attggttcca tttgacagat gaagaaattg aggcagattg cgttaagtgc tgtaccctaa 120

ggtgatatgc agctaattaa atggcagatt ttaatccagg tctgtctcac tctaaagccc 180

tttacattat atgctcttta cattataagg ccttacccac cctatattgg agttggcccc 240

aattccaata tgtgttccag atcggacctc cctgaggaca gcaacactct gtatcctggg 300

tcacatttgt ggctgaggca cgcccttaat tccaaggtgg gctccaaggg tgagactttc 360

aggctctggg gcagtcagcg ggaggaggta tttctggaca atgaagaccc catcaaatca 420

caggctggtg ggcagggcag aggaggcgca cccctgcccc ccactggcct gtcagttcag 480

ctcagcggtg tggactccca gcttctgagc tgtgggaata gagtctgcac aacgcagggc 540

cccgaagagc tctcacggtt tctttggcag gttcactggg tgtggggcac cgagtgtcct 600

ccccagccag ggcagagctg gtggcaagcc cagggcactt ctcgctgctc tagcccaggc 660

agcccctcct ctgggcctgg gcggagaggt taatttgggg aatggacaca aagagggagg 720

atcgaagagt cagagcctca ggaatgccca gccagagcag ttcagacatt gagaggagaa 780

gctggctctg aggaagattt atttgctctg aggctctgca tcccccagcc atccccccag 840

agcctctccc tgtggggaac acaggacaca ggcagagtcc tccccacagc ttgcatctgt 900

cccctgaaca gggcaacctg gacgccaggc tggatggagg ggagaaggcg atgcagccgc 960

aatggtagtc tccatggtgt gtgaggagcc ggcacctgct ctcacacgat gccatcaaag 1020

ccctgcaggc cacacttctt gccggccacc tggcacccga atctcagtgc ttcctgcacg 1080

ctcctcccct gggagaggct gaagatgacg gaggcattga aggtgtctcc agctcccagt 1140

gtatccacca cgcggggtgg cgggaaagca tccgagtgga gcaatttgcc atcagggccc 1200

agggcgtcgg cgccctcctc agcccaggca cagacaagca cagccccttt cctcacacga 1260

ccatacaagc ccctcaaggc ttcctctgct gactggaacc ccaagtgctt ggccacatct 1320
 
ttgctgacaa acaccacgtc tccgtagcca aacagctgga agagctcctc tcgtggcttc 1380

tccacctcca cggacacccg gatcttctgc tctggaggct gcctggtgtt gtgtgcgtct 1440

atccgctgca gcatcttcac ctgctccgat gcgttccggc cctcaatgtg gatccacttg 1500

aactgggtca gatcaacctt ctcaaagtct gtagcagaca catctggcag gctccttatc 1560

tccgagtcct ccttagggta cttgtccacc aggctgatga cgtccagcac cactagcccc 1620

acgcacagga tctgcttctc ttccatgagg ctactcccag agcttcctgc ccgcccggct 1680

gacggggttc ctcccggcct ctgcctctta tccaggagga tggtgtcccc gctcccagct 1740

gccctggctc ctcctccgcg tgggagctcc tcctccaggg agccacctgc agcggccagg 1800

ggtgcaggga acgaaaggag cagggccctt cctcgcgaca cccgaagacc cgagcgggtc 1860

taggaaagcc aacccagagg tcttggtccc cggtcttccc gtagcaggtt gcctgggcct 1920

gcagatggca acgcctccca cttctcagcc ttagtttcct cgcttgtcag atggactcac 1980

agctgatgca aaccttttta aagcgcggct aagcgatctc gagaatgcaa agagaaaatg 2040

cgctacttgc cccgcactct cctgcacctg cgtctccgac tcccgacccg ggacacgcga 2100

ggtacagctg ggcctcgcat ctgcagccct gcctgaagcc cgcggt 2146
<210> 7
   <211> 1117
   <212> DNA
   <213> Mus musculus
<400> 7 
ggaagcttgg ggagcagcct catggaagag aagcagatcc tgtgcgtggg gctggtggtg 60

ctggacatca tcaatgtggt ggacaaatac ccagaggaag acacggatcg caggtgcctg 120

tcccagagat ggcagcgtgg aggcaacgca tccaactcct gcactgtcct ttccttgctt 180

ggagcccgct gtgccttcat gggctctttg gcccctggcc acgttgccga cttcctggtg 240

gctgacttca ggcagagggg cgtggatgtg tctcaagtga cttggcagag ccagggagat 300

accccttgct cttgctgcat cgtcaacaac tccaatggct cccgtaccat tatactctac 360

gacacgaacc tgccagatgt gtctgctaag gactttgaga aggtcgatct gacccggttc 420

aagtggatcc acattgaggg ccggaatgca tcggaacagg tgaagatgct gcagcggata 480

gaggagcaca atgccaagca gcctctgcca cagaaggtcc gggtgtcggt ggagatagag 540

aagccccgtg aggagctctt ccagttgttt agctatggtg aggtggtgtt tgtcagcaaa 600

gatgtggcca agcacctggg gttccagtca gcagtggagg ccctgagggg cttgtacagt 660

cgagtgaaga aaggggctac gcttgtctgt gcctgggctg aggagggtgc cgatgccctg 720

ggccccgatg gtcagctgct ccactcagat gccttcccac cgccccgagt agtagacact 780

cttggggctg gagacacctt caatgcctct gtcatcttca gcctctcgaa gggaaacagc 840

atgcaagagg ccctgagatt cgggtgccag gtggctggca agaagtgtgg cttgcagggg 900

tttgatggca ttgtgtgaga ggcaagcggc accagctcga tacctcagag gctggcacca 960

tgcctgccac tgccttctct acttcctcca gcttagcatc cagctgccat tccccggcag 1020

gtgtgggatg tgggacagcc tctgtctgtg tctgcgtctc tgtataccta tctcctctct 1080

gcagatacct ggagcaaata aatcttcccc tgagcca 1117
<210> 8
   <211> 1117
   <212> DNA
   <213> Mus musculus
<400> 8 
tggctcaggg gaagatttat ttgctccagg tatctgcaga gaggagatag gtatacagag 60

acgcagacac agacagaggc tgtcccacat cccacacctg ccggggaatg gcagctggat 120

gctaagctgg aggaagtaga gaaggcagtg gcaggcatgg tgccagcctc tgaggtatcg 180

agctggtgcc gcttgcctct cacacaatgc catcaaaccc ctgcaagcca cacttcttgc 240

cagccacctg gcacccgaat ctcagggcct cttgcatgct gtttcccttc gagaggctga 300

agatgacaga ggcattgaag gtgtctccag ccccaagagt gtctactact cggggcggtg 360

ggaaggcatc tgagtggagc agctgaccat cggggcccag ggcatcggca ccctcctcag 420

cccaggcaca gacaagcgta gcccctttct tcactcgact gtacaagccc ctcagggcct 480

ccactgctga ctggaacccc aggtgcttgg ccacatcttt gctgacaaac accacctcac 540

catagctaaa caactggaag agctcctcac ggggcttctc tatctccacc gacacccgga 600

ccttctgtgg cagaggctgc ttggcattgt gctcctctat ccgctgcagc atcttcacct 660

gttccgatgc attccggccc tcaatgtgga tccacttgaa ccgggtcaga tcgaccttct 720

caaagtcctt agcagacaca tctggcaggt tcgtgtcgta gagtataatg gtacgggagc 780

cattggagtt gttgacgatg cagcaagagc aaggggtatc tccctggctc tgccaagtca 840

cttgagacac atccacgccc ctctgcctga agtcagccac caggaagtcg gcaacgtggc 900

caggggccaa agagcccatg aaggcacagc gggctccaag caaggaaagg acagtgcagg 960

agttggatgc gttgcctcca cgctgccatc tctgggacag gcacctgcga tccgtgtctt 1020

cctctgggta tttgtccacc acattgatga tgtccagcac caccagcccc acgcacagga 1080

tctgcttctc ttccatgagg ctgctcccca agcttcc 1117
<210> 9
   <211> 1307
   <212> DNA
   <213> Rattus norvegicus
<400> 9 gtgagagggt ctgccattgg ccggactagg taaccaaacc ctcgcaacag cagaaagcac 60 
cctgcgggag gagctccgca ggcagaggag gagccagggt agcccctgag aagttgggac 120

acggtcctgc ggtagataag agacagagtc tagcaggaat cccctccgct tgcgggtagg 180

aagcttgggg agcagcctca tggaagagaa gcagatcctg tgcgtggggc tggtggtgct 240

ggacatcatc aatgtggtgg acaaataccc agaggaagac acggatcgca ggtgcctatc 300

ccagagatgg cagcgtggag gcaacgcgtc caactcctgc actgtgcttt ccttgctcgg 360

agcccgctgt gccttcatgg gctcgctggc ccatggccat gttgccgact tcctggtggc 420

cgacttcagg cggaggggtg tggatgtgtc tcaagtggcc tggcagagcc agggagatac 480

cccttgctcc tgctgcatcg tcaacaactc caatggctcc cgtaccatta ttctctacga 540

cacgaacctg ccagatgtgt ctgctaagga ctttgagaag gtcgatctga cccggttcaa 600

gtggatccac attgagggcc ggaatgcatc ggaacaggta aagatgctac agcggataga 660

acagtacaat gccacgcagc ctctgcagca gaaggtccgg gtgtccgtgg agatagagaa 720

gccccgagag gaactcttcc agctgttcgg ctatggagag gtggtgtttg tcagcaaaga 780

tgtggccaag cacctggggt tccggtcagc aggggaggcc ctgaagggct tgtacagtcg 840

tgtgaagaaa ggggctacgc tcatctgtgc ctgggctgag gagggagccg atgccctggg 900

ccccgacggc cagctgctcc actcagatgc cttcccacca ccccgagtag tagacactct 960

cggggctgga gacaccttca atgcctctgt catcttcagc ctctccaagg gaaacagcat 1020

gcaggaggcc ctgagattcg ggtgccaggt ggctggcaag aagtgtggct tgcaggggtt 1080

tgatggcatt gtgtgagaga tgagcggtgg gaggtagcag ctcgacacct cagaggctgg 1140

caccactgcc tgccattgcc ttcttcattt catccagcct ggcgtctggc tgcccagttc 1200

cctgggccag tgtaggctgt ggaacgggtc tttctgtctc ttctctgcag acacctggag 1260

caaataaatc ttcccctgag ccaaaaaaaa aaaaaaaaaa aaaaaaa 1307
<210> 10
   <211> 1307
   <212> DNA
   <213> Rattus norvegicus
<400> 10 
tttttttttt tttttttttt tttttggctc aggggaagat ttatttgctc caggtgtctg 60

cagagaagag acagaaagac ccgttccaca gcctacactg gcccagggaa ctgggcagcc 120

agacgccagg ctggatgaaa tgaagaaggc aatggcaggc agtggtgcca gcctctgagg 180

tgtcgagctg ctacctccca ccgctcatct ctcacacaat gccatcaaac ccctgcaagc 240

cacacttctt gccagccacc tggcacccga atctcagggc ctcctgcatg ctgtttccct 300

tggagaggct gaagatgaca gaggcattga aggtgtctcc agccccgaga gtgtctacta 360

ctcggggtgg tgggaaggca tctgagtgga gcagctggcc gtcgggcccc agggcatcgg 420

ctccctcctc agcccaggca cagatgagcg tagccccttt cttcacacga ctgtacaagc 480

ccttcagggc ctcccctgct gaccggaacc ccaggtgctt ggccacatct ttgctgacaa 540

acaccacctc tccatagccg aacagctgga agagttcctc tcggggcttc tctatctcca 600

cggacacccg gaccttctgc tgcagaggct gcgtggcatt gtactgttct atccgctgta 660

gcatctttac ctgttccgat gcattccggc cctcaatgtg gatccacttg aaccgggtca 720

gatcgacctt ctcaaagtcc ttagcagaca catctggcag gttcgtgtcg tagagaataa 780

tggtacggga gccattggag ttgttgacga tgcagcagga gcaaggggta tctccctggc 840

tctgccaggc cacttgagac acatccacac ccctccgcct gaagtcggcc accaggaagt 900

cggcaacatg gccatgggcc agcgagccca tgaaggcaca gcgggctccg agcaaggaaa 960

gcacagtgca ggagttggac gcgttgcctc cacgctgcca tctctgggat aggcacctgc 1020

gatccgtgtc ttcctctggg tatttgtcca ccacattgat gatgtccagc accaccagcc 1080

ccacgcacag gatctgcttc tcttccatga ggctgctccc caagcttcct acccgcaagc 1140

ggaggggatt cctgctagac tctgtctctt atctaccgca ggaccgtgtc ccaacttctc 1200

aggggctacc ctggctcctc ctctgcctgc ggagctcctc ccgcagggtg ctttctgctg 1260

ttgcgagggt ttggttacct agtccggcca atggcagacc ctctcac 1307
<210> 11
   <211> 1497
   <212> DNA
   <213> Macaca fascicularis
<400> 11 
cccgggcggg gccgggcagc cgcgaccacg gtcttcaggc agggctgcag atgcaggccc 60

agctctacct cgcgggtcca gggtcgggag tccgagacgc aggtggctcc ccggaggagg 120

agctcccacg cggaggagga gccagggcag ctgggagcga ggacaccatc ctcctggata 180

acaggcagag gccgggagga acccgtcagt cgggcgggca ggaagctctg ggatcagcct 240

catggaagag aagcagatcc tgtgcgtggg gctagtggtg ctggacgtca tcagcctggt 300

ggacaagtac cctaaggagg actcagagat aaggtgcttg tcccagagat ggcaacgcgg 360

aggcaacgcg tccaactcct gcaccgttct ctccctgctc ggagccccct gtgccttcat 420

gggctcaatg gcccctggcc atgttgctga ttttgtcctg gatgacctcc gccgctattc 480

tgtggaccta cgctacacgg tctttcagac cacgggctcc gtccccatcg ccacggtcat 540

catcaacgag gccagtggta gccgcaccat cctatactac gacagcttcc tggtggccga 600

cttcaggcgg cggggtgtgg acgtgtctca ggtggcctgg cagagcaagg gggacacccc 660

cagctcctgc tgcatcatca acaactccaa tggcaaccgt accattgtgc tccatgacac 720

gagcctgcca gatgtgtctg ctacggactt tgagaaggtt gatctgaccc agttcaagtg 780

gatccacatt gagggccgga atgcatcgga gcaggtgaag atgctgcagc ggatagacgc 840

gcacaacacc aggcagcctc cagagcagaa gatccgggtg tccgtggagg tggagaagcc 900

acaagaggag ctctttcagc tgtttggcta cggagacgtg gtgtttgtca gcaaagatgt 960

ggccaagcac ttggggttcc agtcagcagg ggaagccctg aggggcttgt atggtcgtgt 1020

gaggaaaggg gctgtgcttg tctgtgcctg ggctgaggag ggcgccgacg ccctgggccc 1080

tgatggcaaa ctgatccact cggatgcttt cccgccaccc cgcgtggtgg ataccctggg 1140

ggctggagac accttcaatg cctccgtcat cttcagcctc tcccagggga ggagcgtgca 1200

ggaagcactg agattcggat gccaggtggc cggcaagaag tgtggccagc agggctttga 1260

tggcatcgtg tcagagccgg tgcggtagga ggtgccggct ccccgcacac tatggaggct 1320

gacattgcgg ctgcatcgcc ttctcccctc catccagcct ggcatccagg ttgccctgct 1380

caggggacag atgcaggctg tggggaggac tccgcctgtg tcctgtgttc cccacacgtc 1440

tctccctgca gagcctcaga gcgaataaat cttcctcgga gccagcttcc cctggca 1497
<210> 12
   <211> 1497
   <212> DNA
   <213> Macaca fascicularis
<400> 12 
tgccagggga agctggctcc gaggaagatt tattcgctct gaggctctgc agggagagac 60

gtgtggggaa cacaggacac aggcggagtc ctccccacag cctgcatctg tcccctgagc 120

agggcaacct ggatgccagg ctggatggag gggagaaggc gatgcagccg caatgtcagc 180

ctccatagtg tgcggggagc cggcacctcc taccgcaccg gctctgacac gatgccatca 240

aagccctgct ggccacactt cttgccggcc acctggcatc cgaatctcag tgcttcctgc 300

acgctcctcc cctgggagag gctgaagatg acggaggcat tgaaggtgtc tccagccccc 360

agggtatcca ccacgcgggg tggcgggaaa gcatccgagt ggatcagttt gccatcaggg 420

cccagggcgt cggcgccctc ctcagcccag gcacagacaa gcacagcccc tttcctcaca 480

cgaccataca agcccctcag ggcttcccct gctgactgga accccaagtg cttggccaca 540

tctttgctga caaacaccac gtctccgtag ccaaacagct gaaagagctc ctcttgtggc 600

ttctccacct ccacggacac ccggatcttc tgctctggag gctgcctggt gttgtgcgcg 660

tctatccgct gcagcatctt cacctgctcc gatgcattcc ggccctcaat gtggatccac 720

ttgaactggg tcagatcaac cttctcaaag tccgtagcag acacatctgg caggctcgtg 780

tcatggagca caatggtacg gttgccattg gagttgttga tgatgcagca ggagctgggg 840

gtgtccccct tgctctgcca ggccacctga gacacgtcca caccccgccg cctgaagtcg 900

gccaccagga agctgtcgta gtataggatg gtgcggctac cactggcctc gttgatgatg 960

accgtggcga tggggacgga gcccgtggtc tgaaagaccg tgtagcgtag gtccacagaa 1020

tagcggcgga ggtcatccag gacaaaatca gcaacatggc caggggccat tgagcccatg 1080

aaggcacagg gggctccgag cagggagaga acggtgcagg agttggacgc gttgcctccg 1140

cgttgccatc tctgggacaa gcaccttatc tctgagtcct ccttagggta cttgtccacc 1200

aggctgatga cgtccagcac cactagcccc acgcacagga tctgcttctc ttccatgagg 1260

ctgatcccag agcttcctgc ccgcccgact gacgggttcc tcccggcctc tgcctgttat 1320

ccaggaggat ggtgtcctcg ctcccagctg ccctggctcc tcctccgcgt gggagctcct 1380

cctccgggga gccacctgcg tctcggactc ccgaccctgg acccgcgagg tagagctggg 1440

cctgcatctg cagccctgcc tgaagaccgt ggtcgcggct gcccggcccc gcccggg 1497
<210> 13
   <211> 16
   <212> PRT
   <213> Unknown
<220>
   <221> source
   <223> /note="Description of Unknown: RFGF hydrophobic membrane translocation peptide"
<400> 13 
Ala Ala Val Ala Leu Leu Pro Ala Val Leu Leu Ala Leu Leu Ala Pro
1               5                   10                  15
<210> 14
   <211> 11
   <212> PRT
   <213> Unknown
<220>
   <221> source
   <223> /note="Description of Unknown: RFGF analogue peptide"
<400> 14 
Ala Ala Leu Leu Pro Val Leu Leu Ala Ala Pro
1               5                   10
<210> 15
   <211> 13
   <212> PRT
   <213> Human immunodeficiency virus
<400> 15 
Gly Arg Lys Lys Arg Arg Gln Arg Arg Arg Pro Pro Gln
1               5                   10
<210> 16
   <211> 16
   <212> PRT
   <213> Drosophila sp.
<400> 16 
Arg Gln Ile Lys Ile Trp Phe Gln Asn Arg Arg Met Lys Trp Lys Lys
1               5                   10                  15
<210> 17
   <211> 21
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<400> 17
   cuuacgcuga guacuucgat t 21
<210> 18
   <211> 21
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<400> 18
   ucgaaguacu cagcguaagt t 21
<210> 19
   <211> 21
   <212> RNA
   <213> Artificial Sequence
<220>
   <221> source
   <223> /note="Description of Artificial Sequence: Synthetic oligonucleotide"
<400> 19
   ugucagcaaa gauguggcca a 21
<210> 20
   <211> 21
   <212> RNA
   <213> Artificial Sequence
<220>
   <221> source
   <223> /note="Description of Artificial Sequence: Synthetic oligonucleotide"
<400> 20
   agagaagcag auccugugcg u 21
<210> 21
   <211> 21
   <212> RNA
   <213> Artificial Sequence
<220>
   <221> source
   <223> /note="Description of Artificial Sequence: Synthetic oligonucleotide"
<400> 21
   gguguuuguc agcaaagaug u 21
<210> 22
   <211> 21
   <212> RNA
   <213> Artificial Sequence
<220>
   <221> source
   <223> /note="Description of Artificial Sequence: Synthetic oligonucleotide"
<400> 22
   gauccacauu gagggccgga a 21
<210> 23
   <211> 21
   <212> RNA
   <213> Artificial Sequence
<220>
   <221> source
   <223> /note="Description of Artificial Sequence: Synthetic oligonucleotide"
<400> 23
   uugucagcaa agauguggcc a 21
<210> 24
   <211> 21
   <212> RNA
   <213> Artificial Sequence
<220>
   <221> source
   <223> /note="Description of Artificial Sequence: Synthetic oligonucleotide"
<400> 24
   ggauccacau ugagggccgg a 21
<210> 25
   <211> 21
   <212> RNA
   <213> Artificial Sequence
<220>
   <221> source
   <223> /note="Description of Artificial Sequence: Synthetic oligonucleotide"
<400> 25
   gcagauccug ugcguggggc u 21
<210> 26
   <211> 21
   <212> RNA
   <213> Artificial Sequence
<220>
   <221> source
   <223> /note="Description of Artificial Sequence: Synthetic oligonucleotide"
<400> 26
   guuugucagc aaagaugugg a 21
<210> 27
   <211> 21
   <212> RNA
   <213> Artificial Sequence
<220>
   <221> source
   <223> /note="Description of Artificial Sequence: Synthetic oligonucleotide"
<400> 27
   gagaagcaga uccugugcgu a 21
<210> 28
   <211> 21
   <212> RNA
   <213> Artificial Sequence
<220>
   <221> source
   <223> /note="Description of Artificial Sequence: Synthetic oligonucleotide"
<400> 28
   ucaaguggau ccacauugag a 21
<210> 29
   <211> 21
   <212> RNA
   <213> Artificial Sequence
<220>
   <221> source
   <223> /note="Description of Artificial Sequence: Synthetic oligonucleotide"
<400> 29
   guggauccac auugagggcc a 21
<210> 30
   <211> 21
   <212> RNA
   <213> Artificial Sequence
<220>
   <221> source
   <223> /note="Description of Artificial Sequence: Synthetic oligonucleotide"
<400> 30
   aaguggaucc acauugaggg a 21
<210> 31
   <211> 21
   <212> RNA
   <213> Artificial Sequence
<220>
   <221> source
   <223> /note="Description of Artificial Sequence: Synthetic oligonucleotide"
<400> 31
   guguuuguca gcaaagaugu a 21
<210> 32
   <211> 21
   <212> RNA
   <213> Artificial Sequence
<220>
   <221> source
   <223> /note="Description of Artificial Sequence: Synthetic oligonucleotide"
<400> 32
   uguuugucag caaagaugug a 21
<210> 33
   <211> 21
   <212> RNA
   <213> Artificial Sequence
<220>
   <221> source
   <223> /note="Description of Artificial Sequence: Synthetic oligonucleotide"
<400> 33
   uggauccaca uugagggccg a 21
<210> 34
   <211> 21
   <212> RNA
   <213> Artificial Sequence
<220>
   <221> source
   <223> /note="Description of Artificial Sequence: Synthetic oligonucleotide"
<400> 34
   gaagcagauc cugugcgugg a 21
<210> 35
   <211> 21
   <212> RNA
   <213> Artificial Sequence
<220>
   <221> source
   <223> /note="Description of Artificial Sequence: Synthetic oligonucleotide"
<400> 35
   gaagagaagc agauccugug a 21
<210> 36
   <211> 21
   <212> RNA
   <213> Artificial Sequence
<220>
   <221> source
   <223> /note="Description of Artificial Sequence: Synthetic oligonucleotide"
<400> 36
   caaguggauc cacauugagg a 21
<210> 37
   <211> 21
   <212> RNA
   <213> Artificial Sequence
<220>
   <221> source
   <223> /note="Description of Artificial Sequence: Synthetic oligonucleotide"
<400> 37
   aguggaucca cauugagggc a 21
<210> 38
   <211> 21
   <212> RNA
   <213> Artificial Sequence
<220>
   <221> source
   <223> /note="Description of Artificial Sequence: Synthetic oligonucleotide"
<400> 38
   aagagaagca gauccugugc a 21
<210> 39
   <211> 21
   <212> RNA
   <213> Artificial Sequence
<220>
   <221> source
   <223> /note="Description of Artificial Sequence: Synthetic oligonucleotide"
<400> 39
   agaagcagau ccugugcgug a 21
<210> 40
   <211> 23
   <212> RNA
   <213> Artificial Sequence
<220>
   <221> source
   <223> /note="Description of Artificial Sequence: Synthetic oligonucleotide"
<400> 40
   uuggccacau cuuugcugac aaa 23
<210> 41
   <211> 23
   <212> RNA
   <213> Artificial Sequence
<220>
   <221> source
   <223> /note="Description of Artificial Sequence: Synthetic oligonucleotide"
<400> 41
   acgcacagga ucugcuucuc uuc 23
<210> 42
   <211> 23
   <212> RNA
   <213> Artificial Sequence
<220>
   <221> source
   <223> /note="Description of Artificial Sequence: Synthetic oligonucleotide"
<400> 42
   acaucuuugc ugacaaacac cac 23
<210> 43
   <211> 23
   <212> RNA
   <213> Artificial Sequence
<220>
   <221> source
   <223> /note="Description of Artificial Sequence: Synthetic oligonucleotide"
<400> 43
   uuccggcccu caauguggau cca 23
<210> 44
   <211> 23
   <212> RNA
   <213> Artificial Sequence
<220>
   <221> source
   <223> /note="Description of Artificial Sequence: Synthetic oligonucleotide"
<400> 44
   uggccacauc uuugcugaca aac 23
<210> 45
   <211> 23
   <212> RNA
   <213> Artificial Sequence
<220>
   <221> source
   <223> /note="Description of Artificial Sequence: Synthetic oligonucleotide"
<400> 45
   uccggcccuc aauguggauc cac 23
<210> 46
   <211> 23
   <212> RNA
   <213> Artificial Sequence
<220>
   <221> source
   <223> /note="Description of Artificial Sequence: Synthetic oligonucleotide"
<400> 46
   agccccacgc acaggaucug cuu 23
<210> 47
   <211> 23
   <212> RNA
   <213> Artificial Sequence
<220>
   <221> source
   <223> /note="Description of Artificial Sequence: Synthetic oligonucleotide"
<400> 47
   uccacaucuu ugcugacaaa cac 23
<210> 48
   <211> 23
   <212> RNA
   <213> Artificial Sequence
<220>
   <221> source
   <223> /note="Description of Artificial Sequence: Synthetic oligonucleotide"
<400> 48
   uacgcacagg aucugcuucu cuu 23
<210> 49
   <211> 23
   <212> RNA
   <213> Artificial Sequence
<220>
   <221> source
   <223> /note="Description of Artificial Sequence: Synthetic oligonucleotide"
<400> 49
   ucucaaugug gauccacuug aac 23
<210> 50
   <211> 23
   <212> RNA
   <213> Artificial Sequence
<220>
   <221> source
   <223> /note="Description of Artificial Sequence: Synthetic oligonucleotide"
<400> 50
   uggcccucaa uguggaucca cuu 23
<210> 51
   <211> 23
   <212> RNA
   <213> Artificial Sequence
<220>
   <221> source
   <223> /note="Description of Artificial Sequence: Synthetic oligonucleotide"
<400> 51
   ucccucaaug uggauccacu uga 23
<210> 52
   <211> 23
   <212> RNA
   <213> Artificial Sequence
<220>
   <221> source
   <223> /note="Description of Artificial Sequence: Synthetic oligonucleotide"
<400> 52
   uacaucuuug cugacaaaca cca 23
<210> 53
   <211> 23
   <212> RNA
   <213> Artificial Sequence
<220>
   <221> source
   <223> /note="Description of Artificial Sequence: Synthetic oligonucleotide"
<400> 53
   ucacaucuuu gcugacaaac acc 23
<210> 54
   <211> 23
   <212> RNA
   <213> Artificial Sequence
<220>
   <221> source
   <223> /note="Description of Artificial Sequence: Synthetic oligonucleotide"
<400> 54
   ucggcccuca auguggaucc acu 23
<210> 55
   <211> 23
   <212> RNA
   <213> Artificial Sequence
<220>
   <221> source
   <223> /note="Description of Artificial Sequence: Synthetic oligonucleotide"
<400> 55
   uccacgcaca ggaucugcuu cuc 23
<210> 56
   <211> 23
   <212> RNA
   <213> Artificial Sequence
<220>
   <221> source
   <223> /note="Description of Artificial Sequence: Synthetic oligonucleotide"
<400> 56
   ucacaggauc ugcuucucuu cca 23
<210> 57
   <211> 23
   <212> RNA
   <213> Artificial Sequence
<220>
   <221> source
   <223> /note="Description of Artificial Sequence: Synthetic oligonucleotide"
<400> 57
   uccucaaugu ggauccacuu gaa 23
<210> 58
   <211> 23
   <212> RNA
   <213> Artificial Sequence
<220>
   <221> source
   <223> /note="Description of Artificial Sequence: Synthetic oligonucleotide"
<400> 58
   ugcccucaau guggauccac uug 23
<210> 59
   <211> 23
   <212> RNA
   <213> Artificial Sequence
<220>
   <221> source
   <223> /note="Description of Artificial Sequence: Synthetic oligonucleotide"
<400> 59
   ugcacaggau cugcuucucu ucc 23
<210> 60
   <211> 23
   <212> RNA
   <213> Artificial Sequence
<220>
   <221> source
   <223> /note="Description of Artificial Sequence: Synthetic oligonucleotide"
<400> 60
   ucacgcacag gaucugcuuc ucu 23
<210> 61
   <211> 21
   <212> RNA
   <213> Artificial Sequence
<220>
   <221> source
   <223> /note="Description of Artificial Sequence: Synthetic oligonucleotide"
<400> 61
   gccugccaga ugugucugcu a 21
<210> 62
   <211> 21
   <212> RNA
   <213> Artificial Sequence
<220>
   <221> source
   <223> /note="Description of Artificial Sequence: Synthetic oligonucleotide"
<400> 62
   guucaagugg auccacauug a 21
<210> 63
   <211> 21
   <212> RNA
   <213> Artificial Sequence
<220>
   <221> source
   <223> /note="Description of Artificial Sequence: Synthetic oligonucleotide"
<400> 63
   caguucaagu ggauccacau u 21
<210> 64
   <211> 21
   <212> RNA
   <213> Artificial Sequence
<220>
   <221> source
   <223> /note="Description of Artificial Sequence: Synthetic oligonucleotide"
<400> 64
   gugguguuug ucagcaaaga u 21
<210> 65
   <211> 21
   <212> RNA
   <213> Artificial Sequence
<220>
   <221> source
   <223> /note="Description of Artificial Sequence: Synthetic oligonucleotide"
<400> 65
   uucaagugga uccacauuga a 21
<210> 66
   <211> 21
   <212> RNA
   <213> Artificial Sequence
<220>
   <221> source
   <223> /note="Description of Artificial Sequence: Synthetic oligonucleotide"
<400> 66
   acgugguguu ugucagcaaa u 21
<210> 67
   <211> 21
   <212> RNA
   <213> Artificial Sequence
<220>
   <221> source
   <223> /note="Description of Artificial Sequence: Synthetic oligonucleotide"
<400> 67
   aguucaagug gauccacauu a 21
<210> 68
   <211> 21
   <212> RNA
   <213> Artificial Sequence
<220>
   <221> source
   <223> /note="Description of Artificial Sequence: Synthetic oligonucleotide"
<400> 68
   uuguaugguc gugugaggaa a 21
<210> 69
   <211> 21
   <212> RNA
   <213> Artificial Sequence
<220>
   <221> source
   <223> /note="Description of Artificial Sequence: Synthetic oligonucleotide"
<400> 69
   uggcuacgga gacguggugu u 21
<210> 70
   <211> 21
   <212> RNA
   <213> Artificial Sequence
<220>
   <221> source
   <223> /note="Description of Artificial Sequence: Synthetic oligonucleotide"
<400> 70
   uguggccaag cacuuggggu u 21
<210> 71
   <211> 21
   <212> RNA
   <213> Artificial Sequence
<220>
   <221> source
   <223> /note="Description of Artificial Sequence: Synthetic oligonucleotide"
<400> 71
   ccaguucaag uggauccaca u 21
<210> 72
   <211> 21
   <212> RNA
   <213> Artificial Sequence
<220>
   <221> source
   <223> /note="Description of Artificial Sequence: Synthetic oligonucleotide"
<400> 72
   agacguggug uuugucagca a 21
<210> 73
   <211> 21
   <212> RNA
   <213> Artificial Sequence
<220>
   <221> source
   <223> /note="Description of Artificial Sequence: Synthetic oligonucleotide"
<400> 73
   gguggacaag uacccuaagg a 21
<210> 74
   <211> 21
   <212> RNA
   <213> Artificial Sequence
<220>
   <221> source
   <223> /note="Description of Artificial Sequence: Synthetic oligonucleotide"
<400> 74
   aucugaccca guucaagugg a 21
<210> 75
   <211> 21
   <212> RNA
   <213> Artificial Sequence
<220>
   <221> source
   <223> /note="Description of Artificial Sequence: Synthetic oligonucleotide"
<400> 75
   ggucguguga ggaaaggggc u 21
<210> 76
   <211> 21
   <212> RNA
   <213> Artificial Sequence
<220>
   <221> source
   <223> /note="Description of Artificial Sequence: Synthetic oligonucleotide"
<400> 76
   aucggagcag gugaagaugc u 21
<210> 77
   <211> 21
   <212> RNA
   <213> Artificial Sequence
<220>
   <221> source
   <223> /note="Description of Artificial Sequence: Synthetic oligonucleotide"
<400> 77
   acggagacgu gguguuuguc a 21
<210> 78
   <211> 21
   <212> RNA
   <213> Artificial Sequence
<220>
   <221> source
   <223> /note="Description of Artificial Sequence: Synthetic oligonucleotide"
<400> 78
   gauccugugc guggggcuag u 21
<210> 79
   <211> 21
   <212> RNA
   <213> Artificial Sequence
<220>
   <221> source
   <223> /note="Description of Artificial Sequence: Synthetic oligonucleotide"
<400> 79
   gcuuguaugg ucgugugagg a 21
<210> 80
   <211> 21
   <212> RNA
   <213> Artificial Sequence
<220>
   <221> source
   <223> /note="Description of Artificial Sequence: Synthetic oligonucleotide"
<400> 80
   ggcuacggag acgugguguu u 21
<210> 81
   <211> 21
   <212> RNA
   <213> Artificial Sequence
<220>
   <221> source
   <223> /note="Description of Artificial Sequence: Synthetic oligonucleotide"
<400> 81
   gaggaaaggg gcugugcuug u 21
<210> 82
   <211> 21
   <212> RNA
   <213> Artificial Sequence
<220>
   <221> source
   <223> /note="Description of Artificial Sequence: Synthetic oligonucleotide"
<400> 82
   gaagguugau cugacccagu u 21
<210> 83
   <211> 21
   <212> RNA
   <213> Artificial Sequence
<220>
   <221> source
   <223> /note="Description of Artificial Sequence: Synthetic oligonucleotide"
<400> 83
   ccugguggac aaguacccua a 21
<210> 84
   <211> 21
   <212> RNA
   <213> Artificial Sequence
<220>
   <221> source
   <223> /note="Description of Artificial Sequence: Synthetic oligonucleotide"
<400> 84
   uuugagaagg uugaucugac a 1
<210> 85
   <211> 21
   <212> RNA
   <213> Artificial Sequence
<220>
   <221> source
   <223> /note="Description of Artificial Sequence: Synthetic oligonucleotide"
<400> 85
   guugaucuga cccaguucaa a 21
<210> 86
   <211> 21
   <212> RNA
   <213> Artificial Sequence
<220>
   <221> source
   <223> /note="Description of Artificial Sequence: Synthetic oligonucleotide"
<400> 86
   gggcuuguau ggucguguga a 21
<210> 87
   <211> 21
   <212> RNA
   <213> Artificial Sequence
<220>
   <221> source
   <223> /note="Description of Artificial Sequence: Synthetic oligonucleotide"
<400> 87
   agcacuuggg guuccaguca a 21
<210> 88
   <211> 21
   <212> RNA
   <213> Artificial Sequence
<220>
   <221> source
   <223> /note="Description of Artificial Sequence: Synthetic oligonucleotide"
<400> 88
   gcuacggaga cgugguguuu a 21
<210> 89
   <211> 21
   <212> RNA
   <213> Artificial Sequence
<220>
   <221> source
   <223> /note="Description of Artificial Sequence: Synthetic oligonucleotide"
<400> 89
   ggagacgugg uguuugucag a 21
<210> 90
   <211> 23
   <212> RNA
   <213> Artificial Sequence
<220>
   <221> source
   <223> /note="Description of Artificial Sequence: Synthetic oligonucleotide"
<400> 90
   uagcagacac aucuggcagg cuc 23
<210> 91
   <211> 23
   <212> RNA
   <213> Artificial Sequence
<220>
   <221> source
   <223> /note="Description of Artificial Sequence: Synthetic oligonucleotide"
<400> 91
   ucaaugugga uccacuugaa cug 23
<210> 92
   <211> 23
   <212> RNA
   <213> Artificial Sequence
<220>
   <221> source
   <223> /note="Description of Artificial Sequence: Synthetic oligonucleotide"
<400> 92
   aauguggauc cacuugaacu ggg 23
<210> 93
   <211> 23
   <212> RNA
   <213> Artificial Sequence
<220>
   <221> source
   <223> /note="Description of Artificial Sequence: Synthetic oligonucleotide"
<400> 93
   aucuuugcug acaaacacca cgu 23
<210> 94
   <211> 23
   <212> RNA
   <213> Artificial Sequence
<220>
   <221> source
   <223> /note="Description of Artificial Sequence: Synthetic oligonucleotide"
<400> 94
   uucaaugugg auccacuuga acu 23
<210> 95
   <211> 23
   <212> RNA
   <213> Artificial Sequence
<220>
   <221> source
   <223> /note="Description of Artificial Sequence: Synthetic oligonucleotide"
<400> 95
   auuugcugac aaacaccacg ucu 23
<210> 96
   <211> 23
   <212> RNA
   <213> Artificial Sequence
<220>
   <221> source
   <223> /note="Description of Artificial Sequence: Synthetic oligonucleotide"
<400> 96
   uaauguggau ccacuugaac ugg 23
<210> 97
   <211> 23
   <212> RNA
   <213> Artificial Sequence
<220>
   <221> source
   <223> /note="Description of Artificial Sequence: Synthetic oligonucleotide"
<400> 97
   uuuccucaca cgaccauaca age 23
<210> 98
   <211> 23
   <212> RNA
   <213> Artificial Sequence
<220>
   <221> source
   <223> /note="Description of Artificial Sequence: Synthetic oligonucleotide"
<400> 98
   aacaccacgu cuccguagcc aaa 23
<210> 99
   <211> 23
   <212> RNA
   <213> Artificial Sequence
<220>
   <221> source
   <223> /note="Description of Artificial Sequence: Synthetic oligonucleotide"
<400> 99
   aaccccaagu gcuuggccac auc 23
<210> 100
   <211> 23
   <212> RNA
   <213> Artificial Sequence
<220>
   <221> source
   <223> /note="Description of Artificial Sequence: Synthetic oligonucleotide"
<400> 100
   auguggaucc acuugaacug ggu 23
<210> 101
   <211> 23
   <212> RNA
   <213> Artificial Sequence
<220>
   <221> source
   <223> /note="Description of Artificial Sequence: Synthetic oligonucleotide"
<400> 101
   uugcugacaa acaccacguc ucc 23
<210> 102
   <211> 23
   <212> RNA
   <213> Artificial Sequence
<220>
   <221> source
   <223> /note="Description of Artificial Sequence: Synthetic oligonucleotide"
<400> 102
   uccuuagggu acuuguccac cag 23
<210> 103
   <211> 23
   <212> RNA
   <213> Artificial Sequence
<220>
   <221> source
   <223> /note="Description of Artificial Sequence: Synthetic oligonucleotide"
<400> 103
   uccacuugaa cugggucaga uca 23
<210> 104
   <211> 23
   <212> RNA
   <213> Artificial Sequence
<220>
   <221> source
   <223> /note="Description of Artificial Sequence: Synthetic oligonucleotide"
<400> 104
   agccccuuuc cucacacgac cau 23
<210> 105
   <211> 23
   <212> RNA
   <213> Artificial Sequence
<220>
   <221> source
   <223> /note="Description of Artificial Sequence: Synthetic oligonucleotide"
<400> 105
   agcaucuuca ccugcuccga ugc 23
<210> 106
   <211> 23
   <212> RNA
   <213> Artificial Sequence
<220>
   <221> source
   <223> /note="Description of Artificial Sequence: Synthetic oligonucleotide"
<400> 106
   ugacaaacac cacgucuccg uag 23
<210> 107
   <211> 23
   <212> RNA
   <213> Artificial Sequence
<220>
   <221> source
   <223> /note="Description of Artificial Sequence: Synthetic oligonucleotide"
<400> 107
   acuagcccca cgcacaggau cug 23
<210> 108
   <211> 23
   <212> RNA
   <213> Artificial Sequence
<220>
   <221> source
   <223> /note="Description of Artificial Sequence: Synthetic oligonucleotide"
<400> 108
   uccucacacg accauacaag ccc 23
<210> 109
   <211> 23
   <212> RNA
   <213> Artificial Sequence
<220>
   <221> source
   <223> /note="Description of Artificial Sequence: Synthetic oligonucleotide"
<400> 109
   aaacaccacg ucuccguagc caa 23
<210> 110
   <211> 23
   <212> RNA
   <213> Artificial Sequence
<220>
   <221> source
   <223> /note="Description of Artificial Sequence: Synthetic oligonucleotide"
<400> 110
   acaagcacag ccccuuuccu cac 23
<210> 111
   <211> 23
   <212> RNA
   <213> Artificial Sequence
<220>
   <221> source
   <223> /note="Description of Artificial Sequence: Synthetic oligonucleotide"
<400> 111
   aacuggguca gaucaaccuu cuc 23
<210> 112
   <211> 23
   <212> RNA
   <213> Artificial Sequence
<220>
   <221> source
   <223> /note="Description of Artificial Sequence: Synthetic oligonucleotide"
<400> 112
   uuaggguacu uguccaccag gcu 23
<210> 113
   <211> 23
   <212> RNA
   <213> Artificial Sequence
<220>
   <221> source
   <223> /note="Description of Artificial Sequence: Synthetic oligonucleotide"
<400> 113
   ugucagauca accuucucaa agu 23
<210> 114
   <211> 23
   <212> RNA
   <213> Artificial Sequence
<220>
   <221> source
   <223> /note="Description of Artificial Sequence: Synthetic oligonucleotide"
<400> 114
   uuugaacugg gucagaucaa ccu 23
<210> 115
   <211> 23
   <212> RNA
   <213> Artificial Sequence
<220>
   <221> source
   <223> /note="Description of Artificial Sequence: Synthetic oligonucleotide"
<400> 115
   uucacacgac cauacaagcc ccu 23
<210> 116
   <211> 23
   <212> RNA
   <213> Artificial Sequence
<220>
   <221> source
   <223> /note="Description of Artificial Sequence: Synthetic oligonucleotide"
<400> 116
   uugacuggaa ccccaagugc uug 23
<210> 117
   <211> 23
   <212> RNA
   <213> Artificial Sequence
<220>
   <221> source
   <223> /note="Description of Artificial Sequence: Synthetic oligonucleotide"
<400> 117
   uaaacaccac gucuccguag cca 23
<210> 118
   <211> 23
   <212> RNA
   <213> Artificial Sequence
<220>
   <221> source
   <223> /note="Description of Artificial Sequence: Synthetic oligonucleotide"
<400> 118
   ucugacaaac accacgucuc cgu 23
<210> 119
   <211> 21
   <212> RNA
   <213> Artificial Sequence
<220>
   <221> source
   <223> /note="Description of Artificial Sequence: Synthetic oligonucleotide"
<400> 119
   gugaagaugc ugcagcggau a 21
<210> 120
   <211> 21
   <212> RNA
   <213> Artificial Sequence
<220>
   <221> source
   <223> /note="Description of Artificial Sequence: Synthetic oligonucleotide"
<400> 120
   gaagaugcug cagcggauag a 21
<210> 121
   <211> 21
   <212> RNA
   <213> Artificial Sequence
<220>
   <221> source
   <223> /note="Description of Artificial Sequence: Synthetic oligonucleotide"
<400> 121
   ugaagaugcu gcagcggaua a 21
<210> 122
   <211> 23
   <212> RNA
   <213> Artificial Sequence
<220>
   <221> source
   <223> /note="Description of Artificial Sequence: Synthetic oligonucleotide"
<400> 122
   uauccgcugc agcaucuuca ccu 23
<210> 123
   <211> 23
   <212> RNA
   <213> Artificial Sequence
<220>
   <221> source
   <223> /note="Description of Artificial Sequence: Synthetic oligonucleotide"
<400> 123
   ucuauccgcu gcagcaucuu cac 23
<210> 124
   <211> 23
   <212> RNA
   <213> Artificial Sequence
<220>
   <221> source
   <223> /note="Description of Artificial Sequence: Synthetic oligonucleotide"
<400> 124
   uuauccgcug cagcaucuuc acc 23
<210> 125
   <211> 21
   <212> RNA
   <213> Artificial Sequence
<220>
   <221> source
   <223> /note="Description of Artificial Sequence: Synthetic oligonucleotide"
<400> 125
   aucaaugugg uggacaaaua a 21
<210> 126
   <211> 21
   <212> RNA
   <213> Artificial Sequence
<220>
   <221> source
   <223> /note="Description of Artificial Sequence: Synthetic oligonucleotide"
<400> 126
   gguggacaaa uacccagagg a 21
<210> 127
   <211> 21
   <212> RNA
   <213> Artificial Sequence
<220>
   <221> source
   <223> /note="Description of Artificial Sequence: Synthetic oligonucleotide"
<400> 127
   cuuugagaag gucgaucuga a 21
<210> 128
   <211> 21
   <212> RNA
   <213> Artificial Sequence
<220>
   <221> source
   <223> /note="Description of Artificial Sequence: Synthetic oligonucleotide"
<400> 128
   cccgguucaa guggauccac a 21
<210> 129
   <211> 21
   <212> RNA
   <213> Artificial Sequence
<220>
   <221> source
   <223> /note="Description of Artificial Sequence: Synthetic oligonucleotide"
<400> 129
   uguggccaag caccuggggu u 21
<210> 130
   <211> 21
   <212> RNA
   <213> Artificial Sequence
<220>
   <221> source
   <223> /note="Description of Artificial Sequence: Synthetic oligonucleotide"
<400> 130
   uugcaggggu uugauggcau u 21
<210> 131
   <211> 21
   <212> RNA
   <213> Artificial Sequence
<220>
   <221> source
   <223> /note="Description of Artificial Sequence: Synthetic oligonucleotide"
<400> 131
   gaagagaagc agauccugug a 21
<210> 132
   <211> 21
   <212> RNA
   <213> Artificial Sequence
<220>
   <221> source
   <223> /note="Description of Artificial Sequence: Synthetic oligonucleotide"
<400> 132
   aagagaagca gauccugugc a 21
<210> 133
   <211> 21
   <212> RNA
   <213> Artificial Sequence
<220>
   <221> source
   <223> /note="Description of Artificial Sequence: Synthetic oligonucleotide"
<400> 133
   gagaagcaga uccugugcgu a 21
<210> 134
   <211> 21
   <212> RNA
   <213> Artificial Sequence
<220>
   <221> source
   <223> /note="Description of Artificial Sequence: Synthetic oligonucleotide"
<400> 134
   agaagcagau ccugugcgug a 21
<210> 135
   <211> 21
   <212> RNA
   <213> Artificial Sequence
<220>
   <221> source
   <223> /note="Description of Artificial Sequence: Synthetic oligonucleotide"
<400> 135
   gaagcagauc cugugcgugg a 21
<210> 136
   <211> 21
   <212> RNA
   <213> Artificial Sequence
<220>
   <221> source
   <223> /note="Description of Artificial Sequence: Synthetic oligonucleotide"
<400> 136
   gcagauccug ugcguggggc u 21
<210> 137
   <211> 21
   <212> RNA
   <213> Artificial Sequence
<220>
   <221> source
   <223> /note="Description of Artificial Sequence: Synthetic oligonucleotide"
<400> 137
   gauccugugc guggggcuag u 21
<210> 138
   <211> 21
   <212> RNA
   <213> Artificial Sequence
<220>
   <221> source
   <223> /note="Description of Artificial Sequence: Synthetic oligonucleotide"
<400> 138
   gguggacaag uacccuaagg a 21
<210> 139
   <211> 21
   <212> RNA
   <213> Artificial Sequence
<220>
   <221> source
   <223> /note="Description of Artificial Sequence: Synthetic oligonucleotide"
<400> 139
   gccugccaga ugugucugcu a 21
<210> 140
   <211> 21
   <212> RNA
   <213> Artificial Sequence
<220>
   <221> source
   <223> /note="Description of Artificial Sequence: Synthetic oligonucleotide"
<400> 140
   uuugagaagg uugaucugac a 21
<210> 141
   <211> 21
   <212> RNA
   <213> Artificial Sequence
<220>
   <221> source
   <223> /note="Description of Artificial Sequence: Synthetic oligonucleotide"
<400> 141
   guugaucuga cccaguucaa a 21
<210> 142
   <211> 21
   <212> RNA
   <213> Artificial Sequence
<220>
   <221> source
   <223> /note="Description of Artificial Sequence: Synthetic oligonucleotide"
<400> 142
   aucugaccca guucaagugg a 21
<210> 143
   <211> 21
   <212> RNA
   <213> Artificial Sequence
<220>
   <221> source
   <223> /note="Description of Artificial Sequence: Synthetic oligonucleotide"
<400> 143
   ccaguucaag uggauccaca u 21
<210> 144
   <211> 21
   <212> RNA
   <213> Artificial Sequence
<220>
   <221> source
   <223> /note="Description of Artificial Sequence: Synthetic oligonucleotide"
<400> 144
   caguucaagu ggauccacau u 21
<210> 145
   <211> 21
   <212> RNA
   <213> Artificial Sequence
<220>
   <221> source
   <223> /note="Description of Artificial Sequence: Synthetic oligonucleotide"
<400> 145
   aguucaagug gauccacauu a 21
<210> 146
   <211> 21
   <212> RNA
   <213> Artificial Sequence
<220>
   <221> source
   <223> /note="Description of Artificial Sequence: Synthetic oligonucleotide"
<400> 146
   guucaagugg auccacauug a 21
<210> 147
   <211> 21
   <212> RNA
   <213> Artificial Sequence
<220>
   <221> source
   <223> /note="Description of Artificial Sequence: Synthetic oligonucleotide"
<400> 147
   uucaagugga uccacauuga a 21
<210> 148
   <211> 21
   <212> RNA
   <213> Artificial Sequence
<220>
   <221> source
   <223> /note="Description of Artificial Sequence: Synthetic oligonucleotide"
<400> 148
   ucaaguggau ccacauugag a 21
<210> 149
   <211> 21
   <212> RNA
   <213> Artificial Sequence
<220>
   <221> source
   <223> /note="Description of Artificial Sequence: Synthetic oligonucleotide"
<400> 149
   caaguggauc cacauugagg a 21
<210> 150
   <211> 21
   <212> RNA
   <213> Artificial Sequence
<220>
   <221> source
   <223> /note="Description of Artificial Sequence: Synthetic oligonucleotide"
<400> 150
   aaguggaucc acauugaggg a 21
<210> 151
   <211> 21
   <212> RNA
   <213> Artificial Sequence
<220>
   <221> source
   <223> /note="Description of Artificial Sequence: Synthetic oligonucleotide"
<400> 151
   aguggaucca cauugagggc a 21
<210> 152
   <211> 21
   <212> RNA
   <213> Artificial Sequence
<220>
   <221> source
   <223> /note="Description of Artificial Sequence: Synthetic oligonucleotide"
<400> 152
   guggauccac auugagggcc a 21
<210> 153
   <211> 21
   <212> RNA
   <213> Artificial Sequence
<220>
   <221> source
   <223> /note="Description of Artificial Sequence: Synthetic oligonucleotide"
<400> 153
   uggauccaca uugagggccg a 21
<210> 154
   <211> 21
   <212> RNA
   <213> Artificial Sequence
<220>
   <221> source
   <223> /note="Description of Artificial Sequence: Synthetic oligonucleotide"
<400> 154
   gauccacauu gagggccgga a 21
<210> 155
   <211> 21
   <212> RNA
   <213> Artificial Sequence
<220>
   <221> source
   <223> /note="Description of Artificial Sequence: Synthetic oligonucleotide"
<400> 155
   gugaagaugc ugcagcggau a 21
<210> 156
   <211> 21
   <212> RNA
   <213> Artificial Sequence
<220>
   <221> source
   <223> /note="Description of Artificial Sequence: Synthetic oligonucleotide"
<400> 156
   gaagaugcug cagcggauag a 21
<210> 157
   <211> 21
   <212> RNA
   <213> Artificial Sequence
<220>
   <221> source
   <223> /note="Description of Artificial Sequence: Synthetic oligonucleotide"
<400> 157
   uggcuacgga gacguggugu u 21
<210> 158
   <211> 21
   <212> RNA
   <213> Artificial Sequence
<220>
   <221> source
   <223> /note="Description of Artificial Sequence: Synthetic oligonucleotide"
<400> 158
   ggcuacggag acgugguguu u 21
<210> 159
   <211> 21
   <212> RNA
   <213> Artificial Sequence
<220>
   <221> source
   <223> /note="Description of Artificial Sequence: Synthetic oligonucleotide"
<400> 159
   gcuacggaga cgugguguuu a 21
<210> 160
   <211> 21
   <212> RNA
   <213> Artificial Sequence
<220>
   <221> source
   <223> /note="Description of Artificial Sequence: Synthetic oligonucleotide"
<400> 160
   ggagacgugg uguuugucag a 21
<210> 161
   <211> 21
   <212> RNA
   <213> Artificial Sequence
<220>
   <221> source
   <223> /note="Description of Artificial Sequence: Synthetic oligonucleotide"
<400> 161
   acgugguguu ugucagcaaa u 21
<210> 162
   <211> 21
   <212> RNA
   <213> Artificial Sequence
<220>
   <221> source
   <223> /note="Description of Artificial Sequence: Synthetic oligonucleotide"
<400> 162
   gugguguuug ucagcaaaga u 21
<210> 163
   <211> 21
   <212> RNA
   <213> Artificial Sequence
<220>
   <221> source
   <223> /note="Description of Artificial Sequence: Synthetic oligonucleotide"
<400> 163
   gguguuuguc agcaaagaug u 21
<210> 164
   <211> 21
   <212> RNA
   <213> Artificial Sequence
<220>
   <221> source
   <223> /note="Description of Artificial Sequence: Synthetic oligonucleotide"
<400> 164
   guguuuguca gcaaagaugu a 21
<210> 165
   <211> 21
   <212> RNA
   <213> Artificial Sequence
<220>
   <221> source
   <223> /note="Description of Artificial Sequence: Synthetic oligonucleotide"
<400> 165
   uguuugucag caaagaugug a 21
<210> 166
   <211> 21
   <212> RNA
   <213> Artificial Sequence
<220>
   <221> source
   <223> /note="Description of Artificial Sequence: Synthetic oligonucleotide"
<400> 166
   guuugucagc aaagaugugg a 21
<210> 167
   <211> 21
   <212> RNA
   <213> Artificial Sequence
<220>
   <221> source
   <223> /note="Description of Artificial Sequence: Synthetic oligonucleotide"
<400> 167
   uugucagcaa agauguggcc a 21
<210> 168
   <211> 21
   <212> RNA
   <213> Artificial Sequence
<220>
   <221> source
   <223> /note="Description of Artificial Sequence: Synthetic oligonucleotide"
<400> 168
   ugucagcaaa gauguggcca a 21
<210> 169
   <211> 21
   <212> RNA
   <213> Artificial Sequence
<220>
   <221> source
   <223> /note="Description of Artificial Sequence: Synthetic oligonucleotide"
<400> 169
   uguggccaag cacuuggggu u 21
<210> 170
   <211> 21
   <212> RNA
   <213> Artificial Sequence
<220>
   <221> source
   <223> /note="Description of Artificial Sequence: Synthetic oligonucleotide"
<400> 170
   agcacuuggg guuccaguca a 21
<210> 171
   <211> 21
   <212> RNA
   <213> Artificial Sequence
<220>
   <221> source
   <223> /note="Description of Artificial Sequence: Synthetic oligonucleotide"
<400> 171
   gggcuuguau ggucguguga a 21
<210> 172
   <211> 21
   <212> RNA
   <213> Artificial Sequence
<220>
   <221> source
   <223> /note="Description of Artificial Sequence: Synthetic oligonucleotide"
<400> 172
   uuguaugguc gugugaggaa a 21
<210> 173
   <211> 21
   <212> RNA
   <213> Artificial Sequence
<220>
   <221> source
   <223> /note="Description of Artificial Sequence: Synthetic oligonucleotide"
<400> 173
   ggucguguga ggaaaggggc u 21
<210> 174
   <211> 21
   <212> RNA
   <213> Artificial Sequence
<220>
   <221> source
   <223> /note="Description of Artificial Sequence: Synthetic oligonucleotide"
<400> 174
   gaggaaaggg gcugugcuug u 21
<210> 175
   <211> 23
   <212> RNA
   <213> Artificial Sequence
<220>
   <221> source
   <223> /note="Description of Artificial Sequence: Synthetic oligonucleotide"
<400> 175
   uuauuugucc accacauuga uga 23
<210> 176
   <211> 23
   <212> RNA
   <213> Artificial Sequence
<220>
   <221> source
   <223> /note="Description of Artificial Sequence: Synthetic oligonucleotide"
<400> 176
   uccucugggu auuuguccac cac 23
<210> 177
   <211> 23
   <212> RNA
   <213> Artificial Sequence
<220>
   <221> source
   <223> /note="Description of Artificial Sequence: Synthetic oligonucleotide"
<400> 177
   uucagaucga ccuucucaaa guc 23
<210> 178
   <211> 23
   <212> RNA
   <213> Artificial Sequence
<220>
   <221> source
   <223> /note="Description of Artificial Sequence: Synthetic oligonucleotide"
<400> 178
   uguggaucca cuugaaccgg guc 23
<210> 179
   <211> 23
   <212> RNA
   <213> Artificial Sequence
<220>
   <221> source
   <223> /note="Description of Artificial Sequence: Synthetic oligonucleotide"
<400> 179
   aaccccaggu gcuuggccac auc 23
<210> 180
   <211> 23
   <212> RNA
   <213> Artificial Sequence
<220>
   <221> source
   <223> /note="Description of Artificial Sequence: Synthetic oligonucleotide"
<400> 180
   aaugccauca aaccccugca age 23
<210> 181
   <211> 23
   <212> RNA
   <213> Artificial Sequence
<220>
   <221> source
   <223> /note="Description of Artificial Sequence: Synthetic oligonucleotide"
<400> 181
   ucacaggauc ugcuucucuu cca 23
<210> 182
   <211> 23
   <212> RNA
   <213> Artificial Sequence
<220>
   <221> source
   <223> /note="Description of Artificial Sequence: Synthetic oligonucleotide"
<400> 182
   ugcacaggau cugcuucucu ucc 23
<210> 183
   <211> 23
   <212> RNA
   <213> Artificial Sequence
<220>
   <221> source
   <223> /note="Description of Artificial Sequence: Synthetic oligonucleotide"
<400> 183
   uacgcacagg aucugcuucu cuu 23
<210> 184
   <211> 23
   <212> RNA
   <213> Artificial Sequence
<220>
   <221> source
   <223> /note="Description of Artificial Sequence: Synthetic oligonucleotide"
<400> 184
   ucacgcacag gaucugcuuc ucu 23
<210> 185
   <211> 23
   <212> RNA
   <213> Artificial Sequence
<220>
   <221> source
   <223> /note="Description of Artificial Sequence: Synthetic oligonucleotide"
<400> 185
   uccacgcaca ggaucugcuu cuc 23
<210> 186
   <211> 23
   <212> RNA
   <213> Artificial Sequence
<220>
   <221> source
   <223> /note="Description of Artificial Sequence: Synthetic oligonucleotide"
<400> 186
   agccccacgc acaggaucug cuu 23
<210> 187
   <211> 23
   <212> RNA
   <213> Artificial Sequence
<220>
   <221> source
   <223> /note="Description of Artificial Sequence: Synthetic oligonucleotide"
<400> 187
   acuagcccca cgcacaggau cug 23
<210> 188
   <211> 23
   <212> RNA
   <213> Artificial Sequence
<220>
   <221> source
   <223> /note="Description of Artificial Sequence: Synthetic oligonucleotide"
<400> 188
   uccuuagggu acuuguccac cag 23
<210> 189
   <211> 23
   <212> RNA
   <213> Artificial Sequence
<220>
   <221> source
   <223> /note="Description of Artificial Sequence: Synthetic oligonucleotide"
<400> 189
   uagcagacac aucuggcagg cuc 23
<210> 190
   <211> 23
   <212> RNA
   <213> Artificial Sequence
<220>
   <221> source
   <223> /note="Description of Artificial Sequence: Synthetic oligonucleotide"
<400> 190
   ugucagauca accuucucaa agu 23
<210> 191
   <211> 23
   <212> RNA
   <213> Artificial Sequence
<220>
   <221> source
   <223> /note="Description of Artificial Sequence: Synthetic oligonucleotide"
<400> 191
   uuugaacugg gucagaucaa ccu 23
<210> 192
   <211> 23
   <212> RNA
   <213> Artificial Sequence
<220>
   <221> source
   <223> /note="Description of Artificial Sequence: Synthetic oligonucleotide"
<400> 192
   uccacuugaa cugggucaga uca 23
<210> 193
   <211> 23
   <212> RNA
   <213> Artificial Sequence
<220>
   <221> source
   <223> /note="Description of Artificial Sequence: Synthetic oligonucleotide"
<400> 193
   auguggaucc acuugaacug ggu 23
<210> 194
   <211> 23
   <212> RNA
   <213> Artificial Sequence
<220>
   <221> source
   <223> /note="Description of Artificial Sequence: Synthetic oligonucleotide"
<400> 194
   aauguggauc cacuugaacu ggg 23
<210> 195
   <211> 23
   <212> RNA
   <213> Artificial Sequence
<220>
   <221> source
   <223> /note="Description of Artificial Sequence: Synthetic oligonucleotide"
<400> 195
   uaauguggau ccacuugaac ugg 23
<210> 196
   <211> 23
   <212> RNA
   <213> Artificial Sequence
<220>
   <221> source
   <223> /note="Description of Artificial Sequence: Synthetic oligonucleotide"
<400> 196
   ucaaugugga uccacuugaa cug 23
<210> 197
   <211> 23
   <212> RNA
   <213> Artificial Sequence
<220>
   <221> source
   <223> /note="Description of Artificial Sequence: Synthetic oligonucleotide"
<400> 197
   uucaaugugg auccacuuga acu 23
<210> 198
   <211> 23
   <212> RNA
   <213> Artificial Sequence
<220>
   <221> source
   <223> /note="Description of Artificial Sequence: Synthetic oligonucleotide"
<400> 198
   ucucaaugug gauccacuug aac 23
<210> 199
   <211> 23
   <212> RNA
   <213> Artificial Sequence
<220>
   <221> source
   <223> /note="Description of Artificial Sequence: Synthetic oligonucleotide"
<400> 199
   uccucaaugu ggauccacuu gaa 23
<210> 200
   <211> 23
   <212> RNA
   <213> Artificial Sequence
<220>
   <221> source
   <223> /note="Description of Artificial Sequence: Synthetic oligonucleotide"
<400> 200
   ucccucaaug uggauccacu uga 23
<210> 201
   <211> 23
   <212> RNA
   <213> Artificial Sequence
<220>
   <221> source
   <223> /note="Description of Artificial Sequence: Synthetic oligonucleotide"
<400> 201
   ugcccucaau guggauccac uug 23
<210> 202
   <211> 23
   <212> RNA
   <213> Artificial Sequence
<220>
   <221> source
   <223> /note="Description of Artificial Sequence: Synthetic oligonucleotide"
<400> 202
   uggcccucaa uguggaucca cuu 23
<210> 203
   <211> 23
   <212> RNA
   <213> Artificial Sequence
<220>
   <221> source
   <223> /note="Description of Artificial Sequence: Synthetic oligonucleotide"
<400> 203
   ucggcccuca auguggaucc acu 23
<210> 204
   <211> 23
   <212> RNA
   <213> Artificial Sequence
<220>
   <221> source
   <223> /note="Description of Artificial Sequence: Synthetic oligonucleotide"
<400> 204
   uuccggcccu caauguggau cca 23
<210> 205
   <211> 23
   <212> RNA
   <213> Artificial Sequence
<220>
   <221> source
   <223> /note="Description of Artificial Sequence: Synthetic oligonucleotide"
<400> 205
   uauccgcugc agcaucuuca ccu 23
<210> 206
   <211> 23
   <212> RNA
   <213> Artificial Sequence
<220>
   <221> source
   <223> /note="Description of Artificial Sequence: Synthetic oligonucleotide"
<400> 206
   ucuauccgcu gcagcaucuu cac 23
<210> 207
   <211> 23
   <212> RNA
   <213> Artificial Sequence
<220>
   <221> source
   <223> /note="Description of Artificial Sequence: Synthetic oligonucleotide"
<400> 207
   aacaccacgu cuccguagcc aaa 23
<210> 208
   <211> 23
   <212> RNA
   <213> Artificial Sequence
<220>
   <221> source
   <223> /note="Description of Artificial Sequence: Synthetic oligonucleotide"
<400> 208
   aaacaccacg ucuccguagc caa 23
<210> 209
   <211> 23
   <212> RNA
   <213> Artificial Sequence
<220>
   <221> source
   <223> /note="Description of Artificial Sequence: Synthetic oligonucleotide"
<400> 209
   uaaacaccac gucuccguag cca 23
<210> 210
   <211> 23
   <212> RNA
   <213> Artificial Sequence
<220>
   <221> source
   <223> /note="Description of Artificial Sequence: Synthetic oligonucleotide"
<400> 210
   ucugacaaac accacgucuc cgu 23
<210> 211
   <211> 23
   <212> RNA
   <213> Artificial Sequence
<220>
   <221> source
   <223> /note="Description of Artificial Sequence: Synthetic oligonucleotide"
<400> 211
   auuugcugac aaacaccacg ucu 23
<210> 212
   <211> 23
   <212> RNA
   <213> Artificial Sequence
<220>
   <221> source
   <223> /note="Description of Artificial Sequence: Synthetic oligonucleotide"
<400> 212
   aucuuugcug acaaacacca cgu 23
<210> 213
   <211> 23
   <212> RNA
   <213> Artificial Sequence
<220>
   <221> source
   <223> /note="Description of Artificial Sequence: Synthetic oligonucleotide"
<400> 213
   acaucuuugc ugacaaacac cac 23
<210> 214
   <211> 23
   <212> RNA
   <213> Artificial Sequence
<220>
   <221> source
   <223> /note="Description of Artificial Sequence: Synthetic oligonucleotide"
<400> 214
   uacaucuuug cugacaaaca cca 23
<210> 215
   <211> 23
   <212> RNA
   <213> Artificial Sequence
<220>
   <221> source
   <223> /note="Description of Artificial Sequence: Synthetic oligonucleotide"
<400> 215
   ucacaucuuu gcugacaaac acc 23
<210> 216
   <211> 23
   <212> RNA
   <213> Artificial Sequence
<220>
   <221> source
   <223> /note="Description of Artificial Sequence: Synthetic oligonucleotide"
<400> 216
   uccacaucuu ugcugacaaa cac 23
<210> 217
   <211> 23
   <212> RNA
   <213> Artificial Sequence
<220>
   <221> source
   <223> /note="Description of Artificial Sequence: Synthetic oligonucleotide"
<400> 217
   uggccacauc uuugcugaca aac 23
<210> 218
   <211> 23
   <212> RNA
   <213> Artificial Sequence
<220>
   <221> source
   <223> /note="Description of Artificial Sequence: Synthetic oligonucleotide"
<400> 218
   uuggccacau cuuugcugac aaa 23
<210> 219
   <211> 23
   <212> RNA
   <213> Artificial Sequence
<220>
   <221> source
   <223> /note="Description of Artificial Sequence: Synthetic oligonucleotide"
<400> 219
   aaccccaagu gcuuggccac auc 23
<210> 220
   <211> 23
   <212> RNA
   <213> Artificial Sequence
<220>
   <221> source
   <223> /note="Description of Artificial Sequence: Synthetic oligonucleotide"
<400> 220
   uugacuggaa ccccaagugc uug 23
<210> 221
   <211> 23
   <212> RNA
   <213> Artificial Sequence
<220>
   <221> source
   <223> /note="Description of Artificial Sequence: Synthetic oligonucleotide"
<400> 221
   uucacacgac cauacaagcc ccu 23
<210> 222
   <211> 23
   <212> RNA
   <213> Artificial Sequence
<220>
   <221> source
   <223> /note="Description of Artificial Sequence: Synthetic oligonucleotide"
<400> 222
   uuuccucaca cgaccauaca age 23
<210> 223
   <211> 23
   <212> RNA
   <213> Artificial Sequence
<220>
   <221> source
   <223> /note="Description of Artificial Sequence: Synthetic oligonucleotide"
<400> 223
   agccccuuuc cucacacgac cau 23
<210> 224
   <211> 23
   <212> RNA
   <213> Artificial Sequence
<220>
   <221> source
   <223> /note="Description of Artificial Sequence: Synthetic oligonucleotide"
<400> 224
   acaagcacag ccccuuuccu cac 23
<210> 225
   <211> 21
   <212> RNA
   <213> Artificial Sequence
<220>
   <221> source
   <223> /note="Description of Artificial Sequence: Synthetic oligonucleotide"
<400> 225
   aucaaugugg uggacaaaua a 21
<210> 226
   <211> 21
   <212> RNA
   <213> Artificial Sequence
<220>
   <221> source
   <223> /note="Description of Artificial Sequence: Synthetic oligonucleotide"
<400> 226
   gguggacaaa uacccagagg a 21
<210> 227
   <211> 21
   <212> RNA
   <213> Artificial Sequence
<220>
   <221> source
   <223> /note="Description of Artificial Sequence: Synthetic oligonucleotide"
<400> 227
   cuuugagaag gucgaucuga a 21
<210> 228
   <211> 21
   <212> RNA
   <213> Artificial Sequence
<220>
   <221> source
   <223> /note="Description of Artificial Sequence: Synthetic oligonucleotide"
<400> 228
   cccgguucaa guggauccac a 21
<210> 229
   <211> 21
   <212> RNA
   <213> Artificial Sequence
<220>
   <221> source
   <223> /note="Description of Artificial Sequence: Synthetic oligonucleotide"
<400> 229
   uguggccaag caccuggggu u 21
<210> 230
   <211> 21
   <212> RNA
   <213> Artificial Sequence
<220>
   <221> source
   <223> /note="Description of Artificial Sequence: Synthetic oligonucleotide"
<400> 230
   uugcaggggu uugauggcau u 21
<210> 231
   <211> 21
   <212> RNA
   <213> Artificial Sequence
<220>
   <221> source
   <223> /note="Description of Artificial Sequence: Synthetic oligonucleotide"
<400> 231
   gaagagaagc agauccugug a 21
<210> 232
   <211> 21
   <212> RNA
   <213> Artificial Sequence
<220>
   <221> source
   <223> /note="Description of Artificial Sequence: Synthetic oligonucleotide"
<400> 232
   aagagaagca gauccugugc a 21
<210> 233
   <211> 21
   <212> RNA
   <213> Artificial Sequence
<220>
   <221> source
   <223> /note="Description of Artificial Sequence: Synthetic oligonucleotide"
<400> 233
   gagaagcaga uccugugcgu a 21
<210> 234
   <211> 21
   <212> RNA
   <213> Artificial Sequence
<220>
   <221> source
   <223> /note="Description of Artificial Sequence: Synthetic oligonucleotide"
<400> 234
   agaagcagau ccugugcgug a 21
<210> 235
   <211> 21
   <212> RNA
   <213> Artificial Sequence
<220>
   <221> source
   <223> /note="Description of Artificial Sequence: Synthetic oligonucleotide"
<400> 235
   gaagcagauc cugugcgugg a 21
<210> 236
   <211> 21
   <212> RNA
   <213> Artificial Sequence
<220>
   <221> source
   <223> /note="Description of Artificial Sequence: Synthetic oligonucleotide"
<400> 236
   gcagauccug ugcguggggc u 21
<210> 237
   <211> 21
   <212> RNA
   <213> Artificial Sequence
<220>
   <221> source
   <223> /note="Description of Artificial Sequence: Synthetic oligonucleotide"
<400> 237
   gauccugugc guggggcuag u 21
<210> 238
   <211> 21
   <212> RNA
   <213> Artificial Sequence
<220>
   <221> source
   <223> /note="Description of Artificial Sequence: Synthetic oligonucleotide"
<400> 238
   gguggacaag uacccuaagg a 21
<210> 239
   <211> 21
   <212> RNA
   <213> Artificial Sequence
<220>
   <221> source
   <223> /note="Description of Artificial Sequence: Synthetic oligonucleotide"
<400> 239
   gccugccaga ugugucugcu a 21
<210> 240
   <211> 21
   <212> RNA
   <213> Artificial Sequence
<220>
   <221> source
   <223> /note="Description of Artificial Sequence: Synthetic oligonucleotide"
<400> 240
   uuugagaagg uugaucugac a 21
<210> 241
   <211> 21
   <212> RNA
   <213> Artificial Sequence
<220>
   <221> source
   <223> /note="Description of Artificial Sequence: Synthetic oligonucleotide"
<400> 241
   guugaucuga cccaguucaa a 21
<210> 242
   <211> 21
   <212> RNA
   <213> Artificial Sequence
<220>
   <221> source
   <223> /note="Description of Artificial Sequence: Synthetic oligonucleotide"
<400> 242
   aucugaccca guucaagugg a 21
<210> 243
   <211> 21
   <212> RNA
   <213> Artificial Sequence
<220>
   <221> source
   <223> /note="Description of Artificial Sequence: Synthetic oligonucleotide"
<400> 243
   ccaguucaag uggauccaca u 21
<210> 244
   <211> 21
   <212> RNA
   <213> Artificial Sequence
<220>
   <221> source
   <223> /note="Description of Artificial Sequence: Synthetic oligonucleotide"
<400> 244
   caguucaagu ggauccacau u 21
<210> 245
   <211> 21
   <212> RNA
   <213> Artificial Sequence
<220>
   <221> source
   <223> /note="Description of Artificial Sequence: Synthetic oligonucleotide"
<400> 245
   aguucaagug gauccacauu a 21
<210> 246
   <211> 21
   <212> RNA
   <213> Artificial Sequence
<220>
   <221> source
   <223> /note="Description of Artificial Sequence: Synthetic oligonucleotide"
<400> 246
   guucaagugg auccacauug a 21
<210> 247
   <211> 21
   <212> RNA
   <213> Artificial Sequence
<220>
   <221> source
   <223> /note="Description of Artificial Sequence: Synthetic oligonucleotide"
<400> 247
   uucaagugga uccacauuga a 21
<210> 248
   <211> 21
   <212> RNA
   <213> Artificial Sequence
<220>
   <221> source
   <223> /note="Description of Artificial Sequence: Synthetic oligonucleotide"
<400> 248
   ucaaguggau ccacauugag a 21
<210> 249
   <211> 21
   <212> RNA
   <213> Artificial Sequence
<220>
   <221> source
   <223> /note="Description of Artificial Sequence: Synthetic oligonucleotide"
<400> 249
   caaguggauc cacauugagg a 21
<210> 250
   <211> 21
   <212> RNA
   <213> Artificial Sequence
<220>
   <221> source
   <223> /note="Description of Artificial Sequence: Synthetic oligonucleotide"
<400> 250
   aaguggaucc acauugaggg a 21
<210> 251
   <211> 21
   <212> RNA
   <213> Artificial Sequence
<220>
   <221> source
   <223> /note="Description of Artificial Sequence: Synthetic oligonucleotide"
<400> 251
   aguggaucca cauugagggc a 21
<210> 252
   <211> 21
   <212> RNA
   <213> Artificial Sequence
<220>
   <221> source
   <223> /note="Description of Artificial Sequence: Synthetic oligonucleotide"
<400> 252
   guggauccac auugagggcc a 21
<210> 253
   <211> 21
   <212> RNA
   <213> Artificial Sequence
<220>
   <221> source
   <223> /note="Description of Artificial Sequence: Synthetic oligonucleotide"
<400> 253
   uggauccaca uugagggccg a 21
<210> 254
   <211> 21
   <212> RNA
   <213> Artificial Sequence
<220>
   <221> source
   <223> /note="Description of Artificial Sequence: Synthetic oligonucleotide"
<400> 254
   gauccacauu gagggccgga a 21
<210> 255
   <211> 21
   <212> RNA
   <213> Artificial Sequence
<220>
   <221> source
   <223> /note="Description of Artificial Sequence: Synthetic oligonucleotide"
<400> 255
   gugaagaugc ugcagcggau a 21
<210> 256
   <211> 21
   <212> RNA
   <213> Artificial Sequence
<220>
   <221> source
   <223> /note="Description of Artificial Sequence: Synthetic oligonucleotide"
<400> 256
   gaagaugcug cagcggauag a 21
<210> 257
   <211> 21
   <212> RNA
   <213> Artificial Sequence
<220>
   <221> source
   <223> /note="Description of Artificial Sequence: Synthetic oligonucleotide"
<400> 257
   uggcuacgga gacguggugu u 21
<210> 258
   <211> 21
   <212> RNA
   <213> Artificial Sequence
<220>
   <221> source
   <223> /note="Description of Artificial Sequence: Synthetic oligonucleotide"
<400> 258
   ggcuacggag acgugguguu u 21
<210> 259
   <211> 21
   <212> RNA
   <213> Artificial Sequence
<220>
   <221> source
   <223> /note="Description of Artificial Sequence: Synthetic oligonucleotide"
<400> 259
   gcuacggaga cgugguguuu a 21
<210> 260
   <211> 21
   <212> RNA
   <213> Artificial Sequence
<220>
   <221> source
   <223> /note="Description of Artificial Sequence: Synthetic oligonucleotide"
<400> 260
   ggagacgugg uguuugucag a 21
<210> 261
   <211> 21
   <212> RNA
   <213> Artificial Sequence
<220>
   <221> source
   <223> /note="Description of Artificial Sequence: Synthetic oligonucleotide"
<400> 261
   acgugguguu ugucagcaaa u 21
<210> 262
   <211> 21
   <212> RNA
   <213> Artificial Sequence
<220>
   <221> source
   <223> /note="Description of Artificial Sequence: Synthetic oligonucleotide"
<400> 262
   gugguguuug ucagcaaaga u 21
<210> 263
   <211> 21
   <212> RNA
   <213> Artificial Sequence
<220>
   <221> source
   <223> /note="Description of Artificial Sequence: Synthetic oligonucleotide"
<400> 263
   gguguuuguc agcaaagaug u 21
<210> 264
   <211> 21
   <212> RNA
   <213> Artificial Sequence
<220>
   <221> source
   <223> /note="Description of Artificial Sequence: Synthetic oligonucleotide"
<400> 264
   guguuuguca gcaaagaugu a 21
<210> 265
   <211> 21
   <212> RNA
   <213> Artificial Sequence
<220>
   <221> source
   <223> /note="Description of Artificial Sequence: Synthetic oligonucleotide"
<400> 265
   uguuugucag caaagaugug a 21
<210> 266
   <211> 21
   <212> RNA
   <213> Artificial Sequence
<220>
   <221> source
   <223> /note="Description of Artificial Sequence: Synthetic oligonucleotide"
<400> 266
   guuugucagc aaagaugugg a 21
<210> 267
   <211> 21
   <212> RNA
   <213> Artificial Sequence
<220>
   <221> source
   <223> /note="Description of Artificial Sequence: Synthetic oligonucleotide"
<400> 267
   uugucagcaa agauguggcc a 21
<210> 268
   <211> 21
   <212> RNA
   <213> Artificial Sequence
<220>
   <221> source
   <223> /note="Description of Artificial Sequence: Synthetic oligonucleotide"
<400> 268
   ugucagcaaa gauguggcca a 21
<210> 269
   <211> 21
   <212> RNA
   <213> Artificial Sequence
<220>
   <221> source
   <223> /note="Description of Artificial Sequence: Synthetic oligonucleotide"
<400> 269
   uguggccaag cacuuggggu u 21
<210> 270
   <211> 21
   <212> RNA
   <213> Artificial Sequence
<220>
   <221> source
   <223> /note="Description of Artificial Sequence: Synthetic oligonucleotide"
<400> 270
   agcacuuggg guuccaguca a 21
<210> 271
   <211> 21
   <212> RNA
   <213> Artificial Sequence
<220>
   <221> source
   <223> /note="Description of Artificial Sequence: Synthetic oligonucleotide"
<400> 271
   gggcuuguau ggucguguga a 21
<210> 272
   <211> 21
   <212> RNA
   <213> Artificial Sequence
<220>
   <221> source
   <223> /note="Description of Artificial Sequence: Synthetic oligonucleotide"
<400> 272
   uuguaugguc gugugaggaa a 21
<210> 273
   <211> 21
   <212> RNA
   <213> Artificial Sequence
<220>
   <221> source
   <223> /note="Description of Artificial Sequence: Synthetic oligonucleotide"
<400> 273
   ggucguguga ggaaaggggc u 21
<210> 274
   <211> 21
   <212> RNA
   <213> Artificial Sequence
<220>
   <221> source
   <223> /note="Description of Artificial Sequence: Synthetic oligonucleotide"
<400> 274
   gaggaaaggg gcugugcuug u 21
<210> 275
   <211> 23
   <212> RNA
   <213> Artificial Sequence
<220>
   <221> source
   <223> /note="Description of Artificial Sequence: Synthetic oligonucleotide"
<400> 275
   uuauuugucc accacauuga uga 23
<210> 276
   <211> 23
   <212> RNA
   <213> Artificial Sequence
<220>
   <221> source
   <223> /note="Description of Artificial Sequence: Synthetic oligonucleotide"
<400> 276
   uccucugggu auuuguccac cac 23
<210> 277
   <211> 23
   <212> RNA
   <213> Artificial Sequence
<220>
   <221> source
   <223> /note="Description of Artificial Sequence: Synthetic oligonucleotide"
<400> 277
   uucagaucga ccuucucaaa guc 23
<210> 278
   <211> 23
   <212> RNA
   <213> Artificial Sequence
<220>
   <221> source
   <223> /note="Description of Artificial Sequence: Synthetic oligonucleotide"
<400> 278
   uguggaucca cuugaaccgg guc 23
<210> 279
   <211> 23
   <212> RNA
   <213> Artificial Sequence
<220>
   <221> source
   <223> /note="Description of Artificial Sequence: Synthetic oligonucleotide"
<400> 279
   aaccccaggu gcuuggccac auc 23
<210> 280
   <211> 23
   <212> RNA
   <213> Artificial Sequence
<220>
   <221> source
   <223> /note="Description of Artificial Sequence: Synthetic oligonucleotide"
<400> 280
   aaugccauca aaccccugca agc 23
<210> 281
   <211> 23
   <212> RNA
   <213> Artificial Sequence
<220>
   <221> source
   <223> /note="Description of Artificial Sequence: Synthetic oligonucleotide"
<400> 281
   ucacaggauc ugcuucucuu cca 23
<210> 282
   <211> 23
   <212> RNA
   <213> Artificial Sequence
<220>
   <221> source
   <223> /note="Description of Artificial Sequence: Synthetic oligonucleotide"
<400> 282
   ugcacaggau cugcuucucu ucc 23
<210> 283
   <211> 23
   <212> RNA
   <213> Artificial Sequence
<220>
   <221> source
   <223> /note="Description of Artificial Sequence: Synthetic oligonucleotide"
<400> 283
   uacgcacagg aucugcuucu cuu 23
<210> 284
   <211> 23
   <212> RNA
   <213> Artificial Sequence
<220>
   <221> source
   <223> /note="Description of Artificial Sequence: Synthetic oligonucleotide"
<400> 284
   ucacgcacag gaucugcuuc ucu 23
<210> 285
   <211> 23
   <212> RNA
   <213> Artificial Sequence
<220>
   <221> source
   <223> /note="Description of Artificial Sequence: Synthetic oligonucleotide"
<400> 285
   uccacgcaca ggaucugcuu cuc 23
<210> 286
   <211> 23
   <212> RNA
   <213> Artificial Sequence
<220>
   <221> source
   <223> /note="Description of Artificial Sequence: Synthetic oligonucleotide"
<400> 286
   agccccacgc acaggaucug cuu 23
<210> 287
   <211> 23
   <212> RNA
   <213> Artificial Sequence
<220>
   <221> source
   <223> /note="Description of Artificial Sequence: Synthetic oligonucleotide"
<400> 287
   acuagcccca cgcacaggau cug 23
<210> 288
   <211> 23
   <212> RNA
   <213> Artificial Sequence
<220>
   <221> source
   <223> /note="Description of Artificial Sequence: Synthetic oligonucleotide"
<400> 288
   uccuuagggu acuuguccac cag 23
<210> 289
   <211> 23
   <212> RNA
   <213> Artificial Sequence
<220>
   <221> source
   <223> /note="Description of Artificial Sequence: Synthetic oligonucleotide"
<400> 289
   uagcagacac aucuggcagg cuc 23
<210> 290
   <211> 23
   <212> RNA
   <213> Artificial Sequence
<220>
   <221> source
   <223> /note="Description of Artificial Sequence: Synthetic oligonucleotide"
<400> 290
   ugucagauca accuucucaa agu 23
<210> 291
   <211> 23
   <212> RNA
   <213> Artificial Sequence
<220>
   <221> source
   <223> /note="Description of Artificial Sequence: Synthetic oligonucleotide"
<400> 291
   uuugaacugg gucagaucaa ccu 23
<210> 292
   <211> 23
   <212> RNA
   <213> Artificial Sequence
<220>
   <221> source
   <223> /note="Description of Artificial Sequence: Synthetic oligonucleotide"
<400> 292
   uccacuugaa cugggucaga uca 23
<210> 293
   <211> 23
   <212> RNA
   <213> Artificial Sequence
<220>
   <221> source
   <223> /note="Description of Artificial Sequence: Synthetic oligonucleotide"
<400> 293
   auguggaucc acuugaacug ggu 23
<210> 294
   <211> 23
   <212> RNA
   <213> Artificial Sequence
<220>
   <221> source
   <223> /note="Description of Artificial Sequence: Synthetic oligonucleotide"
<400> 294
   aauguggauc cacuugaacu ggg 23
<210> 295
   <211> 23
   <212> RNA
   <213> Artificial Sequence
<220>
   <221> source
   <223> /note="Description of Artificial Sequence: Synthetic oligonucleotide"
<400> 295
   uaauguggau ccacuugaac ugg 23
<210> 296
   <211> 23
   <212> RNA
   <213> Artificial Sequence
<220>
   <221> source
   <223> /note="Description of Artificial Sequence: Synthetic oligonucleotide"
<400> 296
   ucaaugugga uccacuugaa cug 23
<210> 297
   <211> 23
   <212> RNA
   <213> Artificial Sequence
<220>
   <221> source
   <223> /note="Description of Artificial Sequence: Synthetic oligonucleotide"
<400> 297
   uucaaugugg auccacuuga acu 23
<210> 298
   <211> 23
   <212> RNA
   <213> Artificial Sequence
<220>
   <221> source
   <223> /note="Description of Artificial Sequence: Synthetic oligonucleotide"
<400> 298
   ucucaaugug gauccacuug aac 23
<210> 299
   <211> 23
   <212> RNA
   <213> Artificial Sequence
<220>
   <221> source
   <223> /note="Description of Artificial Sequence: Synthetic oligonucleotide"
<400> 299
   uccucaaugu ggauccacuu gaa 23
<210> 300
   <211> 23
   <212> RNA
   <213> Artificial Sequence
<220>
   <221> source
   <223> /note="Description of Artificial Sequence: Synthetic oligonucleotide"
<400> 300
   ucccucaaug uggauccacu uga 23
<210> 301
   <211> 23
   <212> RNA
   <213> Artificial Sequence
<220>
   <221> source
   <223> /note="Description of Artificial Sequence: Synthetic oligonucleotide"
<400> 301
   ugcccucaau guggauccac uug 23
<210> 302
   <211> 23
   <212> RNA
   <213> Artificial Sequence
<220>
   <221> source
   <223> /note="Description of Artificial Sequence: Synthetic oligonucleotide"
<400> 302
   uggcccucaa uguggaucca cuu 23
<210> 303
   <211> 23
   <212> RNA
   <213> Artificial Sequence
<220>
   <221> source
   <223> /note="Description of Artificial Sequence: Synthetic oligonucleotide"
<400> 303
   ucggcccuca auguggaucc acu 23
<210> 304
   <211> 23
   <212> RNA
   <213> Artificial Sequence
<220>
   <221> source
   <223> /note="Description of Artificial Sequence: Synthetic oligonucleotide"
<400> 304
   uuccggcccu caauguggau cca 23
<210> 305
   <211> 23
   <212> RNA
   <213> Artificial Sequence
<220>
   <221> source
   <223> /note="Description of Artificial Sequence: Synthetic oligonucleotide"
<400> 305
   uauccgcugc agcaucuuca ccu 23
<210> 306
   <211> 23
   <212> RNA
   <213> Artificial Sequence
<220>
   <221> source
   <223> /note="Description of Artificial Sequence: Synthetic oligonucleotide"
<400> 306
   ucuauccgcu gcagcaucuu cac 23
<210> 307
   <211> 23
   <212> RNA
   <213> Artificial Sequence
<220>
   <221> source
   <223> /note="Description of Artificial Sequence: Synthetic oligonucleotide"
<400> 307
   aacaccacgu cuccguagcc aaa 23
<210> 308
   <211> 23
   <212> RNA
   <213> Artificial Sequence
<220>
   <221> source
   <223> /note="Description of Artificial Sequence: Synthetic oligonucleotide"
<400> 308
   aaacaccacg ucuccguagc caa 23
<210> 309
   <211> 23
   <212> RNA
   <213> Artificial Sequence
<220>
   <221> source
   <223> /note="Description of Artificial Sequence: Synthetic oligonucleotide"
<400> 309
   uaaacaccac gucuccguag cca 23
<210> 310
   <211> 23
   <212> RNA
   <213> Artificial Sequence
<220>
   <221> source
   <223> /note="Description of Artificial Sequence: Synthetic oligonucleotide"
<400> 310
   ucugacaaac accacgucuc cgu 23
<210> 311
   <211> 23
   <212> RNA
   <213> Artificial Sequence
<220>
   <221> source
   <223> /note="Description of Artificial Sequence: Synthetic oligonucleotide"
<400> 311
   auuugcugac aaacaccacg ucu 23
<210> 312
   <211> 23
   <212> RNA
   <213> Artificial Sequence
<220>
   <221> source
   <223> /note="Description of Artificial Sequence: Synthetic oligonucleotide"
<400> 312
   aucuuugcug acaaacacca cgu 23
<210> 313
   <211> 23
   <212> RNA
   <213> Artificial Sequence
<220>
   <221> source
   <223> /note="Description of Artificial Sequence: Synthetic oligonucleotide"
<400> 313
   acaucuuugc ugacaaacac cac 23
<210> 314
   <211> 23
   <212> RNA
   <213> Artificial Sequence
<220>
   <221> source
   <223> /note="Description of Artificial Sequence: Synthetic oligonucleotide"
<400> 314
   uacaucuuug cugacaaaca cca 23
<210> 315
   <211> 23
   <212> RNA
   <213> Artificial Sequence
<220>
   <221> source
   <223> /note="Description of Artificial Sequence: Synthetic oligonucleotide"
<400> 315
   ucacaucuuu gcugacaaac acc 23
<210> 316
   <211> 23
   <212> RNA
   <213> Artificial Sequence
<220>
   <221> source
   <223> /note="Description of Artificial Sequence: Synthetic oligonucleotide"
<400> 316
   uccacaucuu ugcugacaaa cac 23
<210> 317
   <211> 23
   <212> RNA
   <213> Artificial Sequence
<220>
   <221> source
   <223> /note="Description of Artificial Sequence: Synthetic oligonucleotide"
<400> 317
   uggccacauc uuugcugaca aac 23
<210> 318
   <211> 23
   <212> RNA
   <213> Artificial Sequence
<220>
   <221> source
   <223> /note="Description of Artificial Sequence: Synthetic oligonucleotide"
<400> 318
   uuggccacau cuuugcugac aaa 23
<210> 319
   <211> 23
   <212> RNA
   <213> Artificial Sequence
<220>
   <221> source
   <223> /note="Description of Artificial Sequence: Synthetic oligonucleotide"
<400> 319
   aaccccaagu gcuuggccac auc 23
<210> 320
   <211> 23
   <212> RNA
   <213> Artificial Sequence
<220>
   <221> source
   <223> /note="Description of Artificial Sequence: Synthetic oligonucleotide"
<400> 320
   uugacuggaa ccccaagugc uug 23
<210> 321
   <211> 23
   <212> RNA
   <213> Artificial Sequence
<220>
   <221> source
   <223> /note="Description of Artificial Sequence: Synthetic oligonucleotide"
<400> 321
   uucacacgac cauacaagcc ccu 23
<210> 322
   <211> 23
   <212> RNA
   <213> Artificial Sequence
<220>
   <221> source
   <223> /note="Description of Artificial Sequence: Synthetic oligonucleotide"
<400> 322
   uuuccucaca cgaccauaca agev23
<210> 323
   <211> 23
   <212> RNA
   <213> Artificial Sequence
<220>
   <221> source
   <223> /note="Description of Artificial Sequence: Synthetic oligonucleotide"
<400> 323
   agccccuuuc cucacacgac cau 23
<210> 324
   <211> 23
   <212> RNA
   <213> Artificial Sequence
<220>
   <221> source
   <223> /note="Description of Artificial Sequence: Synthetic oligonucleotide"
<400> 324
   acaagcacag ccccuuuccu cac 23
<210> 325
   <211> 2047
   <212> DNA
   <213> Macaca fascicularis
<400> 325 
ccccagtgtt gcaatgttct agccgtaaat atgtttggat aacccagcag agccatttaa 60

cctttgggaa cagggcaact agcgtatggc agcaggaatc caaccagtgc cctgagtgct 120

gaggcagaga ggaggacaga aaacgagagg ctggagattg tcaaattcag tatcccagtt 180

ggctcttgat tcttggtgaa accatccctc agctcctaga gggagactgt tagatcatga 240

aactaattat catccttttc ttctgctcca ggctgctact aagtttaacc caggaatcac 300

agtccgagga aattgactgc aatgataagg atttatttaa agctgtggat gctgctctga 360
 
agaaatataa cagtcaaaac caaagtaaca accagttcgt actgtaccgc ataactgaag 420

ccactaagat ggttcgctct gacacatttt attccttcaa gtacgaaatc aaggaggggg 480

attgtcctgt tcaaagtggc aaaacctggc aggactgtga ctacaaggat gctgcagaag 540

cggccactgg agaatgcaca gcaactgtgg ggaagagggc gagtatgaaa ttctctgtgg 600

ctacccagac ctgccagatt actccagccg agggccctgt ggtgacagcc cagtataact 660

gccttggctg tgtgcatcct atatcaacgc agagcccaga cctggagccc attctgagac 720

acggcgttca gtactttaac aacaatactc aacattcctc cctcttcacg cttagtgaag 780

taaaacgggc ccaaagacag gataccggtg aatgtacaga taatgcatac gtcgatactc 840

agctacaaat tgcttccttc tcacagaagt gtgacattta tccaggggag gattttgtac 900

aaccaccttc caagatttgc gtgggctgcc ccagagatat acccaccaac agcccagagc 960

tggaggagac actgactcac accatcacaa agcttaatgc ggagaataac gcaactttct 1020

atttcaagat tgacaatgtg aaaaaagcaa gagtacaggt ggtggctggc aagaaatatt 1080

ttattgactt tgtggccagg gaaaccacat gttccaagga aagtaatgaa gagttgaccg 1140

aaagctgtga gaccaaaaaa cttggtcaaa gcctagattg caatgctgaa gtttatgtgg 1200

taccctggga gaagaaaatt taccctactg tcaactgtca accactggga atgatctcat 1260

tgatgaaaag gcctccaggt tttcaccttt tccgatcaac acaagtaggg gaaataaaag 1320

aagaaacaac tagtcaccta aggtcctgcg agtacaaggg tcgaccccca aaggcagggg 1380

cagagccagc atctgagagg gaggtctctt gaccaatggg cagaatcttc actccaggca 1440

catagcccca gccacctctg ccagcaacct tgagaggaag gacaagaaga aagatgggat 1500

agaatttaaa tagagaagaa tgccatttta tcactctgcc tctgggtgaa ataaagatca 1560

gtcttgatgt tcttactcct ggttaattca cagtggtctc ctttcggcca tacccatcct 1620

gcagcaaatt ccagctggtc agagagtcag tgctgtggct ctgccatgga ggttcataac 1680

ccaacactgg aacattctct aaccaaggca gaagtgcttt ggcgggactt ccttttagca 1740

ccatgggtgc taaaagaaga gttagcaggt catgctacta atctaaggac tctctctacc 1800

ttctcttctt cctctttatc cagatttcca agccttagct aagagtaatt tggcttgttt 1860

agtattgttt tcttatagtc cagttgatta ccaaaaataa tttttaaaat catctctgtt 1920

aatatgatgt ctaccaactt ctcattatca gaaaatacct aacctccaaa gaaatttgaa 1980

attatctttc tgatccaggt aaagaagcaa atagaaaatc aataaaataa aaagtgacag 2040

acatttt 2047
<210> 326
   <211> 2047
   <212> DNA
   <213> Macaca fascicularis
<400> 326

aaaatgtctg tcacttttta ttttattgat tttctatttg cttctttacc tggatcagaa 60

agataatttc aaatttcttt ggaggttagg tattttctga taatgagaag ttggtagaca 120

tcatattaac agagatgatt ttaaaaatta tttttggtaa tcaactggac tataagaaaa 180

caatactaaa caagccaaat tactcttagc taaggcttgg aaatctggat aaagaggaag 240

aagagaaggt agagagagtc cttagattag tagcatgacc tgctaactct tcttttagca 300

cccatggtgc taaaaggaag tcccgccaaa gcacttctgc cttggttaga gaatgttcca 360

gtgttgggtt atgaacctcc atggcagagc cacagcactg actctctgac cagctggaat 420

ttgctgcagg atgggtatgg ccgaaaggag accactgtga attaaccagg agtaagaaca 480

tcaagactga tctttatttc acccagaggc agagtgataa aatggcattc ttctctattt 540

aaattctatc ccatctttct tcttgtcctt cctctcaagg ttgctggcag aggtggctgg 600

ggctatgtgc ctggagtgaa gattctgccc attggtcaag agacctccct ctcagatgct 660

ggctctgccc ctgcctttgg gggtcgaccc ttgtactcgc aggaccttag gtgactagtt 720

gtttcttctt ttatttcccc tacttgtgtt gatcggaaag gtgaaaaacc tggaggcctt 780

ttcatcaatg agatcattcc cagtggttga cagttgacag tagggtaaat tttcttctcc 840

cagggtacca cataaacttc agcattgcaa tctaggcttt gaccaagttt tttggtctca 900

cagctttcgg tcaactcttc attactttcc ttggaacatg tggtttccct ggccacaaag 960

tcaataaaat atttcttgcc agccaccacc tgtactcttg cttttttcac attgtcaatc 1020

ttgaaataga aagttgcgtt attctccgca ttaagctttg tgatggtgtg agtcagtgtc 1080

tcctccagct ctgggctgtt ggtgggtata tctctggggc agcccacgca aatcttggaa 1140

ggtggttgta caaaatcctc ccctggataa atgtcacact tctgtgagaa ggaagcaatt 1200

tgtagctgag tatcgacgta tgcattatct gtacattcac cggtatcctg tctttgggcc 1260

cgttttactt cactaagcgt gaagagggag gaatgttgag tattgttgtt aaagtactga 1320

acgccgtgtc tcagaatggg ctccaggtct gggctctgcg ttgatatagg atgcacacag 1380

ccaaggcagt tatactgggc tgtcaccaca gggccctcgg ctggagtaat ctggcaggtc 1440

tgggtagcca cagagaattt catactcgcc ctcttcccca cagttgctgt gcattctcca 1500

gtggccgctt ctgcagcatc cttgtagtca cagtcctgcc aggttttgcc actttgaaca 1560

ggacaatccc cctccttgat ttcgtacttg aaggaataaa atgtgtcaga gcgaaccatc 1620

ttagtggctt cagttatgcg gtacagtacg aactggttgt tactttggtt ttgactgtta 1680

tatttcttca gagcagcatc cacagcttta aataaatcct tatcattgca gtcaatttcc 1740

tcggactgtg attcctgggt taaacttagt agcagcctgg agcagaagaa aaggatgata 1800

attagtttca tgatctaaca gtctccctct aggagctgag ggatggtttc accaagaatc 1860

aagagccaac tgggatactg aatttgacaa tctccagcct ctcgttttct gtcctcctct 1920

ctgcctcagc actcagggca ctggttggat tcctgctgcc atacgctagt tgccctgttc 1980

ccaaaggtta aatggctctg ctgggttatc caaacatatt tacggctaga acattgcaac 2040

actgggg 2047

## Description

### Background of the Invention

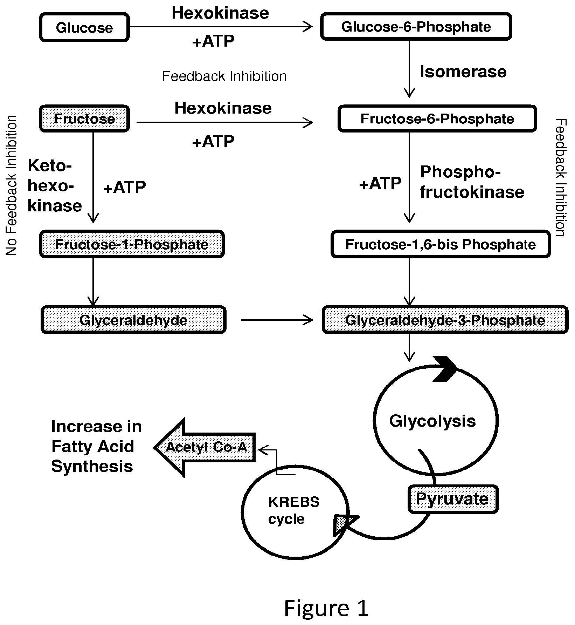

Epidemiological studies have shown that the western diet is one of the leading causes of the modern obesity pandemic. Increase in fructose uptake, associated with the use of enriched soft drinks and processed food, is proposed to be a major contributing factor to the epidemic. High fructose corn sweeteners started gaining widespread use in the food industry by 1967. Although glucose and fructose have the same caloric value per molecule, the two sugars are metabolized differently and utilize different GLUT transporters. Fructose is almost exclusively metabolized in the liver, and unlike the glucose metabolism pathway, the fructose metabolism pathway is not regulated by feedback inhibition by the product (Khaitan Z et al., (2013) J. Nutr. Metab. 2013, Article ID 682673, 1-12). While hexokinase and phosphofructokinase (PFK) regulate the production of glyceraldehyde-3-P from glucose, fructokinase or ketohexokinase (KHK) which is responsible for phosphorylation of fructose to fructose-1-phosphate in the liver, is not down regulated by increasing concentrations of fructose-1-phosphate. As a result, all fructose entering the cell is rapidly phosphorylated. (Cirillo P. et al., (2009) J. Am. Soc. Nephrol. 20: 545-553). Continued utilization of ATP to phosphorylate the fructose to fructose-1-phosphate results in intracellular phosphate depletion, ATP depletion, activation of AMP deaminase and formation of uric acid (Khaitan Z. et al., (2013) J. Nutr. Metab. Article ID 682673, 1-12). Increased uric acid further stimulates the up-regulation of KHK (Lanaspa M.A. et al., (2012) PLOS ONE 7(10): 1-11) and causes endothelial cell and adipocyte dysfunction. Fructose-1-phosphate is subsequently converted to glyceraldehyde by the action of aldolase B and is phosphorylated to glyceraldehyde-3-phosphate. The latter proceeds downstream to the glycolysis pathway to form pyruvate, which enters the citric acid cycle, wherefrom, under well-fed conditions, citrate is exported to the cytosol from the mitochondria, providing Acetyl Coenzyme A for lipogenesis (Figure 1).

The phosphorylation of fructose by KHK, and subsequent activation of lipogenesis leads to, for example, fatty liver, hypertriglyceridemia, dyslipidemia, and insulin resistance. Proinflammatory changes in renal proximal tubular cells have also been shown to be induced by KHK activity (Cirillo P. et al., (2009) J. Am. Soc. Nephrol. 20: 545-553). The phosphorylation of fructose by KHK is associated with diseases, disorders and/or conditions such as liver disease *(e.g*., fatty liver, steatohepatitis), dyslipidemia (*e.g*., hyperlipidemia, high LDL cholesterol, low HDL cholesterol, hypertriglyceridemia, postprandial hypertriglyceridemia), disorders of glycemic control *(e.g*., insulin resistance, diabetes), cardiovascular disease *(e.g*., hypertension, endothelial cell dysfunction), kidney disease (e.g., acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving. Accordingly, there is a need in the art for compositions and methods for treating diseases, disorders, and/or conditions associated with KHK activity.

Cirillo et al, Journal of the American Society of Nephrology, 20, 545-553 (2009) describes that ketohexokinase-dependent metabolism of fructose induces proinflammatory mediators in proximal tubular cells. Kay Lh Wu et al, Journal of Biomedical Science, 21, 8-14 (2014) describes an increase in adenosine-5'-triphosphate (ATP) content in rostral ventrolateral medulla is engaged in the high fructose diet-induced hypertension. WO 2012/019188 describes methods and compositions for the inhibition of fructokinase.

### Summary of the Invention

The present invention is defined by the claims. It provides a double stranded RNAi agent for inhibiting expression of human ketohexokinase (KHK), wherein said double stranded RNAi agent comprises a sense strand and an antisense strand forming a double stranded region, wherein the sense and antisense strands comprise sequences selected from the group consisting of SEQ ID NOs: 161 and 211, SEQ ID NOs: 162 and 212, SEQ ID NOs: 163 and 213, SEQ ID NOs: 164 and 214; and SEQ ID NOs: 165 and 215, wherein said double stranded RNAi agent comprises at least one modified nucleotide, and wherein at least one strand is conjugated to a ligand.

Described are compositions comprising RNAi agents, *e.g*., double-stranded RNAi agents, targeting ketohexokinase (KHK). The present disclosure also provides methods of using the compositions of the invention for inhibiting KHK expression and/or for treating a subject having a disorder that would benefit from inhibiting or reducing the expression of a KHK gene, *e.g.,* a KHK-associated disease, such as liver disease (*e.g*., fatty liver, steatohepatitis), dyslipidemia (*e.g*., hyperlipidemia, high LDL cholesterol, low HDL cholesterol, hypertriglyceridemia, postprandial hypertriglyceridemia), disorders of glycemic control (*e.g*., insulin resistance, diabetes), cardiovascular disease (*e.g*., hypertension, endothelial cell dysfunction), kidney disease (*e.g*., acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving.

Accordingly, described is a double stranded RNAi agent for inhibiting expression of ketohexokinase (KHK) comprising a sense strand and an antisense strand forming a double stranded region, wherein the sense strand comprises at least 15 contiguous nucleotides differing by no more than 3 nucleotides from any one of the nucleotide sequences of SEQ ID NO: 1, SEQ ID NO:3 or SEQ ID NO:5 and the antisense strand comprises at least 15 contiguous nucleotides differing by no more than 3 nucleotides from the nucleotide sequences of SEQ ID NO:2, SEQ ID NO:4 or SEQ ID NO:6.

In another aspect, the present disclosure provides a double stranded RNAi agent for inhibiting expression of ketohexokinase (KHK), comprising a sense strand and an antisense strand forming a double stranded region, the antisense strand comprising a region of complementarity which comprises at least 15 contiguous nucleotides differing by no more than 3 nucleotides from any one of the antisense sequences listed in any one of Tables 3, 4, and 5.

In one case, the sense and antisense strands comprise sequences are selected from the group consisting of AD-63851, AD-63820, AD-63853, AD-63839, AD-63854, AD-63855, and AD-63886 and any one of the sequences disclosed in any one of Tables 3, 4, 8, 11, 12, 14, and 15.

In one embodiment, the at least one modified nucleotide is selected from the group consisting of a 2'-O-methyl modified nucleotide, a nucleotide comprising a 5'-phosphorothioate group, a deoxy-nucleotide, a 3'-terminal deoxy-thymine (dT) nucleotide, a 2'-O-methyl modified nucleotide, a 2'-fluoro modified nucleotide, a 2'-deoxy-modified nucleotide, a terminal nucleotide linked to a cholesteryl derivative or a dodecanoic acid bisdecylamide group, a 2'-deoxy-2'-fluoro modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, 2'-C-alkyl-modified nucleotide, 2'-hydroxyl-modified nucleotide, a 2'-methoxyethyl modified nucleotide, a 2'-O-alkyl-modified nucleotide, a morpholino nucleotide, a phosphoramidate, a non-natural base comprising nucleotide, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate, and a nucleotide comprising a 5'-phosphate mimic.

In another case, the at least one modified nucleotides is selected from the group consisting of a 2'-O-methyl modified nucleotide, a nucleotide comprising a 5'-phosphorothioate group, and a terminal nucleotide linked to a cholesteryl derivative or a dodecanoic acid bisdecylamide group.

In another aspect, the present disclosure provides double stranded RNAi agents for inhibiting expression of ketohexokinase (KHK), which comprise a sense strand and an antisense strand forming a double stranded region, wherein the sense strand comprises at least 15 contiguous nucleotides differing by no more than 3 nucleotides from any one of the nucleotide sequences of SEQ ID NO:1, SEQ ID NO:3 or SEQ ID NO:5 and the antisense strand comprises at least 15 contiguous nucleotides differing by no more than 3 nucleotides from the nucleotide sequences of SEQ ID NO:2, SEQ ID NO:4 or SEQ ID NO:6, wherein substantially all of the nucleotides of the sense strand and substantially all of the nucleotides of the antisense strand are modified nucleotides, and wherein the sense strand is conjugated to a ligand attached at the 3'-terminus.

In one embodiment, all of the nucleotides of the sense strand and all of the nucleotides of the antisense strand are modified nucleotides.

In another case, the sense strand and the antisense strand comprise a region of complementarity which comprises at least 15 contiguous nucleotides differing by no more than 3 nucleotides from any one of the antisense sequences listed in any one of Tables 3, 4, 5, 6, and 7.

In some cases, the modified nucleotides is selected from the group consisting of 2'-O-methyl modified nucleotide, a nucleotide comprising a 5'-phosphorothioate group, a deoxy-nucleotide, a 3'-terminal deoxy-thymine (dT) nucleotide, a 2'-O-methyl modified nucleotide, a 2'-fluoro modified nucleotide, a 2'-deoxy-modified nucleotide, a terminal nucleotide linked to a cholesteryl derivative or a dodecanoic acid bisdecylamide group, a 2'-deoxy-2'-fluoro modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, 2'-C-alkyl-modified nucleotide, 2'-hydroxyl-modified nucleotide, a 2'-methoxyethyl modified nucleotide, a 2'-O-alkyl-modified nucleotide, a morpholino nucleotide, a phosphoramidate, a non-natural base comprising nucleotide, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate, and a nucleotide comprising a 5'-phosphate mimic.

In another case, at least one strand comprises a 3' overhang of at least 1 nucleotide. In another case, at least one strand comprises a 3' overhang of at least 2 nucleotides.

In another case, the double stranded RNAi agent is administered using a pharmaceutical composition and a lipid formulation. In another case, the lipid formulation comprises a lipid nanoparticle (LNP). In yet another case, the lipid nanoparticle (LNP) comprises a MC3 lipid.

In another aspect, the present disclosure provides a composition comprising a modified antisense polynucleotide agent, wherein the agent is capable of inhibiting the expression of ketohexokinase (KHK) in a cell, and comprises a sequence complementary to a sense sequence selected from the group of the sequences listed in any one of Tables 3, 4, and 5, wherein the polynucleotide is about 14 to about 30 nucleotides in length.

In another aspect, the present disclosure provides RNAi agents, *e.g*., double stranded RNAi agent capable of inhibiting the expression of ketohexokinase (KHK) in a cell, wherein the double stranded RNAi agent comprises a sense strand complementary to an antisense strand, wherein the antisense strand comprises a region complementary to part of an mRNA encoding KHK, wherein each strand is about 14 to about 30 nucleotides in length, wherein the double stranded RNAi agent is represented by formula (III):

| | |
|---|---|
| sense: | 5' nₚ -Nₐ -(X X X) ᵢ-N_{b} -Y Y Y -N_{b} -(Z Z Z)ⱼ -Nₐ - n_{q} 3' |
| antisense: | 3' nₚ'-Nₐ'-(X'X'X')ₖ-N_{b}'-Y'Y'Y'-N_{b}'-(Z'Z'Z')ₗ-Nₐ'-n_{q}' 5' (III) |

wherein:
i, j, k, and 1 are each independently 0 or 1;
p, p', q, and q' are each independently 0-6;
each Nₐ and Nₐ' independently represents an oligonucleotide sequence comprising 0-25 nucleotides which are either modified or unmodified or combinations thereof, each sequence comprising at least two differently modified nucleotides;
each N_{b} and N_{b}' independently represents an oligonucleotide sequence comprising 0-10 nucleotides which are either modified or unmodified or combinations thereof;
each nₚ, nₚ', n_{q}, and n_{q}', each of which may or may not be present, independently represents an overhang nucleotide;
XXX, YYY, ZZZ, X'X'X', Y'Y'Y', and Z'Z'Z' each independently represent one motif of three identical modifications on three consecutive nucleotides;
modifications on N_{b} differ from the modification on Y and modifications on N_{b}' differ from the modification on Y'; and
   wherein the sense strand is conjugated to at least one ligand.

In one case, i is 0; j is 0; i is 1; j is 1; both i and j are 0; or both i and j are 1. In another case, k is 0; 1 is 0; k is 1; 1 is 1; both k and 1 are 0; or both k and 1 are 1.

In one case, XXX is complementary to X'X'X', YYY is complementary to Y'Y'Y', and ZZZ is complementary to Z'Z'Z'.

In one case, the YYY motif occurs at or near the cleavage site of the sense strand.

In another case, the Y'Y'Y' motif occurs at the 11, 12 and 13 positions of the antisense strand from the 5'-end.

In one case, Y' is 2'-O-methyl.

In one case, formula (III) is represented by formula (IIIa):

| | |
|---|---|
| sense: | 5' nₚ -Nₐ -Y Y Y -Nₐ - n_{q} 3' |
| antisense: | 3' n_{p'}-N_{a'}- Y'Y'Y'- N_{a'}- n_{q'} 5' (IIIa) |

In another case, formula (III) is represented by formula (IIIb):

| | |
|---|---|
| sense: | 5' nₚ -Nₐ -Y Y Y -N_{b} -Z Z Z -Nₐ - n_{q} 3' |
| antisense: | 3' n_{p'}-N_{a'}-Y'Y'Y'-N_{b'}-Z'Z'Z'- N_{a'}-n_{q'}5' (IIIb) |

wherein each N_{b} and N_{b}' independently represents an oligonucleotide sequence comprising 1-5 modified nucleotides.

In yet another embodiment, formula (III) is represented by formula (IIIc):

| | |
|---|---|
| sense: | 5' nₚ -Nₐ -XXX -N_{b} -Y Y Y -Nₐ - n_{q} 3' |
| antisense: | 3' n_{p'}-N_{a'}- X'X'X'-N_{b'}- Y'Y'Y'- N_{a'}- n_{q'}. 5' (IIIc) |

wherein each N_{b} and N_{b}' independently represents an oligonucleotide sequence comprising 1-5 modified nucleotides.

In a further case, formula (III) is represented by formula (IIId):

| | |
|---|---|
| sense: | 5' nₚ -Nₐ -X X X- N_{b} -Y Y Y -N_{b} -Z Z Z -Nₐ - n_{q} 3' |
| antisense: | 3' n_{p'}-N_{a'},- X'X'X'- N_{b'}-Y'Y'Y'-N_{b'}-Z'Z'Z'- N_{a'}-n_{q'} 5' (IIId) |

wherein each N_{b} and N_{b}' independently represents an oligonucleotide sequence comprising 1-5 modified nucleotides and each Nₐ and Nₐ' independently represents an oligonucleotide sequence comprising 2-10 modified nucleotides.

In one case, the double-stranded region is 15-30 nucleotide pairs in length. In another case, the double-stranded region is 17-23 nucleotide pairs in length. In yet another case, the double-stranded region is 17-25 nucleotide pairs in length. In a further case, the double-stranded region is 23-27 nucleotide pairs in length. In another case, the double-stranded region is 19-21 nucleotide pairs in length. In another case, the double-stranded region is 19-23 nucleotide pairs in length. In another case, the double-stranded region is 21-23 nucleotide pairs in length. In yet another case, each strand has 15-30 nucleotides.

In one case, the modifications on the nucleotides are selected from the group consisting of LNA, CRN, cET, UNA, HNA, CeNA, 2'-methoxyethyl, 2'-O-alkyl, 2'-O-allyl, 2'-C- allyl, 2'-fluoro, 2'-deoxy, 2'-hydroxyl, and combinations thereof. In another embodiment, the modifications on the nucleotides are 2'-O-methyl or 2'-fluoro modifications.

In one embodiment, the ligand is one or more GalNAc derivatives attached through a bivalent or trivalent branched linker. In another embodiment, the ligand is

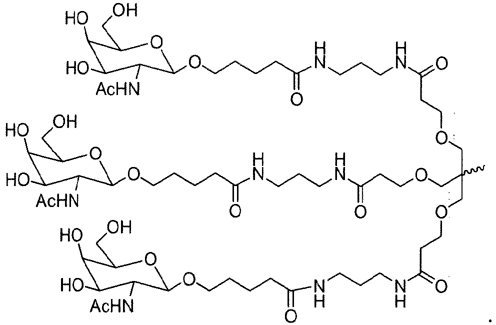

In one embodiment, the ligand is attached to the 3' end of the sense strand.

In another embodiment, the RNAi agent is conjugated to the ligand as shown in the following schematic 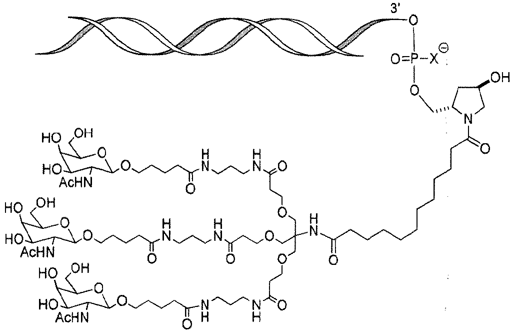 wherein X is O or S. In a specific embodiment, X is O.

In one embodiment, the agent further comprises at least one phosphorothioate or methylphosphonate internucleotide linkage.

In a further embodiment, the phosphorothioate or methylphosphonate internucleotide linkage is at the 3'-terminus of one strand. In another embodiment, the strand is the antisense strand. In another case, the strand is the sense strand.

In one case, the phosphorothioate or methylphosphonate internucleotide linkage is at the 5'-terminus of one strand. In another case, the strand is the antisense strand. In a further case, the strand is the sense strand.

In one embodiment, the phosphorothioate or methylphosphonate internucleotide linkage is at the both the 5'- and 3'-terminus of one strand. In another case, the strand is the antisense strand.

In another embodiment, the double stranded RNAi agent comprises 6-8 phosphorothioate internucleotide linkages. In a further embodiment, the antisense strand comprises two phosphorothioate internucleotide linkages at the 5'-terminus and two phosphorothioate internucleotide linkages at the 3'-terminus, and the sense strand comprises at least two phosphorothioate internucleotide linkages at either the 5'-terminus or the 3'-terminus of the sense strand.

In one case, the base pair at the 1 position of the 5'-end of the antisense strand of the duplex is an AU base pair. In another case, the Y nucleotides contain a 2'-fluoro modification. In a further case, the Y' nucleotides contain a 2'-O-methyl modification.

In one case, p'>0. In another case, p'=2. In a further case, q'=0, p=0, q=0, and p' overhang nucleotides are complementary to the target mRNA. In yet a further case, q'=0, p=0, q=0, and p' overhang nucleotides are non-complementary to the target mRNA.

In one case, the sense strand has a total of 21 nucleotides and the antisense strand has a total of 23 nucleotides.

In another case, at least one nₚ' is linked to a neighboring nucleotide via a phosphorothioate linkage. In a further case, all nₚ' are linked to neighboring nucleotides via phosphorothioate linkages.

In another case, the RNAi agent is selected from the group of RNAi agents listed in any one of Tables 3, 4, and 5.

In one aspect, the present disclosure provides double stranded RNAi agents for inhibiting expression of ketohexokinase (KHK). The double stranded RNAi agents include a sense strand and an antisense strand forming a double stranded region, wherein the sense strand comprises at least 15 contiguous nucleotides differing by no more than 3 nucleotides from any one of the nucleotide sequences of SEQ ID NO:1, SEQ ID NO:3 or SEQ ID NO:5 and the antisense strand comprises at least 15 contiguous nucleotides differing by no more than 3 nucleotides from the nucleotide sequences of SEQ ID NO:2, SEQ ID NO:4 or SEQ ID NO:6, wherein substantially all of the nucleotides of the sense strand comprise a modification selected from the group consisting of a 2'-O-methyl modification and a 2'-fluoro modification wherein the sense strand comprises two phosphorothioate internucleotide linkages at the 5'-terminus, wherein substantially all of the nucleotides of the antisense strand comprise a modification selected from the group consisting of a 2'-O-methyl modification and a 2'-fluoro modification,wherein the antisense strand comprises two phosphorothioate internucleotide linkages at the 5'-terminus and two phosphorothioate internucleotide linkages at the 3'-terminus, and wherein the sense strand is conjugated to one or more GalNAc derivatives attached through a branched bivalent or trivalent linker at the 3'-terminus.

In one case, all of the nucleotides of the sense strand and all of the nucleotides of the antisense strand comprise a modification.

In another aspect, the present disclosure provides RNAi agents, *e.g*., double stranded RNAi agents capable of inhibiting the expression of KHK (ketohexokinase) in a cell, wherein the double stranded RNAi agent comprises a sense strand complementary to an antisense strand, wherein the antisense strand comprises a region complementary to part of an mRNA encoding KHK, wherein each strand is about 14 to about 30 nucleotides in length, wherein the double stranded RNAi agent is represented by formula (III):

| | |
|---|---|
| sense: | 5' nₚ -Nₐ -(X X X) ᵢ-N_{b}-Y Y Y -N_{b} -(Z Z Z)ⱼ -Nₐ - n_{q} 3' |
| antisense: | 3' nₚ'-Nₐ'-(X'X'X')ₖ-N_{b}'-Y'Y'Y'-N_{b}'-(Z'Z'Z')ᵢ-Nₐ'-n_{q}' 5' (III) |

wherein:
i, j, k, and 1 are each independently 0 or 1;
p, p', q, and q' are each independently 0-6;
each Nₐ and Nₐ' independently represents an oligonucleotide sequence comprising 0-25 nucleotides which are either modified or unmodified or combinations thereof, each sequence comprising at least two differently modified nucleotides;
each N_{b} and N_{b}' independently represents an oligonucleotide sequence comprising 0-10 nucleotides which are either modified or unmodified or combinations thereof;
each nₚ, nₚ', n_{q}, and n_{q}', each of which may or may not be present independently represents an overhang nucleotide;
XXX, YYY, ZZZ, X'X'X', Y'Y'Y', and Z'Z'Z' each independently represent one motif of three identical modifications on three consecutive nucleotides, and wherein the modifications are 2'-O-methyl or 2'-fluoro modifications;
modifications on N_{b} differ from the modification on Y and modifications on N_{b}' differ from the modification on Y'; and
wherein the sense strand is conjugated to at least one ligand.

In yet another aspect, the present disclosure provides RNAi agents, *e.g*., double stranded RNAi agents capable of inhibiting the expression of ketohexokinase (KHK) in a cell, wherein the double stranded RNAi agent comprises a sense strand complementary to an antisense strand, wherein the antisense strand comprises a region complementary to part of an mRNA encoding KHK, wherein each strand is about 14 to about 30 nucleotides in length, wherein the double stranded RNAi agent is represented by formula (III):

| | |
|---|---|
| sense: | 5' nₚ -Nₐ -(X X X)ᵢ-N_{b} -Y Y Y -N_{b} -(Z Z Z)ⱼ -Nₐ - n_{q} 3' |
| antisense: | 3' n_{p'}-Nₐ'-(X'X'X')ₖ-N_{b}'-Y'Y'Y'-N_{b}'-(Z'Z'Z')ₗ-Nₐ'-n_{q}' 5' (III) |

wherein:
i, j, k, and 1 are each independently 0 or 1;
each nₚ, n_{q}, and n_{q}', each of which may or may not be present, independently represents an overhang nucleotide;
p, q, and q' are each independently 0-6;
nₚ' >0 and at least one nₚ' is linked to a neighboring nucleotide via a phosphorothioate linkage;
each Nₐ and Nₐ' independently represents an oligonucleotide sequence comprising 0-25 nucleotides which are either modified or unmodified or combinations thereof, each sequence comprising at least two differently modified nucleotides;
each N_{b} and N_{b}' independently represents an oligonucleotide sequence comprising 0-10 nucleotides which are either modified or unmodified or combinations thereof;
XXX, YYY, ZZZ, X'X'X', Y'Y'Y', and Z'Z'Z' each independently represent one motif of three identical modifications on three consecutive nucleotides, and wherein the modifications are 2'-O-methyl or 2'-fluoro modifications;
modifications on N_{b} differ from the modification on Y and modifications on N_{b}' differ from the modification on Y'; and
wherein the sense strand is conjugated to at least one ligand.

In a further aspect, the present disclosure provides RNAi agents, *e.g*., double stranded RNAi agent capable of inhibiting the expression of ketohexokinase (KHK) in a cell, wherein the double stranded RNAi agent comprises a sense strand complementary to an antisense strand, wherein the antisense strand comprises a region complementary to part of an mRNA encoding KHK, wherein each strand is about 14 to about 30 nucleotides in length, wherein the double stranded RNAi agent is represented by formula (III):

| | |
|---|---|
| sense: | 5' nₚ -Nₐ -(X X X) ᵢ-N_{b} -Y Y Y -N_{b} -(Z Z Z)ⱼ -Nₐ - n_{q} 3' |
| antisense: | 3' nₚ'-Nₐ'-(X'X'X')ₖ-N_{b}'-Y'Y'Y'-N_{b}'-(Z'Z'Z')ᵢ-Nₐ'-n_{q}' 5' (III) |

wherein:
i, j, k, and 1 are each independently 0 or 1;
each nₚ, n_{q}, and n_{q}', each of which may or may not be present, independently represents an overhang nucleotide;
p, q, and q' are each independently 0-6;
nₚ' >0 and at least one nₚ' is linked to a neighboring nucleotide via a phosphorothioate linkage;
each Nₐ and Nₐ' independently represents an oligonucleotide sequence comprising 0-25 nucleotides which are either modified or unmodified or combinations thereof, each sequence comprising at least two differently modified nucleotides;
each N_{b} and N_{b}' independently represents an oligonucleotide sequence comprising 0-10 nucleotides which are either modified or unmodified or combinations thereof;
XXX, YYY, ZZZ, X'X'X', Y'Y'Y', and Z'Z'Z' each independently represent one motif of three identical modifications on three consecutive nucleotides, and wherein the modifications are 2'-O-methyl or 2'-fluoro modifications;
modifications on N_{b} differ from the modification on Y and modifications on N_{b}' differ from the modification on Y'; and
wherein the sense strand is conjugated to at least one ligand, wherein the ligand is one or more GalNAc derivatives attached through a bivalent or trivalent branched linker.

In another aspect, the present disclosure provides RNAi agents, *e.g*., double stranded RNAi agents capable of inhibiting the expression of ketohexokinase (KHK) in a cell, wherein the double stranded RNAi agent comprises a sense strand complementary to an antisense strand, wherein the antisense strand comprises a region complementary to part of an mRNA encoding KHK, wherein each strand is about 14 to about 30 nucleotides in length, wherein the double stranded RNAi agent is represented by formula (III):

| | |
|---|---|
| sense: | 5' nₚ -Nₐ -(X X X)ᵢ-N_{b} -Y Y Y -N_{b} -(Z Z Z)ⱼ -Nₐ - n_{q} 3' |
| antisense: | 3' nₚ'-Nₐ'-(X'X'X')ₖ-N_{b}'-Y'Y'Y'-N_{b}'-(Z'Z'Z')ₗ-Nₐ'- n_{q}' 5' (III) |

wherein:
i, j, k, and 1 are each independently 0 or 1;
each nₚ, n_{q}, and n_{q}', each of which may or may not be present, independently represents an overhang nucleotide;
p, q, and q' are each independently 0-6;
nₚ' >0 and at least one nₚ' is linked to a neighboring nucleotide via a phosphorothioate linkage;
each Nₐ and Nₐ' independently represents an oligonucleotide sequence comprising 0-25 nucleotides which are either modified or unmodified or combinations thereof, each sequence comprising at least two differently modified nucleotides;
each N_{b} and N_{b}' independently represents an oligonucleotide sequence comprising 0-10 nucleotides which are either modified or unmodified or combinations thereof;
XXX, YYY, ZZZ, X'X'X', Y'Y'Y', and Z'Z'Z' each independently represent one motif of three identical modifications on three consecutive nucleotides, and wherein the modifications are 2'-O-methyl or 2'-fluoro modifications;
modifications on N_{b} differ from the modification on Y and modifications on N_{b}' differ from the modification on Y';
wherein the sense strand comprises at least one phosphorothioate linkage; and
wherein the sense strand is conjugated to at least one ligand, wherein the ligand is one or more GalNAc derivatives attached through a bivalent or trivalent branched linker.

In yet another aspect, the present disclosure provides RNAi agents, e.g.,double stranded RNAi agent capable of inhibiting the expression of ketohexokinase (KHK) in a cell, wherein the double stranded RNAi agent comprises a sense strand complementary to an antisense strand, wherein the antisense strand comprises a region complementary to part of an mRNA encoding KHK, wherein each strand is about 14 to about 30 nucleotides in length, wherein the double stranded RNAi agent is represented by formula (III):

| | |
|---|---|
| sense: | 5' nₚ-Nₐ -Y Y Y - Nₐ - n_{q} 3' |
| antisense: | 3' nₚ'-Nₐ'- Y'Y'Y'- Nₐ' -n_{q}' 5' (IIIa) |

wherein:
each nₚ, n_{q}, and n_{q}', each of which may or may not be present, independently represents an overhang nucleotide;
p, q, and q' are each independently 0-6;
nₚ' >0 and at least one nₚ' is linked to a neighboring nucleotide via a phosphorothioate linkage;
each Nₐ and Nₐ' independently represents an oligonucleotide sequence comprising 0-25 nucleotides which are either modified or unmodified or combinations thereof, each sequence comprising at least two differently modified nucleotides;
YYY and Y'Y'Y' each independently represent one motif of three identical modifications on three consecutive nucleotides, and wherein the modifications are 2'-O-methyl or 2'-fluoro modifications;
wherein the sense strand comprises at least one phosphorothioate linkage; and
wherein the sense strand is conjugated to at least one ligand, wherein the ligand is one or more GalNAc derivatives attached through a bivalent or trivalent branched linker.

The present invention also provides pharmaceutical compositions comprising the double stranded RNAi agents of the invention.

The present disclosure provides double stranded RNAi agents comprising the RNAi agents listed in any one of Tables 3, 4 5, 6, and 7.

In one case, a cell contains the double stranded RNAi agent.

In another case, a vector encodes at least one strand of a double stranded RNAi agent, wherein the double stranded RNAi agent comprises a region of complementarity to at least a part of an mRNA encoding ketohexokinase, wherein the double stranded RNAi agent is 30 base pairs or less in length, and wherein the double stranded RNAi agent targets the mRNA for cleavage. In a further case, the region of complementarity is at least 15 nucleotides in length. In another case, the region of complementarity is 19 to 21 nucleotides in length. In another case, a cell contains the vector.

In some cases, the double stranded RNAi agent or the composition comprising a modified antisense polynucleotide agent is administered using a pharmaceutical composition.

The double stranded RNAi agent may be administered in a solution. In some cases, the double stranded RNAi agent is administered in an unbuffered solution. In another case, the unbuffered solution is saline or water. In another embodiment, the double stranded RNAi agent is administered with a buffer solution. In yet another case, the buffer solution comprises acetate, citrate, prolamine, carbonate, or phosphate or any combination thereof. In some cases, the buffer solution is phosphate buffered saline (PBS).

In another aspect, the present disclosure provides methods of inhibiting ketohexokinase (KHK) expression in a cell. The methods include contacting the cell with the double stranded RNAi agent, a pharmaceutical composition, a composition comprising a modified antisense polynucleotide agent, or a vector comprising the RNAi agent and maintaining the cell produced for a time sufficient to obtain degradation of the mRNA transcript of a KHK gene, thereby inhibiting expression of the KHK gene in the cell.

In one case, the cell is within a subject. In a further case, the subject is a human. In a further case, the subject suffers from a ketohexokinase-associated disease.

In one case, the KHK expression is inhibited by at least about 30%, about 40%, about 50%, about 60%, about 70%, about 80%, about 90%, about 95%, about 98% or about 100%.

In another aspect, the present disclosure provides methods of treating a subject having a ketohexokinase (KHK)-associated disorder, comprising administering to the subject a therapeutically effective amount of the double stranded RNAi agent, a composition comprising a modified antisense polynucleotide agent, or a pharmaceutical composition comprising the double stranded RNAi agent, thereby treating the subject.

In another aspect, the present disclosure provides methods of treating a subject having a ketohexokinase (KHK)-associated disorder which include subcutaneously administering to the subject a therapeutically effective amount of a double stranded RNAi agent, wherein the double stranded RNAi agent comprises a sense strand and an antisense strand forming a double stranded region, wherein the sense strand comprises at least 15 contiguous nucleotides differing by no more than 3 nucleotides from any one of the nucleotide sequences of SEQ ID NO: 1, SEQ ID NO:3 or SEQ ID NO:5 and the antisense strand comprises at least 15 contiguous nucleotides differing by no more than 3 nucleotides from the nucleotide sequences of SEQ ID NO:2, SEQ ID NO:4 or SEQ ID NO:6, wherein substantially all of the nucleotides of the antisense strand comprise a modification selected from the group consisting of a 2'-O-methyl modification and a 2'-fluoromodification, wherein the antisense strand comprises two phosphorothioate internucleotide linkages at the 5'-terminus and two phosphorothioate internucleotide linkages at the 3'-terminus, wherein substantially all of the nucleotides of the sense strand comprise a modification selected from the group consisting of a 2'-O-methyl modification and a 2'-fluoromodification, wherein the sense strand comprises two phosphorothioate internucleotide linkages at the 5'-terminus and, wherein the sense strand is conjugated to one or more GalNAc derivatives attached through a branched bivalent or trivalent linker at the 3'-terminus.

In one case, all of the nucleotides of the sense strand and all of the nucleotides of the antisense strand comprise a modification.

In one case, the subject is a human.

In one embodiment, the ketohexokinase-associated disease is selected from the group consisting liver disease, dyslipidemia, disorders of glycemic control, cardiovascular disease, kidney disease, metabolic syndrome, adipocyte dysfunction, visceral adipose deposition, obesity, hyperuricemia, gout, eating disorders, and excessive sugar craving. In a specific embodiment, the liver disease is fatty liver and/or steatohepatitis. In another embodiment, the dyslipidemia is selected from the group consisting of hyperlipidemia, high LDL cholesterol, low HDL cholesterol, hypertriglyceridemia, and postprandial hypertriglyceridemia. In yet another embodiment, the disorder of glycemic control is insulin resistance and/or diabetes. In a further embodiment, the cardiovascular disease is hypertension and/or endothelial cell dysfunction. In yet another embodiment, the kidney disease is selected from the group consisting of acute kidney disorder, tubular dysfunction, and proinflammatory changes to the proximal tubules.

In one embodiment, the double stranded RNAi agent is to be administered at a dose of about 0.01 mg/kg to about 10 mg/kg or about 1 mg/kg to about 10 mg/kg. In a preferred case, the double stranded RNAi agent is administered at a dose of about 0.1 mg/kg, about 1.0 mg/kg, or about 3.0 mg/kg. In a specific case, the double stranded RNAi agent is administered at a dose of about 1 mg/kg to about 10 mg/kg.

In one case, the double stranded RNAi agent is administered subcutaneously or intravenously.

In one case, the RNAi agent is administered in two or more doses.

In yet another case, the methods further comprise administering to the subject, an additional therapeutic agent. In some cases, the additional therapeutic agent is selected from the group consisting of an HMG-CoA reductase inhibitor, a diabetic therapy, an anti-hypertensive drug, and resveratrol.

### Brief Description of Drawings

Figure 1 depicts the metabolism of fructose by ketohexokinase and the metabolism of glucose and fructose by hexokinase.
Figure 2 depicts the exon arrangement on the human KHK gene for the transcript products of ketohexokinase A (NM_000221.2), ketohexokinase C (NM_006488.2) and transcript variant X5 (XM_005264298.1).

### Detailed Description of the Invention

The present disclosure provides compositions comprising RNAi agents, *e.g*., double-stranded iRNA agents, targeting KHK. The present disclosure also provides methods of using the compositions of the invention for inhibiting KHK expression and for treating KHK-associated disease, disorders, and/or conditions, *e.g.,* liver disease *(e.g.,* fatty liver, steatohepatitis), dyslipidemia (*e.g*., hyperlipidemia, high LDL cholesterol, low HDL cholesterol, hypertriglyceridemia, postprandial hypertriglyceridemia), disorders of glycemic control (*e.g*., insulin resistance, diabetes), cardiovascular disease (*e.g*., hypertension, endothelial cell dysfunction), kidney disease (*e.g*., acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving (Khaitan Z. et al., (2013) J. Nutr. Metab., Article ID 682673, 1-12; Diggle C.P. et al., (2009) J. Hisotchem. Cytochem., 57(8): 763-774; Cirillo P. et al., (2009) J. Am. Soc. Nephrol., 20: 545-553; Lanaspa M.A. et al., (2012) PLOS ONE 7(10): 1-11).

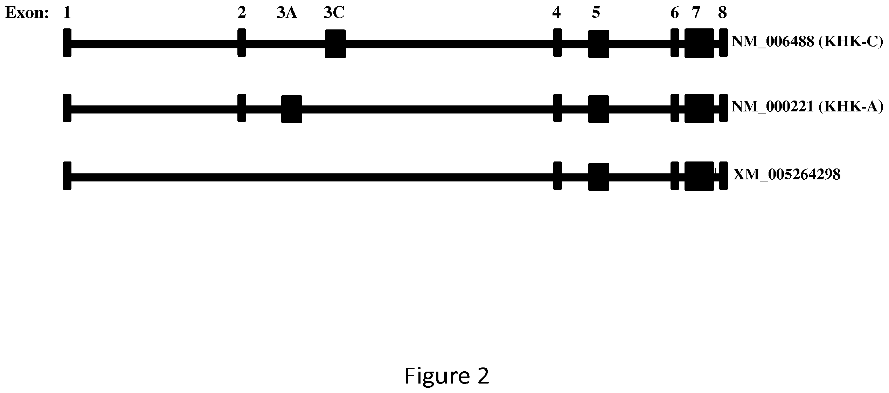

The KHK (Ketohexokinase) gene is located on chromosome 2p23 and encodes ketohexokinase, also known as fructokinase. KHK is a phosphotransferase enzyme with an alcohol as the phosphate acceptor. KHK belongs to the ribokinase family of carbohydrate kinases (Trinh et al., ACTA Cryst., D65: 201-211). Two isoforms of ketohexokinase have been identified, KHK-A and KHK-C, that result from alternative splicing of the full length mRNA. These isoforms differ by inclusion of either exon 3a or 3c, and differ by 32 amino acids between positions 72 and 115 (see, *e.g*., Figure 2). KHK-C mRNA is expressed at high levels, predominantly in the liver, kidney and small intestine. KHK-C has a much lower Kₘ for fructose binding than KHK-A, and as a result, is highly effective in phosphorylating dietary fructose. The sequence of a human KHK-C mRNA transcript may be found at, for example, GenBank Accession No. GI: 153218447 (NM_006488.2; SEQ ID NO:1). The sequence of a human KHK-A mRNA transcript may be found at, for example GenBank Accession No. GI: 153218446 (NM_000221.2; SEQ ID NO:3). The sequence of full-length human KHK mRNA is provided in GenBank Accession No. GI: 530367552 (XM_005264298.1; SEQ ID NO:5) was used (Figure 2).

The present disclosure provides iRNA agents, compositions and methods for modulating the expression of a KHK gene. In certain embodiments, expression of KHK is reduced or inhibited using a KHK-specific iRNA agent, thereby leading to a decrease in the phosphorylation of fructose to fructose-1-phosphate and thereby preventing an increase in uric acid levels and an increase in lipogenesis. Thus, inhibition of KHK gene expression or activity using the iRNA compositions of the invention is useful as a therapy to reduce the lipogenic effects of dietary fructose and preventing the concomitant accumulation of uric acid in a subject. Such inhibition is useful for treating diseases, disorders, and/or conditions such as liver disease (*e.g*., fatty liver, steatohepatitis), dyslipidemia (*e.g*., hyperlipidemia, high LDL cholesterol, low HDL cholesterol, hypertriglyceridemia, postprandial hypertriglyceridemia), disorders of glycemic control (*e.g*., insulin resistance, diabetes), cardiovascular disease (*e.g*., hypertension, endothelial cell dyfunction), kidney disease (*e.g*., acute kidney disorder, tubular dysfunction, proinflammatory changes to the proximal tubules), metabolic syndrome, adiocyte dysfunction, visceral adipose deposition, obesity, hyperuricemia, gout, eating disorders, and excessive sugar craving.

### I. Definitions

In order that the present invention may be more readily understood, certain terms are first defined. In addition, it should be noted that whenever a value or range of values of a parameter are recited, it is intended that values and ranges intermediate to the recited values are also intended to be part of this invention.

The articles "a" and "an" are used herein to refer to one or to more than one *(i.e*., to at least one) of the grammatical object of the article. By way of example, "an element" means one element or more than one element, *e.g*., a plurality of elements.

The term "including" is used herein to mean, and is used interchangeably with, the phrase "including but not limited to".

The term "or" is used herein to mean, and is used interchangeably with, the term "and/or," unless context clearly indicates otherwise.

As used herein, "KHK" refers to the ketohexokinase gene or protein. KHK is also known as fructokinase. The term "KHK" includes human KHK, the amino acid and complete coding sequence of which may be found at for example, GenBank Accession No. BC006233. The sequence of a human KHK-C mRNA transcript may be found at, for example, GenBank Accession No. GI: 153218447 (NM_006488.2; SEQ ID NO:1). The reverse complement of SEQ ID NO: 1 is provided as SEQ ID NO:2. The sequence of a human KHK-A mRNA transcript may be found at, for example GenBank Accession No. GI: 153218446 (NM 000221.2; SEQ ID NO:3). The reverse complement of SEQ ID NO:3 is provided as SEQ ID NO:4. The sequence of a human full-length KHK mRNA transcript is provided in GenBank Accession No. GI: 530367552 (XM_005264298.1; SEQ ID NO:5). The reverse complement of SEQ ID NO:5 is provided as SEQ ID NO:6. The sequence of mouse (*Mus musculus*) KHK mRNA can be found at, for example, GenBank Accession No. GI:118130797 (NM_008439.3; SEQ ID NO:7), and the reverse complement sequence is provided at SEQ ID NO:8. The sequence of rat (*Rattus rattovorus*) KHK mRNA can be found at, for example GenBank Accession No. GI: 126432547 (NM_031855.3; SEQ ID NO:9), and the reverse complement sequence is provided at SEQ ID NO: 10. The sequence of cynomolgus monkey (*Macaca fascicularis*) KHK mRNA, variant X1, can be found at, for example GenBank Accession No. GI:544482340 (XM_005576321.1; SEQ ID NO:11) or GI:, and the reverse complement sequence is provided at SEQ ID NO: 12. The sequence of cynomolgus monkey (*Macaca fascicularis*) KHK mRNA, variant X3, can be found at, for example GenBank Accession No. GI:544482340 (XM_005576321.1; SEQ ID NO:325) or GI:, and the reverse complement sequence is provided at SEQ ID NO:326. Additional examples of KHK mRNA sequences are readily available using publicly available databases, *e.g*., GenBank., UniProt, OMIM, and the *Macaca* genome project web site

As used herein, "target sequence" refers to a contiguous portion of the nucleotide sequence of an mRNA molecule formed during the transcription of a KHK gene, including mRNA that is a product of RNA processing of a primary transcription product.

As used herein, "target sequence" refers to a contiguous portion of the nucleotide sequence of an mRNA molecule formed during the transcription of a KHK gene, including mRNA that is a product of RNA processing of a primary transcription product. In one embodiment, the target portion of the sequence will be at least long enough to serve as a substrate for iRNA-directed cleavage at or near that portion of the nucleotide sequence of an mRNA molecule formed during the transcription of a KHK gene.

The target sequence may be from about 9-36 nucleotides in length, *e.g*., about 15-30 nucleotides in length. For example, the target sequence can be from about 15-30 nucleotides, 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24,20-23, 20-22, 20-21, 21-30, 21-29, 21-28, 21-27, 21-26, 21-25, 21-24, 21-23, or 21-22 nucleotides in length. Ranges and lengths intermediate to the above recited ranges and lengths are also contemplated to be part of the invention.

As used herein, the term "strand comprising a sequence" refers to an oligonucleotide comprising a chain of nucleotides that is described by the sequence referred to using the standard nucleotide nomenclature.

"G," "C," "A," "T" and "U" each generally stand for a nucleotide that contains guanine, cytosine, adenine, thymidine and uracil as a base, respectively. However, it will be understood that the term "ribonucleotide" or "nucleotide" can also refer to a modified nucleotide, as further detailed below, or a surrogate replacement moiety (see, *e.g.,* Table 2). The skilled person is well aware that guanine, cytosine, adenine, and uracil can be replaced by other moieties without substantially altering the base pairing properties of an oligonucleotide comprising a nucleotide bearing such replacement moiety. For example, without limitation, a nucleotide comprising inosine as its base can base pair with nucleotides containing adenine, cytosine, or uracil. Hence, nucleotides containing uracil, guanine, or adenine can be replaced in the nucleotide sequences of dsRNA featured in the invention by a nucleotide containing, for example, inosine. In another example, adenine and cytosine anywhere in the oligonucleotide can be replaced with guanine and uracil, respectively to form G-U Wobble base pairing with the target mRNA. Sequences containing such replacement moieties are suitable for the compositions and methods featured in the invention.

The terms "iRNA", "RNAi agent," "iRNA agent,", "RNA interference agent" or "small inhibitory RNA" or "siRNA" as used interchangeably herein, refer to an agent that contains RNA as that term is defined herein, and which mediates the targeted cleavage of an RNA transcript *via* an RNA-induced silencing complex (RISC) pathway. iRNA directs the sequence-specific degradation of mRNA through a process known as RNA interference (iRNA). The iRNA modulates, *e.g.,* inhibits, the expression of KHK in a cell, *e.g.,* a cell within a subject, such as a mammalian subject.

In one case, an iRNA agent includes a single stranded RNA that interacts with a target RNA sequence, *e.g*., a KHK target mRNA sequence, to direct the cleavage of the target RNA. Without wishing to be bound by theory it is believed that long double stranded RNA introduced into cells is broken down into siRNA by a Type III endonuclease known as Dicer (Sharp et al. (2001) Genes Dev. 15:485). Dicer, a ribonuclease-III-like enzyme, processes the dsRNA into 19-23 base pair short interfering RNAs with characteristic two base 3' overhangs (Bernstein, et al., (2001) Nature 409:363). The siRNAs are then incorporated into an RNA-induced silencing complex (RISC) where one or more helicases unwind the siRNA duplex, enabling the complementary antisense strand to guide target recognition (Nykanen, et al., (2001) Cell 107:309). Upon binding to the appropriate target mRNA, one or more endonucleases within the RISC cleave the target to induce silencing (Elbashir, et al., (2001) Genes Dev. 15:188). Thus, in one aspect the disclosure relates to a single stranded RNA (siRNA) generated within a cell and which promotes the formation of a RISC complex to effect silencing of the target gene, *i.e.,* a KHK gene. Accordingly, the term "siRNA" is also used herein to refer to an iRNA as described above.

In another case, the iRNA agent may be, a single-stranded siRNA that is introduced into a cell or organism to inhibit a target mRNA. Single-stranded iRNA agents bind to the RISC endonuclease, Argonaute 2, which then cleaves the target mRNA. The single-stranded siRNAs are generally 15-30 nucleotides and are chemically modified. The design and testing of single-stranded siRNAs are described in U.S. Patent No. 8,101,348 and in Lima et al., (2012) Cell 150: 883-894. Any of the antisense nucleotide sequences described herein may be used as a single-stranded s iRNA as described herein or as chemically modified by the methods described in Lima et al., (2012) Cell 150;:883-894.

Sometimes an "iRNA" for use in the described compositions, uses, and methods is a double-stranded RNA and is referred to herein as a "double stranded RNAi agent," "double-stranded RNA (dsRNA) molecule," "dsRNA agent," or "dsRNA". The term "dsRNA", refers to a complex of ribonucleic acid molecules, having a duplex structure comprising two anti-parallel and substantially complementary nucleic acid strands, referred to as having "sense" and "antisense" orientations with respect to a target RNA, *i.e.,* a KHK gene. In some embodiments of the invention, a double-stranded RNA (dsRNA) triggers the degradation of a target RNA, *e.g.,* an mRNA, through a post-transcriptional gene-silencing mechanism referred to herein as RNA interference or RNAi or iRNA.

In general, the majority of nucleotides of each strand of a dsRNA molecule are ribonucleotides, but as described in detail herein, each or both strands can also include one or more non-ribonucleotides, *e.g.,* a deoxyribonucleotide and/or a modified nucleotide. In addition, as used in this specification, an " iRNA agent" may include ribonucleotides with chemical modifications; an iRNA agent may include substantial modifications at multiple nucleotides.

As used herein, the term "modified nucleotide" refers to a nucleotide having, independently, a modified sugar moiety, a modified internucleotide linkage, and/or a modified nucleobase. Thus, the term modified nucleotide encompasses substitutions, additions or removal of, *e.g.,* a functional group or atom, to internucleoside linkages, sugar moieties, or nucleobases. The modifications suitable for use in the agents of the invention include all types of modifications disclosed herein or known in the art. Any such modifications, as used in a siRNA type molecule, are encompassed by "RNAi agent" for the purposes of this specification and claims.

The duplex region may be of any length that permits specific degradation of a desired target RNA through a RISC pathway, and may range from about 9 to 36 base pairs in length, *e.g*., about 15-30 base pairs in length, for example, about 9, 10, 11, 12, 13, 14, 15, 16, 17, 18, 19, 20, 21, 22, 23, 24, 25, 26, 27, 28, 29, 30, 31, 32, 33, 34, 35, or 36 base pairs in length, such as about 15-30, 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24,20-23, 20-22, 20-21, 21-30, 21-29, 21-28, 21-27, 21-26, 21-25, 21-24, 21-23, or 21-22 base pairs in length. Ranges and lengths intermediate to the above recited ranges and lengths are also contemplated.

The two strands forming the duplex structure may be different portions of one larger RNA molecule, or they may be separate RNA molecules. Where the two strands are part of one larger molecule, and therefore are connected by an uninterrupted chain of nucleotides between the 3'-end of one strand and the 5'-end of the respective other strand forming the duplex structure, the connecting RNA chain is referred to as a "hairpin loop." A hairpin loop can comprise at least one unpaired nucleotide. In some cases, the hairpin loop can comprise at least 2, at least 3, at least 4, at least 5, at least 6, at least 7, at least 8, at least 9, at least 10, at least 20, at least 23 or more unpaired nucleotides.

Where the two substantially complementary strands of a dsRNA are comprised by separate RNA molecules, those molecules need not, but can be covalently connected.

Where the two strands are connected covalently by means other than an uninterrupted chain of nucleotides between the 3'-end of one strand and the 5'-end of the respective other strand forming the duplex structure, the connecting structure is referred to as a "linker." The RNA strands may have the same or a different number of nucleotides. The maximum number of base pairs is the number of nucleotides in the shortest strand of the dsRNA minus any overhangs that are present in the duplex. In addition to the duplex structure, an iRNA may comprise one or more nucleotide overhangs.

As used herein, the term "nucleotide overhang" refers to at least one unpaired nucleotide that protrudes from the duplex structure of an iRNA, *e.g.,* a dsRNA. For example, when a 3'-end of one strand of a dsRNA extends beyond the 5'-end of the other strand, or *vice versa*, there is a nucleotide overhang. A dsRNA can comprise an overhang of at least one nucleotide; alternatively the overhang can comprise at least two nucleotides, at least three nucleotides, at least four nucleotides, at least five nucleotides or more. A nucleotide overhang can comprise or consist of a nucleotide/nucleoside analog, including a deoxynucleotide/nucleoside. The overhang(s) can be on the sense strand, the antisense strand or any combination thereof. Furthermore, the nucleotide(s) of an overhang can be present on the 5'-end, 3'-end or both ends of either an antisense or sense strand of a dsRNA.

In one case, the antisense strand of a dsRNA has a 1-10 nucleotide, *e.g*., a 1, 2, 3, 4, 5, 6, 7, 8, 9, or 10 nucleotide, overhang at the 3'-end and/or the 5'-end. In one embodiment, the sense strand of a dsRNA has a 1-10 nucleotide, *e.g*., a 1, 2, 3, 4, 5, 6, 7, 8, 9, or 10 nucleotide, overhang at the 3'-end and/or the 5'-end. In another case, one or more of the nucleotides in the overhang is replaced with a nucleoside thiophosphate.

"Blunt" or "blunt end" means that there are no unpaired nucleotides at that end of the double stranded iRNA agent, *i.e.,* no nucleotide overhang. A "blunt ended" iRNA agent is a dsRNA that is double-stranded over its entire length, *i.e.,* no nucleotide overhang at either end of the molecule. The iRNA agents of the invention include iRNA agents with nucleotide overhangs at one end *(i.e.,* agents with one overhang and one blunt end) or with nucleotide overhangs at both ends.

The term "antisense strand" or "guide strand" refers to the strand of an iRNA, *e.g.,* a dsRNA, which includes a region that is substantially complementary to a target sequence, *e.g.,* a KHK mRNA. As used herein, the term "region of complementarity" refers to the region on the antisense strand that is substantially complementary to a sequence, for example a target sequence, *e.g.,* a KHK nucleotide sequence, as defined herein. Where the region of complementarity is not fully complementary to the target sequence, the mismatches can be in the internal or terminal regions of the molecule. Generally, the most tolerated mismatches are in the terminal regions, *e.g*., within 5, 4, 3, or 2 nucleotides of the 5'- and/or 3'-terminus of the iRNA.

The term "sense strand," or "passenger strand" as used herein, refers to the strand of an iRNA that includes a region that is substantially complementary to a region of the antisense strand as that term is defined herein.

As used herein, the term "cleavage region" refers to a region that is located immediately adjacent to the cleavage site. The cleavage site is the site on the target at which cleavage occurs. In some cases, the cleavage region comprises three bases on either end of, and immediately adjacent to, the cleavage site. In some cases, the cleavage region comprises two bases on either end of, and immediately adjacent to, the cleavage site. In some embodiments, the cleavage site specifically occurs at the site bound by nucleotides 10 and 11 of the antisense strand, and the cleavage region comprises nucleotides 11, 12 and 13.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence, as will be understood by the skilled person. Such conditions can, for example, be stringent conditions, where stringent conditions can include: 400 mM NaCl, 40 mM PIPES pH 6.4, 1 mM EDTA, 50°C or 70°C for 12-16 hours followed by washing (see, *e.g.,* "Molecular Cloning: A Laboratory Manual, Sambrook, et al. (1989) Cold Spring Harbor Laboratory Press). Other conditions, such as physiologically relevant conditions as can be encountered inside an organism, can apply. The skilled person will be able to determine the set of conditions most appropriate for a test of complementarity of two sequences in accordance with the ultimate application of the hybridized nucleotides.

Complementary sequences within an iRNA, *e.g*., within a dsRNA as described herein, include base-pairing of the oligonucleotide or polynucleotide comprising a first nucleotide sequence to an oligonucleotide or polynucleotide comprising a second nucleotide sequence over the entire length of one or both nucleotide sequences. Such sequences can be referred to as "fully complementary" with respect to each other herein. However, where a first sequence is referred to as "substantially complementary" with respect to a second sequence herein, the two sequences can be fully complementary, or they can form one or more, but generally not more than 5, 4, 3 or 2 mismatched base pairs upon hybridization for a duplex up to 30 base pairs, while retaining the ability to hybridize under the conditions most relevant to their ultimate application, *e.g*., inhibition of gene expression via a RISC pathway. However, where two oligonucleotides are designed to form, upon hybridization, one or more single stranded overhangs, such overhangs shall not be regarded as mismatches with regard to the determination of complementarity. For example, a dsRNA comprising one oligonucleotide 21 nucleotides in length and another oligonucleotide 23 nucleotides in length, wherein the longer oligonucleotide comprises a sequence of 21 nucleotides that is fully complementary to the shorter oligonucleotide, can yet be referred to as "fully complementary" for the purposes described herein.

"Complementary" sequences, as used herein, can also include, or be formed entirely from non-Watson-Crick base pairs and/or base pairs formed from non-natural and modified nucleotides, in so far as the above requirements with respect to their ability to hybridize are fulfilled. Such non-Watson-Crick base pairs include, but are not limited to, G:U Wobble or Hoogstein base pairing.

The terms "complementary," "fully complementary" and "substantially complementary" herein can be used with respect to the base matching between the sense strand and the antisense strand of a dsRNA, or between the antisense strand of an iRNA agent and a target sequence, as will be understood from the context of their use.

As used herein, a polynucleotide that is "substantially complementary to at least part of' a messenger RNA (mRNA) refers to a polynucleotide that is substantially complementary to a contiguous portion of the mRNA of interest *(e.g.,* an mRNA encoding KHK). For example, a polynucleotide is complementary to at least a part of a KHK mRNA if the sequence is substantially complementary to a non-interrupted portion of an mRNA encoding KHK.

In general, the majority of nucleotides of each strand are ribonucleotides, but as described in detail herein, each or both strands can also include one or more non-ribonucleotides, *e.g.,* a deoxyribonucleotide and/or a modified nucleotide. In addition, an "iRNA" may include ribonucleotides with chemical modifications. Such modifications may include all types of modifications disclosed herein or known in the art. Any such modifications, as used in an iRNA molecule, are encompassed by "iRNA" for the purposes of this specification and claims.

An agent for use in the methods and compositions may be a single-stranded antisense oligonucleotide molecule that inhibits a target mRNA *via* an antisense inhibition mechanism. The single-stranded antisense oligonucleotide molecule is complementary to a sequence within the target mRNA. The single-stranded antisense oligonucleotides can inhibit translation in a stoichiometric manner by base pairing to the mRNA and physically obstructing the translation machinery, see Dias, N. et al., (2002) Mol Cancer Ther 1:347-355. The single-stranded antisense oligonucleotide molecule may be about 15 to about 30 nucleotides in length and have a sequence that is complementary to a target sequence. For example, the single-stranded antisense oligonucleotide molecule may comprise a sequence that is at least about 15, 16, 17, 18, 19, 20, or more contiguous nucleotides from any one of the antisense sequences described herein.

The term "inhibiting," as used herein, is used interchangeably with "reducing," "silencing," "downregulating," "suppressing" and other similar terms, and includes any level of inhibition.

The phrase "inhibiting expression of a KHK," as used herein, includes inhibition of expression of any KHK gene (such as, *e.g.,* a mouse KHK gene, a rat KHK gene, a monkey KHK gene, or a human KHK gene) as well as variants or mutants of a KHK gene that encode a KHK protein.

"Inhibiting expression of a KHK gene" includes any level of inhibition of a KHK gene, *e.g.,* at least partial suppression of the expression of a KHK gene, such as an inhibition by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

The expression of a KHK gene may be assessed based on the level of any variable associated with KHK gene expression, *e.g*., KHK mRNA level, or KHK protein level. Inhibition may be assessed by a decrease in an absolute or relative level of one or more of these variables compared with a control level. The control level may be any type of control level that is utilized in the art, *e.g.,* a pre-dose baseline level, or a level determined from a similar subject, cell, or sample that is untreated or treated with a control (such as, *e.g*., buffer only control or inactive agent control).

In one case, at least partial suppression of the expression of a KHK gene, is assessed by a reduction of the amount of KHK mRNA which can be isolated from or detected in a first cell or group of cells in which a KHK gene is transcribed and which has or have been treated such that the expression of a KHK gene is inhibited, as compared to a second cell or group of cells substantially identical to the first cell or group of cells but which has or have not been so treated (control cells). The degree of inhibition may be expressed in terms of $\frac{\left(mRNA in control cells\right) - \left(mRNA in treated cells\right)}{\left(mRNA in control cells\right)} ⋅ 100 %$

The phrase "contacting a cell with an iRNA agent," such as a dsRNA, as used herein, includes contacting a cell by any possible means. Contacting a cell with an iRNA agent includes contacting a cell *in vitro* with the iRNA or contacting a cell *in vivo* with the iRNA. The contacting may be done directly or indirectly. Thus, for example, the RNAi agent may be put into physical contact with the cell by the individual performing the method, or alternatively, the iRNA agent may be put into a situation that will permit or cause it to subsequently come into contact with the cell.

Contacting a cell *in vitro* may be done, for example, by incubating the cell with the iRNA agent. Contacting a cell *in vivo* may be done, for example, by injecting the iRNA agent into or near the tissue where the cell is located, or by injecting the iRNA agent into another area, *e.g.,* the bloodstream or the subcutaneous space, such that the agent will subsequently reach the tissue where the cell to be contacted is located. For example, the iRNA agent may contain and/or be coupled to a ligand, *e.g*., GalNAc3, that directs the iRNA agent to a site of interest, *e.g.,* the liver. Combinations of *in vitro* and *in vivo* methods of contacting are also possible. For example, a cell may also be contacted *in vitro* with an iRNA agent and subsequently transplanted into a subject.

In one case, contacting a cell with an iRNA includes "introducing" or "delivering the iRNA into the cell" by facilitating or effecting uptake or absorption into the cell. Absorption or uptake of an iRNA can occur through unaided diffusive or active cellular processes, or by auxiliary agents or devices. Introducing an iRNA into a cell may be *in vitro* and/or *in vivo.* For example, for *in vivo* introduction, iRNA can be injected into a tissue site or administered systemically. *In vivo* delivery can also be done by a beta-glucan delivery system, such as those described in U.S. Patent Nos. 5,032,401 and 5,607,677, and U.S. Publication No. 2005/0281781. *In vitro* introduction into a cell includes methods known in the art such as electroporation and lipofection. Further approaches are described herein below and/or are known in the art.

The term "lipid nanoparticle" or "LNP" is a vesicle comprising a lipid layer encapsulating a pharmaceutically active molecule, such as a nucleic acid molecule, *e.g*., an iRNA or a plasmid from which an iRNA is transcribed. LNPs are described in, for example, U.S. Patent Nos. 6,858,225, 6,815,432, 8,158,601, and 8,058,069.

As used herein, a "subject" is an animal, such as a mammal, including a primate (such as a human, a non-human primate, *e.g*., a monkey, and a chimpanzee), a non-primate (such as a cow, a pig, a camel, a llama, a horse, a goat, a rabbit, a sheep, a hamster, a guinea pig, a cat, a dog, a rat, a mouse, a horse, and a whale), or a bird *(e.g.,* a duck or a goose). In an embodiment, the subject is a human, such as a human being treated or assessed for a disease, disorder and/or condition that would benefit from reduction in KHK expression; a human at risk for a disease, disorder and/or condition that would benefit from reduction in ketohexokinase expression; a human having a disease, disorder or condition that would benefit from reduction in ketohexokinase expression.

As used herein, the terms "treating" or "treatment" refer to a beneficial or desired result including, but not limited to, alleviation or amelioration of one or more symptoms associated with unwanted ketohexokinase activation (*e.g.*, liver disease *(e.g.,* fatty liver, steatohepatitis), dyslipidemia *(e.g.,* hyperlipidemia, high LDL cholesterol, low HDL cholesterol, hypertriglyceridemia, postprandial hypertriglyceridemia), disorders of glycemic control (*e.g*., insulin resistance, diabetes), cardiovascular disease (e.g., hypertension, endothelial cell dysfunction), kidney disease (*e.g*., acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving; stabilization *(i.e.,* not worsening) of the one or more diseases, disorders, and/or conditions associated with ketohexokinase activity; amelioration or palliation of unwanted ketohexokinase activity (*e.g*., phosphorylation of fructose leading to activation of lipogenesis and increased uric acid production) whether detectable or undetectable. "Treatment" can also mean prolonging survival as compared to expected survival in the absence of treatment.

Consequently, reduction or amelioration of any of the symptoms resulting from fructose metabolism via KHK pathway includes reduction or amelioration of any one or more of the symptoms included but not limited to the group comprising fatty liver, steatohepatitis, high blood pressure, hypertension, high cholesterol, high LDL cholesterol, low HDL cholesterol, hyperlipidemia, hypertriglyceridemia, kidney disease, metabolic syndrome, excessive sugar craving, eating disorder, post-prandial hypertriglyceridemia, hepatosteatosis, gout, diabetes, acute kidney disorder, tubular dysfunction, insulin resistance and obesity. In some embodiments, the reduction or amelioration of a KHK-associated disease, disorder or condition means reduction of hepatosteatosis, excess body fat, obesity, high cholesterol, hypertension or high blood pressure.

The term "lower" in the context of the level of ketohexokinase activity in a subject or as disease marker or symptom refers to a statistically significant decrease in such level. The decrease can be, for example, at least 10%,'at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or more and is preferably down to a level accepted as within the range of normal for an individual without such disorder.

As used herein, "prevention" or "preventing," when used in reference to a disease, disorder or condition thereof, that would benefit from a reduction in expression of a KHK gene, refers to a reduction in the likelihood that a subject will develop a symptom associated with such a disease, disorder, or condition, *e.g*., a symptom of phosphorylation of fructose to fructose-1-phosphate such as activation of lipogenesis and increased production of uric acid resulting in, for example, liver disease *(e.g.,* fatty liver, steatohepatitis), dyslipidemia (*e.g*., hyperlipidemia, high LDL cholesterol, low HDL cholesterol, hypertriglyceridemia, postprandial hypertriglyceridemia), disorders of glycemic control (*e.g*., insulin resistance, diabetes), cardiovascular disease *(e.g.,* hypertension, endothelial cell dysfunction), kidney disease *(e.g.,* acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving. The likelihood of developing any or all of these diseases, disorders and/or conditions is reduced, for example, when an individual having one or more risk factors for any or all of these diseases, disorders and or conditions, either fails to develop the disease, disorder and/or condition or develops a disease, disorder and or/condition with less severity relative to a population having the same risk factors and not receiving treatment as described herein. The failure to develop a disease, disorder and/or condition, or the reduction in the development of a symptom associated with such a disease, disorder and/or condition (e.g., by at least about 10% on a clinically accepted scale for that disease or disorder), or the exhibition of delayed symptoms delayed *(e.g.,* by days, weeks, months or years) is considered effective prevention.

As used herein, the term "ketohexokinase associated disease" is a disease, disorder or condition that is caused by, or associated with, the ketohexokinase gene or protein, *e.g.,* a disease, disorder or condition caused by or associated with the phosphorylation of fructose to fructose-1 -phosphate. Such diseases are typically associated with activation of lipogenesis and increase uric acid production. Non-limiting examples of ketohexokinase associated diseases include liver disease *(e.g.,* fatty liver, steatohepatitis), dyslipidemia *(e.g.,* hyperlipidemia, high LDL cholesterol, low HDL cholesterol, hypertriglyceridemia, postprandial hypertriglyceridemia), disorders of glycemic control (e.g., insulin resistance, diabetes), cardiovascular disease *(e.g.,* hypertension, endothelial cell dysfunction), kidney disease *(e.g.,* acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving.

"Therapeutically effective amount" as used herein is intended to include the amount of an iRNA agent, that when administered to a subject having a KHK-associated disease or disorder, is sufficient to effect treatment of the disease or disorder *(e.g.,* by diminishing or ameliorating the disease or one or more symptoms of disease). The "therapeutically effective amount" may vary depending on the RNAi agent, how the agent is administered, the disease and its severity and the history, age, weight, family history, genetic makeup, the types or preceding or concomitant treatments, if any, and other individual characteristics of the subject to be treated.

"Prophylactically effective amount," as used herein, is intended to include the amount of an iRNA agent that, when administered to a subject having a KHK-associated disease but not yet (or currently) experiencing or displaying symptoms of the disease, and/or a subject at risk of developing a KHK-associated disease, *e.g.,* a liver disease, dyslipidemia, a disorder of glycemic control, a cardiovascular disease, a kidney disease, metabolic syndrome, and/or obesity, is sufficient to prevent or ameliorate the disease or one or more symptoms of the disease. Ameliorating the disease includes slowing the course of the disease or reducing the severity of later-developed disease. The "prophylactically effective amount" may vary depending on the iRNA agent, how the agent, is administered, the degree of risk of disease, and the history, age, weight, family history, genetic makeup, the types of preceding or concomitant treatments, if any, and other individual characteristics of the patient to be treated.

A "therapeutically effective amount" or "prophylactically effective amount" also includes an amount of an RNAi agent that produces some desired local or systemic effect at a reasonable benefit/risk ratio applicable to any treatment. iRNA agents employed in the methods of the present invention may be administered in a sufficient amount to produce a reasonable benefit/risk ratio applicable to such treatment. The phrase "pharmaceutically acceptable" is employed herein to refer to those compounds, materials, compositions, and/or dosage forms which are, within the scope of sound medical judgment, suitable for use in contact with the tissues of human subjects and animal subjects without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, manufacturing aid (*e.g*., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the subject being treated. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) lubricating agents, such as magnesium state, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; and (22) other non-toxic compatible substances employed in pharmaceutical formulations.

The term "sample," as used herein, includes a collection of similar fluids, cells, or tissues isolated from a subject, as well as fluids, cells, or tissues present within a subject. Examples of biological fluids include blood, serum and serosal fluids, plasma, urine, lymph, cerebrospinal fluid, ocular fluids, saliva, and the like. Tissue samples may include samples from tissues, organs or localized regions. For example, samples may be derived from particular organs, parts of organs, or fluids or cells within those organs. In certain embodiments, samples may be derived from the liver (*e.g*., whole liver or certain segments of liver or certain types of cells in the liver, such as, *e.g*., hepatocytes). In preferred embodiments, a "sample derived from a subject" refers to blood or plasma drawn from the subject. In further embodiments, a "sample derived from a subject" refers to liver tissue (or subcomponents thereof) derived from the subject.

### II. iRNAs of the invention

The present disclosure also provides iRNAs which inhibit the expression of a KHK gene. In one case, the iRNA agent includes double-stranded ribonucleic acid (dsRNA) molecules for inhibiting the expression of a KHK gene in a cell, such as a cell within a subject, *e.g*., a mammal, such as a human having a KHK-associated disease or disorder, including but not limited to liver disease *(e.g.,* fatty liver, steatohepatitis), dyslipidemia *(e.g.,* hyperlipidemia, high LDL cholesterol, low HDL cholesterol, hypertriglyceridemia, postprandial hypertriglyceridemia), disorders of glycemic control *(e.g.,* insulin resistance, diabetes), cardiovascular disease *(e.g.,* hypertension, endothelial cell dysfunction), kidney disease (*e.g*., acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving. The dsRNA includes an antisense strand having a region of complementarity to at least a part of an mRNA formed in the expression of a KHK gene. The region of complementarity is about 30 nucleotides or less in length (*e.g*., about 30, 29, 28, 27, 26, 25, 24, 23, 22, 21, 20, 19, or 18 nucleotides or less in length). Upon contact with a cell expressing the KHK gene, the iRNA inhibits the expression of the KHK gene *(e.g.,* a human, a primate, a non-primate, or a bird KHK gene) by. at least about 10% as assayed by, for example, a PCR or branched DNA (bDNA)-based method, or by a protein-based method, such as by immunofluorescence analysis, using, for example, Western Blotting or flowcytometric techniques.

A dsRNA includes two RNA strands that are complementary and hybridize to form a duplex structure under conditions in which the dsRNA will be used. One strand of a dsRNA (the antisense strand) includes a region of complementarity that is substantially complementary, and generally fully complementary, to a target sequence. The target sequence can be derived from the sequence of an mRNA formed during the expression of a KHK gene. The other strand (the sense strand) includes a region that is complementary to the antisense strand, such that the two strands hybridize and form a duplex structure when combined under suitable conditions. As described elsewhere herein and as known in the art, the complementary sequences of a dsRNA can also be contained as self-complementary regions of a single nucleic acid molecule, as opposed to being on separate oligonucleotides.

Generally, the duplex structure is between 15 and 30 base pairs in length, *e.g*., between, 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24,20-23, 20-22, 20-21, 21-30, 21-29, 21-28, 21-27, 21-26, 21-25, 21-24, 21-23, or 21-22 base pairs in length. Ranges and lengths intermediate to the above recited ranges and lengths are also contemplated.

Similarly, the region of complementarity to the target sequence is between 15 and 30 nucleotides in length, *e.g*., between 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24,20-23, 20-22, 20-21, 21-30, 21-29, 21-28, 21-27, 21-26, 21-25, 21-24, 21-23, or 21-22 nucleotides in length. Ranges and lengths intermediate to the above recited ranges and lengths are also contemplated.

In some cases, the dsRNA is between about 15 and about 20 nucleotides in length, or between about 25 and about 30 nucleotides in length. In general, the dsRNA is long enough to serve as a substrate for the Dicer enzyme. For example, it is well-known in the art that dsRNAs longer than about 21-23 nucleotides in length may serve as substrates for Dicer. As the ordinarily skilled person will also recognize, the region of an RNA targeted for cleavage will most often be part of a larger RNA molecule, often an mRNA molecule. Where relevant, a "part" of an mRNA target is a contiguous sequence of an mRNA target of sufficient length to allow it to be a substrate for iRNA-directed cleavage *(i.e.,* cleavage through a RISC pathway).

One of skill in the art will also recognize that the duplex region is a primary functional portion of a dsRNA, *e.g.,* a duplex region of about 9 to 36 base pairs, *e.g.,* about 10-36, 11-36, 12-36, 13-36, 14-36, 15-36, 9-35, 10-35, 11-35, 12-35, 13-35, 14-35, 15-35, 9-34, 10-34, 11-34, 12-34, 13-34, 14-34, 15-34, 9-33, 10-33, 11-33, 12-33, 13-33, 14-33, 15-33, 9-32, 10-32, 11-32, 12-32, 13-32, 14-32, 15-32, 9-31, 10-31, 11-31, 12-31, 13-32, 14-31, 15-31, 15-30, 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24,20-23, 20-22, 20-21, 21-30, 21-29, 21-28, 21-27, 21-26, 21-25, 21-24, 21-23, or 21-22 base pairs. Thus, in one case, to the extent that it becomes processed to a functional duplex, of *e.g.,* 15-30 base pairs, that targets a desired RNA for cleavage, an RNA molecule or complex of RNA molecules having a duplex region greater than 30 base pairs is a dsRNA. Thus, an ordinarily skilled artisan will recognize that in one embodiment, a miRNA is a dsRNA. In another case, a dsRNA is not a naturally occurring miRNA. In another case, an iRNA agent useful to target KHK expression is not generated in the target cell by cleavage of a larger dsRNA.

A dsRNA as described herein can further include one or more single-stranded nucleotide overhangs *e.g*., 1, 2, 3, or 4 nucleotides. dsRNAs having at least one nucleotide overhang can have unexpectedly superior inhibitory properties relative to their blunt-ended counterparts. A nucleotide overhang can comprise or consist of a nucleotide/nucleoside analog, including a deoxynucleotide/nucleoside. The overhang(s) can be on the sense strand, the antisense strand or any combination thereof. Furthermore, the nucleotide(s) of an overhang can be present on the 5'-end, 3'-end or both ends of either an antisense or sense strand of a dsRNA.

A dsRNA can be synthesized by standard methods known in the art as further discussed below, *e.g.,* by use of an automated DNA synthesizer, such as are commercially available from, for example, Biosearch, Applied Biosystems, Inc.

iRNA compounds of the invention may be prepared using a two-step procedure. First, the individual strands of the double-stranded RNA molecule are prepared separately. Then, the component strands are annealed. The individual strands of the siRNA iRNA compound can be prepared using solution-phase or solid-phase organic synthesis or both. Organic synthesis offers the advantage that the oligonucleotide strands comprising unnatural or modified nucleotides can be easily prepared. Single-stranded oligonucleotides of the invention can be prepared using solution-phase or solid-phase organic synthesis or both.

In one aspect, a dsRNA of the disclosure includes at least two nucleotide sequences, a sense sequence and an anti-sense sequence. The sense strand is selected from the group of sequences provided in any one of Tables 3, 4 5, 6, and 7, and the corresponding antisense strand of the sense strand is selected from the group of sequences provided in any one of Tables 3, 4 5, 6, and 7. In this aspect, one of the two sequences is complementary to the other of the two sequences, with one of the sequences being substantially complementary to a sequence of an mRNA generated in the expression of a KHK gene. As such, in this aspect, a dsRNA will include two oligonucleotides, where one oligonucleotide is described as the sense strand in any one of Tables 3, 4 5, 6, and 7, and the second oligonucleotide is described as the corresponding antisense strand of the sense strand in any one of Tables 3, 4 5, 6, and 7. In one case, the substantially complementary sequences of the dsRNA are contained on separate oligonucleotides. In another case, the substantially complementary sequences of the dsRNA are contained on a single oligonucleotide.

The skilled person is well aware that dsRNAs having a duplex structure of between about 20 and 23 base pairs, *e.g.,* 21, base pairs have been hailed as particularly effective in inducing RNA interference (Elbashir et al., EMBO 2001, 20:6877-6888). However, others have found that shorter or longer RNA duplex structures can also be effective (Chu and Rana (2007) RNA 14:1714-1719; Kim et al. (2005) Nat Biotech 23:222-226). In the embodiments described above, by virtue of the nature of the oligonucleotide sequences provided in any one of Tables 3, 4 5, 6, and 7. dsRNAs described herein can include at least one strand of a length of minimally 21 nucleotides. It can be reasonably expected that shorter duplexes having one of the sequences provided in any one of Tables 3, 4 5, 6, and 7 minus only a few nucleotides on one or both ends can be similarly effective as compared to the dsRNAs described above. Hence, dsRNAs having a sequence of at least 15, 16, 17, 18, 19, 20, or more contiguous nucleotides derived from one of the sequences provided in any one of Tables 3, 4 5, 6, and 7 and differing in their ability to inhibit the expression of a KHK gene by not more than about 5, 10, 15, 20, 25, or 30 % inhibition from a dsRNA comprising the full sequence, are contemplated.

In addition, the RNAs provided in any one of Tables 3, 4 5, 6, and 7 identify a site(s) in a KHK transcript that is susceptible to RISC-mediated cleavage. As such, the present invention further features iRNAs that target within one of these sites. As used herein, an iRNA is said to target within a particular site of an RNA transcript if the iRNA promotes cleavage of the transcript anywhere within that particular site. Such an iRNA will generally include at least about 15 contiguous nucleotides from one of the sequences provided in any one of Tables 3, 4 5, 6, and 7 coupled to additional nucleotide sequences taken from the region contiguous to the selected sequence in a KHK gene.

While a target sequence is generally about 15-30 nucleotides in length, there is wide variation in the suitability of particular sequences in this range for directing cleavage of any given target RNA. Various software packages and the guidelines set out herein provide guidance for the identification of optimal target sequences for any given gene target, but an empirical approach can also be taken in which a "window" or "mask" of a given size (as a non-limiting example, 21 nucleotides) is literally or figuratively (including, *e.g*., in silico) placed on the target RNA sequence to identify sequences in the size range that can serve as target sequences. By moving the sequence "window" progressively one nucleotide upstream or downstream of an initial target sequence location, the next potential target sequence can be identified, until the complete set of possible sequences is identified for any given target size selected. This process, coupled with systematic synthesis and testing of the identified sequences (using assays as described herein or as known in the art) to identify those sequences that perform optimally can identify those RNA sequences that, when targeted with an iRNA agent, mediate the best inhibition of target gene expression. Thus, while the sequences identified, for example, in any one of Tables 3, 4 5, 6, and 7 represent effective target sequences, it is contemplated that further optimization of inhibition efficiency can be achieved by progressively "walking the window" one nucleotide upstream or downstream of the given sequences to identify sequences with equal or better inhibition characteristics.

Further, it is contemplated that for any sequence identified, *e.g*., in any one of Tables 3, 4 5, 6, and 7 further optimization could be achieved by systematically either adding or removing nucleotides to generate longer or shorter sequences and testing those sequences generated by walking a window of the longer or shorter size up or down the target RNA from that point. Again, coupling this approach to generating new candidate targets with testing for effectiveness of iRNAs based on those target sequences in an inhibition assay as known in the art and/or as described herein can lead to further improvements in the efficiency of inhibition. Further still, such optimized sequences can be adjusted by, *e.g.,* the introduction of modified nucleotides as described herein or as known in the art, addition or changes in overhang, or other modifications as known in the art and/or discussed herein to further optimize the molecule (e.g., increasing serum stability or circulating half-life, increasing thermal stability, enhancing transmembrane delivery, targeting to a particular location or cell type, increasing interaction with silencing pathway enzymes, increasing release from endosomes) as an expression inhibitor.

An iRNA as described herein can contain one or more mismatches to the target sequence. In one embodiment, an iRNA as described herein contains no more than 3 mismatches. If the antisense strand of the iRNA contains mismatches to a target sequence, it is preferable that the area of mismatch is not located in the center of the region of complementarity. If the antisense strand of the iRNA contains mismatches to the target sequence, it is preferable that the mismatch be restricted to be within the last 5 nucleotides from either the 5'- or 3'-end of the region of complementarity. For example, for a 23 nucleotide iRNA agent the strand which is complementary to a region of a KHK gene, generally does not contain any mismatch within the central 13 nucleotides. The methods described herein or methods known in the art can be used to determine whether an iRNA containing a mismatch to a target sequence is effective in inhibiting the expression of a KHK gene. Consideration of the efficacy of iRNAs with mismatches in inhibiting expression of a KHK gene is important, especially if the particular region of complementarity in a KHK gene is known to have polymorphic sequence variation within the population.

### III. Modified iRNAs of the Invention

In one embodiment, the RNA of the iRNA of the invention *e.g*., a dsRNA, is unmodified, and does not comprise, *e.g*., chemical modifications and/or conjugations known in the art and described herein. In another embodiment, the RNA of an iRNA of the invention, *e.g*., a dsRNA, is chemically modified to enhance stability or other beneficial characteristics. In certain embodiments of the invention, substantially all of the nucleotides of an iRNA of the invention are modified. In other embodiments of the invention, all of the nucleotides of an iRNA of the invention are modified. iRNAs of the invention in which "substantially all of the nucleotides are modified" are largely but not wholly modified and can include not more than 5, 4, 3, 2, or 1 unmodified nucleotides.

The nucleic acids featured in the invention can be synthesized and/or modified by methods well established in the art, such as those described in "Current protocols in nucleic acid chemistry," Beaucage, S.L. et al. (Edrs.), John Wiley & Sons, Inc., New York, NY, USA. Modifications include, for example, end modifications, *e.g*., 5'-end modifications (phosphorylation, conjugation, inverted linkages) or 3'-end modifications (conjugation, DNA nucleotides, inverted linkages, *etc*.); base modifications, *e.g.,* replacement with stabilizing bases, destabilizing bases, or bases that base pair with an expanded repertoire of partners, removal of bases (abasic nucleotides), or conjugated bases; sugar modifications (e.g., at the 2'-position or 4'-position) or replacement of the sugar; and/or backbone modifications, including modification or replacement of the phosphodiester linkages. Specific examples of iRNA compounds described herein include, but are not limited to RNAs containing modified backbones or no natural internucleoside linkages. RNAs having modified backbones include, among others, those that do not have a phosphorus atom in the backbone. For the purposes of this specification, and as sometimes referenced in the art, modified RNAs that do not have a phosphorus atom in their internucleoside backbone can also be considered to be oligonucleosides. In some cases, a modified iRNA will have a phosphorus atom in its internucleoside backbone.

Modified RNA backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5'-linked analogs of these, and those having inverted polarity wherein the adjacent pairs of nucleoside units are linked 3'-5' to 5'-3' or 2'-5' to 5'-2'. Various salts, mixed salts and free acid forms are also included.

Representative U.S. patents that teach the preparation of the above phosphorus-containing linkages include, but are not limited to, U.S. Patent Nos. 3,687,808; 4,469,863; 4,476,301; 5,023,243; 5,177,195; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,316; 5,550,111; 5,563,253; 5,571,799; 5,587,361; 5,625,050; 6,028,188; 6,124,445; 6,160,109; 6,169,170; 6,172,209; 6, 239,265; 6,277,603; 6,326,199; 6,346,614; 6,444,423; 6,531,590; 6,534,639; 6,608,035; 6,683,167; 6,858,715; 6,867,294; 6,878,805; 7,015,315; 7,041,816; 7,273,933; 7,321,029; and US Pat RE39464.

Modified RNA backbones that do not include a phosphorus atom therein have backbones that are formed by short chain alkyl or cycloalkyl internucleoside linkages, mixed heteroatoms and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts.

Representative U.S. patents that teach the preparation of the above oligonucleosides include, but are not limited to, U.S. Patent Nos. 5,034,506; 5,166,315; 5,185,444; 5,214,134; 5,216,141; 5,235,033; 5,64,562; 5,264,564; 5,405,938; 5,434,257; 5,466,677; 5,470,967; 5,489,677; 5,541,307; 5,561,225; 5,596,086; 5,602,240; 5,608,046; 5,610,289; 5,618,704; 5,623,070; 5,663,312; 5,633,360; 5,677,437; and, 5,677,439.

In other cases, suitable RNA mimetics are contemplated for use in iRNAs, in which both the sugar and the internucleoside linkage, *i.e.,* the backbone, of the nucleotide units are replaced with novel groups. The base units are maintained for hybridization with an appropriate nucleic acid target compound. One such oligomeric compound, an RNA mimetic that has been shown to have excellent hybridization properties, is referred to as a peptide nucleic acid (PNA). In PNA compounds, the sugar backbone of an RNA is replaced with an amide containing backbone, in particular an aminoethylglycine backbone. The nucleobases are retained and are bound directly or indirectly to aza nitrogen atoms of the amide portion of the backbone. Representative U.S. patents that teach the preparation of PNA compounds include, but are not limited to, U.S. Patent Nos. 5,539,082; 5,714,331; and 5,719,262. Additional PNA compounds suitable for use in the iRNAs of the invention are described in, for example, in Nielsen et al., Science, 1991, 254, 1497-1500.

Described are RNAs with phosphorothioate backbones and oligonucleosides with heteroatom backbones, and in particular --CH₂--NH--CH₂-, --CH₂--N(CH₃)--O--CH₂-- [known as a methylene (methylimino) or MMI backbone],-CH₂--O--N(CH₃)--CH₂--, --CH₂--N(CH₃)-N(CH₃)--CH₂-- and -N(CH₃)-CH₂-CH₂--[wherein the native phosphodiester backbone is represented as --O--P--O--CH₂--] of the above-referenced U.S. Patent No. 5,489,677, and the amide backbones of the above-referenced U.S. Patent No. 5,602,240. In some cases, the RNAs featured herein have morpholino backbone structures of the above-referenced U.S. Patent No. 5,034,506.

Modified RNAs can also contain one or more substituted sugar moieties. The iRNAs, *e.g*., dsRNAs, featured herein can include one of the following at the 2'-position: OH; F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted C) to C₁₀ alkyl or C₂ to C₁₀ alkenyl and alkynyl. Exemplary suitable modifications include O[(CH₂)ₙO] ₘCH₃, O(CH₂).ₙOCH₃, O(CH₂)ₙNH₂, O(CH₂) ₙCH₃, O(CH₂)ₙONH₂, and O(CH₂)ₙON[(CH₂)_{b}CH₃)]₂, where n and m are from 1 to about 10. In other embodiments, dsRNAs include one of the following at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an iRNA, or a group for improving the pharmacodynamic properties of an iRNA, and other substituents having similar properties. In some embodiments, the modification includes a 2'-methoxyethoxy (2'-O--CH₂CH₂OCH₃, also known as 2'-O-(2-methoxyethyl) or 2'-MOE) (Martin et al., Helv. Chim. Acta, 1995, 78:486-504) *i.e.,* an alkoxy-alkoxy group. Another exemplary modification is 2'-dimethylaminooxyethoxy, *i.e.,* a O(CH₂)₂ON(CH₃)₂ group, also known as 2'-DMAOE, as described in examples herein below, and 2'-dimethylaminoethoxyethoxy (also known in the art as 2'-O-dimethylaminoethoxyethyl or 2'-DMAEOE), *i.e*., 2'-O-CH₂--O--CH₂--N(CH₂)₂.

Other modifications include 2'-methoxy (2'-OCH₃), 2'-aminopropoxy (2'-OCH₂CH₂CH₂NH₂) and 2'-fluoro (2'-F). Similar modifications can also be made at other positions on the RNA of an iRNA, particularly the 3' position of the sugar on the 3' terminal nucleotide or in 2'-5' linked dsRNAs and the 5' position of 5' terminal nucleotide. iRNAs can also have sugar mimetics such as cyclobutyl moieties in place of the pentofuranosyl sugar. Representative U.S. patents that teach the preparation of such modified sugar structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, certain of which are commonly owned with the instant application. An iRNA can also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as deoxy-thymine (dT), 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deazaguanine and 3-deazaadenine. Further nucleobases include those disclosed in U.S. Pat. No. 3,687,808, those disclosed in Modified Nucleosides in Biochemistry, Biotechnology and Medicine, Herdewijn, P. ed. Wiley-VCH, 2008; those disclosed in The Concise Encyclopedia Of Polymer Science And Engineering, pages 858-859, Kroschwitz, J. L, ed. John Wiley & Sons, 1990, these disclosed by Englisch et al., Angewandte Chemie, International Edition, 1991, 30, 613, and those disclosed by Sanghvi, Y S., Chapter 15, dsRNA Research and Applications, pages 289-302, Crooke, S. T. and Lebleu, B., Ed., CRC Press, 1993. Certain of these nucleobases are particularly useful for increasing the binding affinity of the oligomeric compounds featured in the invention. These include 5-substituted pyrimidines, 6-azapyrimidines and N-2, N-6 and 0-6 substituted purines, including 2-aminopropyladenine, 5-propynyluracil and 5-propynylcytosine. 5-methylcytosine substitutions have been shown to increase nucleic acid duplex stability by 0.6-1.2°C (Sanghvi, Y. S., Crooke, S. T. and Lebleu, B., Eds., dsRNA Research and Applications, CRC Press, Boca Raton, 1993, pp. 276-278) and are exemplary base substitutions, even more particularly when combined with 2'-O-methoxyethyl sugar modifications.

Representative U.S. patents that teach the preparation of certain of the above noted modified nucleobases as well as other modified nucleobases include, but are not limited to, the above noted U.S. Patent Nos. 3,687,808, 4,845,205; 5,130,30; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,681,941; 5,750,692; 6,015,886; 6,147,200; 6,166,197; 6,222,025; 6,235,887; 6,380,368; 6,528,64.0; 6,639,062; 6,617,438; 7,045,610; 7,427,672; and 7,495,088.

The RNA of an iRNA can also be modified to include one or more bicyclic sugar moities. A "bicyclic sugar" is a furanosyl ring modified by the bridging of two atoms. A "bicyclic nucleoside" ("BNA") is a nucleoside having a sugar moiety comprising a bridge connecting two carbon atoms of the sugar ring, thereby forming a bicyclic ring system. In certain embodiments, the bridge connects the 4' -carbon and the 2' -carbon of the sugar ring. Thus, in some embodiments an agent of the invention may include one or more locked nucleic acids (LNA). A locked nucleic acid is a nucleotide having a modified ribose moiety in which the ribose moiety comprises an extra bridge connecting the 2' and 4' carbons. In other words, an LNA is a nucleotide comprising a bicyclic sugar moiety comprising a 4-CH2-O-2' bridge. This structure effectively "locks" the ribose in the 3'-endo structural conformation. The addition of locked nucleic acids to siRNAs has been shown to increase siRNA stability in serum, and to reduce off-target effects (Elmen, J. et al, (2005) Nucleic Acids Research 33(1):439-447; Mook, OR. et al., (2007) Mol Canc Ther 6(3):833-843; Grunweller, A. et al., (2003) Nucleic Acids Research 31(12):3185-3193). Examples of bicyclic nucleosides for use in the polynucleotides of the invention include without limitation nucleosides comprising a bridge between the 4' and the 2' ribosyl ring atoms. In certain embodiments, the antisense polynucleotide agents of the invention include one or more bicyclic nucleosides comprising a 4' to 2' bridge. Examples of such 4' to 2' bridged bicyclic nucleosides, include but are not limited to 4' -(CH2)-O-2' (LNA); 4' -(CH2)-S-2' ; 4' -(CH2)2-O-2' (ENA); 4' -CH(CH3)-O-2' (also referred to as "constrained ethyl" or "cEt") and 4' -CH(CH2OCH3)-O-2' (and analogs thereof; see, *e.g.,* U.S. Pat. No. 7,399,845); 4' -C(CH3)(CH3)-O-2' (and analogs thereof; see *e.g.,* US Patent No. 8,278,283); 4' -CH2-N(OCH3)-2' (and analogs thereof; see *e.g.,* US Patent No. 8,278,425); 4' -CH2-O-N(CH3)-2' (see, e.g.,U.S. Patent Publication No. 2004/0171570); 4' -CH2-N(R)-O-2' , wherein R is H, C1-C12 alkyl, or a protecting group (see, *e.g.,* U.S. Pat. No. 7,427,672); 4' -CH2-C(H)(CH3)-2' (see, *e.g.,* Chattopadhyaya et al., J. Org. Chem., 2009, 74, 118-134); and 4' -CH2-C(=CH2)-2' (and analogs thereof; see, *e.g*., US Patent No. 8,278,426). Additional representative U.S. Patents and US Patent Publications that teach the preparation of locked nucleic acid nucleotides include, but are not limited to, the following: U.S. Patent Nos. 6,268,490; 6,525,191; 6,670,461; 6,770,748; 6,794,499; 6,998,484; 7,053,207; 7,034,133;7,084,125; 7,399,845; 7,427,672; 7,569,686; 7,741,457; 8,022,193; 8,030,467; 8,278,425; 8,278,426; 8,278,283; US 2008/0039618; and US 2009/0012281.

Any of the foregoing bicyclic nucleosides can be prepared having one or more stereochemical sugar configurations including for example α-L-ribofuranose and β-D-ribofuranose (see WO 99/14226).

The RNA of an iRNA can also be modified to include one or more constrained ethyl nucleotides. As used herein, a "constrained ethyl nucleotide" or "cEt" is a locked nucleic acid comprising a bicyclic sugar moiety comprising a 4'-CH(CH3)-0-2' bridge. In one embodiment, a constrained ethyl nucleotide is in the S conformation referred to herein as "S-cEt."

An iRNA may also include one or more "conformationally restricted nucleotides" ("CRN"). CRN are nucleotide analogs with a linker connecting the C2'and C4' carbons of ribose or the C3 and -C5' carbons of ribose . CRN lock the ribose ring into a stable conformation and increase the hybridization affinity to mRNA. The linker is of sufficient length to place the oxygen in an optimal position for stability and affinity resulting in less ribose ring puckering.

Representative publications that teach the preparation of certain of the above noted CRN include, but are not limited to, US Patent Publication No. 2013/0190383; and PCT publication WO 2013/036868.

One or more of the nucleotides of an iRNA of the invention may also include a hydroxymethyl substituted nucleotide. A "hydroxymethyl substituted nucleotide" is an acyclic 2'-3'-seco-nucleotide, also referred to as an "unlocked nucleic acid" ("UNA") modification

Representative U.S. publications that teach the preparation of UNA include, but are not limited to, US Patent No. 8,314,227; and US Patent Publication Nos. 2013/0096289; 2013/0011922; and 2011/0313020Potentially stabilizing modifications to the ends of RNA molecules can include N- (acetylaminocaproyl)-4-hydroxyprolinol (Hyp-C6-NHAc), N-(caproyl-4-hydroxyprolinol (Hyp-C6), N-(acetyl-4-hydroxyprolinol (Hyp-NHAc), thymidine-2'-0-deoxythymidine (ether), N-(aminocaproyl)-4-hydroxyprolinol (Hyp-C6-amino), 2-docosanoyl-uridine-3"- phosphate, inverted base dT(idT) and others. Disclosure of this modification can be found in PCT Publication No. WO 2011/005861.

### A. Modified iRNAs Comprising Motifs of the Invention

The described double-stranded iRNA agents include agents with chemical modifications as disclosed, for example, in U.S. Provisional Application No. 61/561,710, filed on November 18, 2011, or in PCT/US2012/065691, filed on November 16, 2012.

As shown herein and in Provisional Application No. 61/561,710 or in PCT Application No. PCT/US2012/065691, a superior result may be obtained by introducing one or more motifs of three identical modifications on three consecutive nucleotides into a sense strand and/or antisense strand of an iRNA agent, particularly at or near the cleavage site. In some embodiments, the sense strand and antisense strand of the iRNA agent may otherwise be completely modified. The introduction of these motifs interrupts the modification pattern, if present, of the sense and/or antisense strand. The iRNA agent may be optionally conjugated with a GalNAc derivative ligand, for instance on the sense strand. The resulting iRNA agents present superior gene silencing activity.

More specifically, it has been surprisingly discovered that when the sense strand and antisense strand of the double-stranded iRNA agent are completely modified to have one or more motifs of three identical modifications on three consecutive nucleotides at or near the cleavage site of at least one strand of an iRNA agent, the gene silencing acitivity of the iRNA agent was superiorly enhanced.

Accordingly, the invention provides double-stranded iRNA agents capable of inhibiting the expression of a target gene *(i.e.,* KHK gene) *in vivo.* The iRNA agent comprises a sense strand and an antisense strand. Each strand of the iRNA agent may range from 12-30 nucleotides in length. For example, each strand may be between 14-30 nucleotides in length, 17-30 nucleotides in length, 25-30 nucleotides in length, 27-30 nucleotides in length, 17-23 nucleotides in length, 17-21 nucleotides in length, 17-19 nucleotides in length, 19-25 nucleotides in length, 19-23 nucleotides in length, 19-21 nucleotides in length, 21-25 nucleotides in length, or 21-23 nucleotides in length.

The sense strand and antisense strand typically form a duplex double stranded RNA ("dsRNA"), also referred to herein as an"iRNA agent" or " iRNA agent." The duplex region of an iRNA agent may be 12-30 nucleotide pairs in length. For example, the duplex region can be between 14-30 nucleotide pairs in length, 17-30 nucleotide pairs in length, 27-30 nucleotide pairs in length, 17 - 23 nucleotide pairs in length, 17-21 nucleotide pairs in length, 17-19 nucleotide pairs in length, 19-25 nucleotide pairs in length, 19-23 nucleotide pairs in length, 19- 21 nucleotide pairs in length, 21-25 nucleotide pairs in length, or 21-23 nucleotide pairs in length. In another example, the duplex region is selected from 15, 16, 17, 18, 19, 20, 21, 22, 23, 24, 25, 26, and 27 nucleotides in length.

In one case, the iRNA agent may contain one or more overhang regions and/or capping groups at the 3'-end, 5'-end, or both ends of one or both strands. The overhang can be 1-6 nucleotides in length, for instance 2-6 nucleotides in length, 1-5 nucleotides in length, 2-5 nucleotides in length, 1-4 nucleotides in length,-2-4 nucleotides in length, 1-3 nucleotides in length, 2-3 nucleotides in length, or 1-2 nucleotides in length. The overhangs can be the result of one strand being longer than the other, or the result of two strands of the same length being staggered. The overhang can form a mismatch with the target mRNA or it can be complementary to the gene sequences being targeted or can be another sequence. The first and second strands can also be joined, *e.g.,* by additional bases to form a hairpin, or by other non-base linkers.

In one case, the nucleotides in the overhang region of the iRNA agent can each independently be a modified or unmodified nucleotide including, but not limited to 2'-sugar modified, such as, 2-F, 2'-Omethyl, thymidine (T), 2'-O-methoxyethyl-5-methyluridine (Teo), 2'-O-methoxyethyladenosine (Aeo), 2-O-methoxyethyl-5-methylcytidine (m5Ceo), and any combinations thereof. For example, TT can be an overhang sequence for either end on either strand. The overhang can form a mismatch with the target mRNA or it can be complementary to the gene sequences being targeted or can be another sequence.

The 5'- or 3'- overhangs at the sense strand, antisense strand or both strands of the iRNA agent may be phosphorylated. In some cases, the overhang region(s) contains two nucleotides having a phosphorothioate between the two nucleotides, where the two nucleotides can be the same or different. In one case, the overhang is present at the 3'-end of the sense strand, antisense strand, or both strands. In one case, this 3'-overhang is present in the antisense strand. In one embodiment, this 3'-overhang is present in the sense strand.

The iRNA agent may contain only a single overhang, which can strengthen the interference activity of the iRNA, without affecting its overall stability. For example, the single-stranded overhang may be located at the 3'-terminal end of the sense strand or, alternatively, at the 3'-terminal end of the antisense strand. The iRNA may also have a blunt end, located at the 5'-end of the antisense strand (or the 3'-end of the sense strand) or *vice versa.* Generally, the antisense strand of the iRNA has a nucleotide overhang at the 3'-end, and the 5'-end is blunt. While not wishing to be bound by theory, the asymmetric blunt end at the 5'-end of the antisense strand and 3'-end overhang of the antisense strand favor the guide strand loading into RISC process.

In one case, the iRNA agent is a double ended bluntmer of 19 nucleotides in length, wherein the sense strand contains at least one motif of three 2'-F modifications on three consecutive nucleotides at positions 7, 8, 9 from the 5'end. The antisense strand contains at least one motif of three 2'-O-methyl modifications on three consecutive nucleotides at positions 11, 12, 13 from the 5'end.

In another case, the iRNA agent is a double ended bluntmer of 20 nucleotides in length, wherein the sense strand contains at least one motif of three 2'-F modifications on three consecutive nucleotides at positions 8, 9, 10 from the 5'end. The antisense strand contains at least one motif of three 2'-O-methyl modifications on three consecutive nucleotides at positions 11, 12, 13 from the 5'end.

In yet another case, the iRNA agent is a double ended bluntmer of 21 nucleotides in length, wherein the sense strand contains at least one motif of three 2'-F modifications on three consecutive nucleotides at positions 9, 10, 11 from the 5'end. The antisense strand contains at least one motif of three 2'-O-methyl modifications on three consecutive nucleotides at positions 11, 12, 13 from the 5'end.

In one case, the iRNA agent comprises a 21 nucleotide sense strand and a 23 nucleotide antisense strand, wherein the sense strand contains at least one motif of three 2'-F modifications on three consecutive nucleotides at positions 9, 10, 11 from the 5'end; the antisense strand contains at least one motif of three 2'-O-methyl modifications on three consecutive nucleotides at positions 11, 12, 13 from the 5'end, wherein one end of the iRNA agent is blunt, while the other end comprises a 2 nucleotide overhang. Preferably, the 2 nucleotide overhang is at the 3'-end of the antisense strand. When the 2 nucleotide overhang is at the 3'-end of the antisense strand, there may be two phosphorothioate internucleotide linkages between the terminal three nucleotides, wherein two of the three nucleotides are the overhang nucleotides, and the third nucleotide is a paired nucleotide next to the overhang nucleotide. In one case, the iRNA agent additionally has two phosphorothioate internucleotide linkages between the terminal three nucleotides at both the 5'-end of the sense strand and at the 5'-end of the antisense strand. In one case, every nucleotide in the sense strand and the antisense strand of the iRNA agent, including the nucleotides that are part of the motifs are modified nucleotides. In one case each residue is independently modified with a 2'-O-methyl or 3'-fluoro, *e.g.,* in an alternating motif. Optionally, the iRNA agent further comprises a ligand (preferably GalNAc₃).

In one case, the iRNA agent comprises a sense and an antisense strand, wherein the sense strand is 25-30 nucleotide residues in length, wherein starting from the 5' terminal nucleotide (position 1) positions 1 to 23 of the first strand comprise at least 8 ribonucleotides; the antisense strand is 36-66 nucleotide residues in length and, starting from the 3' terminal nucleotide, comprises at least 8 ribonucleotides in the positions paired with positions 1- 23 of sense strand to form a duplex; wherein at least the 3 ' terminal nucleotide of antisense strand is unpaired with sense strand, and up to 6 consecutive 3' terminal nucleotides are unpaired with sense strand, thereby forming a 3' single stranded overhang of 1-6 nucleotides; wherein the 5' terminus of antisense strand comprises from 10-30 consecutive nucleotides which are unpaired with sense strand, thereby forming a 10-30 nucleotide single stranded 5' overhang; wherein at least the sense strand 5' terminal and 3' terminal nucleotides are base paired with nucleotides of antisense strand when sense and antisense strands are aligned for maximum complementarity, thereby forming a substantially duplexed region between sense and antisense strands; and antisense strand is sufficiently complementary to a target RNA along at least 19 ribonucleotides of antisense strand length to reduce target gene expression when the double stranded nucleic acid is introduced into a mammalian cell; and wherein the sense strand contains at least one motif of three 2'-F modifications on three consecutive nucleotides, where at least one of the motifs occurs at or near the cleavage site. The antisense strand contains at least one motif of three 2'-O-methyl modifications on three consecutive nucleotides at or near the cleavage site.

In one case, the iRNA agent comprises sense and antisense strands, wherein the iRNA agent comprises a first strand having a length which is at least 25 and at most 29 nucleotides and a second strand having a length which is at most 30 nucleotides with at least one motif of three 2'-O-methyl modifications on three consecutive nucleotides at position 11, 12, 13 from the 5' end; wherein the 3' end of the first strand and the 5' end of the second strand form a blunt end and the second strand is 1-4 nucleotides longer at its 3' end than the first strand, wherein the duplex region region which is at least 25 nucleotides in length, and the second strand is sufficiently complemenatary to a target mRNA along at least 19 nucleotide of the second strand length to reduce target gene expression when the iRNA agent is introduced into a mammalian cell, and wherein dicer cleavage of the iRNA agent preferentially results in an siRNA comprising the 3' end of the second strand, thereby reducing expression of the target gene in the mammal. Optionally, the iRNA agent further comprises a ligand.

In one case, the sense strand of the iRNA agent contains at least one motif of three identical modifications on three consecutive nucleotides, where one of the motifs occurs at the cleavage site in the sense strand.

In one case, the antisense strand of the iRNA agent can also contain at least one motif of three identical modifications on three consecutive nucleotides, where one of the motifs occurs at or near the cleavage site in the antisense strand

For an iRNA agent having a duplex region of 17-23 nucleotide in length, the cleavage site of the antisense strand is typically around the 10, 11 and 12 positions from the 5'-end. Thus the motifs of three identical modifications may occur at the 9, 10, 11 positions; 10, 11, 12 positions; 11, 12, 13 positions; 12, 13, 14 positions; or 13, 14, 15 positions of the antisense strand, the count starting from the 1^{st} nucleotide from the 5'-end of the antisense strand, or, the count starting from the 1^{st} paired nucleotide within the duplex region from the 5'- end of the antisense strand. The cleavage site in the antisense strand may also change according to the length of the duplex region of the iRNA from the 5'-end.

The sense strand of the iRNA agent may contain at least one motif of three identical modifications on three consecutive nucleotides at the cleavage site of the strand; and the antisense strand may have at least one motif of three identical modifications on three consecutive nucleotides at or near the cleavage site of the strand. When the sense strand and the antisense strand form a dsRNA duplex, the sense strand and the antisense strand can be so aligned that one motif of the three nucleotides on the sense strand and one motif of the three nucleotides on the antisense strand have at least one nucleotide overlap, *i.e.,* at least one of the three nucleotides of the motif in the sense strand forms a base pair with at least one of the three nucleotides of the motif in the antisense strand. Alternatively, at least two nucleotides may overlap, or all three nucleotides may overlap.

In one case, the sense strand of the iRNA agent may contain more than one motif of three identical modifications on three consecutive nucleotides. The first motif may occur at or near the cleavage site of the strand and the other motifs may be a wing modification. The term "wing modification" herein refers to a motif occurring at another portion of the strand that is separated from the motif at or near the cleavage site of the same strand. The wing modification is either adjacent to the first motif or is separated by at least one or more nucleotides. When the motifs are immediately adjacent to each other then the chemistry of the motifs are distinct from each other and when the motifs are separated by one or more nucleotide than the chemistries can be the same or different. Two or more wing modifications may be present. For instance, when two wing modifications are present, each wing modification may occur at one end relative to the first motif which is at or near cleavage site or on either side of the lead motif.

Like the sense strand, the antisense strand of the iRNA agent may contain more than one motifs of three identical modifications on three consecutive nucleotides, with at least one of the motifs occurring at or near the cleavage site of the strand. This antisense strand may also contain one or more wing modifications in an alignment similar to the wing modifications that may be present on the sense strand.

In one case, the wing modification on the sense strand or antisense strand of the iRNA agent typically does not include the first one or two terminal nucleotides at the 3'-end, 5'-end or both ends of the strand.

In another case, the wing modification on the sense strand or antisense strand of the iRNA agent typically does not include the first one or two paired nucleotides within the duplex region at the 3'-end, 5'-end or both ends of the strand.

When the sense strand and the antisense strand of the iRNA agent each contain at least one wing modification, the wing modifications may fall on the same end of the duplex region, and have an overlap of one, two or three nucleotides.

When the sense strand and the antisense strand of the iRNA agent each contain at least two wing modifications, the sense strand and the antisense strand can be so aligned that two modifications each from one strand fall on one end of the duplex region, having an overlap of one, two or three nucleotides; two modifications each from one strand fall on the other end of the duplex region, having an overlap of one, two or three nucleotides; two modifications one strand fall on each side of the lead motif, having an overlap of one, two or three nucleotides in the duplex region.

In one case, every nucleotide in the sense strand and antisense strand of the iRNA agent, including the nucleotides that are part of the motifs, may be modified. Each nucleotide may be modified with the same or different modification which can include one or more alteration of one or both of the non-linking phosphate oxygens and/or of one or more of the linking phosphate oxygens; alteration of a constituent of the ribose sugar, *e.g.,* of the 2' hydroxyl on the ribose sugar; wholesale replacement of the phosphate moiety with "dephospho" linkers; modification or replacement of a naturally occurring base; and replacement or modification of the ribose-phosphate backbone.

As nucleic acids are polymers of subunits, many of the modifications occur at a position which is repeated within a nucleic acid, *e.g.,* a modification of a base, or a phosphate moiety, or a non-linking O of a phosphate moiety. In some cases the modification will occur at all of the subject positions in the nucleic acid but in many cases it will not. By way of example, a modification may only occur at a 3' or 5' terminal position, may only occur in a terminal region, *e.g.,* at a position on a terminal nucleotide or in the last 2, 3, 4, 5, or 10 nucleotides of a strand. A modification may occur in a double strand region, a single strand region, or in both. A modification may occur only in the double strand region of a RNA or may only occur in a single strand region of a RNA. For example, a phosphorothioate modification at a non-linking O position may only occur at one or both termini, may only occur in a terminal region, *e.g.,* at a position on a terminal nucleotide or in the last 2, 3, 4, 5, or 10 nucleotides of a strand, or may occur in double strand and single strand regions, particularly at termini. The 5' end or ends can be phosphorylated.

It may be possible, *e.g.,* to enhance stability, to include particular bases in overhangs, or to include modified nucleotides or nucleotide surrogates, in single strand overhangs, *e.g*., in a 5' or 3' overhang, or in both. For example, it can be desirable to include purine nucleotides in overhangs. In some embodiments all or some of the bases in a 3' or 5' overhang may be modified, *e.g.,* with a modification described herein. Modifications can include, *e.g.,* the use of modifications at the 2' position of the ribose sugar with modifications that are known in the art, *e.g.,* the use of deoxyribonucleotides, 2'-deoxy-2'-fluoro (2'-F) or 2'-O-methyl modified instead of the ribosugar of the nucleobase, and modifications in the phosphate group, *e.g*., phosphorothioate modifications. Overhangs need not be homologous with the target sequence.

In one case, each residue of the sense strand and antisense strand is independently modified with LNA, CRN, cET, UNA, HNA, CeNA, 2'-methoxyethyl, 2'- O-methyl, 2'-O-allyl, 2'-C- allyl, 2'-deoxy, 2'-hydroxyl, or 2'-fluoro. The strands can contain more than one modification. In one case, each residue of the sense strand and antisense strand is independently modified with 2'- O-methyl or 2'-fluoro.

At least two different modifications are typically present on the sense strand and antisense strand. Those two modifications may be the 2'- O-methyl or 2'-fluoro modifications, or others.

In one case, the Nₐ and/or N_{b} comprise modifications of an alternating pattern. The term "alternating motif' as used herein refers to a motif having one or more modifications, each modification occurring on alternating nucleotides of one strand. The alternating nucleotide may refer to one per every other nucleotide or one per every three nucleotides, or a similar pattern. For example, if A, B and C each represent one type of modification to the nucleotide, the alternating motif can be "ABABABABABAB...," "AABBAABBAABB...," "AABAABAABAAB...," "AAABAAABAAAB...," "AAABBBAAABBB...," or "ABCABCABCABC...," etc.

The type of modifications contained in the alternating motif may be the same or different. For example, if A, B, C, D each represent one type of modification on the nucleotide, the alternating pattern, *i.e.,* modifications on every other nucleotide, may be the same, but each of the sense strand or antisense strand can be selected from several possibilities of modifications within the alternating motif such as "ABABAB...", "ACACAC..." "BDBDBD..." or "CDCDCD...," etc.

In one case, the iRNA agent of the invention comprises the modification pattern for the alternating motif on the sense strand relative to the modification pattern for the alternating motif on the antisense strand is shifted. The shift may be such that the modified group of nucleotides of the sense strand corresponds to a differently modified group of nucleotides of the antisense strand and *vice versa.* For example, the sense strand when paired with the antisense strand in the dsRNA duplex, the alternating motif in the sense strand may start with "ABABAB" from 5'-3' of the strand and the alternating motif in the antisense strand may start with "BABABA" from 5'-3'of the strand within the duplex region. As another example, the alternating motif in the sense strand may start with "AABBAABB" from 5'-3' of the strand and the alternating motif in the antisenese strand may start with "BBAABBAA" from 5'-3' of the strand within the duplex region, so that there is a complete or partial shift of the modification patterns between the sense strand and the antisense strand.

In one case, the iRNA agent comprises the pattern of the alternating motif of 2'-O-methyl modification and 2'-F modification on the sense strand initially has a shift relative to the pattern of the alternating motif of 2'-O-methyl modification and 2'-F modification on the antisense strand initially, *i.e.,* the 2'-O-methyl modified nucleotide on the sense strand base pairs with a 2'-F modified nucleotide on the antisense strand and vice versa. The 1 position of the sense strand may start with the 2'-F modification, and the 1 position of the antisense strand may start with the 2'- O-methyl modification.

The introduction of one or more motifs of three identical modifications on three consecutive nucleotides to the sense strand and/or antisense strand interrupts the initial modification pattern present in the sense strand and/or antisense strand. This interruption of the modification pattern of the sense and/or antisense strand by introducing one or more motifs of three identical modifications on three consecutive nucleotides to the sense and/or antisense strand surprisingly enhances the gene silencing acitivty to the target gene.

In one case, when the motif of three identical modifications on three consecutive nucleotides is introduced to any of the strands, the modification of the nucleotide next to the motif is a different modification than the modification of the motif. For example, the portion of the sequence containing the motif is "...NₐYYYN_{b}...," where "Y" represents the modification of the motif of three identical modifications on three consecutive nucleotide, and "Nₐ" and "N_{b}" represent a modification to the nucleotide next to the motif "YYY" that is different than the modification of Y, and where Nₐ and N_{b} can be the same or different modifications. Altnernatively, Nₐ and/or N_{b} may be present or absent when there is a wing modification present.

The iRNA agent may further comprise at least one phosphorothioate or methylphosphonate internucleotide linkage. The phosphorothioate or methylphosphonate internucleotide linkage modification may occur on any nucleotide of the sense strand or antisense strand or both strands in any position of the strand. For instance, the internucleotide linkage modification may occur on every nucleotide on the sense strand and/or antisense strand; each internucleotide linkage modification may occur in an alternating pattern on the sense strand and/or antisense strand; or the sense strand or antisense strand may contain both internucleotide linkage modifications in an alternating pattern. The alternating pattern of the internucleotide linkage modification on the sense strand may be the same or different from the antisense strand, and the alternating pattern of the internucleotide linkage modification on the sense strand may have a shift relative to the alternating pattern of the internucleotide linkage modification on the antisense strand.

In one case, the iRNA comprises a phosphorothioate or methylphosphonate internucleotide linkage modification in the overhang region. For example, the overhang region may contain two nucleotides having a phosphorothioate or methylphosphonate internucleotide linkage between the two nucleotides. Internucleotide linkage modifications also may be made to link the overhang nucleotides with the terminal paired nucleotides within the duplex region. For example, at least 2, 3, 4, or all the overhang nucleotides may be linked through phosphorothioate or methylphosphonate internucleotide linkage, and optionally, there may be additional phosphorothioate or methylphosphonate internucleotide linkages linking the overhang nucleotide with a paired nucleotide that is next to the overhang nucleotide. For instance, there may be at least two phosphorothioate internucleotide linkages between the terminal three nucleotides, in which two of the three nucleotides are overhang nucleotides, and the third is a paired nucleotide next to the overhang nucleotide. These terminal three nucleotides may be at the 3'-end of the antisense strand, the 3'-end of the sense strand, the 5'-end of the antisense strand, and/or the 5'end of the antisense strand.

In one case, the 2 nucleotide overhang is at the 3'-end of the antisense strand, and there are two phosphorothioate internucleotide linkages between the terminal three nucleotides, wherein two of the three nucleotides are the overhang nucleotides, and the third nucleotide is a paired nucleotide next to the overhang nucleotide. Optionally, the iRNA agent may additionally have two phosphorothioate internucleotide linkages between the terminal three nucleotides at both the 5'-end of the sense strand and at the 5'-end of the antisense strand.

In one case, a double-standed RNAi agent comprises 6-8 phosphorothioate internucleotide linkages. In one embodiment, the antisense strand comprises two phosphorothioate internucleotide linkages at the 5'-terminus and two phosphorothioate internucleotide linkages at the 3'-terminus, and the sense strand comprises at least two phosphorothioate internucleotide linkages at either the 5'-terminus or the 3'-terminus.

In one case, the iRNA agent comprises mismatch(es) with the target, within the duplex, or combinations thereof. The mistmatch may occur in the overhang region or the duplex region. The base pair may be ranked on the basis of their propensity to promote dissociation or melting *(e.g.,* on the free energy of association or dissociation of a particular pairing, the simplest approach is to examine the pairs on an individual pair basis, though next neighbor or similar analysis can also be used). In terms of promoting dissociation: A:U is preferred over G:C; G:U is preferred over G:C; and I:C is preferred over G:C (I=inosine). Mismatches, *e.g*., non-canonical or other than canonical pairings (as described elsewhere herein) are preferred over canonical (A:T, A:U, G:C) pairings; and pairings which include a universal base are preferred over canonical pairings.

In one case, the iRNA agent comprises at least one of the first 1, 2, 3, 4, or 5 base pairs within the duplex regions from the 5'- end of the antisense strand independently selected from the group of: A:U, G:U, I:C, and mismatched pairs, *e.g*., non-canonical or other than canonical pairings or pairings which include a universal base, to promote the dissociation of the antisense strand at the 5'-end of the duplex.

In one case, the nucleotide at the 1 position within the duplex region from the 5'-end in the antisense strand is selected from the group consisting of A, dA, dU, U, and dT. Alternatively, at least one of the first 1, 2 or 3 base pair within the duplex region from the 5'- end of the antisense strand is an AU base pair. For example, the first base pair within the duplex region from the 5'- end of the antisense strand is an AU base pair.

In one case, the sense strand sequence may be represented by formula (I):

5' nₚ-Nₐ-(X X X)ᵢ-N_{b}-Y Y Y -N_{b}-(Z Z Z)ⱼ-Nₐ-n_{q} 3' (I)

wherein:
i and j are each independently 0 or 1;
p and q are each independently 0-6;
each Nₐ independently represents an oligonucleotide sequence comprising 0-25 modified nucleotides, each sequence comprising at least two differently modified nucleotides;
each N_{b} independently represents an oligonucleotide sequence comprising 0-10 modified nucleotides;
each nₚ and n_{q} independently represent an overhang nucleotide;
wherein Nb and Y do not have the same modification; and
XXX, YYY and ZZZ each independently represent one motif of three identical modifications on three consecutive nucleotides. Preferably YYY is all 2'-F modified nucleotides.

In one case, the Nₐ and/or N_{b} comprise modifications of alternating pattern.

In one case, the YYY motif occurs at or near the cleavage site of the sense strand. For example, when the iRNA agent has a duplex region of 17-23 nucleotides in length, the YYY motif can occur at or the vicinity of the cleavage site (*e.g*.: can occur at positions 6, 7, 8, 7, 8, 9, 8, 9, 10, 9, 10, 11, 10, 11,12 or 11, 12, 13) of - the sense strand, the count starting from the 1^{st} nucleotide, from the 5'-end; or optionally, the count starting at the 1^{st} paired nucleotide within the duplex region, from the 5'- end.

In one case, i is 1 and j is 0, or i is 0 and j is 1, or both i and j are 1. The sense strand can therefore be represented by the following formulas:

5' nₚ-Nₐ-YYY-N_{b}-ZZZ-Nₐ-n_{q} 3' (Ib);

5' nₚ-Nₐ-XXX-N_{b}-YYY-Nₐ₋n_{q} 3' (Ic);

or

5' nₚ-Nₐ₋XXX-N_{b}-YYY-N_{b}-ZZZ-Nₐ-n_{q} 3' (Id).

When the sense strand is represented by formula (Ib), N_{b} represents an oligonucleotide sequence comprising 0-10, 0-7, 0-5, 0-4, 0-2 or 0 modified nucleotides. Each Nₐ independently can represent an oligonucleotide sequence comprising 2-20, 2-15, or 2-10 modified nucleotides.

When the sense strand is represented as formula (Ic), N_{b} represents an oligonucleotide sequence comprising 0-10, 0-7, 0-10, 0-7, 0-5, 0-4, 0-2 or 0 modified nucleotides. Each Nₐ can independently represent an oligonucleotide sequence comprising 2-20, 2-15, or 2-10 modified nucleotides.

When the sense strand is represented as formula (Id), each N_{b} independently represents an oligonucleotide sequence comprising 0-10, 0-7, 0-5, 0-4, 0-2 or 0 modified nucleotides. Preferably, N_{b} is 0, 1, 2, 3, 4, 5 or 6 Each Nₐ can independently represent an oligonucleotide sequence comprising 2-20, 2-15, or 2-10 modified nucleotides.

Each of X, Y and Z may be the same or different from each other.

In other case, i is 0 and j is 0, and the sense strand may be represented by the formula:

5' nₚ-Nₐ-YYY- Nₐ-n_{q} 3' (Ia).

When the sense strand is represented by formula (Ia), each Nₐ independently can represent an oligonucleotide sequence comprising 2-20, 2-15, or 2-10 modified nucleotides.

In one case, the antisense strand sequence of the iRNA may be represented by formula (II):

5' n_{q'}-Nₐ'-(Z'Z'Z')ₖ-N_{b}'-Y'Y'Y'-N_{b}'-(X'X'X')ₗ-N'ₐ-nₚ' 3' (II)

wherein:
k and 1 are each independently 0 or 1;
p' and q' are each independently 0-6;
each Nₐ' independently represents an oligonucleotide sequence comprising 0-25 modified nucleotides, each sequence comprising at least two differently modified nucleotides;
each N_{b}' independently represents an oligonucleotide sequence comprising 0-10 modified nucleotides;
each nₚ' and n_{q}' independently represent an overhang nucleotide;
wherein N_{b}' and Y' do not have the same modification; and
X'X'X', Y'Y'Y' and Z'Z'Z' each independently represent one motif of three identical modifications on three consecutive nucleotides.

In one case, the Nₐ' and/or N_{b}' comprise modifications of alternating pattern.

The Y'Y'Y' motif occurs at or near the cleavage site of the antisense strand. For example, when the iRNA agent has a duplex region of 17-23nucleotidein length, the Y'Y'Y' motif can occur at positions 9, 10, 1 1; 10, 11, 12; 11, 12, 13; 12, 13, 14 ; or 13, 14, 15 of the antisense strand, with the count starting from the 1^{st} nucleotide, from the 5'-end; or optionally, the count starting at the 1^{st} paired nucleotide within the duplex region, from the 5'- end. Preferably, the Y'Y'Y' motif occurs at positions 11, 12, 13.

In one case, Y'Y'Y' motif is all 2'-OMe modified nucleotides.

In one case, k is 1 and 1 is 0, or k is 0 and 1 is 1, or both k and 1 are 1.

The antisense strand can therefore be represented by the following formulas:

5' n_{q'}-Nₐ'-Z'Z'Z'-N_{b}'-Y'Y'Y'-Nₐ'-n_{p'} 3' (IIb);

5' n_{q}-Nₐ'-Y'Y'Y'-N_{b}'-X'X'X'-n_{p<} 3' (IIc);

or

5' n_{q'}-Nₐ'- Z'Z'Z'-N_{b}'-Y'Y'Y'-N_{b}'- X'X'X'-Nₐ'-nₚ- 3' (IId).

When the antisense strand is represented by formula (IIb), N_{b}' represents an oligonucleotide sequence comprising 0-10, 0-7, 0-10, 0-7, 0-5, 0-4, 0-2 or 0 modified nucleotides. Each Nₐ' independently represents an oligonucleotide sequence comprising 2-20, 2-15, or 2-10 modified nucleotides.

When the antisense strand is represented as formula (IIc), N_{b}' represents an oligonucleotide sequence comprising 0-10, 0-7, 0-10, 0-7, 0-5, 0-4, 0-2 or 0 modified nucleotides. Each Nₐ' independently represents an oligonucleotide sequence comprising 2-20, 2-15, or 2-10 modified nucleotides.

When the antisense strand is represented as formula (IId), each N_{b}' independently represents an oligonucleotide sequence comprising 0-10, 0-7, 0-10, 0-7, 0-5, 0-4, 0-2 or 0 modified nucleotides. Each Nₐ' independently represents an oligonucleotide sequence comprising 2-20, 2-15, or 2-10 modified nucleotides. Preferably, N_{b} is 0, 1, 2, 3, 4, 5 or 6. In other embodiments, k is 0 and 1 is 0 and the antisense strand may be represented by the formula:

5' n_{p'}-Nₐ'-Y'Y'Y'- N_{a'}-n_{q'}. 3' (Ia).

When the antisense strand is represented as formula (IIa), each Nₐ' independently represents an oligonucleotide sequence comprising 2-20, 2-15, or 2-10 modified nucleotides.

Each of X', Y' and Z' may be the same or different from each other.

Each nucleotide of the sense strand and antisense strand may be independently modified with LNA, CRN, cET, UNA, HNA, CeNA, 2'-methoxyethyl, 2'-O-methyl, 2'-O-allyl, 2'-C-allyl, 2'-hydroxyl, or 2'-fluoro. For example, each nucleotide of the sense strand and antisense strand is independently modified with 2'-O-methyl or 2'-fluoro. Each X, Y, Z, X', Y' and Z', in particular, may represent a 2'-O-methyl modification or a 2'-fluoro modification.

In one case, the sense strand of the iRNA agent may contain YYY motif occurring at 9, 10 and 11 positions of the strand when the duplex region is 21 nt, the count starting from the 1^{st} nucleotide from the 5'-end, or optionally, the count starting at the 1^{st} paired nucleotide within the duplex region, from the 5'- end; and Y represents 2'-F modification. The sense strand may additionally contain XXX motif or ZZZ motifs as wing modifications at the opposite end of the duplex region; and XXX and ZZZ each independently represents a 2'-OMe modification or 2'-F modification.

In one case the antisense strand may contain Y'Y'Y' motif occurring at positions 11, 12, 13 of the strand, the count starting from the 1^{st} nucleotide from the 5'-end, or optionally, the count starting at the 1^{st} paired nucleotide within the duplex region, from the 5'- end; and Y' represents 2'-O-methyl modification. The antisense strand may additionally contain X'X'X' motif or Z'Z'Z' motifs as wing modifications at the opposite end of the duplex region; and X'X'X' and Z'Z'Z' each independently represents a 2'-OMe modification or 2'-F modification.

The sense strand represented by any one of the above formulas (Ia), (Ib), (Ic), and (Id) forms a duplex with a antisense strand being represented by any one of formulas (IIa), (IIb), (IIc), and (IId), respectively.

Accordingly, the iRNA agents for use in the described methods may comprise a sense strand and an antisense strand, each strand having 14 to 30 nucleotides, the iRNA duplex represented by formula (III): 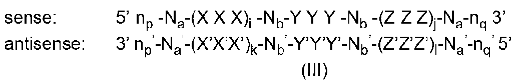 wherein:
i, j, k, and l are each independently 0 or 1;
p, p', q, and q' are each independently 0-6;
each Nₐ and Nₐ^{'} independently represents an oligonucleotide sequence comprising 0-25 modified nucleotides, each sequence comprising at least two differently modified nucleotides;
each N_{b} and N_{b}^{'} independently represents an oligonucleotide sequence comprising 0-10 modified nucleotides;
wherein
each nₚ^{'}, nₚ, n_{q}^{'}, and n_{q}, each of which may or may not be present, independently represents an overhang nucleotide; and
XXX, YYY, ZZZ, X'X'X', Y'Y'Y', and Z'Z'Z' each independently represent one motif of three identical modifications on three consecutive nucleotides.

In one case, i is 0 and j is 0; or i is 1 and j is 0; or i is 0 and j is 1; or both i and j are 0; or both i and j are 1. In another case, k is 0 and 1 is 0; or k is 1 and 1 is 0; k is 0 and 1 is 1; or both k and 1 are 0; or both k and 1 are 1.

Exemplary combinations of the sense strand and antisense strand forming a iRNA duplex include the formulas below:

5'nₚ - Nₐ -Y Y Y -Nₐ-n_{q} 3'

3' nₚ^{'}-Nₐ^{'}-Y'Y'Y' -Nₐ^{'}n_{q}^{'} 5' (IIIa)

5' nₚ-Nₐ -YYY-N_{b}-ZZZ-Nₐ-n_{q} 3'

3' np^{'}-Nₐ^{'}-Y'Y'Y'-N_{b}^{'}-Z'Z'Z'-Nₐ^{'}n_{q}^{'}5' (IIIb)

5' nₚ-Nₐ₋XXX-N_{b}-YYY-Nₐ₋n_{q} 3'

3' nₚ^{'}-Nₐ^{'}-X'X'X'-N_{b}^{'}-Y'Y'Y'-Nₐ^{'}-n_{q}^{'} 5' (IIIc)

5' nₚ-Nₐ-XXX-N_{b}-YYY-N_{b}-ZZZ-Nₐ-n_{q} 3'

3' nₚ^{'}-Nₐ^{'}-X'X'X'-N_{b}^{'}-Y'Y'Y'-N_{b}^{'}-Z'Z'Z'-Nₐ-n_{q}^{'} 5' (IIId)

When the iRNA agent is represented by formula (IIIa), each Nₐ independently represents an oligonucleotide sequence comprising 2-20, 2-15, or 2-10 modified nucleotides.

When the iRNA agent is represented by formula (IIIb), each N_{b} independently represents an oligonucleotide sequence comprising 1-10, 1-7, 1-5 or 1-4 modified nucleotides. Each Nₐ independently represents an oligonucleotide sequence comprising 2-20, 2-15, or 2-10 modified nucleotides.

When the iRNA agent is represented as formula (IIIc), each N_{b}, N_{b}' independently represents an oligonucleotide sequence comprising 0-10, 0-7, 0-10, 0-7, 0-5, 0-4, 0-2 or 0modified nucleotides. Each Nₐ independently represents an oligonucleotide sequence comprising 2-20, 2-15, or 2-10 modified nucleotides.

When the iRNA agent is represented as formula (IIId), each N_{b}, N_{b}' independently represents an oligonucleotide sequence comprising 0-10, 0-7, 0-10, 0-7, 0-5, 0-4, 0-2 or 0modified nucleotides. Each Nₐ, Nₐ^{'} independently represents an oligonucleotide sequence comprising 2-20, 2-15, or 2-10 modified nucleotides. Each of Nₐ, Nₐ', N_{b} and N_{b}^{'} independently comprises modifications of alternating pattern.

Each of X, Y and Z in formulas (III), (IIIa), (IIIb), (IIIc), and (IIId) may be the same or different from each other.

When the iRNA agent is represented by formula (III), (IIIa), (IIIb), (IIIc), and (IIId), at least one of the Y nucleotides may form a base pair with one of the Y' nucleotides. Alternatively, at least two of the Y nucleotides form base pairs with the corresponding Y' nucleotides; or all three of the Y nucleotides all form base pairs with the corresponding Y' nucleotides.

When the iRNA agent is represented by formula (IIIb) or (IIId), at least one of the Z nucleotides may form a base pair with one of the Z' nucleotides. Alternatively, at least two of the Z nucleotides form base pairs with the corresponding Z' nucleotides; or all three of the Z nucleotides all form base pairs with the corresponding Z' nucleotides.

When the iRNA agent is represented as formula (IIIc) or (IIId), at least one of the X nucleotides may form a base pair with one of the X' nucleotides. Alternatively, at least two of the X nucleotides form base pairs with the corresponding X' nucleotides; or all three of the X nucleotides all form base pairs with the corresponding X' nucleotides.

In one case, the modification on the Y nucleotide is different than the modification on the Y' nucleotide, the modification on the Z nucleotide is different than the modification on the Z' nucleotide, and/or the modification on the X nucleotide is different than the modification on the X' nucleotide.

In one case, when the iRNA agent is represented by formula (IIId), the Nₐ modifications are 2'-O-methyl or 2'-fluoro modifications. In another case, when the iRNA agent is represented by formula (IIId), the Nₐ modifications are 2'-O-methyl or 2'-fluoro modifications and nₚ' >0 and at least one nₚ' is linked to a neighboring nucleotide a via phosphorothioate linkage. In yet another case, when the iRNA agent is represented by formula (IIId), the Nₐ modifications are 2'-O-methyl or 2'-fluoro modifications , nₚ^{'} >0 and at least one nₚ^{'} is linked to a neighboring nucleotide via phosphorothioate linkage, and the sense strand is conjugated to one or more GalNAc derivatives attached through a bivalent or trivalent branched linker (described below). In another case, when the iRNA agent is represented by formula (IIId), the Nₐ modifications are 2'-O-methyl or 2'-fluoro modifications , nₚ^{'} >0 and at least one nₚ^{'} is linked to a neighboring nucleotide via phosphorothioate linkage, the sense strand comprises at least one phosphorothioate linkage, and the sense strand is conjugated to one or more GalNAc derivatives attached through a bivalent or trivalent branched linker.

In one case, when the iRNA agent is represented by formula (IIIa), the Nₐ modifications are 2'-O-methyl or 2'-fluoro modifications , nₚ^{'} >0 and at least one nₚ^{'} is linked to a neighboring nucleotide via phosphorothioate linkage, the sense strand comprises at least one phosphorothioate linkage, and the sense strand is conjugated to one or more GalNAc derivatives attached through a bivalent or trivalent branched linker.

In one case, the iRNA agent is a multimer containing at least two duplexes represented by formula (III), (IIIa), (IIIb), (IIIc), and (IIId), wherein the duplexes are connected by a linker. The linker can be cleavable or non-cleavable. Optionally, the multimer further comprises a ligand. Each of the duplexes can target the same gene or two different genes; or each of the duplexes can target same gene at two different target sites.

In one case, the iRNA agent is a multimer containing three, four, five, six or more duplexes represented by formula (III), (IIIa), (IIIb), (IIIc), and (IIId), wherein the duplexes are connected by a linker. The linker can be cleavable or non-cleavable. Optionally, the multimer further comprises a ligand. Each of the duplexes can target the same gene or two different genes; or each of the duplexes can target same gene at two different target sites.

In one case, two iRNA agents represented by formula (III), (IIIa), (IIIb), (IIIc), and (IIId) are linked to each other at the 5' end, and one or both of the 3' ends and are optionally conjugated to to a ligand. Each of the agents can target the same gene or two different genes; or each of the agents can target same gene at two different target sites.

Various publications describe multimeric iRNA agents that can be used in the methods of the invention. Such publications include WO2007/091269, US Patent No. 7858769, WO2010/141511, WO2007/117686, WO2009/014887 and WO2011/031520.

As described in more detail below, the iRNA agent that contains conjugations of one or more carbohydrate moieties to a iRNA agent can optimize one or more properties of the iRNA agent. In many cases, the carbohydrate moiety will be attached to a modified subunit of the iRNA agent. For example, the ribose sugar of one or more ribonucleotide subunits of a dsRNA agent can be replaced with another moiety, *e.g.,* a non-carbohydrate (preferably cyclic) carrier to which is attached a carbohydrate ligand. A ribonucleotide subunit in which the ribose sugar of the subunit has been so replaced is referred to herein as a ribose replacement modification subunit (RRMS). A cyclic carrier may be a carbocyclic ring system, *i.e.,* all ring atoms are carbon atoms, or a heterocyclic ring system, *i.e.,* one or more ring atoms may be a heteroatom, *e.g.,* nitrogen, oxygen, sulfur. The cyclic carrier may be a monocyclic ring system, or may contain two or more rings, *e.g.* fused rings. The cyclic carrier may be a fully saturated ring system, or it may contain one or more double bonds.

The ligand may be attached to the polynucleotide via a carrier. The carriers include (i) at least one "backbone attachment point," preferably two "backbone attachment points" and (ii) at least one "tethering attachment point." A "backbone attachment point" as used herein refers to a functional group, *e.g.* a hydroxyl group, or generally, a bond available for, and that is suitable for incorporation of the carrier into the backbone, *e.g.,* the phosphate, or modified phosphate, *e.g.,* sulfur containing, backbone, of a ribonucleic acid. A "tethering attachment point" (TAP) in some embodiments refers to a constituent ring atom of the cyclic carrier, *e.g.,* a carbon atom or a heteroatom (distinct from an atom which provides a backbone attachment point), that connects a selected moiety. The moiety can be, *e.g.,* a carbohydrate, *e.g.* monosaccharide, disaccharide, trisaccharide, tetrasaccharide, oligosaccharide and polysaccharide. Optionally, the selected moiety is connected by an intervening tether to the cyclic carrier. Thus, the cyclic carrier will often include a functional group, *e.g.,* an amino group, or generally, provide a bond, that is suitable for incorporation or tethering of another chemical entity, *e.g.,* a ligand to the constituent ring.

The iRNA agents may be conjugated to a ligand *via* a carrier, wherein the carrier can be cyclic group or acyclic group; preferably, the cyclic group is selected from pyrrolidinyl, pyrazolinyl, pyrazolidinyl, imidazolinyl, imidazolidinyl, piperidinyl, piperazinyl, [1,3]dioxolane, oxazolidinyl, isoxazolidinyl, morpholinyl, thiazolidinyl, isothiazolidinyl, quinoxalinyl, pyridazinonyl, tetrahydrofuryl and and decalin; preferably, the acyclic group is selected from serinol backbone or diethanolamine backbone.

In certain specific cases, the iRNA agent for use in the described methods is an agent selected from the group of agents listed in any one of Tables 3, 4 5, 6, and 7. These agents may further comprise a ligand.

### IV. iRNAs Conjugated to Ligands

Another modification of the RNA of an iRNA of the invention involves chemically linking to the RNA one or more ligands, moieties or conjugates that enhance the activity, cellular distribution or cellular uptake of the iRNA. Such moieties include but are not limited to lipid moieties such as a cholesterol moiety (Letsinger et al., Proc. Natl. Acid. Sci. USA, 1989, 86: 6553-6556), cholic acid (Manoharan et al., Biorg. Med. Chem. Let., 1994, 4:1053-1060), a thioether, *e.g.,* beryl-S-tritylthiol (Manoharan et al., Ann. N.Y. Acad. Sci., 1992, 660:306-309; Manoharan et al., Biorg. Med. Chem. Let., 1993, 3:2765-2770), a thiocholesterol (Oberhauser et al., Nucl. Acids Res., 1992, 20:533-538), an aliphatic chain, *e.g.,* dodecandiol or undecyl residues (Saison-Behmoaras et al., EMBO J, 1991, 10:1111-1118; Kabanov et al., FEBS Lett., 1990, 259:327-330; Svinarchuk et al., Biochimie, 1993, 75:49-54), a phospholipid, *e.g.,* di-hexadecyl-rac-glycerol or triethyl-ammonium 1,2-di-O-hexadecyl-rac-glycero-3-phosphonate (Manoharan et al., Tetrahedron Lett., 1995, 36:3651-3654; Shea et al., Nucl. Acids Res., 1990, 18:3777-3783), a polyamine or a polyethylene glycol chain (Manoharan et al., Nucleosides & Nucleotides, 1995, 14:969-973), or adamantane acetic acid (Manoharan et al., Tetrahedron Lett., 1995, 36:3651-3654), a palmityl moiety (Mishra et al., Biochim. Biophys. Acta, 1995, 1264:229-237), or an octadecylamine or hexylamino-carbonyloxycholesterol moiety (Crooke et al., J. Pharmacol. Exp. Ther., 1996, 277:923-937).

In one case, a ligand alters the distribution, targeting or lifetime of an iRNA agent into which it is incorporated. In preferred cases a ligand provides an enhanced affinity for a selected target, *e.g.,* molecule, cell or cell type, compartment, *e.g.,* a cellular or organ compartment, tissue, organ or region of the body, as, *e.g.,* compared to a species absent such a ligand. Preferred ligands will not take part in duplex pairing in a duplexed nucleic acid.

Ligands can include a naturally occurring substance, such as a protein *(e.g.,* human serum albumin (HSA), low-density lipoprotein (LDL), or globulin); carbohydrate *(e.g.,* a dextran, pullulan, chitin, chitosan, inulin, cyclodextrin, N-acetylgalactosamine, or hyaluronic acid); or a lipid. The ligand can also be a recombinant or synthetic molecule, such as a synthetic polymer, *e.g.,* a synthetic polyamino acid. Examples of polyamino acids include polyamino acid is a polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an alpha helical peptide.

Ligands can also include targeting groups, *e.g.,* a cell or tissue targeting agent, *e.g.,* a lectin, glycoprotein, lipid or protein, *e.g.,* an antibody, that binds to a specified cell type such as a kidney cell. A targeting group can be a thyrotropin, melanotropin, lectin, glycoprotein, surfactant protein A, Mucin carbohydrate, multivalent lactose, multivalent galactose, N-acetyl-galactosamine, N-acetyl-gulucoseamine multivalent mannose, multivalent fucose, glycosylated polyaminoacids, multivalent galactose, transferrin, bisphosphonate, polyglutamate, polyaspartate, a lipid, cholesterol, a steroid, bile acid, folate, vitamin B12, vitamin A, biotin, or an RGD peptide or RGD peptide mimetic.

Other examples of ligands include dyes, intercalating agents *(e.g.* acridines), cross-linkers *(e.g.* psoralene, mitomycin C), porphyrins (TPPC4, texaphyrin, Sapphyrin), polycyclic aromatic hydrocarbons *(e.g.,* phenazine, dihydrophenazine), artificial endonucleases *(e.g.* EDTA), lipophilic molecules, *e.g.,* cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine)and peptide conjugates *(e.g.,* antennapedia peptide, Tat peptide), alkylating agents, phosphate, amino, mercapto, PEG *(e.g.,* PEG-40K), MPEG, [MPEG]₂, polyamino, alkyl, substituted alkyl, radiolabeled markers, enzymes, haptens *(e.g.* biotin), transport/absorption facilitators *(e.g.,* aspirin, vitamin E, folic acid), synthetic ribonucleases *(e.g.,* imidazole, bisimidazole, histamine, imidazole clusters, acridine-imidazole conjugates, Eu3+ complexes of tetraazamacrocycles), dinitrophenyl, HRP, or AP.

Ligands can be proteins, *e.g.,* glycoproteins, or peptides, *e.g.,* molecules having a specific affinity for a co-ligand, or antibodies *e.g.,* an antibody, that binds to a specified cell type such as a hepatic cell. Ligands can also include hormones and hormone receptors. They can also include non-peptidic species, such as lipids, lectins, carbohydrates, vitamins, cofactors, multivalent lactose, multivalent galactose, N-acetyl-galactosamine, N-acetyl-gulucosamine multivalent mannose, or multivalent fucose. The ligand can be, for example, a lipopolysaccharide, an activator of p38 MAP kinase, or an activator of NF-κB.

The ligand can be a substance, *e.g.,* a drug, which can increase the uptake of the iRNA agent into the cell, for example, by disrupting the cell's cytoskeleton, *e.g.,* by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin.

In some cases, a ligand attached to an iRNA as described herein acts as a pharmacokinetic modulator (PK modulator). PK modulators include lipophiles, bile acids, steroids, phospholipid analogues, peptides, protein binding agents, PEG, vitamins *etc.* Exemplary PK modulators include, but are not limited to, cholesterol, fatty acids, cholic acid, lithocholic acid, dialkylglycerides, diacylglyceride, phospholipids, sphingolipids, naproxen, ibuprofen, vitamin E, biotin *etc.* Oligonucleotides that comprise a number of phosphorothioate linkages are also known to bind to serum protein, thus short oligonucleotides, *e.g.,* oligonucleotides of about 5 bases, 10 bases, 15 bases or 20 bases, comprising multiple of phosphorothioate linkages in the backbone are also amenable to the present invention as ligands *(e.g.* as PK modulating ligands). In addition, aptamers that bind serum components *(e.g.* serum proteins) are also suitable for use as PK modulating ligands in the embodiments described herein.

Ligand-conjugated oligonucleotides of the invention may be synthesized by the use of an oligonucleotide that bears a pendant reactive functionality, such as that derived from the attachment of a linking molecule onto the oligonucleotide (described below). This reactive oligonucleotide may be reacted directly with commercially-available ligands, ligands that are synthesized bearing any of a variety of protecting groups, or ligands that have a linking moiety attached thereto.

The oligonucleotides used in the conjugates of the present invention may be conveniently and routinely made through the well-known technique of solid-phase synthesis. Equipment for such synthesis is sold by several vendors including, for example, Applied Biosystems (Foster City, Calif.). Any other means for such synthesis known in the art may additionally or alternatively be employed. It is also known to use similar techniques to prepare other oligonucleotides, such as the phosphorothioates and alkylated derivatives.

In the ligand-conjugated oligonucleotides and ligand-molecule bearing sequence-specific linked nucleosides of the present invention, the oligonucleotides and oligonucleosides may be assembled on a suitable DNA synthesizer utilizing standard nucleotide or nucleoside precursors, or nucleotide or nucleoside conjugate precursors that already bear the linking moiety, ligand-nucleotide or nucleoside-conjugate precursors that already bear the ligand molecule, or non-nucleoside ligand-bearing building blocks.

When using nucleotide-conjugate precursors that already bear a linking moiety, the synthesis of the sequence-specific linked nucleosides is typically completed, and the ligand molecule is then reacted with the linking moiety to form the ligand-conjugated oligonucleotide. In some cases, the oligonucleotides or linked nucleosides of the present invention are synthesized by an automated synthesizer using phosphoramidites derived from ligand-nucleoside conjugates in addition to the standard phosphoramidites and non-standard phosphoramidites that are commercially available and routinely used in oligonucleotide synthesis.

### A. Lipid Conjugates

In one case, the ligand or conjugate is a lipid or lipid-based molecule. Such a lipid or lipid-based molecule preferably binds a serum protein, *e.g.,* human serum albumin (HSA). An HSA binding ligand allows for distribution of the conjugate to a target tissue, *e.g.,* a non-kidney target tissue of the body. For example, the target tissue can be the liver, including parenchymal cells of the liver. Other molecules that can bind HSA can also be used as ligands. For example, naproxen or aspirin can be used. A lipid or lipid-based ligand can (a) increase resistance to degradation of the conjugate, (b) increase targeting or transport into a target cell or cell membrane, and/or (c) can be used to adjust binding to a serum protein, *e.g.,* HSA.

A lipid based ligand can be used to inhibit, *e.g.,* control the binding of the conjugate to a target tissue. For example, a lipid or lipid-based ligand that binds to HSA more strongly will be less likely to be targeted to the kidney and therefore less likely to be cleared from the body. A lipid or lipid-based ligand that binds to HSA less strongly can be used to target the conjugate to the kidney.

In a preferred case, the lipid based ligand binds HSA. Preferably, it binds HSA with a sufficient affinity such that the conjugate will be preferably distributed to a non-kidney tissue. However, it is preferred that the affinity not be so strong that the HSA-ligand binding cannot be reversed.

In another preferred case, the lipid based ligand binds HSA weakly or not at all, such that the conjugate will be preferably distributed to the kidney. Other moieties that target to kidney cells can also be used in place of or in addition to the lipid based ligand.

In another aspect, the ligand is a moiety, *e.g.,* a vitamin, which is taken up by a target cell, *e.g.,* a proliferating cell. These are particularly useful for treating disorders characterized by unwanted cell proliferation, *e.g.,* of the malignant or non-malignant type, *e.g.,* cancer cells. Exemplary vitamins include vitamin A, E, and K. Other exemplary vitamins include are B vitamin, *e.g.,* folic acid, B12, riboflavin, biotin, pyridoxal or other vitamins or nutrients taken up by target cells such as liver cells. Also included are HSA and low density lipoprotein (LDL).

### B. Cell Permeation Agents

In another aspect, the ligand is a cell-permeation agent, preferably a helical cell-permeation agent. Preferably, the agent is amphipathic. An exemplary agent is a peptide such as tat or antennopedia. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

The ligand can be a peptide or peptidomimetic. A peptidomimetic (also referred to herein as an oligopeptidomimetic) is a molecule capable of folding into a defined three-dimensional structure similar to a natural peptide. The attachment of peptide and peptidomimetics to iRNA agents can affect pharmacokinetic distribution of the iRNA, such as by enhancing cellular recognition and absorption. The peptide or peptidomimetic moiety can be about 5-50 amino acids long, *e.g.,* about 5, 10, 15, 20, 25, 30, 35, 40, 45, or 50 amino acids long.

A peptide or peptidomimetic can be, for example, a cell permeation peptide, cationic peptide, amphipathic peptide, or hydrophobic peptide *(e.g.,* consisting primarily of Tyr, Trp or Phe). The peptide moiety can be a dendrimer peptide, constrained peptide or crosslinked peptide. In another alternative, the peptide moiety can include a hydrophobic membrane translocation sequence (MTS). An exemplary hydrophobic MTS-containing peptide is RFGF having the amino acid sequence AAVALLPAVLLALLAP (SEQ ID NO: 13). An RFGF analogue *(e.g.,* amino acid sequence AALLPVLLAAP (SEQ ID NO: 14) containing a hydrophobic MTS can also be a targeting moiety. The peptide moiety can be a "delivery" peptide, which can carry large polar molecules including peptides, oligonucleotides, and protein across cell membranes. For example, sequences from the HIV Tat protein (GRKKRRQRRRPPQ (SEQ ID NO: 15) and the Drosophila Antennapedia protein (RQIKIWFQNRRMKWKK (SEQ ID NO: 16) have been found to be capable of functioning as delivery peptides. A peptide or peptidomimetic can be encoded by a random sequence of DNA, such as a peptide identified from a phage-display library, or one-bead-one-compound (OBOC) combinatorial library (Lam et al., Nature, 354:82-84, 1991). Examples of a peptide or peptidomimetic tethered to a dsRNA agent via an incorporated monomer unit for cell targeting purposes is an arginine-glycine-aspartic acid (RGD)-peptide, or RGD mimic. A peptide moiety can range in length from about 5 amino acids to about 40 amino acids. The peptide moieties can have a structural modification, such as to increase stability or direct conformational properties. Any of the structural modifications described below can be utilized.

An RGD peptide for use in the described compositions and methods may be linear or cyclic, and may be modified, *e.g.,* glycosylated or methylated, to facilitate targeting to a specific tissue(s). RGD-containing peptides and peptidiomimemtics may include D-amino acids, as well as synthetic RGD mimics. In addition to RGD, one can use other moieties that target the integrin ligand. Preferred conjugates of this ligand target PECAM-1 or VEGF.

A "cell permeation peptide" is capable of permeating a cell, *e.g.,* a microbial cell, such as a bacterial or fungal cell, or a mammalian cell, such as a human cell. A microbial cell-permeating peptide can be, for example, a α-helical linear peptide *(e.g.,* LL-37 or Ceropin PI), a disulfide bond-containing peptide *(e.g.,* α-defensin, β-defensin or bactenecin), or a peptide containing only one or two dominating amino acids *(e.g.,* PR-39 or indolicidin). A cell permeation peptide can also include a nuclear localization signal (NLS). For example, a cell permeation peptide can be a bipartite amphipathic peptide, such as MPG, which is derived from the fusion peptide domain of HIV-1 gp41 and the NLS of SV40 large T antigen (Simeoni et al., Nucl. Acids Res. 31:2717-2724, 2003).

### C. Carbohydrate Conjugates

An iRNA oligonucleotide may further comprise a carbohydrate. The carbohydrate conjugated iRNA are advantageous for the *in vivo* delivery of nucleic acids, as well as compositions suitable for *in vivo* therapeutic use, as described herein. As used herein, "carbohydrate" refers to a compound which is either a carbohydrate *per se* made up of one or more monosaccharide units having at least 6 carbon atoms (which can be linear, branched or cyclic) with an oxygen, nitrogen or sulfur atom bonded to each carbon atom; or a compound having as a part thereof a carbohydrate moiety made up of one or more monosaccharide units each having at least six carbon atoms (which can be linear, branched or cyclic), with an oxygen, nitrogen or sulfur atom bonded to each carbon atom. Representative carbohydrates include the sugars (mono-, di-, tri- and oligosaccharides containing from about 4, 5, 6, 7, 8, or 9 monosaccharide units), and polysaccharides such as starches, glycogen, cellulose and polysaccharide gums. Specific monosaccharides include C5 and above *(e.g.,* C5, C6, C7, or C8) sugars; di- and trisaccharides include sugars having two or three monosaccharide units *(e.g.,* C5, C6, C7, or C8).

In one case, a carbohydrate conjugate for use in the compositions and methods of the invention is a monosaccharide. In one case, the monosaccharide is an N-acetylgalactosamine, such as

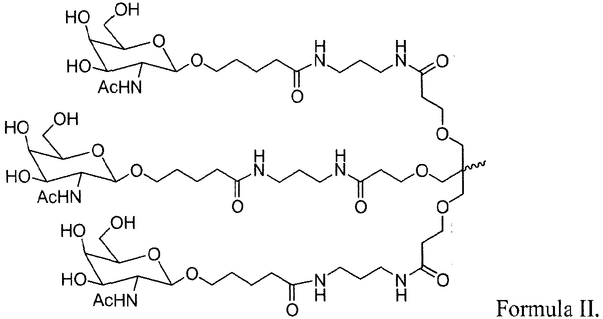

In another case, a carbohydrate conjugate for use in the compositions and methods is selected from the group consisting of:

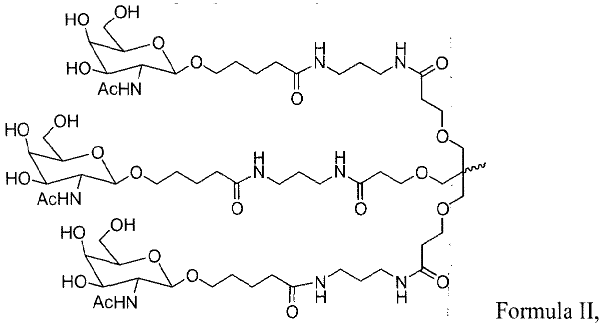

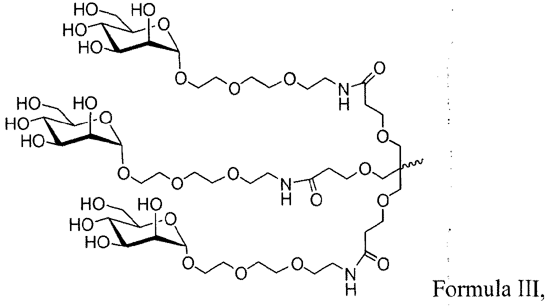

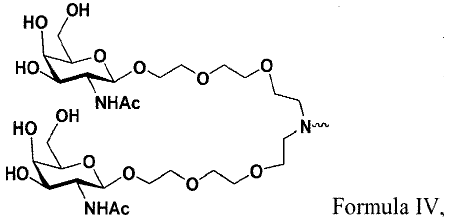

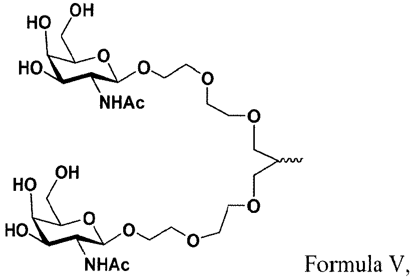

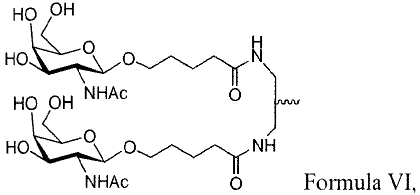

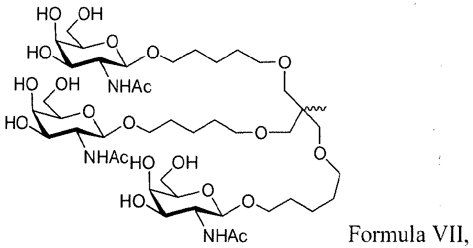

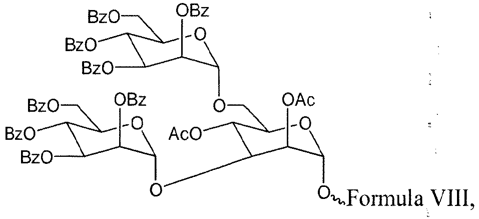

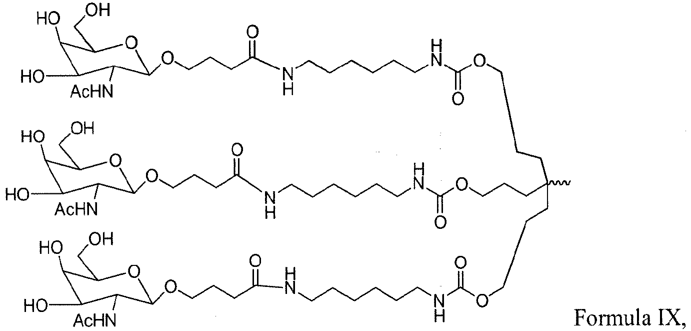

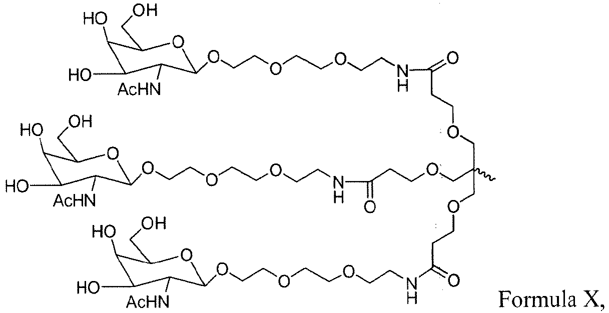

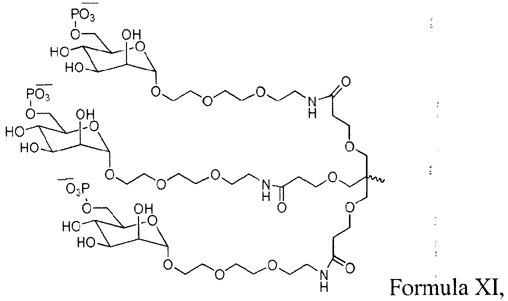

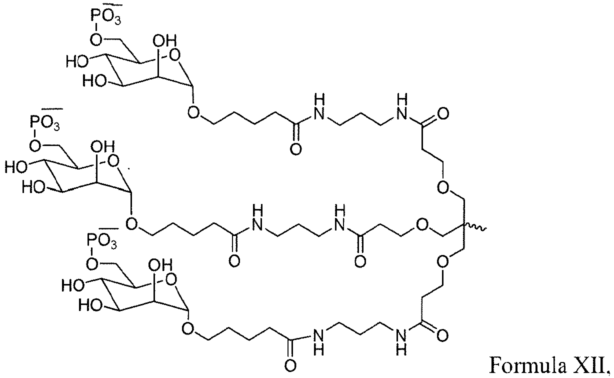

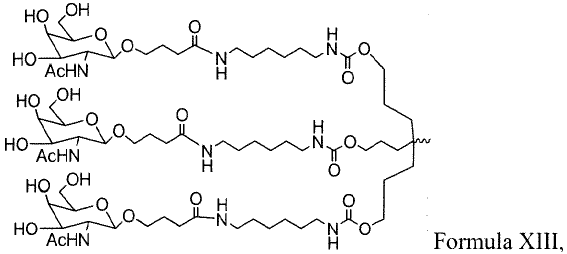

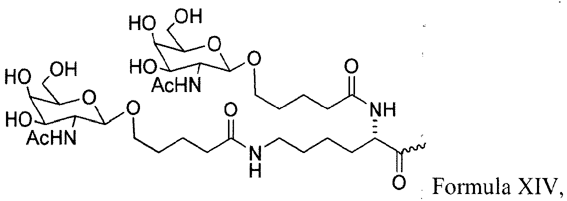

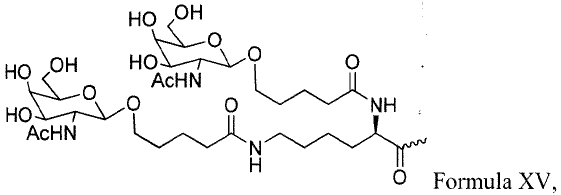

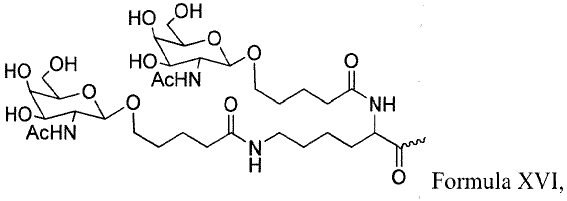

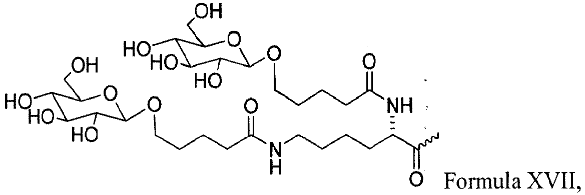

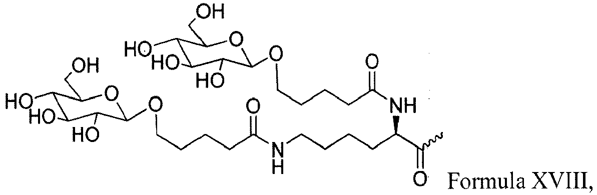

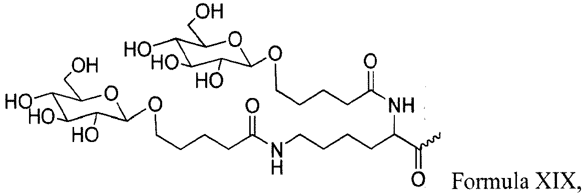

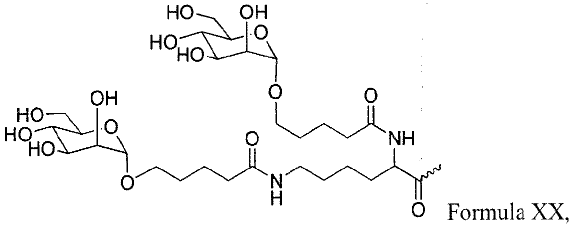

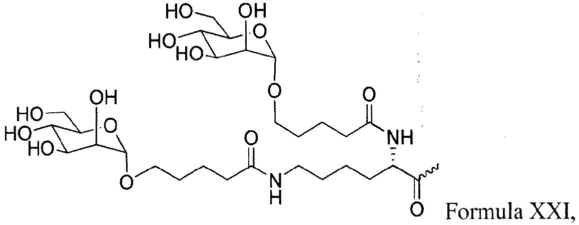

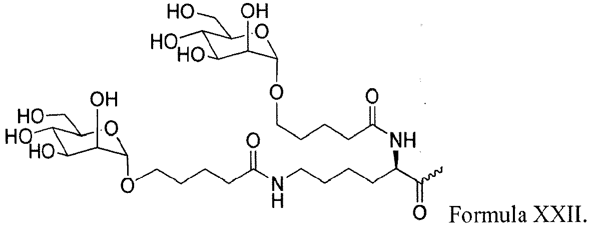

Another representative carbohydrate conjugate for use in the embodiments described herein includes, but is not limited to,

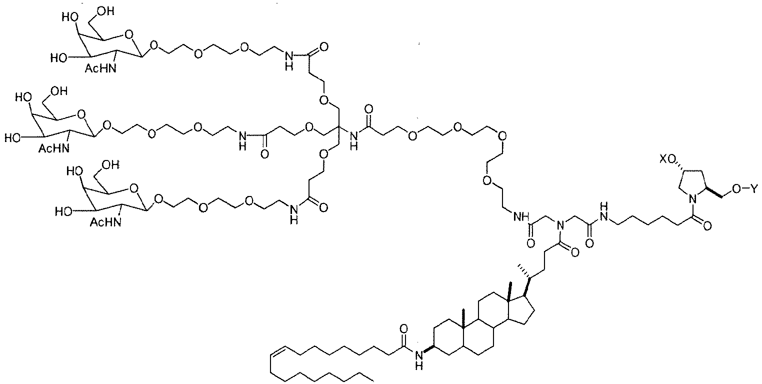

(Formula XXIII), when one of X or Y is an oligonucleotide, the other is a hydrogen.

In some embodiments, the carbohydrate conjugate further comprises one or more additional ligands as described above, such as, but not limited to, a PK modulator and/or a cell permeation peptide.

Additional carbohydrate conjugates suitable for use include those described in PCT Publication Nos. WO 2014/179620 and WO 2014/179627.

### D. Linkers

In some cases, the conjugate or ligand described herein can be attached to an iRNA oligonucleotide with various linkers that can be cleavable or non-cleavable.

The term "linker" or "linking group" means an organic moiety that connects two parts of a compound, *e.g.,* covalently attaches two parts of a compound. Linkers typically comprise a direct bond or an atom such as oxygen or sulfur, a unit such as NR8, C(O), C(O)NH, SO, SO₂, SO₂NH or a chain of atoms, such as, but not limited to, substituted or unsubstituted alkyl, substituted or unsubstituted alkenyl, substituted or unsubstituted alkynyl, arylalkyl, arylalkenyl, arylalkynyl, heteroarylalkyl, heteroarylalkenyl, heteroarylalkynyl, heterocyclylalkyl, heterocyclylalkenyl, heterocyclylalkynyl, aryl, heteroaryl, heterocyclyl, cycloalkyl, cycloalkenyl, alkylarylalkyl, alkylarylalkenyl, alkylarylalkynyl, alkenylarylalkyl, alkenylarylalkenyl, alkenylarylalkynyl, alkynylarylalkyl, alkynylarylalkenyl, alkynylarylalkynyl, alkylheteroarylalkyl, alkylheteroarylalkenyl, alkylheteroarylalkynyl, alkenylheteroarylalkyl, alkenylheteroarylalkenyl, alkenylheteroarylalkynyl, alkynylheteroarylalkyl, alkynylheteroarylalkenyl, alkynylheteroarylalkynyl, alkylheterocyclylalkyl, alkylheterocyclylalkenyl, alkylhererocyclylalkynyl, alkenylheterocyclylalkyl, alkenylheterocyclylalkenyl, alkenylheterocyclylalkynyl, alkynylheterocyclylalkyl, alkynylheterocyclylalkenyl, alkynylheterocyclylalkynyl, alkylaryl, alkenylaryl, alkynylaryl, alkylheteroaryl, alkenylheteroaryl, alkynylhereroaryl, which one or more methylenes can be interrupted or terminated by O, S, S(O), SO₂, N(R8), C(O), substituted or unsubstituted aryl, substituted or unsubstituted heteroaryl, substituted or unsubstituted heterocyclic; where R8 is hydrogen, acyl, aliphatic or substituted aliphatic. In one embodiment, the linker is between about 1-24 atoms, 2-24, 3-24, 4-24, 5-24, 6-24, 6-18, 7-18, 8-18 atoms, 7-17, 8-17, 6-16, 7-16, or 8-16 atoms.

A cleavable linking group is one which is sufficiently stable outside the cell, but which upon entry into a target cell is cleaved to release the two parts the linker is holding together. In a preferred embodiment, the cleavable linking group is cleaved at least about 10 times, 20, times, 30 times, 40 times, 50 times, 60 times, 70 times, 80 times, 90 times or more, or at least about 100 times faster in a target cell or under a first reference condition (which can, *e.g.,* be selected to mimic or represent intracellular conditions) than in the blood of a subject, or under a second reference condition (which can, *e.g.,* be selected to mimic or represent conditions found in the blood or serum).

Cleavable linking groups are susceptible to cleavage agents, *e.g.,* pH, redox potential or the presence of degradative molecules. Generally, cleavage agents are more prevalent or found at higher levels or activities inside cells than in serum or blood. Examples of such degradative agents include: redox agents which are selected for particular substrates or which have no substrate specificity, including, *e.g.,* oxidative or reductive enzymes or reductive agents such as mercaptans, present in cells, that can degrade a redox cleavable linking group by reduction; esterases; endosomes or agents that can create an acidic environment, *e.g.,* those that result in a pH of five or lower; enzymes that can hydrolyze or degrade an acid cleavable linking group by acting as a general acid, peptidases (which can be substrate specific), and phosphatases.

A cleavable linkage group, such as a disulfide bond can be susceptible to pH. The pH of human serum is 7.4, while the average intracellular pH is slightly lower, ranging from about 7.1-7.3. Endosomes have a more acidic pH, in the range of 5.5-6.0, and lysosomes have an even more acidic pH at around 5.0. Some linkers will have a cleavable linking group that is cleaved at a preferred pH, thereby releasing a cationic lipid from the ligand inside the cell, or into the desired compartment of the cell.

A linker can include a cleavable linking group that is cleavable by a particular enzyme. The type of cleavable linking group incorporated into a linker can depend on the cell to be targeted. For example, a liver-targeting ligand can be linked to a cationic lipid through a linker that includes an ester group. Liver cells are rich in esterases, and therefore the linker will be cleaved more efficiently in liver cells than in cell types that are not esterase-rich. Other cell-types rich in esterases include cells of the lung, renal cortex, and testis.

Linkers that contain peptide bonds can be used when targeting cell types rich in peptidases, such as liver cells and synoviocytes.

In general, the suitability of a candidate cleavable linking group can be evaluated by testing the ability of a degradative agent (or condition) to cleave the candidate linking group. It will also be desirable to also test the candidate cleavable linking group for the ability to resist cleavage in the blood or when in contact with other non-target tissue. Thus, one can determine the relative susceptibility to cleavage between a first and a second condition, where the first is selected to be indicative of cleavage in a target cell and the second is selected to be indicative of cleavage in other tissues or biological fluids, *e.g.,* blood or serum. The evaluations can be carried out in cell free systems, in cells, in cell culture, in organ or tissue culture, or in whole animals. It can be useful to make initial evaluations in cell-free or culture conditions and to confirm by further evaluations in whole animals. In preferred embodiments, useful candidate compounds are cleaved at least about 2, 4, 10, 20, 30, 40, 50, 60, 70, 80, 90, or about 100 times faster in the cell (or under *in vitro* conditions selected to mimic intracellular conditions) as compared to blood or serum (or under *in vitro* conditions selected to mimic extracellular conditions).

### i. Redox cleavable linking groups

In one case, a cleavable linking group is a redox cleavable linking group that is cleaved upon reduction or oxidation. An example of reductively cleavable linking group is a disulphide linking group (-S-S-). To determine if a candidate cleavable linking group is a suitable "reductively cleavable linking group," or for example is suitable for use with a particular iRNA moiety and particular targeting agent one can look to methods described herein. For example, a candidate can be evaluated by incubation with dithiothreitol (DTT), or other reducing agent using reagents know in the art, which mimic the rate of cleavage which would be observed in a cell, *e.g.,* a target cell. The candidates can also be evaluated under conditions which are selected to mimic blood or serum conditions. In one, candidate compounds are cleaved by at most about 10% in the blood. In other embodiments, useful candidate compounds are degraded at least about 2, 4, 10, 20, 30, 40, 50, 60, 70, 80, 90, or about 100 times faster in the cell (or under *in vitro* conditions selected to mimic intracellular conditions) as compared to blood (or under *in vitro* conditions selected to mimic extracellular conditions). The rate of cleavage of candidate compounds can be determined using standard enzyme kinetics assays under conditions chosen to mimic intracellular media and compared to conditions chosen to mimic extracellular media.

### ii. Phosphate-based cleavable linking groups

In another case, a cleavable linker comprises a phosphate-based cleavable linking group. A phosphate-based cleavable linking group is cleaved by agents that degrade or hydrolyze the phosphate group. An example of an agent that cleaves phosphate groups in cells are enzymes such as phosphatases in cells. Examples of phosphate-based linking groups are -O-P(O)(ORk)-O-, -O-P(S)(ORk)-O-, -O-P(S)(SRk)-O-, -S-P(O)(ORk)-O-, -O-P(O)(ORk)-S-, -S-P(O)(ORk)-S-, -O-P(S)(ORk)-S-, -S-P(S)(ORk)-O-, -O-P(O)(Rk)-O-, -O-P(S)(Rk)-O-, -S-P(O)(Rk)-O-, -S-P(S)(Rk)-O-, -S-P(O)(Rk)-S-, -O-P(S)(Rk)-S-. Preferred embodiments are -O-P(O)(OH)-O-, - O-P(S)(OH)-O-, -O-P(S)(SH)-O-, -S-P(O)(OH)-O-, -O-P(O)(OH)-S-, -S-P(O)(OH)-S-, -OP(S)(OH)-S-, -S-P(S)(OH)-O-, -O-P(O)(H)-O-, -O-P(S)(H)-O-, -S-P(O)(H)-O, -S-P(S)(H)-O-, - S-P(O)(H)-S-, -O-P(S)(H)-S-. A preferred case is -O-P(O)(OH)-O-. These candidates can be evaluated using methods analogous to those described above.

### iii. Acid cleavable linking groups

In another case, a cleavable linker comprises an acid cleavable linking group. An acid cleavable linking group is a linking group that is cleaved under acidic conditions. In preferred embodiments acid cleavable linking groups are cleaved in an acidic environment with a pH of about 6.5 or lower *(e.g.,* about 6.0, 5.75, 5.5, 5.25, 5.0, or lower), or by agents such as enzymes that can act as a general acid. In a cell, specific low pH organelles, such as endosomes and lysosomes can provide a cleaving environment for acid cleavable linking groups. Examples of acid cleavable linking groups include but are not limited to hydrazones, esters, and esters of amino acids. Acid cleavable groups can have the general formula -C=NN-, C(O)O, or -OC(O). A preferred case is when the carbon attached to the oxygen of the ester (the alkoxy group) is an aryl group, substituted alkyl group, or tertiary alkyl group such as dimethyl pentyl or t-butyl. These candidates can be evaluated using methods analogous to those described above.

### iv. Ester-based linking groups

In another case, a cleavable linker comprises an ester-based cleavable linking group. An ester-based cleavable linking group is cleaved by enzymes such as esterases and amidases in cells. Examples of ester-based cleavable linking groups include but are not limited to esters of alkylene, alkenylene and alkynylene groups. Ester cleavable linking groups have the general formula -C(O)O-, or -OC(O)-. These candidates can be evaluated using methods analogous to those described above.

### v. Peptide-based cleaving groups

In yet another case, a cleavable linker comprises a peptide-based cleavable linking group. A peptide-based cleavable linking group is cleaved by enzymes such as peptidases and proteases in cells. Peptide-based cleavable linking groups are peptide bonds formed between amino acids to yield oligopeptides *(e.g.,* dipeptides, tripeptides *etc.)* and polypeptides. Peptide-based cleavable groups do not include the amide group (-C(O)NH-). The amide group can be formed between any alkylene, alkenylene or alkynelene. A peptide bond is a special type of amide bond formed between amino acids to yield peptides and proteins. The peptide based cleavage group is generally limited to the peptide bond *(i.e.,* the amide bond) formed between amino acids yielding peptides and proteins and does not include the entire amide functional group. Peptide-based cleavable linking groups have the general formula - NHCHRAC(O)NHCHRBC(O)-, where RA and RB are the R groups of the two adjacent amino acids. These candidates can be evaluated using methods analogous to those described above.

In one case, an iRNA is conjugated to a carbohydrate through a linker. Non-limiting examples of iRNA carbohydrate conjugates with linkers of the compositions and methods include, but are not limited to, 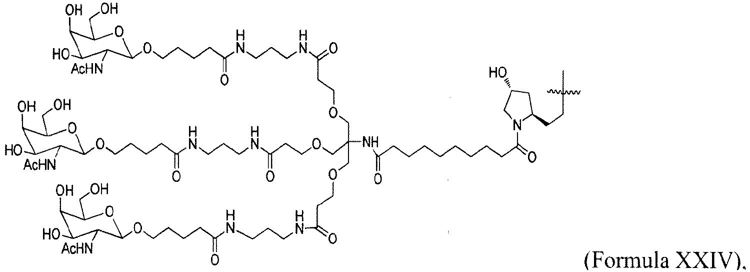 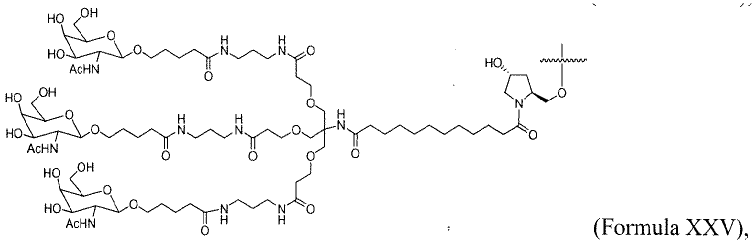 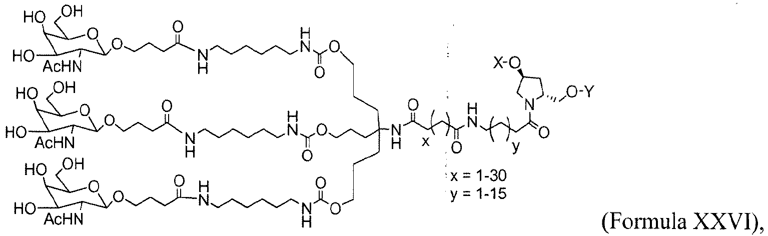 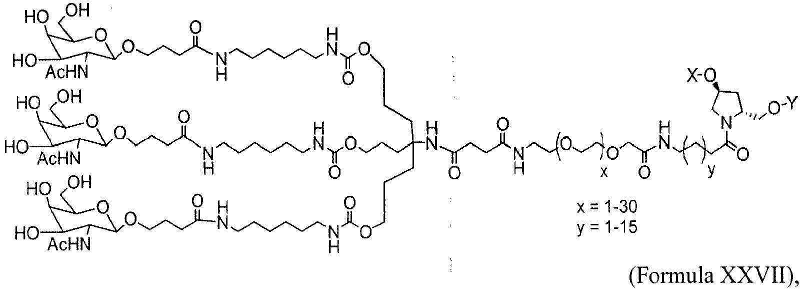 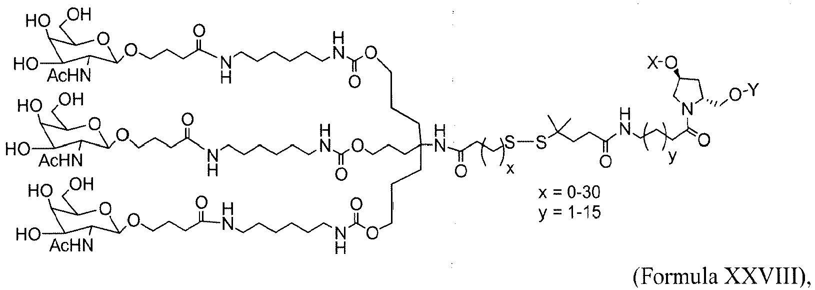 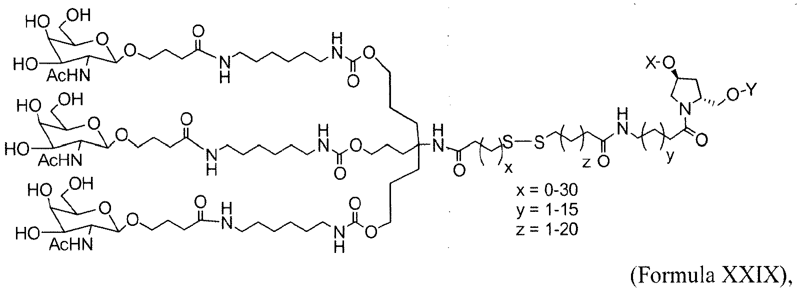 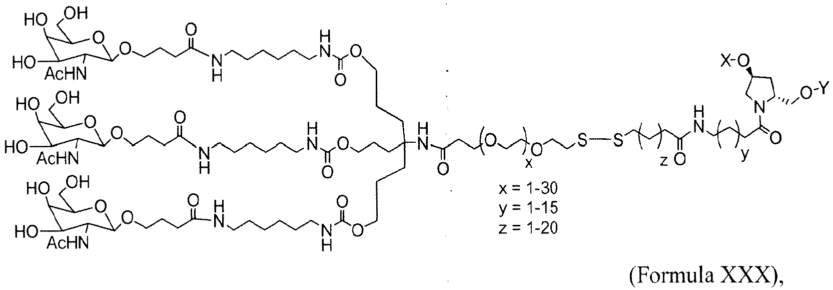 and 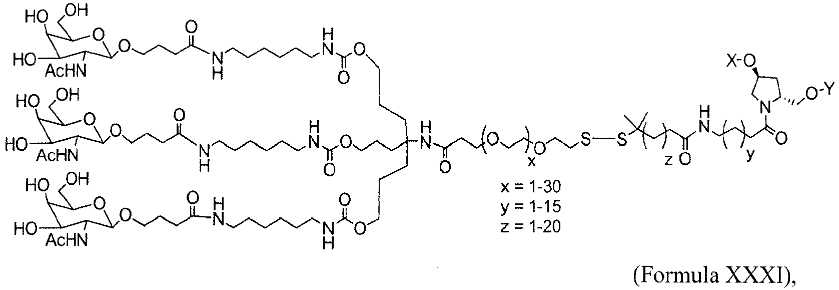 when one of X or Y is an oligonucleotide, the other is a hydrogen.

In certain embodiments of the compositions and methods of the invention, a ligand is one or more "GalNAc" (N-acetylgalactosamine) derivatives attached through a bivalent or trivalent branched linker.

In one case, a dsRNA of the invention is conjugated to a bivalent or trivalent branched linker selected from the group of structures shown in any of formula (XXXII) - (XXXV): 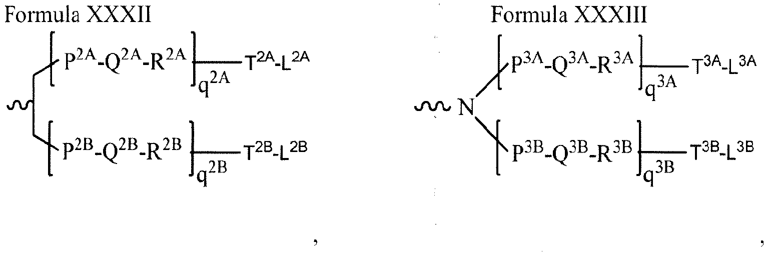 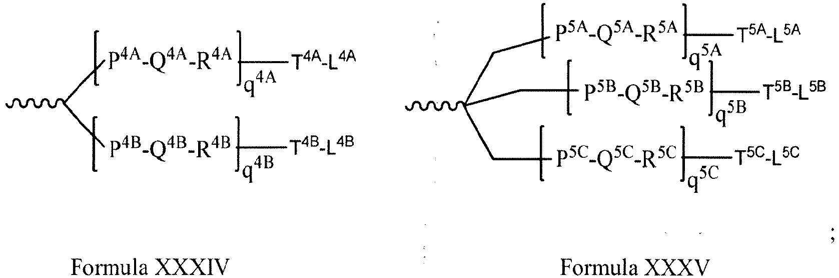 wherein:
q2A, q2B, q3A, q3B, q4A, q4B, q5A, q5B and q5C represent independently for each occurrence 0-20 and wherein the repeating unit can be the same or different;
P^{2A}, P^{2B}, P^{3A}, P^{3B}, P^{4A}, P^{4B}, P^{5A}, P^{5B}, P^{5C}, T^{2A}, T^{2B}, T^{3A}, T^{3B}, T^{4A}, T^{4B}, T^{4A}, T^{5B}, T^{5C} are each independently for each occurrence absent, CO, NH, O, S, OC(O), NHC(O), CH₂, CH₂NH or CH₂O;
Q^{2A}, Q^{2B}, Q^{3A}, Q^{3B}, Q^{4A}, Q^{4B}, Q^{5A}, Q^{5B}, Q^{5C} are independently for each occurrence absent, alkylene, substituted alkylene wherin one or more methylenes can be interrupted or terminated by one or more of O, S, S(O), SO₂, N(R^{N}), C(R')=C(R"), C=C or C(O);
R^{2A}, R^{2B}, R^{3A}, R^{3B}, R^{4A}, R^{4B}, R^{5A}, R^{5B}, R^{5C} are each independently for each occurrence absent, NH, O, S, CH₂, C(O)O, C(O)NH, NHCH(R^{a})C(O), -C(O)-CH(R^{a})-NH- CO, CH=N-O 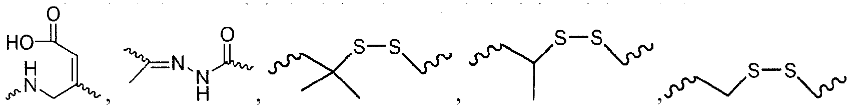
or heterocyclyl;
L^{2A}, L^{2B}, L^{3A}, L^{3B}, L^{4A}, L^{4B}, L^{5A}, L^{5B} and L^{5C} represent the ligand; *i.e.* each independently for each occurrence a monosaccharide (such as GalNAc), disaccharide, trisaccharide, tetrasaccharide, oligosaccharide, or polysaccharide; and R^{a} is H or amino acid side chain.Trivalent conjugating GalNAc derivatives are particularly useful for use with RNAi agents for inhibiting the expression of a target gene, such as those of formula (XXXV): 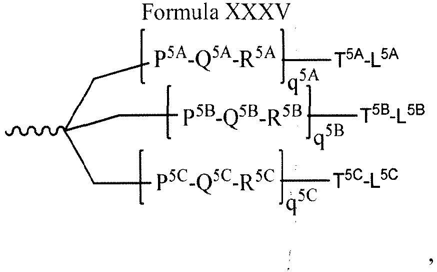
wherein L^{5A}, L^{5B} and L^{5C} represent a monosaccharide, such as GalNAc derivative.

Examples of suitable bivalent and trivalent branched linker groups conjugating GalNAc derivatives include, but are not limited to, the structures recited above as formulas II, VII, XI, X, and XIII.

Representative U.S. patents that teach the preparation of RNA conjugates include, but are not limited to, U.S. Pat. Nos. 4,828,979; 4,948,882; 5,218,105; 5,525,465; 5,541,313; 5,545,730; 5,552,538; 5,578,717, 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941; 6,294,664; 6,320,017; 6,576,752; 6,783,931; 6,900,297; 7,037,646; 8,106,022.

It is not necessary for all positions in a given compound to be uniformly modified, and in fact more than one of the aforementioned modifications can be incorporated in a single compound or even at a single nucleoside within an iRNA. The present disclosure also includes iRNA compounds that are chimeric compounds.

"Chimeric" iRNA compounds or "chimeras" are iRNA compounds, preferably dsRNAs, which contain two or more chemically distinct regions, each made up of at least one monomer unit, *i.e.,* a nucleotide in the case of a dsRNA compound. These iRNAs typically contain at least one region wherein the RNA is modified so as to confer upon the iRNA increased resistance to nuclease degradation, increased cellular uptake, and/or increased binding affinity for the target nucleic acid. An additional region of the iRNA can serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of iRNA inhibition of gene expression. Consequently, comparable results can often be obtained with shorter iRNAs when chimeric dsRNAs are used, compared to phosphorothioate deoxy dsRNAs hybridizing to the same target region. Cleavage of the RNA target can be routinely detected by gel electrophoresis and, if necessary, associated nucleic acid hybridization techniques known in the art.

In certain instances, the RNA of an iRNA can be modified by a non-ligand group. A number of non-ligand molecules have been conjugated to iRNAs in order to enhance the activity, cellular distribution or cellular uptake of the iRNA, and procedures for performing such conjugations are available in the scientific literature. Such non-ligand moieties have included lipid moieties, such as cholesterol (Kubo, T. et al., Biochem. Biophys. Res. Comm., 2007, 365(1):54-61; Letsinger et al., Proc. Natl. Acad. Sci. USA, 1989, 86:6553), cholic acid (Manoharan et al., Bioorg. Med. Chem. Lett., 1994, 4:1053), a thioether, *e.g.,* hexyl-S-tritylthiol (Manoharan et al., Ann. N.Y. Acad. Sci., 1992, 660:306; Manoharan et al., Bioorg. Med. Chem. Let., 1993, 3:2765), a thiocholesterol (Oberhauser et al., Nucl. Acids Res., 1992, 20:533), an aliphatic chain, *e.g.,* dodecandiol or undecyl residues (Saison-Behmoaras et al., EMBO J., 1991, 10:111; Kabanov et al., FEBS Lett., 1990, 259:327; Svinarchuk et al., Biochimie, 1993, 75:49), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36:3651; Shea et al., Nucl. Acids Res., 1990, 18:3777), a polyamine or a polyethylene glycol chain (Manoharan et al., Nucleosides & Nucleotides, 1995, 14:969), or adamantane acetic acid (Manoharan et al., Tetrahedron Lett., 1995, 36:3651), a palmityl moiety (Mishra et al., Biochim. Biophys. Acta, 1995, 1264:229), or an octadecylamine or hexylamino-carbonyl-oxycholesterol moiety (Crooke et al., J. Pharmacol. Exp. Ther., 1996, 277:923). Representative United States patents that teach the preparation of such RNA conjugates have been listed above. Typical conjugation protocols involve the synthesis of an RNAs bearing an aminolinker at one or more positions of the sequence. The amino group is then reacted with the molecule being conjugated using appropriate coupling or activating reagents. The conjugation reaction can be performed either with the RNA still bound to the solid support or following cleavage of the RNA, in solution phase. Purification of the RNA conjugate by HPLC typically affords the pure conjugate.

### V. Delivery of an iRNA of the Invention

The delivery of an iRNA of the invention to a cell *e.g.,* a cell within a subject, such as a human subject *(e.g.,* a subject in need thereof, such as a subject having a disease, disorder or condition associated with phosphorylation of fructose by KHK) can be achieved in a number of different ways. For example, delivery may be performed by contacting a cell with an iRNA of the invention either *in vitro* or *in vivo. In vivo* delivery may also be performed directly by administering a composition comprising an iRNA, *e.g.,* a dsRNA, to a subject. Alternatively, *in vivo* delivery may be performed indirectly by administering one or more vectors that encode and direct the expression of the iRNA. These alternatives are discussed further below.

In general, any method of delivering a nucleic acid molecule *(in vitro* or *in vivo)* can be adapted for use with an iRNA of the invention (see *e.g.,* Akhtar S. and Julian RL. (1992) Trends Cell. Biol. 2(5):139-144 and WO94/02595. For *in vivo* delivery, factors to consider in order to deliver an iRNA molecule include, for example, biological stability of the delivered molecule, prevention of non-specific effects, and accumulation of the delivered molecule in the target tissue. The non-specific effects of an iRNA can be minimized by local administration, for example, by direct injection or implantation into a tissue or topically administering the preparation. Local administration to a treatment site maximizes local concentration of the agent, limits the exposure of the agent to systemic tissues that can otherwise be harmed by the agent or that can degrade the agent, and permits a lower total dose of the iRNA molecule to be administered. Several studies have shown successful knockdown of gene products when an iRNA is administered locally. For example, intraocular delivery of a VEGF dsRNA by intravitreal injection in cynomolgus monkeys (Tolentino, MJ., et al (2004) Retina 24:132-138) and subretinal injections in mice (Reich, SJ., et al (2003) Mol. Vis. 9:210-216) were both shown to prevent neovascularization in an experimental model of age-related macular degeneration. In addition, direct intratumoral injection of a dsRNA in mice reduces tumor volume (Pille, J., et al (2005) Mol. Ther. 11:267-274) and can prolong survival of tumor-bearing mice (Kim, WJ., et al (2006) Mol. Ther. 14:343-350; Li, S., et al (2007) Mol. Ther. 15:515-523). RNA interference has also shown success with local delivery to the CNS by direct injection (Dorn, G., et al. (2004) Nucleic Acids 32:e49; Tan, PH., et al (2005) Gene Ther. 12:59-66; Makimura, H., et al (2002) BMC Neurosci. 3:18; Shishkina, GT., et al (2004) Neuroscience 129:521-528; Thakker, ER., et al (2004) Proc. Natl. Acad. Sci. U.S.A. 101:17270-17275; Akaneya,Y., et al (2005) J. Neurophysiol. 93:594-602) and to the lungs by intranasal administration (Howard, KA., et al (2006) Mol. Ther. 14:476-484; Zhang, X., et al (2004) J. Biol. Chem. 279:10677-10684; Bitko, V., et al (2005) Nat. Med. 11:50-55). For administering an iRNA systemically for the treatment of a disease, the RNA can be modified or alternatively delivered using a drug delivery system; both methods act to prevent the rapid degradation of the dsRNA by endo- and exo-nucleases *in* vivo. Modification of the RNA or the pharmaceutical carrier can also permit targeting of the iRNA composition to the target tissue and avoid undesirable off-target effects. iRNA molecules can be modified by chemical conjugation to lipophilic groups such as cholesterol to enhance cellular uptake and prevent degradation. For example, an iRNA directed against ApoB conjugated to a lipophilic cholesterol moiety was injected systemically into mice and resulted in knockdown of apoB mRNA in both the liver and jejunum (Soutschek, J., et al (2004) Nature 432:173-178). Conjugation of an iRNA to an aptamer has been shown to inhibit tumor growth and mediate tumor regression in a mouse model of prostate cancer (McNamara, JO., et al (2006) Nat. Biotechnol. 24:1005-1015). In an alternative embodiment, the iRNA can be delivered using drug delivery systems such as a nanoparticle, a dendrimer, a polymer, liposomes, or a cationic delivery system. Positively charged cationic delivery systems facilitate binding of an iRNA molecule (negatively charged) and also enhance interactions at the negatively charged cell membrane to permit efficient uptake of an iRNA by the cell. Cationic lipids, dendrimers, or polymers can either be bound to an iRNA, or induced to form a vesicle or micelle (see *e.g.,* Kim SH., et al (2008) Journal of Controlled Release 129(2):107-116) that encases an iRNA. The formation of vesicles or micelles further prevents degradation of the iRNA when administered systemically. Methods for making and administering cationic- iRNA complexes are well within the abilities of one skilled in the art (see *e.g.;* Sorensen, DR., et al (2003) J. Mol. Biol 327:761-766; Verma, UN., et al (2003) Clin. Cancer Res. 9:1291-1300; Arnold, AS et al (2007) J. Hypertens. 25:197-205, which are incorporated herein by reference in their entirety). Some non-limiting examples of drug delivery systems useful for systemic delivery of iRNAs include DOTAP (Sorensen, DR., *et al* (2003), supra; Verma, UN., *et al* (2003), *supra*), Oligofectamine, "solid nucleic acid lipid particles" (Zimmermann, TS., et al (2006) Nature 441:111-114), cardiolipin (Chien, PY., et al (2005) Cancer Gene Ther. 12:321-328; Pal, A., et al (2005) Int J. Oncol. 26:1087-1091), polyethyleneimine (Bonnet ME., et al (2008) Pharm. Res. Aug 16 Epub ahead of print; Aigner, A. (2006) J. Biomed. Biotechnol. 71659), Arg-Gly-Asp (RGD) peptides (Liu, S. (2006) Mol. Pharm. 3:472-487), and polyamidoamines (Tomalia, DA., et al (2007) Biochem. Soc. Trans. 35:61-67; Yoo, H., et al (1999) Pharm. Res. 16:1799-1804). In some embodiments, an iRNA forms a complex with cyclodextrin for systemic administration. Methods for administration and pharmaceutical compositions of iRNAs and cyclodextrins can be found in U.S. Patent No. 7,427,605, which is herein incorporated by reference in its entirety.

### A. Vector encoded iRNAs of the Invention

iRNA targeting the C5 gene can be expressed from transcription units inserted into DNA or RNA vectors (see, *e.g.,* Couture, A, et al., TIG. (1996), 12:5-10; Skillern, A., et al., International PCT Publication No. WO 00/22113, Conrad, International PCT Publication No. WO 00/22114, and Conrad, U.S. Pat. No. 6,054,299). Expression can be transient (on the order of hours to weeks) or sustained (weeks to months or longer), depending upon the specific construct used and the target tissue or cell type. These transgenes can be introduced as a linear construct, a circular plasmid, or a viral vector, which can be an integrating or non-integrating vector. The transgene can also be constructed to permit it to be inherited as an extrachromosomal plasmid (Gassmann, et al., Proc. Natl. Acad. Sci. USA (1995) 92:1292).

The individual strand or strands of an iRNA can be transcribed from a promoter on an expression vector. Where two separate strands are to be expressed to generate, for example, a dsRNA, two separate expression vectors can be co-introduced *(e.g.,* by transfection or infection) into a target cell. Alternatively each individual strand of a dsRNA can be transcribed by promoters both of which are located on the same expression plasmid. In one embodiment, a dsRNA is expressed as inverted repeat polynucleotides joined by a linker polynucleotide sequence such that the dsRNA has a stem and loop structure.

iRNA expression vectors are generally DNA plasmids or viral vectors. Expression vectors compatible with eukaryotic cells, preferably those compatible with vertebrate cells, can be used to produce recombinant constructs for the expression of an iRNA as described herein. Eukaryotic cell expression vectors are well known in the art and are available from a number of commercial sources. Typically, such vectors are provided containing convenient restriction sites for insertion of the desired nucleic acid segment. Delivery of iRNA expressing vectors can be systemic, such as by intravenous or intramuscular administration, by administration to target cells ex-planted from the patient followed by reintroduction into the patient, or by any other means that allows for introduction into a desired target cell.

iRNA expression plasmids can be transfected into target cells as a complex with cationic lipid carriers *(e.g.,* Oligofectamine) or non-eationic lipid-based carriers *(e.g.,* Transit-TKO^{™}). Multiple lipid transfections for iRNA-mediated knockdowns targeting different regions of a target RNA over a period of a week or more are also contemplated by the invention. Successful introduction of vectors into host cells can be monitored using various known methods. For example, transient transfection can be signaled with a reporter, such as a fluorescent marker, such as Green Fluorescent Protein (GFP). Stable transfection of cells *ex vivo* can be ensured using markers that provide the transfected cell with resistance to specific environmental factors *(e.g.,* antibiotics and drugs), such as hygromycin B resistance.

Viral vector systems which can be utilized with the methods and compositions described herein include, but are not limited to, (a) adenovirus vectors; (b) retrovirus vectors, including but not limited to lentiviral vectors, moloney murine leukemia virus, *etc.;* (c) adeno- associated virus vectors; (d) herpes simplex virus vectors; (e) SV 40 vectors; (f) polyoma virus vectors; (g) papilloma virus vectors; (h) picornavirus vectors; (i) pox virus vectors such as an orthopox, *e.g.,* vaccinia virus vectors or avipox, *e.g.* canary pox or fowl pox; and (j) a helper-dependent or gutless adenovirus. Replication-defective viruses can also be advantageous. Different vectors will or will not become incorporated into the cells' genome. The constructs can include viral sequences for transfection, if desired. Alternatively, the construct can be incorporated into vectors capable of episomal replication, *e.g.* EPV and EBV vectors. Constructs for the recombinant expression of an iRNA will generally require regulatory elements, *e.g.,* promoters, enhancers, *etc.,* to ensure the expression of the iRNA in target cells. Other aspects to consider for vectors and constructs are further described below.

Vectors useful for the delivery of an iRNA will include regulatory elements (promoter, enhancer, *etc.)* sufficient for expression of the iRNA in the desired target cell or tissue. The regulatory elements can be chosen to provide either constitutive or regulated/inducible expression.

Expression of the iRNA can be precisely regulated, for example, by using an inducible regulatory sequence that is sensitive to certain physiological regulators, *e.g.,* circulating glucose levels, or hormones (Docherty et al., 1994, FASEB J. 8:20-24). Such inducible expression systems, suitable for the control of dsRNA expression in cells or in mammals include, for example, regulation by ecdysone, by estrogen, progesterone, tetracycline, chemical inducers of dimerization, and isopropyl-beta-D1 -thiogalactopyranoside (IPTG). A person skilled in the art would be able to choose the appropriate regulatory/promoter sequence based on the intended use of the iRNA transgene.

Viral vectors that contain nucleic acid sequences encoding an iRNA can be used. For example, a retroviral vector can be used (see Miller et al., Meth. Enzymol. 217:581-599 (1993)). These retroviral vectors contain the components necessary for the correct packaging of the viral genome and integration into the host cell DNA. The nucleic acid sequences encoding an iRNA are cloned into one or more vectors, which facilitate delivery of the nucleic acid into a patient. More detail about retroviral vectors can be found, for example, in Boesen et al., Biotherapy 6:291-302 (1994), which describes the use of a retroviral vector to deliver the mdr1 gene to hematopoietic stem cells in order to make the stem cells more resistant to chemotherapy. Other references illustrating the use of retroviral vectors in gene therapy are: Clowes et al., J. Clin. Invest. 93:644-651 (1994); Kiem et al., Blood 83:1467-1473 (1994); Salmons and Gunzberg, Human Gene Therapy 4:129-141 (1993); and Grossman and Wilson, Curr. Opin. in Genetics and Devel. 3:110-114 (1993). Lentiviral vectors contemplated for use include, for example, the HIV based vectors described in U.S. Patent Nos. 6,143,520; 5,665,557; and 5,981,276.

Adenoviruses are also contemplated for use in delivery of iRNAs of the invention. Adenoviruses are especially attractive vehicles, *e.g.,* for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, Current Opinion in Genetics and Development 3:499-503 (1993) present a review of adenovirus-based gene therapy. Bout et al., Human Gene Therapy 5:3-10 (1994) demonstrated the use of adenovirus vectors to transfer genes to the respiratory epithelia of rhesus monkeys. Other instances of the use of adenoviruses in gene therapy can be found in Rosenfeld et al., Science 252:431-434 (1991); Rosenfeld et al., Cell 68:143-155 (1992); Mastrangeli et al., J. Clin. Invest. 91:225-234 (1993); PCT Publication WO94/12649; and Wang, et al., Gene Therapy 2:775-783 (1995). A suitable AV vector for expressing an iRNA featured in the invention, a method for constructing the recombinant AV vector, and a method for delivering the vector into target cells, are described in Xia H et al. (2002), Nat. Biotech. 20: 1006-1010.

Adeno-associated virus (AAV) vectors may also be used to delivery an iRNA of the invention (Walsh et al., Proc. Soc. Exp. Biol. Med. 204:289-300 (1993); U.S. Pat. No. 5,436,146). In one embodiment, the iRNA can be expressed as two separate, complementary single-stranded RNA molecules from a recombinant AAV vector having, for example, either the U6 or H1 RNA promoters, or the cytomegalovirus (CMV) promoter. Suitable AAV vectors for expressing the dsRNA featured in the invention, methods for constructing the recombinant AV vector, and methods for delivering the vectors into target cells are described in Samulski R et al. (1987), J. Virol. 61: 3096-3101; Fisher K J et al. (1996), J. Virol, 70: 520-532; Samulski R et al. (1989), J. Virol. 63: 3822-3826; U.S. Pat. No. 5,252,479; U.S. Pat. No. 5,139,941; International Patent Application No. WO 94/13788; and International Patent Application No. WO 93/24641.

Another viral vector suitable for delivery of an iRNA of the inevtion is a pox virus such as a vaccinia virus, for example an attenuated vaccinia such as Modified Virus Ankara (MVA) or NYVAC, an avipox such as fowl pox or canary pox.

The tropism of viral vectors can be modified by pseudotyping the vectors with envelope proteins or other surface antigens from other viruses, or by substituting different viral capsid proteins, as appropriate. For example, lentiviral vectors can be pseudotyped with surface proteins from vesicular stomatitis virus (VSV), rabies, Ebola, Mokola, and the like. AAV vectors can be made to target different cells by engineering the vectors to express different capsid protein serotypes; see, *e.g.,* Rabinowitz J E et al. (2002), J Virol 76:791-801, the entire disclosure of which is herein incorporated by reference.

The pharmaceutical preparation of a vector can include the vector in an acceptable diluent, or can include a slow release matrix in which the gene delivery vehicle is imbedded. Alternatively, where the complete gene delivery vector can be produced intact from recombinant cells, *e.g.,* retroviral vectors, the pharmaceutical preparation can include one or more cells which produce the gene delivery system.

### VI. Pharmaceutical Compositions of the Invention

The present invention also includes pharmaceutical compositions and formulations which include the iRNAs of the invention. In one embodiment, provided herein are pharmaceutical compositions containing an iRNA, as described herein, and a pharmaceutically acceptable carrier. The pharmaceutical compositions containing the iRNA are useful for treating a disease or disorder associated with the expression or activity of a KHK gene. Such pharmaceutical compositions are formulated based on the mode of delivery. One example is compositions that are formulated for systemic administration *via* parenteral delivery, *e.g.,* by subcutaneous (SC) or intravenous (IV) delivery. Another example is compositions that are formulated for direct delivery into the brain parenchyma, *e.g.,* by infusion into the brain, such as by continuous pump infusion. The pharmaceutical compositions of the invention may be administered in dosages sufficient to inhibit expression of a KHK gene.

The pharmaceutical composition can be administered by intravenous infusion over a period of time, such as over a 5, 6, 7, 8, 9, 10, 11, 12, 13, 14, 15, 16, 17, 18, 19, 20, and 21, 22, 23, 24, or about a 25 minute period. The administration may be repeated, for example, on a regular basis, such as weekly, biweekly *(i.e.,* every two weeks) for one month, two months, three months, four months or longer. After an initial treatment regimen, the treatments can be administered on a less frequent basis. For example, after administration weekly or biweekly for three months, administration can be repeated once per month, for six months or a year or longer.

The pharmaceutical composition can be administered once daily, or the iRNA can be administered as two, three, or more sub-doses at appropriate intervals throughout the day or even using continuous infusion or delivery through a controlled release formulation. In that case, the iRNA contained in each sub-dose must be correspondingly smaller in order to achieve the total daily dosage. The dosage unit can also be compounded for delivery over several days, *e.g.,* using a conventional sustained release formulation which provides sustained release of the iRNA over a several day period. Sustained release formulations are well known in the art and are particularly useful for delivery of agents at a particular site, such as could be used with the agents of the present invention. In this case, the dosage unit contains a corresponding multiple of the daily dose.

In other cases, a single dose of the pharmaceutical compositions can be long lasting, such that subsequent doses are administered at not more than 3, 4, or 5 day intervals, or at not more than 1, 2, 3, or 4 week intervals. In some cases, a single dose of the pharmaceutical compositions of the invention is administered once per week. In other cases, a single dose of the pharmaceutical compositions of the invention is administered bi-monthly.

The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and *in vivo* half-lives for the individual iRNAs encompassed by the invention can be made using conventional methodologies or on the basis of *in vivo* testing using an appropriate animal model, as described elsewhere herein.

The pharmaceutical compositions of the present invention can be administered in a number of ways depending upon whether local or systemic treatment is desired and upon the area to be treated. Administration can be topical *(e.g.,* by a transdermal patch), pulmonary, *e.g.,* by inhalation or insufflation of powders or aerosols, including by nebulizer; intratracheal, intranasal, epidermal and transdermal, oral or parenteral. Parenteral administration includes intravenous, intraarterial, subcutaneous, intraperitoneal or intramuscular injection or infusion; subdermal, *e.g.,* via an implanted device; or intracranial, *e.g.,* by intraparenchymal, intrathecal or intraventricular, administration.

The iRNA can be delivered in a manner to target a particular tissue, such as the liver *(e.g.,* the hepatocytes of the liver).

Pharmaceutical compositions and formulations for topical administration can include transdermal patches, ointments, lotions, creams, gels, drops, suppositories, sprays, liquids and powders. Conventional pharmaceutical carriers, aqueous, powder or oily bases, thickeners and the like can be necessary or desirable. Coated condoms, gloves and the like can also be useful. Suitable topical formulations include those in which the iRNAs featured in the invention are in admixture with a topical delivery agent such as lipids, liposomes, fatty acids, fatty acid esters, steroids, chelating agents and surfactants. Suitable lipids and liposomes include neutral *(e.g.,* dioleoylphosphatidyl DOPE ethanolamine, dimyristoylphosphatidyl choline DMPC, distearolyphosphatidyl choline) negative *(e.g.,* dimyristoylphosphatidyl glycerol DMPG) and cationic *(e.g.,* dioleoyltetramethylaminopropyl DOTAP and dioleoylphosphatidyl ethanolamine DOTMA). iRNAs featured in the invention can be encapsulated within liposomes or can form complexes thereto, in particular to cationic liposomes. Alternatively, iRNAs can be complexed to lipids, in particular to cationic lipids. Suitable fatty acids and esters include but are not limited to arachidonic acid, oleic acid, eicosanoic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a C₁₋₂₀ alkyl ester *(e.g.,* isopropylmyristate IPM), monoglyceride, diglyceride or pharmaceutically acceptable salt thereof). Topical formulations are described in detail in U.S. Patent No. 6,747,014.

### A. iRNA Formulations Comprising Membranous Molecular Assemblies

An iRNA for use in the compositions and methods can be formulated for delivery in a membranous molecular assembly, *e.g.,* a liposome or a micelle. As used herein, the term "liposome" refers to a vesicle composed of amphiphilic lipids arranged in at least one bilayer, *e.g.,* one bilayer or a plurality of bilayers. Liposomes include unilamellar and multilamellar vesicles that have a membrane formed from a lipophilic material and an aqueous interior. The aqueous portion contains the iRNA composition. The lipophilic material isolates the aqueous interior from an aqueous exterior, which typically does not include the iRNA composition, although in some examples, it may. Liposomes are useful for the transfer and delivery of active ingredients to the site of action. Because the liposomal membrane is structurally similar to biological membranes, when liposomes are applied to a tissue, the liposomal bilayer fuses with bilayer of the cellular membranes. As the merging of the liposome and cell progresses, the internal aqueous contents that include the iRNA are delivered into the cell where the iRNA can specifically bind to a target RNA and can mediate iRNA. In some cases the liposomes are also specifically targeted, *e.g.,* to direct the iRNA to particular cell types.

A liposome containing a iRNA agent can be prepared by a variety of methods. In one example, the lipid component of a liposome is dissolved in a detergent so that micelles are formed with the lipid component. For example, the lipid component can be an amphipathic cationic lipid or lipid conjugate. The detergent can have a high critical micelle concentration and may be nonionic. Exemplary detergents include cholate, CHAPS, octylglucoside, deoxycholate, and lauroyl sarcosine. The iRNA agent preparation is then added to the micelles that include the lipid component. The cationic groups on the lipid interact with the iRNA agent and condense around the iRNA agent to form a liposome. After condensation, the detergent is removed, *e.g.,* by dialysis, to yield a liposomal preparation of iRNA agent.

If necessary a carrier compound that assists in condensation can be added during the condensation reaction, *e.g.,* by controlled addition. For example, the carrier compound can be a polymer other than a nucleic acid *(e.g.,* spermine or spermidine). pH can also adjusted to favor condensation.

Methods for producing stable polynucleotide delivery vehicles, which incorporate a polynucleotide/cationic lipid complex as structural components of the delivery vehicle, are further described in, e.g., WO 96/37194. Liposome formation can also include one or more aspects of exemplary methods described in Felgner, P. L. et al., Proc. Natl. Acad. Sci., USA 8:7413-7417, 1987; U.S. Pat. No. 4,897,355; U.S. Pat. No. 5,171,678; Bangham, et al. M. Mol. Biol. 23:238, 1965; Olson, et al. Biochim. Biophys. Acta 557:9, 1979; Szoka, et al. Proc. Natl. Acad. Sci. 75: 4194, 1978; Mayhew, et al. Biochim. Biophys. Acta 775:169, 1984; Kim, et al. Biochim. Biophys. Acta 728:339, 1983; and Fukunaga, et al. Endocrinol. 115:757, 1984. Commonly used techniques for preparing lipid aggregates of appropriate size for use as delivery vehicles include sonication and freeze-thaw plus extrusion (see, *e.g.,* Mayer, et al. Biochim. Biophys. Acta 858:161, 1986). Microfluidization can be used when consistently small (50 to 200 nm) and relatively uniform aggregates are desired (Mayhew, et al. Biochim. Biophys. Acta 775:169, 1984). These methods are readily adapted to packaging iRNA agent preparations into liposomes.

Liposomes fall into two broad classes. Cationic liposomes are positively charged liposomes which interact with the negatively charged nucleic acid molecules to form a stable complex. The positively charged nucleic acid/liposome complex binds to the negatively charged cell surface and is internalized in an endosome. Due to the acidic pH within the endosome, the liposomes are ruptured, releasing their contents into the cell cytoplasm (Wang et al., Biochem. Biophys. Res. Commun., 1987, 147, 980-985).

Liposomes which are pH-sensitive or negatively-charged, entrap nucleic acids rather than complex with it. Since both the nucleic acid and the lipid are similarly charged, repulsion rather than complex formation occurs. Nevertheless, some nucleic acid is entrapped within the aqueous interior of these liposomes. pH-sensitive liposomes have been used to deliver nucleic acids encoding the thymidine kinase gene to cell monolayers in culture. Expression of the exogenous gene was detected in the target cells (Zhou et al., Journal of Controlled Release, 1992, 19, 269-274).

One major type of liposomal composition includes phospholipids other than naturally-derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Examples of other methods to introduce liposomes into cells *in vitro* and *in vivo* include U.S. Pat. No. 5,283,185; U.S. Pat. No. 5,171,678; WO 94/00569; WO 93/24640; WO 91/16024; Feigner, J. Biol. Chem. 269:2550, 1994; Nabel, Proc. Natl. Acad. Sci. 90:11307, 1993; Nabel, Human Gene Ther. 3:649, 1992; Gershon, Biochem. 32:7143, 1993; and Strauss EMBO J. 11:417, 1992.

Non-ionic liposomal systems have also been examined to determine their utility in the delivery of drugs to the skin, in particular systems comprising non-ionic surfactant and cholesterol. Non-ionic liposomal formulations comprising Novasome^{™} I (glyceryl dilaurate/cholesterol/polyoxyethylene-10-stearyl ether) and Novasome^{™} II (glyceryl distearate/cholesterol/polyoxyethylene-10-stearyl ether) were used to deliver cyclosporin-A into the dermis of mouse skin. Results indicated that such non-ionic liposomal systems were effective in facilitating the deposition of cyclosporine A into different layers of the skin (Hu et al. S.T.P.Pharma. Sci., 1994, 4(6) 466).

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome (A) comprises one or more glycolipids, such as monosialoganglioside G_{M1}, or (B) is derivatized with one or more hydrophilic polymers, such as a polyethylene glycol (PEG) moiety. While not wishing to be bound by any particular theory, it is thought in the art that, at least for sterically stabilized liposomes containing gangliosides, sphingomyelin, or PEG-derivatized lipids, the enhanced circulation half-life of these sterically stabilized liposomes derives from a reduced uptake into cells of the reticuloendothelial system (RES) (Allen et al., FEBS Letters, 1987, 223, 42; Wu et al., Cancer Research, 1993, 53, 3765).

Various liposomes comprising one or more glycolipids are known in the art. Papahadjopoulos et al. (Ann. N.Y. Acad. Sci., 1987, 507, 64) reported the ability of monosialoganglioside G_{M1}, galactocerebroside sulfate and phosphatidylinositol to improve blood half-lives of liposomes. These findings were expounded upon by Gabizon et al. (Proc. Natl. Acad. Sci. U.S.A., 1988, 85, 6949). U.S. Pat. No. 4,837,028 and WO 88/04924, both to Allen et al.*,* disclose liposomes comprising (1) sphingomyelin and (2) the ganglioside G_{M1} or a galactocerebroside sulfate ester. U.S. Pat. No. 5,543,152 (Webb et al.) discloses liposomes comprising sphingomyelin. Liposomes comprising 1,2-sn-dimyristoylphosphatidylcholine are disclosed in WO 97/13499 (Lim et al).

In one case, cationic liposomes are used. Cationic liposomes possess the advantage of being able to fuse to the cell membrane. Non-cationic liposomes, although not able to fuse as efficiently with the plasma membrane, are taken up by macrophages *in vivo* and can be used to deliver iRNA agents to macrophages.

Further advantages of liposomes include: liposomes obtained from natural phospholipids are biocompatible and biodegradable; liposomes can incorporate a wide range of water and lipid soluble drugs; liposomes can protect encapsulated iRNA agents in their internal compartments from metabolism and degradation (Rosoff, in "Pharmaceutical Dosage Forms," Lieberman, Rieger and Banker (Eds.), 1988, volume 1, p. 245). Important considerations in the preparation of liposome formulations are the lipid surface charge, vesicle size and the aqueous volume of the liposomes.

A positively charged synthetic cationic lipid, N-[1-(2,3-dioleyloxy)propyl]-N,N,N-trimethylammonium chloride (DOTMA) can be used to form small liposomes that interact spontaneously with nucleic acid to form lipid-nucleic acid complexes which are capable of fusing with the negatively charged lipids of the cell membranes of tissue culture cells, resulting in delivery of iRNA agent (see, e.g., Felgner, P. L. et al., Proc. Natl. Acad. Sci., USA 8:7413-7417, 1987 and U.S. Pat. No. 4,897,355 for a description of DOTMA and its use with DNA).

A DOTMA analogue, 1,2-bis(oleoyloxy)-3-(trimethylammonia)propane (DOTAP) can be used in combination with a phospholipid to form DNA-complexing vesicles. Lipofectin^{™} Bethesda Research Laboratories, Gaithersburg, Md.) is an effective agent for the delivery of highly anionic nucleic acids into living tissue culture cells that comprise positively charged DOTMA liposomes which interact spontaneously with negatively charged polynucleotides to form complexes. When enough positively charged liposomes are used, the net charge on the resulting complexes is also positive. Positively charged complexes prepared in this way spontaneously attach to negatively charged cell surfaces, fuse with the plasma membrane, and efficiently deliver functional nucleic acids into, for example, tissue culture cells. Another commercially available cationic lipid, 1,2-bis(oleoyloxy)-3,3-(trimethylammonia)propane ("DOTAP") (Boehringer Mannheim, Indianapolis, Indiana) differs from DOTMA in that the oleoyl moieties are linked by ester, rather than ether linkages.

Other reported cationic lipid compounds include those that have been conjugated to a variety of moieties including, for example, carboxyspermine which has been conjugated to one of two types of lipids and includes compounds such as 5-carboxyspermylglycine dioctaoleoylamide ("DOGS") (Transfectam^{™}, Promega, Madison, Wisconsin) and dipalmitoylphosphatidylethanolamine 5-carboxyspermyl-amide ("DPPES") (see, e.g., U.S. Pat. No. 5,171,678).

Another cationic lipid conjugate includes derivatization of the lipid with cholesterol ("DC-Chol") which has been formulated into liposomes in combination with DOPE (See, Gao, X. and Huang, L., Biochim. Biophys. Res. Commun. 179:280, 1991). Lipopolylysine, made by conjugating polylysine to DOPE, has been reported to be effective for transfection in the presence of serum (Zhou, X. et al., Biochim. Biophys. Acta 1065:8, 1991). For certain cell lines, these liposomes containing conjugated cationic lipids, are said to exhibit lower toxicity and provide more efficient transfection than the DOTMA-containing compositions. Other commercially available cationic lipid products include DMRIE and DMRIE-HP (Vical, La Jolla, California) and Lipofectamine (DOSPA) (Life Technology, Inc., Gaithersburg, Maryland). Other cationic lipids suitable for the delivery of oligonucleotides are described in WO 98/39359 and WO 96/37194.

Liposomal formulations are particularly suited for topical administration, liposomes present several advantages over other formulations. Such advantages include reduced side effects related to high systemic absorption of the administered drug, increased accumulation of the administered drug at the desired target, and the ability to administer iRNA agent into the skin. In some implementations, liposomes are used for delivering iRNA agent to epidermal cells and also to enhance the penetration of iRNA agent into dermal tissues, *e.g.,* into skin. For example, the liposomes can be applied topically. Topical delivery of drugs formulated as liposomes to the skin has been documented (see, *e.g.,* Weiner et al., Journal of Drug Targeting, 1992, vol. 2,405-410 and du Plessis et al., Antiviral Research, 18, 1992, 259-265; Mannino, R. J. and Fould-Fogerite, S., Biotechniques 6:682-690, 1988; Itani, T. et al. Gene 56:267-276. 1987; Nicolau, C. et al. Meth. Enz. 149:157-176, 1987; Straubinger, R. M. and Papahadjopoulos, D. Meth. Enz. 101:512-527, 1983; Wang, C. Y. and Huang, L., Proc. Natl. Acad. Sci. USA 84:7851-7855, 1987).

Non-ionic liposomal systems have also been examined to determine their utility in the delivery of drugs to the skin, in particular systems comprising non-ionic surfactant and cholesterol. Non-ionic liposomal formulations comprising Novasome I (glyceryl dilaurate/cholesterol/polyoxyethylene-10-stearyl ether) and Novasome II (glyceryl distearate/ cholesterol/polyoxyethylene-10-stearyl ether) were used to deliver a drug into the dermis of mouse skin. Such formulations with iRNA agent are useful for treating a dermatological disorder.

Liposomes that include iRNA can be made highly deformable. Such deformability can enable the liposomes to penetrate through pore that are smaller than the average radius of the liposome. For example, transfersomes are a type of deformable liposomes. Transferosomes can be made by adding surface edge activators, usually surfactants, to a standard liposomal composition. Transfersomes that include iRNA agent can be delivered, for example, subcutaneously by infection in order to deliver iRNA agent to keratinocytes in the skin. In order to cross intact mammalian skin, lipid vesicles must pass through a series of fine pores, each with a diameter less than 50 nm, under the influence of a suitable transdermal gradient. In addition, due to the lipid properties, these transferosomes can be self-optimizing (adaptive to the shape of pores, *e.g.,* in the skin), self-repairing, and can frequently reach their targets without fragmenting, and often self-loading.

Other formulations amenable to the present invention are described in United States provisional application serial Nos. 61/018,616, filed January 2, 2008; 61/018,611, filed January 2, 2008; 61/039,748, filed March 26, 2008; 61/047,087, filed April 22, 2008 and 61/051,528, filed May 8, 2008. PCT application no PCT/US2007/080331, filed October 3, 2007 also describes formulations that are amenable to the present invention.

Transfersomes are yet another type of liposomes, and are highly deformable lipid aggregates which are attractive candidates for drug delivery vehicles. Transfersomes can be described as lipid droplets which are so highly deformable that they are easily able to penetrate through pores which are smaller than the droplet. Transfersomes are adaptable to the environment in which they are used, *e.g.,* they are self-optimizing (adaptive to the shape of pores in the skin), self-repairing, frequently reach their targets without fragmenting, and often self-loading. To make transfersomes it is possible to add surface edge-activators, usually surfactants, to a standard liposomal composition. Transfersomes have been used to deliver serum albumin to the skin. The transfersome-mediated delivery of serum albumin has been shown to be as effective as subcutaneous injection of a solution containing serum albumin.

Surfactants find wide application in formulations such as emulsions (including microemulsions) and liposomes. The most common way of classifying and ranking the properties of the many different types of surfactants, both natural and synthetic, is by the use of the hydrophile/lipophile balance (HLB). The nature of the hydrophilic group (also known as the "head") provides the most useful means for categorizing the different surfactants used in formulations (Rieger, in "Pharmaceutical Dosage Forms", Marcel Dekker, Inc., New York, N.Y., 1988, p. 285).

If the surfactant molecule is not ionized, it is classified as a nonionic surfactant. Nonionic surfactants find wide application in pharmaceutical and cosmetic products and are usable over a wide range of pH values. In general their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides.

The use of surfactants in drug products, formulations and in emulsions has been reviewed (Rieger, in "Pharmaceutical Dosage Forms", Marcel Dekker, Inc., New York, N.Y., 1988, p. 285).

The iRNA for use in the methods of the invention can also be provided as micellar formulations. "Micelles" are defined herein as a particular type of molecular assembly in which amphipathic molecules are arranged in a spherical structure such that all the hydrophobic portions of the molecules are directed inward, leaving the hydrophilic portions in contact with the surrounding aqueous phase. The converse arrangement exists if the environment is hydrophobic.

A mixed micellar formulation suitable for delivery through transdermal membranes may be prepared by mixing an aqueous solution of the siRNA composition, an alkali metal C₈ to C₂₂ alkyl sulphate, and a micelle forming compounds. Exemplary micelle forming compounds include lecithin, hyaluronic acid, pharmaceutically acceptable salts of hyaluronic acid, glycolic acid, lactic acid, chamomile extract, cucumber extract, oleic acid, linoleic acid, linolenic acid, monoolein, monooleates, monolaurates, borage oil, evening of primrose oil, menthol, trihydroxy oxo cholanyl glycine and pharmaceutically acceptable salts thereof, glycerin, polyglycerin, lysine, polylysine, triolein, polyoxyethylene ethers and analogues thereof, polidocanol alkyl ethers and analogues thereof, chenodeoxycholate, deoxycholate, and mixtures thereof. The micelle forming compounds may be added at the same time or after addition of the alkali metal alkyl sulphate. Mixed micelles will form with substantially any kind of mixing of the ingredients but vigorous mixing in order to provide smaller size micelles.

In one method a first micellar composition is prepared which contains the siRNA composition and at least the alkali metal alkyl sulphate. The first micellar composition is then mixed with at least three micelle forming compounds to form a mixed micellar composition. In another method, the micellar composition is prepared by mixing the siRNA composition, the alkali metal alkyl sulphate and at least one of the micelle forming compounds, followed by addition of the remaining micelle forming compounds, with vigorous mixing.

Phenol and/or m-cresol may be added to the mixed micellar composition to stabilize the formulation and protect against bacterial growth. Alternatively, phenol and/or m-cresol may be added with the micelle forming ingredients. An isotonic agent such as glycerin may also be added after formation of the mixed micellar composition.

For delivery of the micellar formulation as a spray, the formulation can be put into an aerosol dispenser and the dispenser is charged with a propellant. The propellant, which is under pressure, is in liquid form in the dispenser. The ratios of the ingredients are adjusted so that the aqueous and propellant phases become one, *i.e.,* there is one phase. If there are two phases, it is necessary to shake the dispenser prior to dispensing a portion of the contents, *e.g.,* through a metered valve. The dispensed dose of pharmaceutical agent is propelled from the metered valve in a fine spray.

Propellants may include hydrogen-containing chlorofluorocarbons, hydrogen-containing fluorocarbons, dimethyl ether and diethyl ether. In certain embodiments, HFA 134a (1,1,1,2 tetrafluoroethane) may be used.

The specific concentrations of the essential ingredients can be determined by relatively straightforward experimentation. For absorption through the oral cavities, it is often desirable to increase, *e.g.,* at least double or triple, the dosage for through injection or administration through the gastrointestinal tract.

### B. Lipid particles

iRNAs, *e.g.,* dsRNAs of in the invention may be fully encapsulated in a lipid formulation, *e.g.,* a LNP, or other nucleic acid-lipid particle.

As used herein, the term "LNP" refers to a stable nucleic acid-lipid particle. LNPs typically contain a cationic lipid, a non-cationic lipid, and a lipid that prevents aggregation of the particle (*e.g.,* a PEG-lipid conjugate). LNPs are extremely useful for systemic applications, as they exhibit extended circulation lifetimes following intravenous (i.v.) injection and accumulate at distal sites (*e.g.,* sites physically separated from the administration site). LNPs include "pSPLP," which include an encapsulated condensing agent-nucleic acid complex as set forth in PCT Publication No. WO 00/03683. The particles of the present invention typically have a mean diameter of about 50 nm to about 150 nm, more typically about 60 nm to about 130 nm, more typically about 70 nm to about 110 nm, most typically about 70 nm to about 90 nm, and are substantially nontoxic. In addition, the nucleic acids when present in the nucleic acid- lipid particles of the present invention are resistant in aqueous solution to degradation with a nuclease. Nucleic acid-lipid particles and their method of preparation are disclosed in, *e.g.,* U.S. Patent Nos. 5,976,567; 5,981,501; 6,534,484; 6,586,410; 6,815,432; U.S. Publication No. 2010/0324120 and PCT Publication No. WO 96/40964.

In one case, the lipid to drug ratio (mass/mass ratio) *(e.g.,* lipid to dsRNA ratio) will be in the range of from about 1:1 to about 50:1, from about 1:1 to about 25:1, from about 3:1 to about 15:1, from about 4:1 to about 10:1, from about 5:1 to about 9:1, or about 6:1 to about 9:1. Ranges intermediate to the above recited ranges are also contemplated.

The cationic lipid can be, for example, N,N-dioleyl-N,N-dimethylammonium chloride (DODAC), N,N-distearyl-N,N-dimethylammonium bromide (DDAB), N-(I -(2,3-dioleoyloxy)propyl)-N,N,N-trimethylammonium chloride (DOTAP), N-(I -(2,3-dioleyloxy)propyl)-N,N,N-trimethylammonium chloride (DOTMA), N,N-dimethyl-2,3-dioleyloxy)propylamine (DODMA), 1,2-DiLinoleyloxy-N,N-dimethylaminopropane (DLinDMA), 1,2-Dilinolenyloxy-N,N-dimethylaminopropane (DLenDMA), 1,2-Dilinoleylcarbamoyloxy-3-dimethylaminopropane (DLin-C-DAP), 1,2-Dilinoleyoxy-3-(dimethylamino)acetoxypropane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S-DMA), 1-Linoleoyl-2-linoleyloxy-3-dimethylaminopropane (DLin-2-DMAP), 1,2-Dilinoleyloxy-3-trimethylaminopropane chloride salt (DLin-TMA.Cl), 1,2-Dilinoleoyl-3-trimethylaminopropane chloride salt (DLin-TAP.Cl), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N-dimethylamino)ethoxypropane (DLin-EG-DMA), 1,2-Dilinolenyloxy-N,N-dimethylaminopropane (DLinDMA), 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech G1), or a mixture thereof. The cationic lipid can comprise from about 20 mol % to about 50 mol % or about 40 mol % of the total lipid present in the particle.

In another case, the compound 2,2-Dilinoleyl-4-dimethylaminoethyl-[1,3]-dioxolane can be used to prepare lipid-siRNA nanoparticles. Synthesis of 2,2-Dilinoleyl-4-dimethylaminoethyl-[1,3]-dioxolane is described in United States provisional patent application number 61/107,998 filed on October 23, 2008.

In one case, the lipid-siRNA particle includes 40% 2, 2-Dilinoleyl-4-dimethylaminoethyl-[1,3]-dioxolane: 10% DSPC: 40% Cholesterol: 10% PEG-C-DOMG (mole percent) with a particle size of 63.0 ± 20 nm and a 0.027 siRNA/Lipid Ratio.

The ionizable/non-cationic lipid can be an anionic lipid or a neutral lipid including, but not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoylphosphatidylethanolamine (POPE), dioleoyl- phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1 -trans PE, 1 -stearoyl-2-oleoyl- phosphatidyethanolamine (SOPE), cholesterol, or a mixture thereof. The non-cationic lipid can be from about 5 mol % to about 90 mol %, about 10 mol %, or about 58 mol % if cholesterol is included, of the total lipid present in the particle.

The conjugated lipid that inhibits aggregation of particles can be, for example, a polyethyleneglycol (PEG)-lipid including, without limitation, a PEG-diacylglycerol (DAG), a PEG-dialkyloxypropyl (DAA), a PEG-phospholipid, a PEG-ceramide (Cer), or a mixture thereof. The PEG-DAA conjugate can be, for example, a PEG-dilauryloxypropyl (Ci₂), a PEG-dimyristyloxypropyl (Ci₄), a PEG-dipalmityloxypropyl (Ci₆), or a PEG- distearyloxypropyl (C]₈). The conjugated lipid that prevents aggregation of particles can be from 0 mol % to about 20 mol % or about 2 mol % of the total lipid present in the particle.

In some cases, the nucleic acid-lipid particle further includes cholesterol at, *e.g.,* about 10 mol % to about 60 mol % or about 48 mol % of the total lipid present in the particle.

In one case, the lipidoid ND98·4HCl (MW 1487) (see U.S. Patent Application No. 12/056,230, filed 3/26/2008, Cholesterol (Sigma-Aldrich), and PEG-Ceramide C16 (Avanti Polar Lipids) can be used to prepare lipid-dsRNA nanoparticles *(i.e.,* LNP01 particles). Stock solutions of each in ethanol can be prepared as follows: ND98, 133 mg/ml; Cholesterol, 25 mg/ml, PEG-Ceramide C16, 100 mg/ml. The ND98, Cholesterol, and PEG-Ceramide C16 stock solutions can then be combined in a, *e.g.,* 42:48:10 molar ratio. The combined lipid solution can be mixed with aqueous dsRNA *(e.g.,* in sodium acetate pH 5) such that the final ethanol concentration is about 35-45% and the final sodium acetate concentration is about 100-300 mM. Lipid-dsRNA nanoparticles typically form spontaneously upon mixing. Depending on the desired particle size distribution, the resultant nanoparticle mixture can be extruded through a polycarbonate membrane *(e.g.,* 100 nm cut-off) using, for example, a thermobarrel extruder, such as Lipex Extruder (Northern Lipids, Inc). In some cases, the extrusion step can be omitted. Ethanol removal and simultaneous buffer exchange can be accomplished by, for example, dialysis or tangential flow filtration. Buffer can be exchanged with, for example, phosphate buffered saline (PBS) at about pH 7, *e.g.,* about pH 6.9, about pH 7.0, about pH 7.1, about pH 7.2, about pH 7.3, or about pH 7.4.

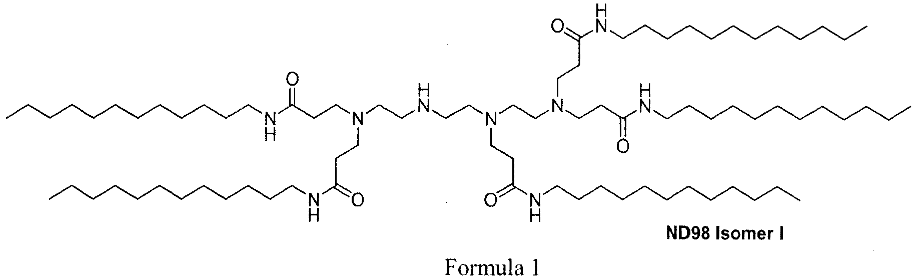

LNP01 formulations are described, *e.g.,* in International Application Publication No. WO 2008/042973.

Additional exemplary lipid-dsRNA formulations are described in Table 1.

**Table 1**

| | **Ionizable/Cationic Lipid** | **cationic lipid/non-cationic lipid/ cholesterol/PEG-lipid conjugate Lipid:siRNA ratio** |
|---|---|---|
| SNALP-1 | 1,2-Dilinolenyloxy-N,N-dimethylaminopropane (DLinDMA) | DLinDMA/DPPC/Cholesterol/PEG-cDMA |
| | | (57.1/7.1/34.4/1.4) |
| | | lipid:siRNA ~ 7:1 |
| 2-XTC | 2,2-Dilinoleyl-4-dimethylaminoethyl-[1,3]-dioxolane (XTC) | XTC/DPPC/Cholesterol/PEG-cD MA |
| | | 57.1/7.1/34.4/1.4 |
| | | lipid:siRNA ∼ 7:1 |
| LNP05 | 2,2-Dilinoleyl-4-dimethylaminoethyl-[1,3]-dioxolane (XTC) | XTC/DSPC/Cholesterol/PEG-DMG |
| | | 57.5/7.5/31.5/3.5 |
| | | lipid:siRNA ~ 6:1 |
| LNP06 | 2,2-Dilinoleyl-4-dimethylaminoethyl-[1,3]-dioxolane (XTC) | XTC/DSPC/Cholesterol/PEG-DMG |
| | | 57.5/7.5/31.5/3.5 |
| | | lipid:siRNA ~ 11:1 |
| LNP07 | 2,2-Dilinoleyl-4-dimethylaminoethyl-[1,3]-dioxolane (XTC) | XTC/DSPC/Cholesterol/PEG-DMG |
| | | 60/7.5/31/1.5, lipid:siRNA ~ 6:1 |
| LNP08 | 2,2-Dilinoleyl-4-dimethylaminoethyl-[1,3]-dioxolane (XTC) | XTC/DSPC/Cholesterol/PEG-DMG |
| | | 60/7.5/31/1.5, |
| | | lipid:siRNA ~ 11: 1 |
| LNP09 | 2,2-Dilinoleyl-4-dimethylaminoethyl-[1,3]-dioxolane (XTC) | XTC/DSPC/Cholesterol/PEG-DMG |
| | | 50/10/38.5/1.5 |
| | | Lipid:siRNA 10:1 |
| LNP10 | (3aR,5s,6aS)-N,N-dimethyl-2,2-di((9Z, 12Z)-octadeca-9,12-dienyl)tetrahydro-3aH-cyclopenta[d][1,3]dioxol-5-amine (ALN100) | ALN100/DSPC/Cholesterol/PEG-DMG |
| | | 50/10/38.5/1.5 |
| | | Lipid:siRNA 10:1 |
| LNP11 | (6Z,9Z,28Z,31Z)-heptatriaconta-6,9,28,31-tetraen-19-yl 4-(dimethylamino)butanoate (MC3) | MC-3/DSPC/Cholesterol/PEG-DMG |
| | | 50/10/38.5/1.5 |
| | | Lipid:siRNA 10:1 |
| LNP12 | 1,1'-(2-(4-(2-((2-(bis(2-hydroxydodecyl)amino)ethyl)(2-hydroxydodecyl)amino)ethyl)piperazin-1-yl)ethylazanediyl) didodecan-2-ol (Tech G1) | Tech G1/DSPC/Cholesterol/PEG-DMG |
| | | 50/10/38.5/1.5 |
| | | Lipid: siRNA 10:1 |
| LNP13 | XTC | XTC/DSPC/Chol/PEG-DMG |
| | | 50/10/38.5/1.5 |
| | | Lipid:siRNA: 33:1 |
| LNP14 | MC3 | MC3/DSPC/Chol/PEG-DMG |
| | | 40/15/40/5 |
| | | Lipid:siRNA: 11:1 |
| LNP15 | MC3 | MC3/DSPC/Chol/PEG-DSG/ |
| | | GalNAc-PEG-DSG |
| | | 50/10/35/4.5/0.5 |
| | | Lipid:siRNA: 11:1 |
| LNP16 | MC3 | MC3/DSPC/Chol/PEG-DMG |
| | | 50/10/38.5/1.5 |
| | | Lipid:siRNA: 7:1 |
| LNP17 | MC3 | MC3/DSPC/Chol/PEG-DSG |
| | | 50/10/38.5/1.5 |
| | | Lipid:siRNA: 10:1 |
| LNP18 | MC3 | MC3/DSPC/Chol/PEG-DMG |
| | | 50/10/38.5/1.5 |
| | | Lipid:siRNA: 12:1 |
| LNP19 | MC3 | MC3/DSPC/Chol/PEG-DMG |
| | | 50/10/35/5 |
| | | Lipid:siRNA: 8:1 |
| LNP20 | MC3 | MC3/DSPC/Chol/PEG-DPG |
| | | 50/10/38.5/1.5 |
| | | Lipid:siRNA: 10:1 |
| LNP21 | C12-200 | C12-200/DSPC/Chol/PEG-DSG |
| | | 50/10/38.5/1.5 |
| | | Lipid:siRNA: 7:1 |
| LNP22 | XTC | XTC/DSPC/Chol/PEG-DSG |
| | | 50/10/38.5/1.5 |
| | | Lipid:siRNA: 10:1 |
| DSPC: distearoylphosphatidylcholine | | |
| DPPC: dipalmitoylphosphatidylcholine | | |
| PEG-DMG: PEG-didimyristoyl glycerol (C14-PEG, or PEG-C14) (PEG with avg mol wt of 2000) | | |
| PEG-DSG: PEG-distyryl glycerol (C18-PEG, or PEG-C18) (PEG with avg mol wt of 2000) PEG-cDMA: PEG-carbamoyl-1,2-dimyristyloxypropylamine (PEG with avg mol wt of 2000) SNALP (1,2-Dilinolenyloxy-N,N-dimethylami- | | |
| nopropane (DLinDMA)) comprising formulations are described in International Publication No. WO2009/127060, filed April 15, 2009. | | |

XTC comprising formulations are described, e.g., in U.S. Provisional Serial No. 61/148,366, filed January 29, 2009; U.S. Provisional Serial No. 61/156,851, filed March 2, 2009; U.S. Provisional Serial No. filed June 10, 2009; U.S. Provisional Serial No. 61/228,373, filed July 24, 2009; U.S. Provisional Serial No. 61/239,686, filed September 3, 2009, and International Application No. PCT/US2010/022614, filed January 29, 2010.

MC3 comprising formulations are described, e.g., in U.S. Publication No. 2010/0324120, filed June 10, 2010.

ALNY-100 comprising formulations are described, e.g., International patent application number PCT/US09/63933, filed on November 10, 2009.

C12-200 comprising formulations are described in U.S. Provisional Serial No. 61/175,770, filed May 5, 2009 and International Application No. PCT/US10/33777, filed May 5, 2010.

### Synthesis of ionizable/cationic lipids

Any of the compounds, *e.g.,* cationic lipids and the like, used in the nucleic acid-lipid particles of the invention can be prepared by known organic synthesis techniques, including the methods described in more detail in the Examples. All substituents are as defined below unless indicated otherwise.

"Alkyl" means a straight chain or branched, noncyclic or cyclic, saturated aliphatic hydrocarbon containing from 1 to 24 carbon atoms. Representative saturated straight chain alkyls include methyl, ethyl, n-propyl, n-butyl, n-pentyl, n-hexyl, and the like; while saturated branched alkyls include isopropyl, sec-butyl, isobutyl, tert-butyl, isopentyl, and the like. Representative saturated cyclic alkyls include cyclopropyl, cyclobutyl, cyclopentyl, cyclohexyl, and the like; while unsaturated cyclic alkyls include cyclopentenyl and cyclohexenyl, and the like.

"Alkenyl" means an alkyl, as defined above, containing at least one double bond between adjacent carbon atoms. Alkenyls include both cis and trans isomers. Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like.

"Alkynyl" means any alkyl or alkenyl, as defined above, which additionally contains at least one triple bond between adjacent carbons. Representative straight chain and branched alkynyls include acetylenyl, propynyl, 1-butynyl, 2-butynyl, 1-pentynyl, 2-pentynyl, 3-methyl-1 butynyl, and the like.

"Acyl" means any alkyl, alkenyl, or alkynyl wherein the carbon at the point of attachment is substituted with an oxo group, as defined below. For example, -C(=O)alkyl, -C(=O)alkenyl, and -C(=O)alkynyl are acyl groups.

"Heterocycle" means a 5- to 7-membered monocyclic, or 7- to 10-membered bicyclic, heterocyclic ring which is either saturated, unsaturated, or aromatic, and which contains from 1 or 2 heteroatoms independently selected from nitrogen, oxygen and sulfur, and wherein the nitrogen and sulfur heteroatoms can be optionally oxidized, and the nitrogen heteroatom can be optionally quaternized, including bicyclic rings in which any of the above heterocycles are fused to a benzene ring. The heterocycle can be attached via any heteroatom or carbon atom. Heterocycles include heteroaryls as defined below. Heterocycles include morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

The terms "optionally substituted alkyl", "optionally substituted alkenyl", "optionally substituted alkynyl", "optionally substituted acyl", and "optionally substituted heterocycle" means that, when substituted, at least one hydrogen atom is replaced with a substituent. In the case of an oxo substituent (=O) two hydrogen atoms are replaced. In this regard, substituents include oxo, halogen, heterocycle, -CN, -ORx, -NRxRy, -NRxC(=O)Ry, -NRxSO2Ry, -C(=O)Rx, -C(=O)ORx, -C(=O)NRxRy, -SOnRx and -SOnNRxRy, wherein n is 0, 1 or 2, Rx and Ry are the same or different and independently hydrogen, alkyl or heterocycle, and each of said alkyl and heterocycle substituents can be further substituted with one or more of oxo, halogen, -OH, -CN, alkyl, -ORx, heterocycle, -NRxRy, -NRxC(=O)Ry, -NRxSO2Ry, -C(=O)Rx, -C(=O)ORx, -C(=O)NRxRy, -SOnRx and -SOnNRxRy.

"Halogen" means fluoro, chloro, bromo and iodo.

In some cases, the methods can require the use of protecting groups. Protecting group methodology is well known to those skilled in the art (see, for example, Protective Groups in Organic Synthesis, Green, T.W. et al., Wiley-Interscience, New York City, 1999). Briefly, protecting groups within the context of this invention are any group that reduces or eliminates unwanted reactivity of a functional group. A protecting group can be added to a functional group to mask its reactivity during certain reactions and then removed to reveal the original functional group. In some embodiments an "alcohol protecting group" is used. An "alcohol protecting group" is any group which decreases or eliminates unwanted reactivity of an alcohol functional group. Protecting groups can be added and removed using techniques well known in the art.

### Synthesis of Formula A

In some cases, nucleic acid-lipid particles are formulated using a cationic lipid of formula A: 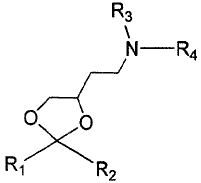 where R1 and R2 are independently alkyl, alkenyl or alkynyl, each can be optionally substituted, and R3 and R4 are independently lower alkyl or R3 and R4 can be taken together to form an optionally substituted heterocyclic ring. In some embodiments, the cationic lipid is XTC (2,2-Dilinoleyl-4-dimethylaminoethyl-[1,3]-dioxolane). In general, the lipid of formula A above can be made by the following Reaction Schemes 1 or 2, wherein all substituents are as defined above unless indicated otherwise. 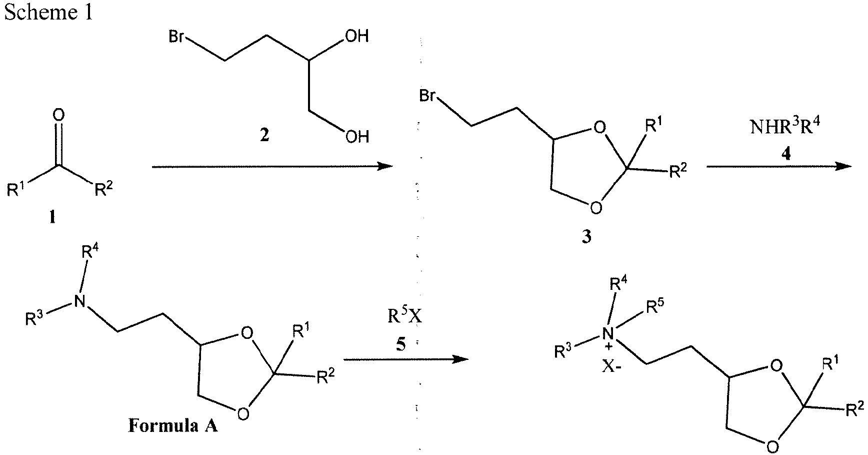 Lipid A, where R1 and R2 are independently alkyl, alkenyl or alkynyl, each can be optionally substituted, and R3 and R4 are independently lower alkyl or R3 and R4 can be taken together to form an optionally substituted heterocyclic ring, can be prepared according to Scheme 1. Ketone 1 and bromide 2 can be purchased or prepared according to methods known to those of ordinary skill in the art. Reaction of 1 and 2 yields ketal 3. Treatment of ketal 3 with amine 4 yields lipids of formula A. The lipids of formula A can be converted to the corresponding ammonium salt with an organic salt of formula 5, where X is anion counter ion selected from halogen, hydroxide, phosphate, sulfate, or the like.

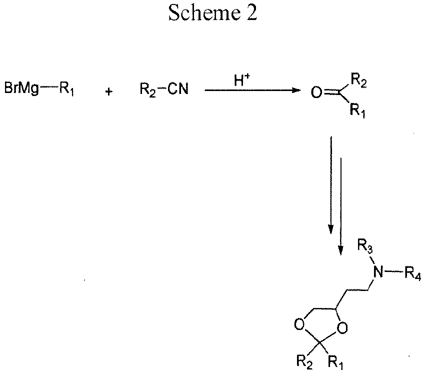

Alternatively, the ketone 1 starting material can be prepared according to Scheme 2. Grignard reagent 6 and cyanide 7 can be purchased or prepared according to methods known to those of ordinary skill in the art. Reaction of 6 and 7 yields ketone 1. Conversion of ketone 1 to the corresponding lipids of formula A is as described in Scheme 1.

### Synthesis of MC3

Preparation of DLin-M-C3-DMA *(i.e.,* (6Z,9Z,28Z,31Z)-heptatriaconta-6,9,28,31-tetraen-19-yl 4-(dimethylamino)butanoate) was as follows. A solution of (6Z,9Z,28Z,31Z)-heptatriaconta-6,9,28,31-tetraen-19-ol (0.53 g), 4-N,N-dimethylaminobutyric acid hydrochloride (0.51 g), 4-N,N-dimethylaminopyridine (0.61g) and 1-ethyl-3-(3-dimethylaminopropyl)carbodiimide hydrochloride (0.53 g) in dichloromethane (5 mL) was stirred at room temperature overnight. The solution was washed with dilute hydrochloric acid followed by dilute aqueous sodium bicarbonate. The organic fractions were dried over anhydrous magnesium sulphate, filtered and the solvent removed on a rotovap. The residue was passed down a silica gel column (20 g) using a 1-5% methanol/dichloromethane elution gradient. Fractions containing the purified product were combined and the solvent removed, yielding a colorless oil (0.54 g).

### Synthesis of ALNY-100

Synthesis of ketal 519 [ALNY-100] was performed using the following scheme 3:

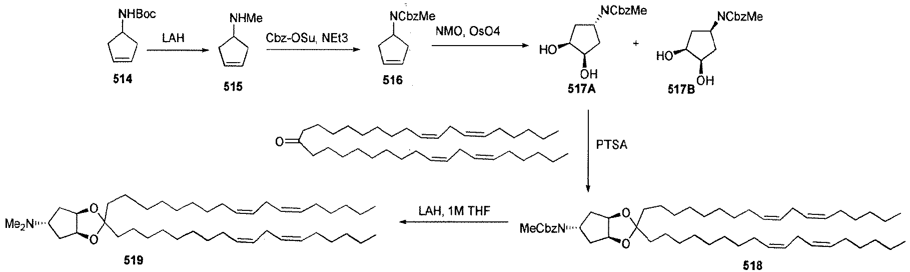

### Synthesis of 515

To a stirred suspension of LiAlH4 (3.74 g, 0.09852 mol) in 200 ml anhydrous THF in a two neck RBF (1L), was added a solution of 514 (10g, 0.04926mol) in 70 mL of THF slowly at 0 °C under nitrogen atmosphere. After complete addition, reaction mixture was warmed to room temperature and then heated to reflux for 4 h. Progress of the reaction was monitored by TLC. After completion of reaction (by TLC) the mixture was cooled to 0 °C and quenched with careful addition of saturated Na2SO4 solution. Reaction mixture was stirred for 4 h at room temperature and filtered off. Residue was washed well with THF. The filtrate and washings were mixed and diluted with 400 mL dioxane and 26 mL conc. HCl and stirred for 20 minutes at room temperature. The volatilities were stripped off under vacuum to furnish the hydrochloride salt of 515 as a white solid. Yield: 7.12 g 1H-NMR (DMSO, 400MHz): δ= 9.34 (broad, 2H), 5.68 (s, 2H), 3.74 (m, 1H), 2.66-2.60 (m, 2H), 2.50-2.45 (m, 5H).

### Synthesis of 516

To a stirred solution of compound 515 in 100 mL dry DCM in a 250 mL two neck RBF, was added NEt3 (37.2 mL, 0.2669 mol) and cooled to 0 °C under nitrogen atmosphere. After a slow addition of N-(benzyloxy-carbonyloxy)-succinimide (20 g, 0.08007 mol) in 50 mL dry DCM, reaction mixture was allowed to warm to room temperature. After completion of the reaction (2-3 h by TLC) mixture was washed successively with 1N HCl solution (1 x 100 mL) and saturated NaHCO3 solution (1 x 50 mL). The organic layer was then dried over anhyd. Na2SO4 and the solvent was evaporated to give crude material which was purified by silica gel column chromatography to get 516 as sticky mass. Yield: 11g (89%). 1H-NMR (CDC13, 400MHz): δ = 7.36-7.27(m, 5H), 5.69 (s, 2H), 5.12 (s, 2H), 4.96 (br., 1H) 2.74 (s, 3H), 2.60(m, 2H), 2.30-2.25(m, 2H). LC-MS [M+H] -232.3 (96.94%).

### Synthesis of 517A and 517B

The cyclopentene 516 (5 g, 0.02164 mol) was dissolved in a solution of 220 mL acetone and water (10:1) in a single neck 500 mL RBF and to it was added N-methyl morpholine-N-oxide (7.6 g, 0.06492 mol) followed by 4.2 mL of 7.6% solution of OsO4 (0.275 g, 0.00108 mol) in tert-butanol at room temperature. After completion of the reaction (∼ 3 h), the mixture was quenched with addition of solid Na2SO3 and resulting mixture was stirred for 1.5 h at room temperature. Reaction mixture was diluted with DCM (300 mL) and washed with water (2 x 100 mL) followed by saturated NaHCO3 (1 x 50 mL) solution, water (1 x 30 mL) and finally with brine (1x 50 mL). Organic phase was dried over an.Na2SO4 and solvent was removed in vacuum. Silica gel column chromatographic purification of the crude material was afforded a mixture of diastereomers, which were separated by prep HPLC. Yield: - 6 g crude

517A - Peak-1 (white solid), 5.13 g (96%). 1H-NMR (DMSO, 400MHz): δ= 7.39-7.31(m, 5H), 5.04(s, 2H), 4.78-4.73 (m, 1H), 4.48-4.47(d, 2H), 3.94-3.93(m, 2H), 2.71(s, 3H), 1.72- 1.67(m, 4H). LC-MS - [M+H]-266.3, [M+NH4 +]-283.5 present, HPLC-97.86%. Stereochemistry confirmed by X-ray.

### Synthesis of 518

Using a procedure analogous to that described for the synthesis of compound 505, compound 518 (1.2 g, 41%) was obtained as a colorless oil. 1H-NMR (CDCl3, 400MHz): δ= 7.35-7.33(m, 4H), 7.30-7.27(m, 1H), 5.37-5.27(m, 8H), 5.12(s, 2H), 4.75(m,1H), 4.58-4.57(m,2H), 2.78-2.74(m,7H), 2.06-2.00(m,8H), 1.96-1.91(m, 2H), 1.62(m, 4H), 1.48(m, 2H), 1.37-1.25(br m, 36H), 0.87(m, 6H). HPLC-98.65%.

### General Procedure for the Synthesis of Compound 519

A solution of compound 518 (1 eq) in hexane (15 mL) was added in a drop-wise fashion to an ice-cold solution of LAH in THF (1 M, 2 eq). After complete addition, the mixture was heated at 40oC over 0.5 h then cooled again on an ice bath. The mixture was carefully hydrolyzed with saturated aqueous Na2SO4 then filtered through celite and reduced to an oil. Column chromatography provided the pure 519 (1.3 g, 68%) which was obtained as a colorless oil. 13C NMR δ= 130.2, 130.1 (x2), 127.9 (x3), 112.3, 79.3, 64.4, 44.7, 38.3, 35.4, 31.5, 29.9 (x2), 29.7, 29.6 (x2), 29.5 (x3), 29.3 (x2), 27.2 (x3), 25.6, 24.5, 23.3, 226, 14.1; Electrospray MS (+ve): Molecular weight for C44H80NO2 (M + H)+ Calc. 654.6, Found 654.6.

Formulations prepared by either the standard or extrusion-free method can be characterized in similar manners. For example, formulations are typically characterized by visual inspection. They should be whitish translucent solutions free from aggregates or sediment. Particle size and particle size distribution of lipid-nanoparticles can be measured by light scattering using, for example, a Malvern Zetasizer Nano ZS (Malvern, USA). Particles should be about 20-300 nm, such as 40-100 nm in size. The particle size distribution should be unimodal. The total dsRNA concentration in the formulation, as well as the entrapped fraction, is estimated using a dye exclusion assay. A sample of the formulated dsRNA can be incubated with an RNA-binding dye, such as Ribogreen (Molecular Probes) in the presence or absence of a formulation disrupting surfactant, *e.g.,* 0.5% Triton-Xl 00. The total dsRNA in the formulation can be determined by the signal from the sample containing the surfactant, relative to a standard curve. The entrapped fraction is determined by subtracting the "free" dsRNA content (as measured by the signal in the absence of surfactant) from the total dsRNA content. Percent entrapped dsRNA is typically >85%. For SNALP formulation, the particle size is at least 30 nm, at least 40 nm, at least 50 nm, at least 60 nm, at least 70 nm, at least 80 nm, at least 90 nm, at least 100 nm, at least 110 nm, and at least 120 nm. The suitable range is typically about at least 50 nm to about at least 110 nm, about at least 60 nm to about at least 100 nm, or about at least 80 nm to about at least 90 nm.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders can be desirable. In some embodiments, oral formulations are those in which dsRNAs featured in the invention are administered in conjunction with one or more penetration enhancer surfactants and chelators. Suitable surfactants include fatty acids and/or esters or salts thereof, bile acids and/or salts thereof. Suitable bile acids/salts include chenodeoxycholic acid (CDCA) and ursodeoxychenodeoxycholic acid (UDCA), cholic acid, dehydrocholic acid, deoxycholic acid, glucholic acid, glycholic acid, glycodeoxycholic acid, taurocholic acid, taurodeoxycholic acid, sodium tauro-24,25-dihydro-fusidate and sodium glycodihydrofusidate. Suitable fatty acids include arachidonic acid, undecanoic acid, oleic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a monoglyceride, a diglyceride or a pharmaceutically acceptable salt thereof (*e.g*., sodium). In some embodiments, combinations of penetration enhancers are used, for example, fatty acids/salts in combination with bile acids/salts. One exemplary combination is the sodium salt of lauric acid, capric acid and UDCA. Further penetration enhancers include polyoxyethylene-9-lauryl ether, polyoxyethylene-20-cetyl ether. DsRNAs featured in the invention can be delivered orally, in granular form including sprayed dried particles, or complexed to form micro or nanoparticles. DsRNA complexing agents include poly-amino acids; polyimines; polyacrylates; polyalkylacrylates, polyoxethanes, polyalkylcyanoacrylates; cationized gelatins, albumins, starches, acrylates, polyethyleneglycols (PEG) and starches; polyalkylcyanoacrylates; DEAE-derivatized polyimines, pollulans, celluloses and starches. Suitable complexing agents include chitosan, N-trimethylchitosan, poly-L-lysine, polyhistidine, polyornithine, polyspermines, protamine, polyvinylpyridine, polythiodiethylaminomethylethylene P(TDAE), polyaminostyrene (*e.g*., p-amino), poly(methylcyanoacrylate), poly(ethylcyanoacrylate), poly(butylcyanoacrylate), poly(isobutylcyanoacrylate), poly(isohexylcynaoacrylate), DEAE-methacrylate, DEAE-hexylacrylate, DEAE-acrylamide, DEAE-albumin and DEAE-dextran, polymethylacrylate, polyhexylacrylate, poly(D,L-lactic acid), poly(DL-lactic-co-glycolic acid (PLGA), alginate, and polyethyleneglycol (PEG). Oral formulations for dsRNAs and their preparation are described in detail in U.S. Patent 6,887,906, US Publn. No. 20030027780, and U.S. Patent No. 6,747,014.

Compositions and formulations for parenteral, intraparenchymal (into the brain), intrathecal, intraventricular or intrahepatic administration can include sterile aqueous solutions which can also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions can be generated from a variety of components that include, but are not limited to, preformed liquids, self-emulsifying solids and self-emulsifying semisolids. Particularly preferred are formulations that target the liver when treating hepatic disorders such as hepatic carcinoma.

The pharmaceutical formulations of the present invention, which can conveniently be presented in unit dosage form, can be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions of the present invention can be formulated into any of many possible dosage forms such as, but not limited to, tablets, capsules, gel capsules, liquid syrups, soft gels, suppositories, and enemas. The compositions of the present invention can also be formulated as suspensions in aqueous, non-aqueous or mixed media. Aqueous suspensions can further contain substances which increase the viscosity of the suspension including, for example, sodium carboxymethylcellulose, sorbitol and/or dextran. The suspension can also contain stabilizers.

### C. Additional Formulations

### i. Emulsions

The compositions of the present invention can be prepared and formulated as emulsions. Emulsions are typically heterogeneous systems of one liquid dispersed in another in the form of droplets usually exceeding 0.1µm in diameter (see e.g., Ansel's Pharmaceutical Dosage Forms and Drug Delivery Systems, Allen, LV., Popovich NG., and Ansel HC., 2004, Lippincott Williams & Wilkins (8th ed.), New York, NY; Idson, in Pharmaceutical Dosage Forms, Lieberman, Rieger and Banker (Eds.), 1988, Marcel Dekker, Inc., New York, N.Y., volume 1, p. 199; Rosoff, in Pharmaceutical Dosage Forms, Lieberman, Rieger and Banker (Eds.), 1988, Marcel Dekker, Inc., New York, N.Y., Volume 1, p. 245; Block in Pharmaceutical Dosage Forms, Lieberman, Rieger and Banker (Eds.), 1988, Marcel Dekker, Inc., New York, N.Y., volume 2, p. 335; Higuchi et al., in Remington's Pharmaceutical Sciences, Mack Publishing Co., Easton, Pa., 1985, p. 301). Emulsions are often biphasic systems comprising two immiscible liquid phases intimately mixed and dispersed with each other. In general, emulsions can be of either the water-in-oil (w/o) or the oil-in-water (o/w) variety. When an aqueous phase is finely divided into and dispersed as minute droplets into a bulk oily phase, the resulting composition is called a water-in-oil (w/o) emulsion. Alternatively, when an oily phase is finely divided into and dispersed as minute droplets into a bulk aqueous phase, the resulting composition is called an oil-in-water (o/w) emulsion. Emulsions can contain additional components in addition to the dispersed phases, and the active drug which can be present as a solution in either the aqueous phase, oily phase or itself as a separate phase. Pharmaceutical excipients such as emulsifiers, stabilizers, dyes, and anti-oxidants can also be present in emulsions as needed. Pharmaceutical emulsions can also be multiple emulsions that are comprised of more than two phases such as, for example, in the case of oil-in-water-in-oil (o/w/o) and water-in-oil-in-water (w/o/w) emulsions. Such complex formulations often provide certain advantages that simple binary emulsions do not. Multiple emulsions in which individual oil droplets of an o/w emulsion enclose small water droplets constitute a w/o/w emulsion. Likewise a system of oil droplets enclosed in globules of water stabilized in an oily continuous phase provides an o/w/o emulsion.

Emulsions are characterized by little or no thermodynamic stability. Often, the dispersed or discontinuous phase of the emulsion is well dispersed into the external or continuous phase and maintained in this form through the means of emulsifiers or the viscosity of the formulation. Either of the phases of the emulsion can be a semisolid or a solid, as is the case of emulsion-style ointment bases and creams. Other means of stabilizing emulsions entail the use of emulsifiers that can be incorporated into either phase of the emulsion. Emulsifiers can broadly be classified into four categories: synthetic surfactants, naturally occurring emulsifiers, absorption bases, and finely dispersed solids (see e.g., Ansel's Pharmaceutical Dosage Forms and Drug Delivery Systems, Allen, LV., Popovich NG., and Ansel HC., 2004, Lippincott Williams & Wilkins (8th ed.), New York, NY; Idson, in Pharmaceutical Dosage Forms, Lieberman, Rieger and Banker (Eds.), 1988, Marcel Dekker, Inc., New York, N.Y., volume 1, p. 199).

Synthetic surfactants, also known as surface active agents, have found wide applicability in the formulation of emulsions and have been reviewed in the literature (see e.g., Ansel's Pharmaceutical Dosage Forms and Drug Delivery Systems, Allen, LV., Popovich NG., and Ansel HC., 2004, Lippincott Williams & Wilkins (8th ed.), New York, NY; Rieger, in Pharmaceutical Dosage Forms, Lieberman, Rieger and Banker (Eds.), 1988, Marcel Dekker, Inc., New York, N.Y., volume 1, p. 285; Idson, in Pharmaceutical Dosage Forms, Lieberman, Rieger and Banker (Eds.), Marcel Dekker, Inc., New York, N.Y., 1988, volume 1, p. 199). Surfactants are typically amphiphilic and comprise a hydrophilic and a hydrophobic portion. The ratio of the hydrophilic to the hydrophobic nature of the surfactant has been termed the hydrophile/lipophile balance (HLB) and is a valuable tool in categorizing and selecting surfactants in the preparation of formulations. Surfactants can be classified into different classes based on the nature of the hydrophilic group: nonionic, anionic, cationic and amphoteric (see e.g., Ansel's Pharmaceutical Dosage Forms and Drug Delivery Systems, Allen, LV., Popovich NG., and Ansel HC., 2004, Lippincott Williams & Wilkins (8th ed.), New York, NY Rieger, in Pharmaceutical Dosage Forms, Lieberman, Rieger and Banker (Eds.), 1988, Marcel Dekker, Inc., New York, N.Y., volume 1, p. 285).

Naturally occurring emulsifiers used in emulsion formulations include lanolin, beeswax, phosphatides, lecithin and acacia. Absorption bases possess hydrophilic properties such that they can soak up water to form w/o emulsions yet retain their semisolid consistencies, such as anhydrous lanolin and hydrophilic petrolatum. Finely divided solids have also been used as good emulsifiers especially in combination with surfactants and in viscous preparations. These include polar inorganic solids, such as heavy metal hydroxides, nonswelling clays such as bentonite, attapulgite, hectorite, kaolin, montmorillonite, colloidal aluminum silicate and colloidal magnesium aluminum silicate, pigments and nonpolar solids such as carbon or glyceryl tristearate.

A large variety of non-emulsifying materials are also included in emulsion formulations and contribute to the properties of emulsions. These include fats, oils, waxes, fatty acids, fatty alcohols, fatty esters, humectants, hydrophilic colloids, preservatives and antioxidants (Block, in Pharmaceutical Dosage Forms, Lieberman, Rieger and Banker (Eds.), 1988, Marcel Dekker, Inc., New York, N.Y., volume 1, p. 335; Idson, in Pharmaceutical Dosage Forms, Lieberman, Rieger and Banker (Eds.), 1988, Marcel Dekker, Inc., New York, N.Y., volume 1, p. 199).

Hydrophilic colloids or hydrocolloids include naturally occurring gums and synthetic polymers such as polysaccharides (for example, acacia, agar, alginic acid, carrageenan, guar gum, karaya gum, and tragacanth), cellulose derivatives (for example, carboxymethylcellulose and carboxypropylcellulose), and synthetic polymers (for example, carbomers, cellulose ethers, and carboxyvinyl polymers). These disperse or swell in water to form colloidal solutions that stabilize emulsions by forming strong interfacial films around the dispersed-phase droplets and by increasing the viscosity of the external phase.

Since emulsions often contain a number of ingredients such as carbohydrates, proteins, sterols and phosphatides that can readily support the growth of microbes, these formulations often incorporate preservatives. Commonly used preservatives included in emulsion formulations include methyl paraben, propyl paraben, quaternary ammonium salts, benzalkonium chloride, esters of p-hydroxybenzoic acid, and boric acid. Antioxidants are also commonly added to emulsion formulations to prevent deterioration of the formulation. Antioxidants used can be free radical scavengers such as tocopherols, alkyl gallates, butylated hydroxyanisole, butylated hydroxytoluene, or reducing agents such as ascorbic acid and sodium metabisulfite, and antioxidant synergists such as citric acid, tartaric acid, and lecithin.

The application of emulsion formulations via dermatological, oral and parenteral routes and methods for their manufacture have been reviewed in the literature (see e.g., Ansel's Pharmaceutical Dosage Forms and Drug Delivery Systems, Allen, LV., Popovich NG., and Ansel HC., 2004, Lippincott Williams & Wilkins (8th ed.), New York, NY; Idson, in Pharmaceutical Dosage Forms, Lieberman, Rieger and Banker (Eds.), 1988, Marcel Dekker, Inc., New York, N.Y., volume 1, p. 199). Emulsion formulations for oral delivery have been very widely used because of ease of formulation, as well as efficacy from an absorption and bioavailability standpoint (see e.g., Ansel's Pharmaceutical Dosage Forms and Drug Delivery Systems, Allen, LV., Popovich NG., and Ansel HC., 2004, Lippincott Williams & Wilkins (8th ed.), New York, NY; Rosoff, in Pharmaceutical Dosage Forms, Lieberman, Rieger and Banker (Eds.), 1988, Marcel Dekker, Inc., New York, N.Y., volume 1, p. 245; Idson, in Pharmaceutical Dosage Forms, Lieberman, Rieger and Banker (Eds.), 1988, Marcel Dekker, Inc., New York, N.Y., volume 1, p. 199). Mineral-oil base laxatives, oil-soluble vitamins and high fat nutritive preparations are among the materials that have commonly been administered orally as o/w emulsions.

### ii. Microemulsions

The compositions of iRNAs and nucleic acids may be formulated as microemulsions. A microemulsion can be defined as a system of water, oil and amphiphile which is a single optically isotropic and thermodynamically stable liquid solution (see e.g., Ansel's Pharmaceutical Dosage Forms and Drug Delivery Systems, Allen, LV., Popovich NG., and Ansel HC., 2004, Lippincott Williams & Wilkins (8th ed.), New York, NY; Rosoff, in Pharmaceutical Dosage Forms, Lieberman, Rieger and Banker (Eds.), 1988, Marcel Dekker, Inc., New York, N.Y., volume 1, p. 245). Typically microemulsions are systems that are prepared by first dispersing an oil in an aqueous surfactant solution and then adding a sufficient amount of a fourth component, generally an intermediate chain-length alcohol to form a transparent system. Therefore, microemulsions have also been described as thermodynamically stable, isotropically clear dispersions of two immiscible liquids that are stabilized by interfacial films of surface-active molecules (Leung and Shah, in: Controlled Release of Drugs: Polymers and Aggregate Systems, Rosoff, M., Ed., 1989, VCH Publishers, New York, pages 185-215). Microemulsions commonly are prepared via a combination of three to five components that include oil, water, surfactant, cosurfactant and electrolyte. Whether the microemulsion is of the water-in-oil (w/o) or an oil-in-water (o/w) type is dependent on the properties of the oil and surfactant used and on the structure and geometric packing of the polar heads and hydrocarbon tails of the surfactant molecules (Schott, in Remington's Pharmaceutical Sciences, Mack Publishing Co., Easton, Pa., 1985, p. 271).

The phenomenological approach utilizing phase diagrams has been extensively studied and has yielded a comprehensive knowledge, to one skilled in the art, of how to formulate microemulsions (see e.g., Ansel's Pharmaceutical Dosage Forms and Drug Delivery Systems, Allen, LV., Popovich NG., and Ansel HC., 2004, Lippincott Williams & Wilkins (8th ed.), New York, NY; Rosoff, in Pharmaceutical Dosage Forms, Lieberman, Rieger and Banker (Eds.), 1988, Marcel Dekker, Inc., New York, N.Y., volume 1, p. 245; Block, in Pharmaceutical Dosage Forms, Lieberman, Rieger and Banker (Eds.), 1988, Marcel Dekker, Inc., New York, N.Y., volume 1, p. 335). Compared to conventional emulsions, microemulsions offer the advantage of solubilizing water-insoluble drugs in a formulation of thermodynamically stable droplets that are formed spontaneously.

Surfactants used in the preparation of microemulsions include, but are not limited to, ionic surfactants, non-ionic surfactants, Brij 96, polyoxyethylene oleyl ethers, polyglycerol fatty acid esters, tetraglycerol monolaurate (ML310), tetraglycerol monooleate (MO310), hexaglycerol monooleate (PO310), hexaglycerol pentaoleate (PO500), decaglycerol monocaprate (MCA750), decaglycerol monooleate (MO750), decaglycerol sequioleate (SO750), decaglycerol decaoleate (DAO750), alone or in combination with cosurfactants. The cosurfactant, usually a short-chain alcohol such as ethanol, 1-propanol, and 1-butanol, serves to increase the interfacial fluidity by penetrating into the surfactant film and consequently creating a disordered film because of the void space generated among surfactant molecules. Microemulsions can, however, be prepared without the use of cosurfactants and alcohol-free self-emulsifying microemulsion systems are known in the art. The aqueous phase can typically be, but is not limited to, water, an aqueous solution of the drug, glycerol, PEG300, PEG400, polyglycerols, propylene glycols, and derivatives of ethylene glycol. The oil phase can include, but is not limited to, materials such as Captex 300, Captex 355, Capmul MCM, fatty acid esters, medium chain (C8-C12) mono, di, and tri-glycerides, polyoxyethylated glyceryl fatty acid esters, fatty alcohols, polyglycolized glycerides, saturated polyglycolized C8-C10 glycerides, vegetable oils and silicone oil.

Microemulsions are particularly of interest from the standpoint of drug solubilization and the enhanced absorption of drugs. Lipid based microemulsions (both o/w and w/o) have been proposed to enhance the oral bioavailability of drugs, including peptides (see e.g., U.S. Patent Nos. 6,191,105; 7,063,860; 7,070,802; 7,157,099; Constantinides et al., Pharmaceutical Research, 1994, 11, 1385-1390; Ritschel, Meth. Find. Exp. Clin. Pharmacol., 1993, 13, 205). Microemulsions afford advantages of improved drug solubilization, protection of drug from enzymatic hydrolysis, possible enhancement of drug absorption due to surfactant-induced alterations in membrane fluidity and permeability, ease of preparation, ease of oral administration over solid dosage forms, improved clinical potency, and decreased toxicity (see e.g., U.S. Patent Nos. 6,191,105; 7,063,860; 7,070,802; 7,157,099; Constantinides et al., Pharmaceutical Research, 1994, 11, 1385; Ho et al., J. Pharm. Sci., 1996, 85, 138-143). Often microemulsions can form spontaneously when their components are brought together at ambient temperature. This can be particularly advantageous when formulating thermolabile drugs, peptides or iRNAs. Microemulsions have also been effective in the transdermal delivery of active components in both cosmetic and pharmaceutical applications. It is expected that the microemulsion compositions and formulations of the present invention will facilitate the increased systemic absorption of iRNAs and nucleic acids from the gastrointestinal tract, as well as improve the local cellular uptake of iRNAs and nucleic acids.

Microemulsions of the present invention can also contain additional components and additives such as sorbitan monostearate (Grill 3), Labrasol, and penetration enhancers to improve the properties of the formulation and to enhance the absorption of the iRNAs and nucleic acids of the present invention. Penetration enhancers used in the microemulsions of the present invention can be classified as belonging to one of five broad categories--surfactants, fatty acids, bile salts, chelating agents, and non-chelating non-surfactants (Lee et al., Critical Reviews in Therapeutic Drug Carrier Systems, 1991, p. 92). Each of these classes has been discussed above.

### iii. Microparticles

An iRNA agent of the invention may be incorporated into a particle, e.g., a microparticle. Microparticles can be produced by spray-drying, but may also be produced by other methods including lyophilization, evaporation, fluid bed drying, vacuum drying, or a combination of these techniques.

### iv. Penetration Enhancers

In one case, the various penetration enhancers are employed to effect the efficient delivery of nucleic acids, particularly iRNAs, to the skin of animals. Most drugs are present in solution in both ionized and nonionized forms. However, usually only lipid soluble or lipophilic drugs readily cross cell membranes. It has been discovered that even non-lipophilic drugs can cross cell membranes if the membrane to be crossed is treated with a penetration enhancer. In addition to aiding the diffusion of non-lipophilic drugs across cell membranes, penetration enhancers also enhance the permeability of lipophilic drugs.

Penetration enhancers can be classified as belonging to one of five broad categories, i.e., surfactants, fatty acids, bile salts, chelating agents, and non-chelating non-surfactants (see e.g., Malmsten, M. Surfactants and polymers in drug delivery, Informa Health Care, New York, NY, 2002; Lee et al., Critical Reviews in Therapeutic Drug Carrier Systems, 1991, p.92). Each of the above mentioned classes of penetration enhancers are described below in greater detail.

Surfactants (or "surface-active agents") are chemical entities which, when dissolved in an aqueous solution, reduce the surface tension of the solution or the interfacial tension between the aqueous solution and another liquid, with the result that absorption of iRNAs through the mucosa is enhanced. In addition to bile salts and fatty acids, these penetration enhancers include, for example, sodium lauryl sulfate, polyoxyethylene-9-lauryl ether and polyoxyethylene-20-cetyl ether) (see e.g., Malmsten, M. Surfactants and polymers in drug delivery, Informa Health Care, New York, NY, 2002; Lee et al., Critical Reviews in Therapeutic Drug Carrier Systems, 1991, p.92); and perfluorochemical emulsions, such as FC-43. Takahashi et al., J. Pharm. Pharmacol., 1988, 40, 252).

Various fatty acids and their derivatives which act as penetration enhancers include, for example, oleic acid, lauric acid, capric acid (n-decanoic acid), myristic acid, palmitic acid, stearic acid, linoleic acid, linolenic acid, dicaprate, tricaprate, monoolein (1-monooleoyl-rac-glycerol), dilaurin, caprylic acid, arachidonic acid, glycerol 1-monocaprate, 1-dodecylazacycloheptan-2-one, acylcamitines, acylcholines, C1-20 alkyl esters thereof (e.g., methyl, isopropyl and t-butyl), and mono- and di-glycerides thereof (i.e., oleate, laurate, caprate, myristate, palmitate, stearate, linoleate, etc.) (see e.g., Touitou, E., et al. Enhancement in Drug Delivery, CRC Press, Danvers, MA, 2006; Lee et al., Critical Reviews in Therapeutic Drug Carrier Systems, 1991, p.92; Muranishi, Critical Reviews in Therapeutic Drug Carrier Systems, 1990, 7, 1-33; El Hariri et al., J. Pharm. Pharmacol., 1992, 44, 651-654).

The physiological role of bile includes the facilitation of dispersion and absorption of lipids and fat-soluble vitamins (see e.g., Malmsten, M. Surfactants and polymers in drug delivery, Informa Health Care, New York, NY, 2002; Brunton, Chapter 38 in: Goodman & Gilman's The Pharmacological Basis of Therapeutics, 9th Ed., Hardman et al. Eds., McGraw-Hill, New York, 1996, pp. 934-935). Various natural bile salts, and their synthetic derivatives, act as penetration enhancers. Thus the term "bile salts" includes any of the naturally occurring components of bile as well as any of their synthetic derivatives. Suitable bile salts include, for example, cholic acid (or its pharmaceutically acceptable sodium salt, sodium cholate), dehydrocholic acid (sodium dehydrocholate), deoxycholic acid (sodium deoxycholate), glucholic acid (sodium glucholate), glycholic acid (sodium glycocholate), glycodeoxycholic acid (sodium glycodeoxycholate), taurocholic acid (sodium taurocholate), taurodeoxycholic acid (sodium taurodeoxycholate), chenodeoxycholic acid (sodium chenodeoxycholate), ursodeoxycholic acid (UDCA), sodium tauro-24,25-dihydro-fusidate (STDHF), sodium glycodihydrofusidate and polyoxyethylene-9-lauryl ether (POE) (see e.g., Malmsten, M. Surfactants and polymers in drug delivery, Informa Health Care, New York, NY, 2002; Lee et al., Critical Reviews in Therapeutic Drug Carrier Systems, 1991, page 92; Swinyard, Chapter 39 In: Remington's Pharmaceutical Sciences, 18th Ed., Gennaro, ed., Mack Publishing Co., Easton, Pa., 1990, pages 782-783; Muranishi, Critical Reviews in Therapeutic Drug Carrier Systems, 1990, 7, 1-33; Yamamoto et al., J. Pharm. Exp. Ther., 1992, 263, 25; Yamashita et al., J. Pharm. Sci., 1990, 79, 579-583).

Chelating agents, as used in connection with the present invention, can be defined as compounds that remove metallic ions from solution by forming complexes therewith, with the result that absorption of iRNAs through the mucosa is enhanced. With regards to their use as penetration enhancers in the present invention, chelating agents have the added advantage of also serving as DNase inhibitors, as most characterized DNA nucleases require a divalent metal ion for catalysis and are thus inhibited by chelating agents (Jarrett, J. Chromatogr., 1993, 618, 315-339). Suitable chelating agents include but are not limited to disodium ethylenediaminetetraacetate (EDTA), citric acid, salicylates (e.g., sodium salicylate, 5-methoxysalicylate and homovanilate), N-acyl derivatives of collagen, laureth-9 and N-amino acyl derivatives of beta-diketones (enamines)(see e.g., Katdare, A. et al., Excipient development for pharmaceutical, biotechnology, and drug delivery, CRC Press, Danvers, MA, 2006; Lee et al., Critical Reviews in Therapeutic Drug Carrier Systems, 1991, page 92; Muranishi, Critical Reviews in Therapeutic Drug Carrier Systems, 1990, 7, 1-33; Buur et al., J. Control Rel., 1990, 14, 43-51).

As used herein, non-chelating non-surfactant penetration enhancing compounds can be defined as compounds that demonstrate insignificant activity as chelating agents or as surfactants but that nonetheless enhance absorption of iRNAs through the alimentary mucosa (see e.g., Muranishi, Critical Reviews in Therapeutic Drug Carrier Systems, 1990, 7, 1-33). This class of penetration enhancers includes, for example, unsaturated cyclic ureas, 1-alkyl- and 1-alkenylazacyclo-alkanone derivatives (Lee et al., Critical Reviews in Therapeutic Drug Carrier Systems, 1991, page 92); and non-steroidal anti-inflammatory agents such as diclofenac sodium, indomethacin and phenylbutazone (Yamashita et al., J. Pharm. Pharmacol., 1987, 39, 621-626).

Agents that enhance uptake of iRNAs at the cellular level can also be added to the pharmaceutical and other compositions of the present invention. For example, cationic lipids, such as lipofectin (Junichi et al, U.S. Pat. No. 5,705,188), cationic glycerol derivatives, and polycationic molecules, such as polylysine (Lollo et al., PCT Application WO 97/30731), are also known to enhance the cellular uptake of dsRNAs. Examples of commercially available transfection reagents include, for example Lipofectamine^{™} (Invitrogen; Carlsbad, CA), Lipofectamine 2000^{™} (Invitrogen; Carlsbad, CA), 293fectin^{™} (Invitrogen; Carlsbad, CA), Cellfectin^{™} (Invitrogen; Carlsbad, CA), DMRIE-C^{™} (Invitrogen; Carlsbad, CA), FreeStyle^{™} MAX (Invitrogen; Carlsbad, CA), Lipofectamine^{™} 2000 CD (Invitrogen; Carlsbad, CA), Lipofectamine^{™} (Invitrogen; Carlsbad, CA), iRNAMAX (Invitrogen; Carlsbad, CA), Oligofectamine^{™} (Invitrogen; Carlsbad, CA), Optifect^{™} (Invitrogen; Carlsbad, CA), X-tremeGENE Q2 Transfection Reagent (Roche; Grenzacherstrasse, Switzerland), DOTAP Liposomal Transfection Reagent (Grenzacherstrasse, Switzerland), DOSPER Liposomal Transfection Reagent (Grenzacherstrasse, Switzerland), or Fugene (Grenzacherstrasse, Switzerland), Transfectam^{®} Reagent (Promega; Madison, WI), TransFast^{™} Transfection Reagent (Promega; Madison, WI), Tfx^{™}-20 Reagent (Promega; Madison, WI), Tfx^{™}-50 Reagent (Promega; Madison, WI), DreamFect^{™} (OZ Biosciences; Marseille, France), EcoTransfect (OZ Biosciences; Marseille, France), TransPass^{a} D1 Transfection Reagent (New England Biolabs; Ipswich, MA, USA), LyoVec^{™}/LipoGen^{™} (Invitrogen; San Diego, CA, USA), PerFectin Transfection Reagent (Genlantis; San Diego, CA, USA), NeuroPORTER Transfection Reagent (Genlantis; San Diego, CA, USA), GenePORTER Transfection reagent (Genlantis; San Diego, CA, USA), GenePORTER 2 Transfection reagent (Genlantis; San Diego, CA, USA), Cytofectin Transfection Reagent (Genlantis; San Diego, CA, USA), BaculoPORTER Transfection Reagent (Genlantis; San Diego, CA, USA), TroganPORTER^{™} transfection Reagent (Genlantis; San Diego, CA, USA), RiboFect (Bioline; Taunton, MA, USA), PlasFect (Bioline; Taunton, MA, USA), UniFECTOR (B-Bridge International; Mountain View, CA, USA), SureFECTOR (B-Bridge International; Mountain View, CA, USA), or HiFect^{™} (B-Bridge International, Mountain View, CA, USA), among others.

Other agents can be utilized to enhance the penetration of the administered nucleic acids, including glycols such as ethylene glycol and propylene glycol, pyrrols such as 2-pyrrol, azones, and terpenes such as limonene and menthone.

### v. Carriers

Certain compositions also incorporate carrier compounds in the formulation. As used herein, "carrier compound" or "carrier" can refer to a nucleic acid, or analog thereof, which is inert (i.e., does not possess biological activity per se) but is recognized as a nucleic acid by *in vivo* processes that reduce the bioavailability of a nucleic acid having biological activity by, for example, degrading the biologically active nucleic acid or promoting its removal from circulation. The coadministration of a nucleic acid and a carrier compound, typically with an excess of the latter substance, can result in a substantial reduction of the amount of nucleic acid recovered in the liver, kidney or other extracirculatory reservoirs, presumably due to competition between the carrier compound and the nucleic acid for a common receptor. For example, the recovery of a partially phosphorothioate dsRNA in hepatic tissue can be reduced when it is coadministered with polyinosinic acid, dextran sulfate, polycytidic acid or 4-acetamido-4'isothiocyano-stilbene-2,2'-disulfonic acid (Miyao et al., DsRNA Res. Dev., 1995, 5, 115-121; Takakura et al., DsRNA & Nucl. Acid Drug Dev., 1996, 6, 177-183.

### vi. Excipients

In contrast to a carrier compound, a "pharmaceutical carrier" or "excipient" is a pharmaceutically acceptable solvent, suspending agent or any other pharmacologically inert vehicle for delivering one or more nucleic acids to an animal. The excipient can be liquid or solid and is selected, with the planned manner of administration in mind, so as to provide for the desired bulk, consistency, etc., when combined with a nucleic acid and the other components of a given pharmaceutical composition. Typical pharmaceutical carriers include, but are not limited to, binding agents (e.g., pregelatinized maize starch, polyvinylpyrrolidone or hydroxypropyl methylcellulose, etc.); fillers (e.g., lactose and other sugars, microcrystalline cellulose, pectin, gelatin, calcium sulfate, ethyl cellulose, polyacrylates or calcium hydrogen phosphate, etc.); lubricants (e.g., magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g., starch, sodium starch glycolate, etc.); and wetting agents (e.g., sodium lauryl sulphate, etc).

Pharmaceutically acceptable organic or inorganic excipients suitable for non-parenteral administration which do not deleteriously react with nucleic acids can also be used to formulate the compositions of the present invention. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like.

Formulations for topical administration of nucleic acids can include sterile and non-sterile aqueous solutions, non-aqueous solutions in common solvents such as alcohols, or solutions of the nucleic acids in liquid or solid oil bases. The solutions can also contain buffers, diluents and other suitable additives. Pharmaceutically acceptable organic or inorganic excipients suitable for non-parenteral administration which do not deleteriously react with nucleic acids can be used.

Suitable pharmaceutically acceptable excipients include, but are not limited to, water, salt solutions, alcohol, polyethylene glycols, gelatin, lactose, amylose, magnesium stearate, talc, silicic acid, viscous paraffin, hydroxymethylcellulose, polyvinylpyrrolidone and the like.

### vii. Other Components

The compositions can additionally contain other adjunct components conventionally found in pharmaceutical compositions, at their art-established usage levels. Thus, for example, the compositions can contain additional, compatible, pharmaceutically-active materials such as, for example, antipruritics, astringents, local anesthetics or anti-inflammatory agents, or can contain additional materials useful in physically formulating various dosage forms of the compositions of the present invention, such as dyes, flavoring agents, preservatives, antioxidants, opacifiers, thickening agents and stabilizers. However, such materials, when added, should not unduly interfere with the biological activities of the components of the compositions of the present invention. The formulations can be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Aqueous suspensions can contain substances which increase the viscosity of the suspension including, for example, sodium carboxymethylcellulose, sorbitol and/or dextran. The suspension can also contain stabilizers.

In some cases, pharmaceutical compositions include (a) one or more iRNA compounds and (b) one or more agents which function by a non- iRNA mechanism and which are useful in treating a hemolytic disorder. Examples of such agents include, but are not Imited to an anti-inflammatory agent, anti-steatosis agent, anti-viral, and/or anti-fibrosis agent. In addition, other substances commonly used to protect the liver, such as silymarin, can also be used in conjunction with the iRNAs described herein. Other agents useful for treating liver diseases include telbivudine, entecavir, and protease inhibitors such as telaprevir and other disclosed, for example, in Tung et al., U.S. Application Publication Nos. 2005/0148548, 2004/0167116, and 2003/0144217; and in Hale et al., U.S. Application Publication No. 2004/0127488.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are preferred.

The data obtained from cell culture assays and animal studies can be used in formulating a range of dosage for use in humans. The dosage of compositions featured herein in the invention lies generally within a range of circulating concentrations that include the ED50 with little or no toxicity. The dosage can vary within this range depending upon the dosage form employed and the route of administration utilized. For any compound used in the methods featured in the invention, the therapeutically effective dose can be estimated initially from cell culture assays. A dose can be formulated in animal models to achieve a circulating plasma concentration range of the compound or, when appropriate, of the polypeptide product of a target sequence (e.g., achieving a decreased concentration of the polypeptide) that includes the IC50 (i.e., the concentration of the test compound which achieves a half-maximal inhibition of symptoms) as determined in cell culture. Such information can be used to more accurately determine useful doses in humans. Levels in plasma can be measured, for example, by high performance liquid chromatography.

In addition to their administration, as discussed above, the iRNAs featured in the invention can be administered in combination with other known agents effective in treatment of pathological processes mediated by KHK expression. In any event, the administering physician can adjust the amount and timing of iRNA administration on the basis of results observed using standard measures of efficacy known in the art or described herein.

### VII. Methods

The present disclosure provides therapeutic and prophylactic methods which include administering to a subject having, or prone to developing, a KHK-associated disease, disorder, and/or condition (*e.g.,* liver disease (*e.g.,* fatty liver, steatohepatitis), dyslipidemia (*e.g.,* hyperlipidemia, high LDL cholesterol, low HDL cholesterol, hypertriglyceridemia, postprandial hypertriglyceridemia), disorders of glycemic control (*e.g.,* insulin resistance, diabetes), cardiovascular disease (*e.g.,* hypertension, endothelial cell dysfunction), kidney disease (*e.g.,* acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving an iRNA agent, pharmaceutical compositions comprising an iRNA agent, or vector comprising an iRNA of the invention.

In one aspect, the present disclosure provides methods of treating a subject having a disorder that would benefit from reduction in KHK expression, *e.g*., a KHK-associated disease, *e.g.,* liver disease (*e.g.,* fatty liver, steatohepatitis), dyslipidemia (*e.g.,* hyperlipidemia, high LDL cholesterol, low HDL cholesterol, hypertriglyceridemia, postprandial hypertriglyceridemia), disorders of glycemic control (*e.g.,* insulin resistance, diabetes), cardiovascular disease (*e.g.,* hypertension, endothelial cell dysfunction), kidney disease (*e.g*., acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving. The treatment methods (and uses) of the invention include administering to the subject, *e.g*., a human, a therapeutically effective amount of an iRNA agent targeting a KHK gene or a pharmaceutical composition comprising an iRNA agent targeting a KHK gene, thereby treating the subject having a disorder that would benefit from reduction in KHK expression.

In one aspect, the disclosure provides methods of preventing at least one symptom in a subject having a disorder that would benefit from reduction in KHK expression, *e.g*., a KHK-associated disease, *e.g.,* liver disease (*e.g.,* fatty liver, steatohepatitis), dyslipidemia (*e.g.,* hyperlipidemia, high LDL cholesterol, low HDL cholesterol, hypertriglyceridemia, postprandial hypertriglyceridemia), disorders of glycemic control (*e.g*., insulin resistance, diabetes), cardiovascular disease (*e.g.,* hypertension, endothelial cell dysfunction), kidney disease (*e.g.,* acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving. The methods include administering to the subject a therapeutically effective amount of the iRNA agent, *e.g*., dsRNA, or vector of the invention, thereby preventing at least one symptom in the subject having a disorder that would benefit from reduction in KHK expression. For example, the invention provides methods for preventing lipogenesis and/or hyperuricemia in a subject suffering from a disorder that would benefit from reduction in KHK expression.

In another aspect, the present disclosure provides uses of a therapeutically effective amount of an iRNA agent of the invention for treating a subject, *e.g*., a subject that would benefit from a reduction and/or inhibition of KHK expression.

In a further aspect, the present disclosure provides uses of an iRNA agent, *e.g.,* a dsRNA, of the invention targeting a KHK gene or pharmaceutical composition comprising an iRNA agent targeting a KHK gene in the manufacture of a medicament for treating a subject, *e.g*., a subject that would benefit from a reduction and/or inhibition of KHK expression, such as a subject having a disorder that would benefit from reduction in KHK expression, *e.g*., a KHK-associated disease, *e.g.,* liver disease (*e.g.,* fatty liver, steatohepatitis), dyslipidemia (*e.g.,* hyperlipidemia, high LDL cholesterol, low HDL cholesterol, hypertriglyceridemia, postprandial hypertriglyceridemia), disorders of glycemic control (*e.g*., insulin resistance, diabetes), cardiovascular disease (e.*g*., hypertension, endothelial cell dysfunction), kidney disease *(e.g.,* acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving.

In another aspect, the disclosure provides uses of an iRNA, *e.g.,* a dsRNA, of the invention for preventing at least one symptom in a subject suffering from a disorder that would benefit from a reduction and/or inhibition of KHK expression, such as a KHK-associated disease, *e.g.,* liver disease (*e.g.,* fatty liver, steatohepatitis), dyslipidemia (*e.g.,* hyperlipidemia, high LDL cholesterol, low HDL cholesterol, hypertriglyceridemia, postprandial hypertriglyceridemia), disorders of glycemic control (*e.g*., insulin resistance, diabetes), cardiovascular disease (*e.g.,* hypertension, endothelial cell dysfunction), kidney disease (*e.g.,* acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving.

In a further aspect, the present disclosure provides uses of an iRNA agent of the invention in the manufacture of a medicament for preventing at least one symptom in a subject suffering from a disorder that would benefit from a reduction and/or inhibition of KHK expression, such as a KHK-associated disease, *e.g.,* liver disease (*e.g.,* fatty liver, steatohepatitis), dyslipidemia *(e.g.,* hyperlipidemia, high LDL cholesterol, low HDL cholesterol, hypertriglyceridemia, postprandial hypertriglyceridemia), disorders of glycemic control (e.g., insulin resistance, diabetes), cardiovascular disease *(e.g.,* hypertension, endothelial cell dysfunction), kidney disease *(e.g*., acute kidney disorder, tubular dysfunction, proinflammatory changes to the proximal tubules), metabolic syndrome, adipocyte dysfunction, visceral adipose deposition, obesity, hyperuricemia, gout, eating disorders, and excessive sugar craving.

In one case, an iRNA agent targeting KHK is administered to a subject having a KHK-associated disease such that KHK levels, *e.g.,* in a cell, tissue, blood or other tissue or fluid of the subject are reduced by at least about 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 62%, 64%, 65%, 66%, 67%, 68%, 69%, 70%, 71%, 72%, 73%, 74%, 75%, 776%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least about 99% or more when the dsRNA agent is administered to the subject.

The methods and uses include administering a composition described herein such that expression of the target KHK gene is decreased, such as for about 1, 2, 3, 4 5, 6, 7, 8, 12, 16, 18, 24, 28, 32, 36, 40, 44, 48, 52, 56, 60, 64, 68, 72, 76, or about 80 hours. In one embodiment, expression of the target KHK gene is decreased for an extended duration, e.*g.,* at least about two, three, four, five, six, seven days or more, *e.g*., about one week, two weeks, three weeks, or about four weeks or longer.

Administration of the dsRNA according to the methods and uses of the invention may result in a reduction of the severity, signs, symptoms, and/or markers of such diseases or disorders in a patient with a KHK-associated disease. By "reduction" in this context is meant a statistically significant decrease in such level. The reduction can be, for example, at least about 5%, 10%, 15%, 20%, 25%, 30%, 35%, 40%, 45%, 50%, 55%, 60%, 65%, 70%, 75%, 80%, 85%, 90%, 95%, or about 100%.

Efficacy of treatment or prevention of disease can be assessed, for example by measuring disease progression, disease remission, symptom severity, reduction in pain, quality of life, dose of a medication required to sustain a treatment effect, level of a disease marker or any other measurable parameter appropriate for a given disease being treated or targeted for prevention. It is well within the ability of one skilled in the art to monitor efficacy of treatment or prevention by measuring any one of such parameters, or any combination of parameters. For example, efficacy of treatment of dyslipidemia may be assessed, for example, by periodic monitoring of LDL cholesterol, HDL cholesterol and triglyceride levels. In a further example, efficacy of treatment of a glucose control disorder may be assessed, for example, by periodic monitoring of insulin and glucose levels. In another example, efficacy of treatment of obesity may be assessed, for example by periodic monitoring of body mass index. In yet another example, efficacy of treatment of hypertension may be assessed, for example, by periodic monitoring of blood pressure. Comparison of the later readings with the initial readings provide a physician an indication of whether the treatment is effective. It is well within the ability of one skilled in the art to monitor efficacy of treatment or prevention by measuring any one of such parameters, or any combination of parameters. In connection with the administration of an iRNA targeting KHK or pharmaceutical composition thereof, "effective against" a KHK-associated disease indicates that administration in a clinically appropriate manner results in a beneficial effect for at least a statistically significant fraction of patients, such as improvement of symptoms, a cure, a reduction in disease, extension of life, improvement in quality of life, or other effect generally recognized as positive by medical doctors familiar with treating a KHK-associated disease and the related causes.

A treatment or preventive effect is evident when there is a statistically significant improvement in one or more parameters of disease status, or by a failure to worsen or to develop symptoms where they would otherwise be anticipated. As an example, a favorable change of at least 10% in a measurable parameter of disease, and preferably at least 20%, 30%, 40%, 50% or more can be indicative of effective treatment. Efficacy for a given iRNA drug or formulation of that drug can also be judged using an experimental animal model for the given disease as known in the art. When using an experimental animal model, efficacy of treatment is evidenced when a statistically significant reduction in a marker or symptom is observed.

Subjects can be administered a therapeutic amount of iRNA, such as about 0.01 mg/kg, 0.02 mg/kg, 0.03 mg/kg, 0.04 mg/kg, 0.05 mg/kg, 0.1 mg/kg, 0.15 mg/kg, 0.2 mg/kg, 0.25 mg/kg, 0.3 mg/kg, 0.35 mg/kg, 0.4 mg/kg, 0.45 mg/kg, 0.5 mg/kg, 0.55 mg/kg, 0.6 mg/kg, 0.65 mg/kg, 0.7 mg/kg, 0.75 mg/kg, 0.8 mg/kg, 0.85 mg/kg, 0.9 mg/kg, 0.95 mg/kg, 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg dsRNA, 2.6 mg/kg dsRNA, 2.7 mg/kg dsRNA, 2.8 mg/kg dsRNA, 2.9 mg/kg dsRNA, 3.0 mg/kg dsRNA, 3.1 mg/kg dsRNA, 3.2 mg/kg dsRNA, 3.3 mg/kg dsRNA, 3.4 mg/kg dsRNA, 3.5 mg/kg dsRNA, 3.6 mg/kg dsRNA, 3.7 mg/kg dsRNA, 3.8 mg/kg dsRNA, 3.9 mg/kg dsRNA, 4.0 mg/kg dsRNA, 4.1 mg/kg dsRNA, 4.2 mg/kg dsRNA, 4.3 mg/kg dsRNA, 4.4 mg/kg dsRNA, 4.5 mg/kg dsRNA, 4.6 mg/kg dsRNA, 4.7 mg/kg dsRNA, 4.8 mg/kg dsRNA, 4.9 mg/kg dsRNA, 5.0 mg/kg dsRNA, 5.1 mg/kg dsRNA, 5.2 mg/kg dsRNA, 5.3 mg/kg dsRNA, 5.4 mg/kg dsRNA, 5.5 mg/kg dsRNA, 5.6 mg/kg dsRNA, 5.7 mg/kg dsRNA, 5.8 mg/kg dsRNA, 5.9 mg/kg dsRNA, 6.0 mg/kg dsRNA, 6.1 mg/kg dsRNA, 6.2 mg/kg dsRNA, 6.3 mg/kg dsRNA, 6.4 mg/kg dsRNA, 6.5 mg/kg dsRNA, 6.6 mg/kg dsRNA, 6.7 mg/kg dsRNA, 6.8 mg/kg dsRNA, 6.9 mg/kg dsRNA, 7.0 mg/kg dsRNA, 7.1 mg/kg dsRNA, 7.2 mg/kg dsRNA, 7.3 mg/kg dsRNA, 7.4 mg/kg dsRNA, 7.5 mg/kg dsRNA, 7.6 mg/kg dsRNA, 7.7 mg/kg dsRNA, 7.8 mg/kg dsRNA, 7.9 mg/kg dsRNA, 8.0 mg/kg dsRNA, 8.1 mg/kg dsRNA, 8.2 mg/kg dsRNA, 8.3 mg/kg dsRNA, 8.4 mg/kg dsRNA, 8.5 mg/kg dsRNA, 8.6 mg/kg dsRNA, 8.7 mg/kg dsRNA, 8.8 mg/kg dsRNA, 8.9 mg/kg dsRNA, 9.0 mg/kg dsRNA, 9.1 mg/kg dsRNA, 9.2 mg/kg dsRNA, 9.3 mg/kg dsRNA, 9.4 mg/kg dsRNA, 9.5 mg/kg dsRNA, 9.6 mg/kg dsRNA, 9.7 mg/kg dsRNA, 9.8 mg/kg dsRNA, 9.9 mg/kg dsRNA, 9.0 mg/kg dsRNA, 10 mg/kg dsRNA, 15 mg/kg dsRNA, 20 mg/kg dsRNA, 25 mg/kg dsRNA, 30 mg/kg dsRNA, 35 mg/kg dsRNA, 40 mg/kg dsRNA, 45 mg/kg dsRNA, or about 50 mg/kg dsRNA. Values and ranges intermediate to the recited values are also intended.

In certain cases, for example, when a composition of the invention comprises a dsRNA as described herein and a lipid, subjects can be administered a therapeutic amount of iRNA, such as about 0.01 mg/kg to about 5 mg/kg, about 0.01 mg/kg to about 10 mg/kg, about 0.05 mg/kg to about 5 mg/kg, about 0.05 mg/kg to about 10 mg/kg, about 0.1 mg/kg to about 5 mg/kg, about 0.1 mg/kg to about 10 mg/kg, about 0.2 mg/kg to about 5 mg/kg, about 0.2 mg/kg to about 10 mg/kg, about 0.3 mg/kg to about 5 mg/kg, about 0.3 mg/kg to about 10 mg/kg, about 0.4 mg/kg to about 5 mg/kg, about 0.4 mg/kg to about 10 mg/kg, about 0.5 mg/kg to about 5 mg/kg, about 0.5 mg/kg to about 10 mg/kg, about 1 mg/kg to about 5 mg/kg, about 1 mg/kg to about 10 mg/kg, about 1.5 mg/kg to about 5 mg/kg, about 1.5 mg/kg to about 10 mg/kg, about 2 mg/kg to about 2.5 mg/kg, about 2 mg/kg to about 10 mg/kg, about 3 mg/kg to about 5 mg/kg, about 3 mg/kg to about 10 mg/kg, about 3.5 mg/kg to about 5 mg/kg, about 4 mg/kg to about 5 mg/kg, about 4.5 mg/kg to about 5 mg/kg, about 4 mg/kg to about 10 mg/kg, about 4.5 mg/kg to about 10 mg/kg, about 5 mg/kg to about 10 mg/kg, about 5.5 mg/kg to about 10 mg/kg, about 6 mg/kg to about 10 mg/kg, about 6.5 mg/kg to about 10 mg/kg, about 7 mg/kg to about 10 mg/kg, about 7.5 mg/kg to about 10 mg/kg, about 8 mg/kg to about 10 mg/kg, about 8.5 mg/kg to about 10 mg/kg, about 9 mg/kg to about 10 mg/kg, or about 9.5 mg/kg to about 10 mg/kg. Values and ranges intermediate to the recited values are also intended.

For example, the dsRNA may be administered at a dose of about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or about 10 mg/kg. Values and ranges intermediate to the recited values are also intended.

In other cases, for example, when a composition of the invention comprises a dsRNA as described herein and an N-acetylgalactosamine, subjects can be administered a therapeutic amount of iRNA, such as a dose of about 0.1 to about 50 mg/kg, about 0.25 to about 50 mg/kg, about 0.5 to about 50 mg/kg, about 0.75 to about 50 mg/kg, about 1 to about 50 mg/kg, about 1.5 to about 50 mg/kg, about 2 to about 50 mg/kg, about 2.5 to about 50 mg/kg, about 3 to about 50 mg/kg, about 3.5 to about 50 mg/kg, about 4 to about 50 mg/kg, about 4.5 to about 50 mg/kg, about 5 to about 50 mg/kg, about 7.5 to about 50 mg/kg, about 10 to about 50 mg/kg, about 15 to about 50 mg/kg, about 20 to about 50 mg/kg, about 20 to about 50 mg/kg, about 25 to about 50 mg/kg, about 25 to about 50 mg/kg, about 30 to about 50 mg/kg, about 35 to about 50 mg/kg, about 40 to about 50 mg/kg, about 45 to about 50 mg/kg, about 0.1 to about 45 mg/kg, about 0.25 to about 45 mg/kg, about 0.5 to about 45 mg/kg, about 0.75 to about 45 mg/kg, about 1 to about 45 mg/kg, about 1.5 to about 45 mg/kg, about 2 to about 45 mg/kg, about 2.5 to about 45 mg/kg, about 3 to about 45 mg/kg, about 3.5 to about 45 mg/kg, about 4 to about 45 mg/kg, about 4.5 to about 45 mg/kg, about 5 to about 45 mg/kg, about 7.5 to about 45 mg/kg, about 10 to about 45 mg/kg, about 15 to about 45 mg/kg, about 20 to about 45 mg/kg, about 20 to about 45 mg/kg, about 25 to about 45 mg/kg, about 25 to about 45 mg/kg, about 30 to about 45 mg/kg, about 35 to about 45 mg/kg, about 40 to about 45 mg/kg, about 0.1 to about 40 mg/kg, about 0.25 to about 40 mg/kg, about 0.5 to about 40 mg/kg, about 0.75 to about 40 mg/kg, about 1 to about 40 mg/kg, about 1.5 to about 40 mg/kg, about 2 to about 40 mg/kg, about 2.5 to about 40 mg/kg, about 3 to about 40 mg/kg, about 3.5 to about 40 mg/kg, about 4 to about 40 mg/kg, about 4.5 to about 40 mg/kg, about 5 to about 40 mg/kg, about 7.5 to about 40 mg/kg, about 10 to about 40 mg/kg, about 15 to about 40 mg/kg, about 20 to about 40 mg/kg, about 20 to about 40 mg/kg, about 25 to about 40 mg/kg, about 25 to about 40 mg/kg, about 30 to about 40 mg/kg, about 35 to about 40 mg/kg, about 0.1 to about 30 mg/kg, about 0.25 to about 30 mg/kg, about 0.5 to about 30 mg/kg, about 0.75 to about 30 mg/kg, about 1 to about 30 mg/kg, about 1.5 to about 30 mg/kg, about 2 to about 30 mg/kg, about 2.5 to about 30 mg/kg, about 3 to about 30 mg/kg, about 3.5 to about 30 mg/kg, about 4 to about 30 mg/kg, about 4.5 to about 30 mg/kg, about 5 to about 30 mg/kg, about 7.5 to about 30 mg/kg, about 10 to about 30 mg/kg, about 15 to about 30 mg/kg, about 20 to about 30 mg/kg, about 20 to about 30 mg/kg, about 25 to about 30 mg/kg, about 0.1 to about 20 mg/kg, about 0.25 to about 20 mg/kg, about 0.5 to about 20 mg/kg, about 0.75 to about 20 mg/kg, about 1 to about 20 mg/kg, about 1.5 to about 20 mg/kg, about 2 to about 20 mg/kg, about 2.5 to about 20 mg/kg, about 3 to about 20 mg/kg, about 3.5 to about 20 mg/kg, about 4 to about 20 mg/kg, about 4.5 to about 20 mg/kg, about 5 to about 20 mg/kg, about 7.5 to about 20 mg/kg, about 10 to about 20 mg/kg, or about 15 to about 20 mg/kg. In one case, when a composition of the invention comprises a dsRNA as described herein and an N-acetylgalactosamine, subjects can be administered a therapeutic amount of about 10 to about 30 mg/kg of dsRNA. Values and ranges intermediate to the recited values are also intended.

For example, subjects can be administered a therapeutic amount of iRNA, such as about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 31, 32, 33, 34, 34, 35, 36, 37, 38, 39, 40, 41, 42, 43, 44, 45, 46, 47, 48, 49, or about 50 mg/kg. Values and ranges intermediate to the recited values are also intended to be part of this invention.

The iRNA can be administered by intravenous infusion over a period of time, such as over a 5, 6, 7, 8, 9, 10, 11, 12, 13, 14, 15, 16, 17, 18, 19, 20, 21, 22, 23, 24, or about a 25 minute period. The administration may be repeated, for example, on a regular basis, such as weekly, biweekly (*i.e.,* every two weeks) for one month, two months, three months, four months or longer. After an initial treatment regimen, the treatments can be administered on a less frequent basis. For example, after administration weekly or biweekly for three months, administration can be repeated once per month, for six months or a year or longer.

Administration of the iRNA can reduce KHK levels, *e.g.,* in a cell, tissue, blood, urine or other compartment of the patient by at least about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least about 99% or more.

Before administration of a full dose of the iRNA, patients can be administered a smaller dose, such as a 5% infusion, and monitored for adverse effects, such as an allergic reaction. In another example, the patient can be monitored for unwanted immunostimulatory effects, such as increased cytokine (*e.g*., TNF-alpha or INF-alpha) levels.

Owing to the inhibitory effects on KHK expression, a composition according to the invention or a pharmaceutical composition prepared therefrom can enhance the quality of life.

An iRNA of the invention may be administered in "naked" form, where the modified or unmodified iRNA agent is directly suspended in aqueous or suitable buffer solvent, as a "free iRNA." A free iRNA is administered in the absence of a pharmaceutical composition. The free iRNA may be in a suitable buffer solution. The buffer solution may comprise acetate, citrate, prolamine, carbonate, or phosphate, or any combination thereof. In one embodiment, the buffer solution is phosphate buffered saline (PBS). The pH and osmolarity of the buffer solution containing the iRNA can be adjusted such that it is suitable for administering to a subject.

Alternatively, an iRNA of the invention may be administered as a pharmaceutical composition, such as a dsRNA liposomal formulation.

Subjects that would benefit from a reduction and/or inhibition of KHK gene expression are those having a KHK-associated disease or disorder as described herein. In one embodiment, a subject having a KHK-associated disease has liver disease (*e.g*., fatty liver, steatohepatitis). In another embodiment, a subject having a KHK-associate disease has dyslipidemia (*e.g*., hyperlipidemia, high LDL cholesterol, low HDL cholesterol, hypertriglyceridemia, postprandial hypertriglyceridemia). In another embodiment, a subject having a KHK-associate disease has a disorder of glycemic control (*e.g*., insulin resistance, diabetes). In yet another embodiment, a subject having a KHK-associate disease has cardiovascular disease (*e.g*., hypertension, endothelial cell dysfunction). In one embodiment, a subject having a KHK-associate disease has kidney disease (*e.g*., acute kidney disorder, tubular dysfunction, proinflammatory changes to the proximal tubules). In another embodiment, a subject having a KHK-associate disease has metabolic syndrome. In a particular embodiment, a subject having a KHK-associate disease has adipocyte dysfunction. In yet another embodiment, a subject having a KHK-associate disease has visceral adipose deposition. In another embodiment, a subject having a KHK-associate disease has obesity. In a particular embodiment, a subject having a KHK-associate disease has hyperuricemia. In another embodiment, a subject having a KHK-associate disease has gout. In another embodiment, a subject having a KHK-associate disease has an eating disorder and/or excessive sugar craving.

Treatment of a subject that would benefit from a reduction and/or inhibition of KHK gene expression includes therapeutic and prophylactic treatment.

The disclosure further provides methods and uses of an iRNA agent or a pharmaceutical composition thereof for treating a subject that would benefit from reduction and/or inhibition of KHK expression, *e.g*., a subject having a KHK-associated disease, in combination with other pharmaceuticals and/or other therapeutic methods, *e.g*., with known pharmaceuticals and/or known therapeutic methods, such as, for example, those which are currently employed for treating these disorders. For example, in certain cases, an iRNA targeting KHK is administered in combination with, *e.g*., an agent useful in treating a KHK-associated disease as described elsewhere herein. For example, additional therapeutics and therapeutic methods suitable for treating a subject that would benefit from reduction in KHK expression, *e.g*., a subject having a KHK-associated disease, include an HMG-CoA reductase inhibitor, a diabetic therapy, an anti-hypertensive drug, resveratrol, or other therapeutic agents for treating a KHK-associated disease. Exemplary HMG-CoA reductase inhibitors include atorvastatin (Pfizer's Lipitor^{®}/Tahor/Sortis/Torvast/Cardyl), pravastatin (Bristol-Myers Squibb's Pravachol, Sankyo's Mevalotin/Sanaprav), simvastatin (Merck's Zocor^{®}/Sinvacor, Boehringer Ingelheim's Denan, Banyu's Lipovas), lovastatin (Merck's Mevacor/Mevinacor, Bexal's Lovastatina, Cepa; Schwarz Pharma's Liposcler), fluvastatin (Novartis' Lescol^{®}/Locol/Lochol, Fujisawa's Cranoc, Solvay's Digaril), cerivastatin (Bayer's Lipobay/GlaxoSmithKline's Baycol), rosuvastatin (AstraZeneca' s Crestor^{®}), and pitivastatin (itavastatin/risivastatin) (Nissan Chemical, Kowa Kogyo, Sankyo, and Novartis). Exemplary diabetic therapies are known in the art and include, for example, insulin sensitizers, such as biguanides (*e.g.,* metformin) and thiazolidinediones (*e.g.,* rosiglitazone, pioglitazone, troglitazone); secretagogues, such as the sulfonylureas (*e.g.,* glyburide, glipizide, glimepiride, tolbutamide, acetohexamide, tolazamide, chlorpropamide, gliclazide, glycopyamide, gliquidone), the nonsulfonylurea secretagogues, *e.g*., meglitinide derivatives (*e.g.,* repaglinide, nateglinide); the dipeptidyl peptidase IV inhibitors (*e.g.,* sitagliptin, saxagliptin, linagliptin, vildagliptin, allogliptin, septagliptin); alpha-glucosidase inhibitors (*e.g*., acarbose, miglitol, voglibose); amylinomimetics (*e.g*., pramlintide acetate); incretin mimetics (*e.g*., exenatide, liraglutide, taspoglutide); insulin and its analogues (*e.g*., rapid acting, slow acting, and intermediate acting); bile acid sequestrants (*e.g*., colesevelam); and dopamine agonists (*e.g*., bromocriptine), alone or in combinations. Exemplary anti-hypertensive drugs are known in the art and include diuretics (*e.g*., thiazide diuretics (*e.g.,* chlorothiazide, chlorthalidone, hydrochlorothiazide, indapamide, metolazone), loop diuretics (*e.g*., bumetanide, ethacrynic acid, furosemide, torsemide), and potassium-sparing diuretics/aldosterone-receptor blockers (*e.g.,* amiloride, spironolactone, triamterene, eplerenone)), anti-adrenergic drugs (*e.g.,* beta blockers (*e.g*., atenolol, metoprolol, metoprolol extended release, nebivolol, nadolol, pindolol, propranolol, sotalol, timolol), alpha-1-blockers (*e*.g., doxazosin, prazosin, terazosin), alpha and beta blockers (*e.g.,* carvedilol, labetalol), centrally acting agents (*e.g.,* clonidine, methyldopa), peripheral nerve-acting agents (*e.g*., guanethidine, reserpine), and direct-acting vasodilators (*e.g.,* hydralazine, minoxidil)), calcium channel blockers (*e.g.,* amlodipine, diltiazem, felodipine, isradipine, nicardipine, nifedipine, verapamil), ace inhibitors (*e.g.,* benazepril, captopril, enalapril, fosinopril, lisinopril, quinapril, ramipril), angiotensin-receptor blockers (*e.g*., candesartan, eprosartan, irbesartan, losartan, olmesartan, telmisartan, valsartan) or any combinations thereof.

The iRNA agent and an additional therapeutic agent and/or treatment may be administered at the same time and/or in the same combination, *e.g*., parenterally, or the additional therapeutic agent can be administered as part of a separate composition or at separate times and/or by another method known in the art or described herein.

The present disclosure also provides methods of using an iRNA agent of the invention and/or a composition containing an iRNA agent of the invention to reduce and/or inhibit KHK expression in a cell. In other aspects, the present disclosure provides an iRNA of the invention and/or a composition comprising an iRNA of the invention for use in reducing and/or inhibiting KHK expression in a cell. In yet other aspects, use of an iRNA of the invention and/or a composition comprising an iRNA of the invention for the manufacture of a medicament for reducing and/or inhibiting KHK expression in a cell are provided.

The methods and uses include contacting the cell with an iRNA, *e.g.,* a dsRNA, of the invention and maintaining the cell for a time sufficient to obtain degradation of the mRNA transcript of a KHK gene, thereby inhibiting expression of the KHK gene in the cell.

Reduction in gene expression can be assessed by any methods known in the art. For example, a reduction in the expression of KHK may be determined by determining the mRNA expression level of KHK using methods routine to one of ordinary skill in the art, *e.g*., Northern blotting, qRT-PCR, by determining the protein level of KHK using methods routine to one of ordinary skill in the art, such as Western blotting, immunological techniques, flow cytometry methods, ELISA, and/or by determining a biological activity of KHK (*e.g*., phosphorylation of fructose to fructose-1-phosphate). In one case, reduction in KHK gene expression can be determined by measuring the level of fructose in the urine.

In the methods and uses the cell may be contacted *in vitro* or *in vivo, i.e.,* the cell may be within a subject.

A cell suitable for treatment using the methods may be any cell that expresses a KHK gene. A cell suitable for use in the methods and uses of the invention may be a mammalian cell, *e.g.,* a primate cell (such as a human cell or a non-human primate cell, *e.g.,* a monkey cell or a chimpanzee cell), a non-primate cell (such as a cow cell, a pig cell, a camel cell, a llama cell, a horse cell, a goat cell, a rabbit cell, a sheep cell, a hamster, a guinea pig cell, a cat cell, a dog cell, a rat cell, a mouse cell, a lion cell, a tiger cell, a bear cell, or a buffalo cell), a bird cell (*e.g.,* a duck cell or a goose cell), or a whale cell. In one embodiment, the cell is a human cell, *e.g.,* a human liver cell.

KHK expression may be inhibited in the cell by at least about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or about 100%.

The *in vivo* methods and uses may include administering to a subject a composition containing an iRNA, where the iRNA includes a nucleotide sequence that is complementary to at least a part of an RNA transcript of the KHK gene of the mammal to be treated. When the organism to be treated is a human, the composition can be administered by any means known in the art including, but not limited to subcutaneous, intravenous, oral, intraperitoneal, or parenteral routes, including intracranial (e.g., intraventricular, intraparenchymal and intrathecal), intramuscular, transdermal, airway (aerosol), nasal, rectal, and topical (including buccal and sublingual) administration. In certain cases, the compositions are administered by subcutaneous or intravenous infusion or injection.

In some cases, the administration is via a depot injection. A depot injection may release the iRNA in a consistent way over a prolonged time period. Thus, a depot injection may reduce the frequency of dosing needed to obtain a desired effect, e.g., a desired inhibition of KHK, or a therapeutic or prophylactic effect. A depot injection may also provide more consistent serum concentrations. Depot injections may include subcutaneous injections or intramuscular injections. In preferred cases, the depot injection is a subcutaneous injection.

In some cases, the administration is via a pump. The pump may be an external pump or a surgically implanted pump. In certain cases, the pump is a subcutaneously implanted osmotic pump. In other cases, the pump is an infusion pump. An infusion pump may be used for intravenous, subcutaneous, arterial, or epidural infusions. In preferred cases, the infusion pump is a subcutaneous infusion pump. In other embodiments, the pump is a surgically implanted pump that delivers the iRNA to the liver.

The mode of administration may be chosen based upon whether local or systemic treatment is desired and based upon the area to be treated. The route and site of administration may be chosen to enhance targeting.

In one aspect, the present disclosure also provides methods for inhibiting the expression of a KHK gene in a mammal, e.g., a human. The present disclosure also provides a composition comprising an iRNA, *e.g.,* a dsRNA, that targets a KHK gene in a cell of a mammal for use in inhibiting expression of the KHK gene in the mammal. In another aspect, the present disclosure provides use of an iRNA, *e.g.,* a dsRNA, that targets a KHK gene in a cell of a mammal in the manufacture of a medicament for inhibiting expression of the KHK gene in the mammal.

The methods and uses include administering to the mammal, *e.g*., a human, a composition comprising an iRNA, *e.g.,* a dsRNA, that targets a KHK gene in a cell of the mammal and maintaining the mammal for a time sufficient to obtain degradation of the mRNA transcript of the KHK gene, thereby inhibiting expression of the KHK gene in the mammal.

Reduction in gene expression can be assessed in peripheral blood sample of the iRNA-administered subject by any methods known it the art, *e.g*. qRT-PCR, described herein. Reduction in protein production can be assessed by any methods known it the art and by methods, *e.g*., ELISA or Western blotting, described herein. In one case, a puncture liver biopsy sample serves as the tissue material for monitoring the reduction in KHK gene and/or protein expression. In another case, a blood sample serves as the tissue material for monitoring the reduction in KHK gene and/or protein expression.

In one case, verification of RISC medicated cleavage of target *in vivo* following administration of iRNA agent is done by performing 5'-RACE or modifications of the protocol as known in the art (Lasham A et al., (2010) Nucleic Acid Res., 38 (3) p-e19) (Zimmermann et al. (2006) Nature 441: 111-4).

Unless otherwise defined, all technical and scientific terms used herein have the same meaning as commonly understood by one of ordinary skill in the art to which this invention belongs. The examples are illustrative only and not intended to be limiting.

### EXAMPLES

### Example 1. iRNA Synthesis

### Source of reagents

Where the source of a reagent is not specifically given herein, such reagent can be obtained from any supplier of reagents for molecular biology at a quality/purity standard for application in molecular biology.

A detailed list of KHK sense and antisense strand sequences is shown in Tables 3, 4 and 5.

### Transcripts

siRNA design was carried out to identify siRNAs targeting human, cynomolgus monkey (*Macaca fascicularis*; henceforth "cyno"), mouse, and rat KHK transcripts annotated in the NCBI Gene database (http://www.ncbi.nlm.nih.gov/gene/). Design used the following transcripts from the NCBI RefSeq collection: Human -XM_005264298.1; Cyno - XM_005576324.1; Mouse - NM_008439.3; Rat -NM_031855.3. Due to high primate/rodent sequence divergence, siRNA duplexes were designed in several separate batches, including but not limited to batches containing duplexes matching human and cyno transcripts only; human, cyno, and mouse transcripts only; and human, cyno, mouse, and rat transcripts only. Most siRNA duplexes were designed that shared 100% identity in the designated region with the listed human transcript and other species transcripts considered in each design batch (above). In some instances, mismatches between duplex and mRNA target were allowed at the first antisense (last sense) position when the antisense strand:target mRNA complementary basepair was a GC or CG pair. In these cases, duplexes were designed with UA or AU pairs at the first antisense:last sense pair. Thus the duplexes maintained complementarity but were mismatched with respect to target (U:C, U:G, A:C, or A:G).

### siRNA Design, Specificity, and Efficacy Prediction

The specificity of all possible 19mers was predicted from each sequence. Candidate 19mers that lacked repeats longer than 7 nucleotides were then selected. These 476 candidate human/cyno, 71 human/cyno/mouse, and 58 human/cyno/mouse/rat siRNAs were used in comprehensive searches against the appropriate transcriptomes (defined as the set of NM_and XM_records within the human, cyno, mouse, or rat NCBI Refseq sets) using an exhaustive "brute-force" algorithm implemented in the python script 'BruteForce.py'. The script next parsed the transcript-oligo alignments to generate a score based on the position and number of mismatches between the siRNA and any potential 'off-target' transcript. The off-target score is weighted to emphasize differences in the 'seed' region of siRNAs, in positions 2-9 from the 5' end of the molecule. Each oligo-transcript pair from the brute-force search was given a mismatch score by summing the individual mismatch scores; mismatches in the position 2-9 were counted as 2.8, mismatches in the cleavage site positions 10-11 were counted as 1.2, and mismatches in region 12-19 counted as 1.0. An additional off-target prediction was carried out by comparing the frequency of heptamers and octomers derived from 3 distinct, seed-derived hexamers of each oligo. The hexamers from positions 2-7 relative to the 5' start were used to create 2 heptamers and one octomer. Heptamerl was created by adding a 3' A to the hexamer; heptamer2 was created by adding a 5' A to the hexamer; the octomer was created by adding an A to both 5' and 3' ends of the hexamer. The frequency of octomers and heptamers in the human, cyno, mouse, or rat 3'UTRome (defined as the subsequence of the transcriptome from NCBI's Refseq database where the end of the coding region, the 'CDS', is clearly defined) was pre-calculated. The octomer frequency was normalized to the heptamer frequency using the median value from the range of octomer frequencies. A 'mirSeedScore' was then calculated by calculating the sum of ((3 X normalized octomer count) + (2 X heptamer2 count) + (1 X heptamer1 count)).

Both siRNAs strands were assigned to a category of specificity according to the calculated scores: a score above 3 qualifies as highly specific, equal to 3 as specific and between 2 and 2.8 as moderately specific. siRNA strands were sorted by the specificity of the antisense strand. Moderately (or higher) specific duplexes whose antisense oligos possessed characteristics of duplexes with high predicted efficacy, including maximal UA content in the seed region and low overall GC content were selected. One additional duplex with an antisense score of 1.2 in the rat (but >= 2 in the other species) was also included.

Candidate GalNaC-conjugated duplexes, 21 and 23 nucleotides long on the sense and antisense strands respectively, were designed by extending antisense 19mers (described above) to 23 nucleotides of target-complementary sequence. All species transcripts included in the design batch were checked for complementarity. For each duplex, the sense 21mer was specified as the reverse complement of the first 21 nucleotides of the antisense strand.

### siRNA sequence selection

A total of 21 sense and 21 antisense derived human/cyno/mouse/rat siRNA 21/23mer oligos (Table 3), 29 sense and 29 antisense derived human/cyno siRNA 21/23mer oligos (Table 4) and 3 sense and 3 antisense derived human/cyno/mouse (Table 5) siRNA 21/23mer oligos were synthesized.

### siRNA Synthesis

### General Small and Medium Scale RNA Synthesis Procedure

RNA oligonucleotides are synthesized at scales between 0.2-500 µmol using commercially available 5'-*O*-(4,4'-dimethoxytrityl)-2'-*O*-t-butyldimethylsilyl-3'-*O*-(2-cyanoethyl-*N,N*-diisopropyl)phosphoramidite monomers of uridine, 4-N-acetylcytidine, 6-*N*-benzoyladenosine and 2-*N*-isobutyrylguanosine and the corresponding 2'-*O*-methyl and 2'-fluoro phosphoramidites according to standard solid phase oligonucleotide synthesis protocols. The amidite solutions are prepared at 0.1-0.15 M concentration and 5-ethylthio-1H-tetrazole (0.25-0.6 M in acetonitrile) is used as the activator. Phosphorothioate backbone modifications are introduced during synthesis using 0.2 M phenylacetyl disulfide (PADS) in lutidine:acetonitrile (1:1) (v;v) or 0.1 M 3-(dimethylaminomethylene) amino-3H-1,2,4-dithiazole-5-thione (DDTT) in pyridine for the oxidation step. After completion of synthesis, the sequences are cleaved from the solid support and deprotected using methylamine followed by triethylamine.3HF to remove any 2'-*O*-t-butyldimethylsilyl protecting groups present.

For synthesis scales between 5-500 µmol and fully 2' modified sequences (2'-fluoro and/ or 2'-*O*-methyl or combinations thereof) the oligonucleotides are deprotected using 3:1 (v/v) ethanol and concentrated (28-32%) aqueous ammonia either at 35°C 16 h or 55°C for 5.5 h. Prior to ammonia deprotection the oligonucleotides are treated with 0.5 M piperidine in acetonitrile for 20 min on the solid support. The crude oligonucleotides are analyzed by LC-MS and anion-exchange HPLC (IEX-HPLC). Purification of the oligonucleotides is carried out by IEX HPLC using: 20 mM phosphate, 10%-15% ACN, pH = 8.5 (buffer A) and 20 mM phosphate, 10%-15% ACN, 1 M NaBr, pH = 8.5 (buffer B). Fractions are analyzed for purity by analytical HPLC. The product-containing fractions with suitable purity are pooled and concentrated on a rotary evaporator prior to desalting. The samples are desalted by size exclusion chromatography and lyophilized to dryness. Equal molar amounts of sense and antisense strands are annealed in 1x PBS buffer to prepare the corresponding siRNA duplexes.

For small scales (0.2-1 µmol), synthesis is performed on a MerMade 192 synthesizer in a 96 well format. In case of fully 2'-modified sequences (2'-fluoro and/or 2'-*O*-methyl or combinations thereof) the oligonucleotides are deprotected using methylamine at room temperature for 30-60 min followed by incubation at 60°C for 30 min or using 3:1 (v/v) ethanol and concentrated (28-32%) aqueous ammonia at room temperature for 30-60 min followed by incubation at 40°C for 1.5 hours. The crude oligonucleotides are then precipitated in a solution of acetonitrile: acetone (9:1) and isolated by centrifugation and decanting the supernatant. The crude oligonucleotide pellet is re-suspended in 20 mM NaOAc buffer and analyzed by LC-MS and anion exchange HPLC. The crude oligonucleotide sequences are desalted in 96 deep well plates on a 5 mL HiTrap Sephadex G25 column (GE Healthcare). In each well about 1.5 mL samples corresponding to an individual sequence is collected. These purified desalted oligonucleotides are analyzed by LC-MS and anion exchange chromatography. Duplexes are prepared by annealing equimolar amounts of sense and antisense sequences on a Tecan robot. Concentration of duplexes is adjusted to 10 µM in 1x PBS buffer.

### Synthesis of GalNAc-Conjugated Oligonucleotides for In Vivo Analysis

Oligonucleotides conjugated with GalNAc ligand at their 3'-terminus are synthesized at scales between 0.2-500 µmol using a solid support pre-loaded with a Y-shaped linker bearing a 4,4'-dimethoxytrityl (DMT)-protected primary hydroxy group for oligonucleotide synthesis and a GalNAc ligand attached through a tether.

For synthesis of GalNAc conjugates in the scales between 5-500 µmol, the above synthesis protocol for RNA is followed with the following adaptions: For polystyrene-based synthesis supports 5% dichloroacetic acid in toluene is used for DMT-cleavage during synthesis. Cleavage from the support and deprotection is performed as described above. Phosphorothioate-rich sequences (usually > 5 phorphorothioates) are synthesized without removing the final 5'-DMT group ("DMT-on") and, after cleavage and deprotection as described above, purified by reverse phase HPLC using 50 mM ammonium acetate in water (buffer A) and 50 mM ammoniumacetate in 80% acetonitirile (buffer B). Fractions are analyzed for purity by analytical HPLC and/or LC-MS. The product-containing fractions with suitable purity are pooled and concentrated on a rotary evaporator. The DMT-group is removed using 20%-25% acetic acid in water until completion. The samples are desalted by size exclusion chromatography and lyophilized to dryness. Equal molar amounts of sense and antisense strands are annealed in 1x PBS buffer to prepare the corresponding siRNA duplexes.

For small scale synthesis of GalNAc conjugates (0.2-1 µmol), including sequences with multiple phosphorothioate linkages, the protocols described above for synthesis of RNA or fully 2'-F/2'-OMe-containing sequences on MerMade platform are applied. Synthesis is performed on pre-packed columns containing GalNAc-functionalized controlled pore glass support.

### Example 2. General In vitro screening of siRNA duplexes

The *in vitro* efficacy of the duplexes can be determined in single dose screens for any RNAi targeted gene expression using the following methods. Similar methods may be used for multi-dose screens to determine the dose response of the duplexes and to calculate the IC₅₀ of the duplexes.

### Cell culture and transfections

Hep3B cells (ATCC, Manassas, VA) are grown to near confluence at 37°C in an atmosphere of 5% CO2 in Eagle's Minimum Essential Medium (ATCC) supplemented with 10% FBS, streptomycin, and glutamine (ATCC) before being released from the plate by trypsinization. Cells are washed and re-suspended at 0.25 x10⁶ cells/ml. During transfections, cells are plated onto a 96-well plate with about 20,000 cells per well.

Primary mouse hepatocytes (PMH) are freshly isolated from a C57BL/6 female mouse (Charles River Labortories International, Inc. Willmington, MA) less than 1 hour prior to transfections and grown in primary hepatocyte media. Cells are resuspended at 0.11x106 cells/ml in InVitroGRO CP Rat (plating) medium (Celsis In Vitro Technologies, catalog number S01494). During transfections, cells are plated onto a BD BioCoat 96 well collagen plate (BD, 356407) at 10,000 cells per well and incubated at 37°C in an atmosphere of 5% CO2.

Cryopreserved Primary Cynomolgus Hepatocytes (Celsis In Vitro Technologies, M003055-P) are thawed at 37°C water bath immediately prior to usage and re-suspended at 0.26x10⁶ cells/ml in InVitroGRO CP (plating) medium (Celsis In Vitro Technologies, catalog number Z99029). During transfections, cells are plated onto a BD BioCoat 96 well collagen plate (BD, 356407) at 25,000 cells per well and incubated at 37°C in an atmosphere of 5% CO2.

For Hep3B, PMH, and primary Cynomolgus hepatocytes, transfection are carried out by adding 14.8 µl of Opti-MEM plus 0.2 µl of Lipofectamine RNAiMax per well (Invitrogen, Carlsbad CA. catalog number 13778-150) to 5 µl of each siRNA duplex to an individual well in a 96-well plate. The mixture is then incubated at room temperature for 20 minutes. Eighty µl of complete growth media without antibiotic containing the appropriate cell number are then added to the siRNA mixture. Cells are incubated for 24 hours prior to RNA purification.

Single dose experiments are performed at 10nM and 0.1nM final duplex concentration for GalNAc modified sequences or at 1nM and 0.01nM final duplex concentration for all other sequences. Dose response experiments are done at 3, 1, 0.3, 0.1, 0.037, 0.0123, 0.00412, and 0.00137 nM final duplex concentration for primary mouse hepatocytes and at 3, 1, 0.3, 0.1, 0.037, 0.0123, 0.00412, 0.00137, 0.00046, 0.00015, 0.00005, and 0.000017 nM final duplex concentration for Hep3B cells.

### Free uptake transfection

Free uptake experiments are performed by adding 10µl of siRNA duplexes in PBS per well into a 96 well plate. Ninety µl of complete growth media containing appropriate cell number for the cell type is then added to the siRNA. Cells are incubated for 24 hours prior to RNA purification. Single dose experiments are performed at 500nM and 5nM final duplex concentration and dose response experiments are done at 1000, 333, 111, 37, 12.3, 4.12, 1.37, 0.46 nM final duplex concentration.

### Total RNA isolation using DYNABEADS mRNA Isolation Kit (Invitrogen, part #: 610-12)

Cells are harvested and lysed in 150 µl of Lysis/Binding Buffer then mixed for 5 minutes at 850 rpm using an Eppendorf Thermomixer (the mixing speed is the same throughout the process). Ten microliters of magnetic beads and 80 µl Lysis/Binding Buffer mixture are added to a round bottom plate and mixed for 1 minute. Magnetic beads are captured using a magnetic stand and the supernatant is removed without disturbing the beads. After removing the supernatant, the lysed cells are added to the remaining beads and mixed for 5 minutes. After removing the supernatant, magnetic beads are washed 2 times with 150 µl Wash Buffer A and mixed for 1 minute. The beads are captured again and the supernatant is removed. The beads are then washed with 150 µl Wash Buffer B, captured and the supernatant is removed. The beads are next washed with 150 µl Elution Buffer, captured and the supernatant removed. Finally, the beads are allowed to dry for 2 minutes. After drying, 50 µl of Elution Buffer is added and mixed for 5 minutes at 70°C. The beads are captured on a magnet for 5 minutes. Forty-five µl of supernatant is removed and added to another 96 well plate.

### General cDNA synthesis using ABI High capacity cDNA reverse transcription kit (Applied Biosystems, Foster City, CA, Cat #4368813)

A master mix of 2 µl 10X Buffer, 0.8 µl 25X dNTPs, 2 µl Random primers, 1 µl Reverse Transcriptase, 1 µl RNase inhibitor and 3.2 µl of H2O per reaction is prepared. Equal volumes master mix and RNA are mixed for a final volume of 12 µl for *in vitro* screened or 20 µl for *in* vivo screened samples. cDNA is generated using a Bio-Rad C-1000 or S-1000 thermal cycler (Hercules, CA) through the following steps: 25°C for 10 minutes, 37°C for 120 minutes, 85°C for 5 seconds, and 4°C hold.

### Real time PCR

Two µl of cDNA are added to a master mix containing 2µl of H₂O, 0.5µl GAPDH TaqMan Probe (Life Technologies catalog number 4326317E for Hep3B cells, catalog number 352339E for primary mouse hepatocytes or custom probe for cynomolgus primary hepatocytes), 0.5µl C5 TaqMan probe (Life Technologies c catalog number Hs00156197_m1 for Hep3B cells or mm00439275_m1 for Primary Mouse Hepatoctyes or custom probe for cynomolgus primary hepatocytes) and 5µl Lightcycler 480 probe master mix (Roche catalog number 04887301001) per well in a 384 well plates (Roche catalog number 04887301001). Real time PCR is performed in an Roche LC480 Real Time PCR system (Roche) using the ΔΔCt(RQ) assay. For *in vitro* screening, each duplex is tested with two biological replicates unless otherwise noted and each Real Time PCR is performed in duplicate technical replicates. For *in vivo* screening, each duplex is tested in one or more experiments (3 mice per group) and each Real Time PCR is run in duplicate technical replicates.

To calculate relative fold change in KHK mRNA levels, real time data are analyzed using the ΔΔCt method and normalized to assays performed with cells transfected with 10 nM AD-1955, or mock transfected cells. IC₅₀s are calculated using a 4 parameter fit model using XLFit and normalized to cells transfected with AD-1955 over the same dose range, or to its own lowest dose.

The sense and antisense sequences of AD-1955 are:
SENSE: cuuAcGcuGAGuAcuucGAdTsdT (SEQ ID NO: 17)
ANTISENSE: UCGAAGuACUcAGCGuAAGdTsdT (SEQ ID NO:18).

**Table 2. Abbreviations of nucleotide monomers used in nucleic acid sequence representation. It will be understood that these monomers, when present in an oligonucleotide, are mutually linked by 5'-3'-phosphodiester bonds.**

| **Abbreviation** | **Nucleotide(s)** |
|---|---|
| A | Adenosine-3'-phosphate |
| Af | 2'-fluoroadenosine-3'-phosphate |
| Afs | 2'-fluoroadenosine-3'-phosphorothioate |
| As | adenosine-3'-phosphorothioate |
| C | cytidine-3'-phosphate |
| Cf | 2'-fluorocytidine-3'-phosphate |
| Cfs | 2'-fluorocytidine-3'-phosphorothioate |
| Cs | cytidine-3'-phosphorothioate |
| G | guanosine-3'-phosphate |
| Gf | 2'-fluoroguanosine-3'-phosphate |
| Gfs | 2'-fluoroguanosine-3'-phosphorothioate |
| Gs | guanosine-3'-phosphorothioate |
| T | 5'-methyluridine-3'-phosphate |
| Tf | 2'-fluoro-5-methyluridine-3'-phosphate |
| Tfs | 2'-fluoro-5-methyluridine-3'-phosphorothioate |
| Ts | 5-methyluridine-3'-phosphorothioate |
| U | Uridine-3'-phosphate |
| Uf | 2'-fluorouridine-3'-phosphate |
| Ufs | 2'-fluorouridine-3'-phosphorothioate |
| Us | uridine -3'-phosphorothioate |
| N | any nucleotide (G, A, C, T or U) |
| a | 2'-O-methyladenosine-3'-phosphate |
| as | 2'-O-methyladenosine-3'-phosphorothioate |
| c | 2'-O-methylcytidine-3'-phosphate |
| cs | 2'-O-methylcytidine-3'-phosphorothioate |
| g | 2'-O-methylguanosine-3'-phosphate |
| gs | 2'-O-methylguanosine-3'-phosphorothioate |
| t | 2'-O-methyl-5-methyluridine-3'-phosphate |
| ts | 2'-O-methyl-5-methyluridine-3'-phosphorothioate |
| u | 2'-O-methyluridine-3'-phosphate |
| us | 2'-O-methyluridine-3'-phosphorothioate |
| s | phosphorothioate linkage |
| L96 | N-[tris(GalNAc-alkyl)-amidodecanoyl)]-4-hydroxyprolinol Hyp-(GalN Ac-alkyl)3 |

**Table 3. Unmodified Sense and Antisense Strand Sequences of KHK dsRNAs**

| Ref Seq; Corresponding mRNA position of sense strand | Target Site | Sense (5'-3') | SEQ ID NO: | Antisense (5'-3') | SEQ ID NO: |
|---|---|---|---|---|---|
| XM_005264298_816-838_s | 816 | UGUCAGCAAAGAUGUGGCCAA | 19 | UUGGCCACAUCUUUGCUGACAAA | 40 |
| XM_005264298_504-526_s | 504 | AGAGAAGCAGAUCCUGUGCGU | 20 | ACGCACAGGAUCUGCUUCUCUUC | 41 |
| XM_005264298_810-832_s | 810 | GGUGUUUGUCAGCAAAGAUGU | 21 | ACAUCUUUGCUGACAAACACCAC | 42 |
| XM_005264298_651-673_s | 651 | GAUCCACAUUGAGGGCCGGAA | 22 | UUCCGGCCCUCAAUGUGGAUCCA | 43 |
| XM_005264298_815-837_s | 815 | UUGUCAGCAAAGAUGUGGCCA | 23 | UGGCCACAUCUUUGCUGACAAAC | 44 |
| XM_005264298_650-672_s | 650 | GGAUCCACAUUGAGGGCCGGA | 24 | UCCGGCCCUCAAUGUGGAUCCAC | 45 |
| XM_005264298_510-532_s | 510 | GCAGAUCCUGUGCGUGGGGCU | 25 | AGCCCCACGCACAGGAUCUGCUU | 46 |
| XM_005264298_813-835_C21A_s | 813 | GUUUGUCAGCAAAGAUGUGGA | 26 | UCCACAUCUUUGCUGACAAACAC | 47 |
| XM_005264298_505-527_G21A_s | 505 | GAGAAGCAGAUCCUGUGCGUA | 27 | UACGCACAGGAUCUGCUUCUCUU | 48 |
| XM_005264298_644-666_G21A_s | 644 | UCAAGUGGAUCCACAUUGAGA | 28 | UCUCAAUGUGGAUCCACUUGAAC | 49 |
| XM_005264298_648-670_G21A_s | 648 | GUGGAUCCACAUUGAGGGCCA | 29 | UGGCCCUCAAUGUGGAUCCACUU | 50 |
| XM_005264298_646-668_C21A_s | 646 | AAGUGGAUCCACAUUGAGGGA | 30 | UCCCUCAAUGUGGAUCCACUUGA | 51 |
| XM_005264298_811-833G21A_s | 811 | GUGUUUGUCAGCAAAGAUGUA | 31 | UACAUCUUUGCUGACAAACACCA | 52 |
| XM_005264298_812-834_G21A_s | 812 | UGUUUGUCAGCAAAGAUGUGA | 32 | UCACAUCUUUGCUGACAAACACC | 53 |
| XM_005264298_649-671_C21A_s | 649 | UGGAUCCACAUUGAGGGCCGA | 33 | UCGGCCCUCAAUGUGGAUCCACU | 54 |
| XM_005264298_507-529_G21A_s | 507 | GAAGCAGAUCCUGUGCGUGGA | 34 | UCCACGCACAGGAUCUGCUUCUC | 55 |
| XM_005264298_502-524_C21A_s | 502 | GAAGAGAAGCAGAUCCUGUGA | 35 | UCACAGGAUCUGCUUCUCUUCCA | 56 |
| XM_005264298_645-667_G21A_s | 645 | CAAGUGGAUCCACAUUGAGGA | 36 | UCCUCAAUGUGGAUCCACUUGAA | 57 |
| XM_005264298_647-669_C21A_s | 647 | AGUGGAUCCACAUUGAGGGCA | 37 | UGCCCUCAAUGUGGAUCCACUUG | 58 |
| XM_005264298_503-525_G21A_s | 503 | AAGAGAAGCAGAUCCUGUGCA | 38 | UGCACAGGAUCUGCUUCUCUUCC | 59 |
| XM_005264298_506-528_G21A_s | 506 | AGAAGCAGAUCCUGUGCGUGA | 39 | UCACGCACAGGAUCUGCUUCUCU | 60 |

**Table 4. Unmodified Sense and Antisense Strand Sequences of KHK dsRNAs**

| Ref Seq; Corresponding mRNA position of sense strand | Target Site | Sense (5'-3') | SEQ ID NO: | Antisense (5'-3') | SEQ ID NO: |
|---|---|---|---|---|---|
| XM_005264298.1_593-615_s | 593 | GCCUGCCAGAUGUGUCUGCUA | 61 | UAGCAGACACAUCUGGCAGGCUC | 90 |
| XM_005264298.1_642-664_s | 642 | GUUCAAGUGGAUCCACAUUGA | 62 | UCAAUGUGGAUCCACUUGAACUG | 91 |
| XM_005264298.1_640-662_s | 640 | CAGUUCAAGUGGAUCCACAUU | 63 | AAUGUGGAUCCACUUGAACUGGG | 92 |
| XM_005264298.1_808-830_s | 808 | GUGGUGUUUGUCAGCAAAGAU | 64 | AUCUUUGCUGACAAACACCACGU | 93 |
| XM_005264298.1_643-665_G21A_s | 643 | UUCAAGUGGAUCCACAUUGAA | 65 | UUCAAUGUGGAUCCACUUGAACU | 94 |
| XM_005264298.1_806-828_G21U_s | 806 | ACGUGGUGUUUGUCAGCAAAU | 66 | AUUUGCUGACAAACACCACGUCU | 95 |
| XM_005264298.1_641-663_G21A_s | 641 | AGUUCAAGUGGAUCCACAUUA | 67 | UAAUGUGGAUCCACUUGAACUGG | 96 |
| XM_005264298.1_877-899_s | 877 | UUGUAUGGUCGUGUGAGGAAA | 68 | UUUCCUCACACGACCAUACAAGC | 97 |
| XM_005264298.1_795-817_s | 795 | UGGCUACGGAGACGUGGUGUU | 69 | AACACCACGUCUCCGUAGCCAAA | 98 |
| XM_005264298.1_828-850_s | 828 | UGUGGCCAAGCACUUGGGGUU | 70 | AACCCCAAGUGCUUGGCCACAUC | 99 |
| XM_005264298.1_639-661_s | 639 | CCAGUUCAAGUGGAUCCACAU | 71 | AUGUGGAUCCACUUGAACUGGGU | 100 |
| XM_005264298.1_804-826_s | 804 | AGACGUGGUGUUUGUCAGCAA | 72 | UUGCUGACAAACACCACGUCUCC | 101 |
| XM_005264298.1_555-577_s | 555 | GGUGGACAAGUACCCUAAGGA | 73 | UCCUUAGGGUACUUGUCCACCAG | 102 |
| XM_005264298.1_632-654_s | 632 | AUCUGACCCAGUUCAAGUGGA | 74 | UCCACUUGAACUGGGUCAGAUCA | 103 |
| XM_005264298.1_883-905_s | 883 | GGUCGUGUGAGGAAAGGGGCU | 75 | AGCCCCUUUCCUCACACGACCAU | 104 |
| XM_005264298.1_675-697_s | 675 | AUCGGAGCAGGUGAAGAUGCU | 76 | AGCAUCUUCACCUGCUCCGAUGC | 105 |
| XM_005264298.1_800-822_s | 800 | ACGGAGACGUGGUGUUUGUCA | 77 | UGACAAACACCACGUCUCCGUAG | 106 |
| XM_005264298.1_513-535_s | 513 | GAUCCUGUGCGUGGGGCUAGU | 78 | ACUAGCCCCACGCACAGGAUCUG | 107 |
| XM_005264298.1_875-897_s | 875 | GCUUGUAUGGUCGUGUGAGGA | 79 | UCCUCACACGACCAUACAAGCCC | 108 |
| XM 005264298.1_796-818_s | 796 | GGCUACGGAGACGUGGUGUUU | 80 | AAACACCACGUCUCCGUAGCCAA | 109 |
| XM_005264298.1_891-913_s | 891 | GAGGAAAGGGGCUGUGCUUGU | 81 | ACAAGCACAGCCCCUUUCCUCAC | 110 |
| XM_005264298.1_624-646_s | 624 | GAAGGUUGAUCUGACCCAGUU | 82 | AACUGGGUCAGAUCAACCUUCUC | 111 |
| XM_005264298.1_552-574_s | 552 | CCUGGUGGACAAGUACCCUAA | 83 | UUAGGGUACUUGUCCACCAGGCU | 112 |
| XM_005264298.1_619-641_C21A_s | 619 | UUUGAGAAGGUUGAUCUGACA | 84 | UGUCAGAUCAACCUUCUCAAAGU | 113 |
| XM_005264298.1_628-650_G21A_s | 628 | GUUGAUCUGACCCAGUUCAAA | 85 | UUUGAACUGGGUCAGAUCAACCU | 114 |
| XM_005264298.1_873-895_G21A_s | 873 | GGGCUUGUAUGGUCGUGUGAA | 86 | UUCACACGACCAUACAAGCCCCU | 115 |
| XM 005264298.1_836-858_G21A_s | 836 | AGCACUUGGGGUUCCAGUCAA | 87 | UUGACUGGAACCCCAAGUGCUUG | 116 |
| XM_005264298.1_797-819_G21A_s | 797 | GCUACGGAGACGUGGUGUUUA | 88 | UAAACACCACGUCUCCGUAGCCA | 117 |
| XM_005264298.1_802-824_C21A_s | 802 | GGAGACGUGGUGUUUGUCAGA | 89 | UCUGACAAACACCACGUCUCCGU | 118 |

**Table 5. Unmodified Sense and Antisense Strand Sequences of KHK dsRNAs**

| Ref Seq; Corresponding mRNA position of sense strand | Target Site | Sense (5'-3') | SEQ ID NO: | Antisense (5'-3') | SEQ ID NO: |
|---|---|---|---|---|---|
| XM_005264298.1_685-707_s | 685 | GUGAAGAUGCUGCAGCGGAUA | 119 | UAUCCGCUGCAGCAUCUUCACCU | 122 |
| XM_005264298.1_687-709_s | 687 | GAAGAUGCUGCAGCGGAUAGA | 120 | UCUAUCCGCUGCAGCAUCUUCAC | 123 |
| XM_005264298.1_686-708_G21A_s | 686 | UGAAGAUGCUGCAGCGGAUAA | 121 | UUAUCCGCUGCAGCAUCUUCACC | 124 |

### Example 3. Design, Synthesis, and in Vitro Screening of Additional siRNAs

### siRNA design

An additional set of siRNAs targeting the human KHK, "ketohexokinase (fructokinase)" (human: NCBI refseqID XM_005264298; NCBI GeneID: 3795), as well as toxicology-species KHK orthologs (cynomolgus monkey: XM_005545463; mouse: NM_008439; rat, NM_031855) were designed using custom R and Python scripts. The human XM_005264298 REFSEQ mRNA has a length of 2146 bases. The rationale and method for the set of siRNA designs is as follows: the predicted efficacy for every potential 19mer siRNA from position 501 through position 2146 (the coding region and 3' UTR) was determined with a linear model derived from the direct measure of mRNA knockdown from more than 20,000 distinct siRNA designs targeting a large number of vertebrate genes. Subsets of the KHK siRNAs were designed with perfect or near-perfect matches between human, cynomolgus and rodent species as well as a subset targeting human and cynomolgus monkey alone. A further subset was designed with perfect or near-perfect matches to mouse and rat KHK orthologs. For each strand of the siRNA, a custom Python script was used in a brute force search to measure the number and positions of mismatches between the siRNA and all potential alignments in the target species transcriptome. Extra weight was given to mismatches in the seed region, defined here as positions 2-9 of the antisense oligonucleotide, as well the cleavage site of the siRNA, defined here as positions 10-11 of the antisense oligonucleotide. The relative weight of the mismatches was 2.8; 1.2: 1 for seed mismatches, cleavage site, and other positions up through antisense position 19. Mismatches in the first position were ignored. A specificity score was calculated for each strand by summing the value of each weighted mismatch. Preference was given to siRNAs whose antisense score in human and cynomolgus monkey was >= 3.0 and predicted efficacy was >= 70% knockdown of the XM_005264298 transcript.

### Synthesis

KHK siRNA sequences were synthesized at a 1 µmol scale on Mermade 192 synthesizer (BioAutomation) using the solid support mediated phosphoramidite chemistry. The solid support was controlled pore glass (500 °A) loaded with custom GalNAc ligand or universal solid support (AM biochemical). Ancillary synthesis reagents, 2'-F and 2'-O-Methyl RNA and deoxy phosphoramidites were obtained from Thermo-Fisher (Milwaukee, WI) and Hongene (China). 2'F, 2'-O-Methyl, RNA, DNA and other modified nucleosides were introduced in the sequences using the corresponding phosphoramidites. Synthesis of 3' GalNAc conjugated single strands was performed on a GalNAc modified CPG support. Custom CPG universal solid support was used for the synthesis of antisense single strands. Coupling time for all phosphoramidites (100 mM in acetonitrile) was 5 minutes employing 5-Ethylthio-1H-tetrazole (ETT) as activator (0.6 M in acetonitrile). Phosphorothioate linkages were generated using a 50 mM solution of 3-((Dimethylamino-methylidene)amino)-3H-1,2, 4-dithiazole-3-thione (DDTT, obtained from Chemgenes (Wilmington, MA, USA) in anhydrous acetonitrile/pyridine (1:1 v/v). Oxidation time was 3 minutes. All sequences were synthesized with final removal of the DMT group ("DMT off").

Upon completion of the solid phase synthesis, oligoribonucleotides were cleaved from the solid support and deprotected in sealed 96 deep well plates using 200 µL Aqueous Methylamine reagent at 60 °C for 20 minutes. For sequences containing 2' ribo residues (2'-OH) that are protected with tert-butyl dimethyl silyl (TBDMS) group, a second step deprotection was performed using TEA.3HF (triethylamine trihydro fluoride) reagent. To the methylamine deprotection solution, 200 µL of dimethyl sulfoxide (DMSO) and 300 µl TEA.3HF reagent was added and the solution was incubated for additional 20 minutes at 60°C. At the end of cleavage and deprotection step, the synthesis plate was allowed to come to room temperature and was precipitated by addition of 1mL of acetontile: ethanol mixture (9:1). The plates were cooled at - 80 °C for 2 hours and the superanatant decanted carefully with the aid of a multi channel pipette. The oligonucleotide pellet was re-suspended in 20 mM NaOAc buffer and were desalted using a 5 mL HiTrap size exclusion column (GE Healthcare) on an AKTA Purifier System equipped with an A905 autosampler and a Frac 950 fraction collector. Desalted samples were collected in 96 well plates. Samples from each sequence were analyzed by LC-MS to confirm the identity, UV (260 nm) for quantification and a selected set of samples by IEX chromatography to determine purity.

Annealing of KHK single strands was performed on a Tecan liquid handling robot. Equimolar mixture of sense and antisense single strands were combined and annealed in 96 well plates. After combining the complementary single strands, the 96 well plate was sealed tightly and heated in an oven at 100 °C for 10 minutes and allowed to come slowly to room temperature over a period 2-3 hours. The concentration of each duplex was normalized to 10 uM in IX PBS and then submitted for in vitro screening assays.

### Cell culture and transfections

Hep3b cells were transfected by adding 4.9µl of Opti-MEM plus 0.1µl of Lipofectamine RNAiMax per well (Invitrogen, Carlsbad CA. cat # 13778-150) to 5µl of siRNA duplexes per well into a 384-well plate and incubated at room temperature for 15 minutes. Forty µl of DMEM containing ∼5 x10³ cells were then added to the siRNA mixture. Cells were incubated for 24 hours prior to RNA purification. Single dose experiments were performed at 10nM and 0.1nM final duplex concentration and dose response experiments were done over a range of doses from 10nM to 36fM final duplex concentration over 8, 6-fold dilutions.

### Total RNA isolation using DYNABEADS mRNA Isolation Kit

RNA was isolated using an automated protocol on a BioTek-EL406 platform using DYNABEADs (Invitrogen, cat#61012). Briefly, 50µl of Lysis/Binding Buffer and 25µl of lysis buffer containing 3µl of magnetic beads were added to the plate with cells. Plates were incubated on an electromagnetic shaker for 10 minutes at room temperature and then magnetic beads were captured and the supernatant was removed. Bead-bound RNA was then washed 2 times with 150µl Wash Buffer A and once with Wash Buffer B. Beads were then washed with 150µl Elution Buffer, re-captured and supernatant removed.

### cDNA synthesis using ABI High capacity cDNA reverse transcription kit (Applied Biosystems, Foster City, CA, Cat #4368813)

Ten µl of a master mix containing 1µl 10X Buffer, 0.4µl 25X dNTPs, 1µl 10x Random primers, 0.5µl Reverse Transcriptase, 0.5µl RNase inhibitor and 6.6µl of H2O per reaction was added to RNA isolated above. Plates were sealed, mixed, and incubated on an electromagnetic shaker for 10 minutes at room temperature, followed by 2h 37°C. Plates were then incubated at 81°C for 8min.

### Real time PCR

Two µl of cDNA were added to a master mix containing 0.5µl of GAPDH TaqMan Probe (Hs99999905), 0.5µl KHK probe (Hs00240827_m1) and 5µl Lightcycler 480 probe master mix (Roche Cat # 04887301001) per well in a 384 well plates (Roche cat # 04887301001). Real time PCR was done in a LightCycler480 Real Time PCR system (Roche) using the ΔΔCt(RQ) assay. Each duplex was tested in four independent transfections.

To calculate relative fold change, real time data were analyzed using the ΔΔCt method and normalized to assays performed with cells transfected with 10nM AD-1955, or mock transfected cells. IC50s were calculated using a 4 parameter fit model using XLFit and normalized to cells transfected with AD-1955 or naive cells.

A detailed list of KHK sense and antisense strand sequences is shown in Tables 6 and 7.

The results of the single dose screens are provided in Table 8. Data are expressed as percent message remaining relative to AD-1955.

Table 9 shows the dose response of a subset of agents in Hep3B cells transfected with the indicated iRNAs. The indicated IC₅₀ values represent the IC₅₀ values relative to untreated cells.

**Table 6. KHK Unmodified Sequences**

| Duplex Name | Sense strand | SEQ ID NO: | Ref Seq; Corresponding target mRNA position | Antisense strand | SEQ ID NO: | Corresponding target mRNA position |
|---|---|---|---|---|---|---|
| AD-63824 | AUCAAUGUGGUGGACAAAUAA | 125 | NM_008439.3_70-90_C21A_s | UUAUUUGUCCACCACAUUGAUGA | 175 | NM_008439.3_68-90_C21A_as |
| AD-63829 | GGUGGACAAAUACCCAGAGGA | 126 | NM_008439.3_78-9880218_s | UCCUCUGGGUAUUUGUCCACCAC | 176 | NM_ 008439.3_76-9880218_as |
| AD-63855 | CUUUGAGAAGGUCGAUCUGAA | 127 | NM_008439.3_393-413_C21A_ s | UUCAGAUCGACCUUCUCAAAGUC | 177 | NM_008439.3_391-413_C21A_ as |
| AD-63835 | CCCGGUUCAAGUGGAUCCACA | 128 | NM_008439.3_413-4335-213_s | UGUGGAUCCACUUGAACCGGGUC | 178 | NM _008439.3_411-4335-213_as |
| AD-63845 | UGUGGCCAAGCACCUGGGGUU | 129 | NM_008439.3_603-6235-213_s | AACCCCAGGUGCUUGGCCACAUC | 179 | NM**_** 008439.3_601-6235-213_as |
| AD-63823 | UUGCAGGGGUUUGAUGGCAUU | 130 | NM_008439.3_892-9124-212_s | AAUGCCAUCAAACCCCUGCAAGC | 180 | NM_ 008439.3_890-9124-212_as |
| AD-63863 | GAAGAGAAGCAGAUCCUGUGA | 131 | XM_ 005264298.1_504-524_C21_A_s | UCACAGGAUCUGCUUCUCUUCCA | 181 | XM_005264298.1_5 02-524_C21A_as |
| AD-63881 | AAGAGAAGCAGAUCCUGUGCA | 132 | XM_ 005264298.1_505-525_G21A_s | UGCACAGGAUCUGCUUCUCUUCC | 182 | XM_005264298.1_5 03-525_G21A_as |
| AD-63862 | GAGAAGCAGAUCCUGUGCGUA | 133 | XM_ 005264298.1_507-527_G21A_s | UACGCACAGGAUCUGCUUCUCUU | 183 | XM_ 005264298.1_505-527_G21A_ as |
| AD-63887 | AGAAGCAGAUCCUGUGCGUGA | 134 | XM_ 005264298.1_508-528_G21A_s | UCACGCACAGGAUCUGCUUCUCU | 184 | XM_ 005264298.1_506-528_G21A_ as |
| AD-63902 | GAAGCAGAUCCUGUGCGUGGA | 135 | XM_ 005264298.1_509-529_G21A_s | UCCACGCACAGGAUCUGCUUCUC | 185 | XM_ 005264298.1_507-529_G21A_ as |
| AD-63896 | GCAGAUCCUGUGCGUGGGGCU | 136 | XM_005264298.1_512-532_s | AGCCCCACGCACAGGAUCUGCUU | 186 | XM_005264298.1_510-532_as |
| AD-63843 | GAUCCUGUGCGUGGGGCUAGU | 137 | XM_005264298.1_515-535_s | ACUAGCCCCACGCACAGGAUCUG | 187 | XM_005264298.1_513-535_as |
| AD-63857 | GGUGGACAAGUACCCUAAGGA | 138 | XM_005264298.1_557-577_s | UCCUUAGGGUACUUGUCCACCAG | 188 | XM_005264298.1_555-577_as |
| AD-63836 | GCCUGCCAGAUGUGUCUGCUA | 139 | XM_005264298.1_595-615_s | UAGCAGACACAUCUGGCAGGCUC | 189 | XM_005264298.1_593-615_as |
| AD-63834 | UUUGAGAAGGUUGAUCUGACA | 140 | XM_005264298.1_621-641_ C21A_ s | UGUCAGAUCAACCUUCUCAAAGU | 190 | XM_ 005264298.1_619-641_C21A_ as |
| AD-63839 | GUUGAUCUGACCCAGUUCAAA | 141 | XM_ 005264298.1_630-650_G21A_s | UUUGAACUGGGUCAGAUCAACCU | 191 | XM 005264298.1_628-650 G21A_as |
| AD-63821 | AUCUGACCCAGUUCAAGUGGA | 142 | XM_005264298.1_634-654_s | UCCACUUGAACUGGGUCAGAUCA | 192 | XM_005264298.1_632-654_as |
| AD-63847 | CCAGUUCAAGUGGAUCCACAU | 143 | XM_005264298.1_641-661_s | AUGUGGAUCCACUUGAACUGGGU | 193 | XM_005264298.1_639-661_as |
| AD-63846 | CAGUUCAAGUGGAUCCACAUU | 144 | XM_005264298.1_642-662_s | AAUGUGGAUCCACUUGAACUGGG | 194 | XM 005264298.1_640-662_as |
| AD-63826 | AGUUCAAGUGGAUCCACAUUA | 145 | XM_ 005264298.1_643-663_G21A_s | UAAUGUGGAUCCACUUGAACUGG | 195 | XM_ 005264298.1_641-663_G21A_ as |
| AD-63841 | GUUCAAGUGGAUCCACAUUGA | 146 | XM_005264298.1644-664_s | UCAAUGUGGAUCCACUUGAACUG | 196 | XM 005264298.1_642-664_as |
| AD-63856 | UUCAAGUGGAUCCACAUUGAA | 147 | XM_ 005264298.1_645-665_G21A_s | UUCAAUGUGGAUCCACUUGAACU | 197 | XM_ 005264298.1_643-665_G21A_ as |
| AD-63868 | UCAAGUGGAUCCACAUUGAGA | 148 | XM_ 005264298.1_646-666_G21A_s | UCUCAAUGUGGAUCCACUUGAAC | 198 | XM_ 005264298.1_644-666_G21A_ as |
| AD-63869 | CAAGUGGAUCCACAUUGAGGA | 149 | XM_ 005264298.1_647-667_G21A_s | UCCUCAAUGUGGAUCCACUUGAA | 199 | XM_ 005264298.1_645-667_G21A_ as |
| AD-63880 | AAGUGGAUCCACAUUGAGGGA | 150 | XM_ 005264298.1_648-668_C21A_s | UCCCUCAAUGUGGAUCCACUUGA | 200 | XM_ 005264298.1_646-668_C21A_ as |
| AD-63875 | AGUGGAUCCACAUUGAGGGCA | 151 | XM_ 005264298.1_649-669_C21A_s | UGCCCUCAAUGUGGAUCCACUUG | 201 | XM_ 005264298.1_647-669_C21A_ as |
| AD-63874 | GUGGAUCCACAUUGAGGGCCA | 152 | XM_ 005264298.1_650-670_G21A_s | UGGCCCUCAAUGUGGAUCCACUU | 202 | XM_ 005264298.1_648-670_G21A_ as |
| AD-63897 | UGGAUCCACAUUGAGGGCCGA | 153 | XM_ 005264298.1_651-671_G21A_s | UCGGCCCUCAAUGUGGAUCCACU | 203 | XM_ 005264298.1_649-671_G21A_ as |
| AD-63879 | GAUCCACAUUGAGGGCCGGAA | 154 | XM_005264298.1_653-673_s | UUCCGGCCCUCAAUGUGGAUCCA | 204 | XM_005264298.1_651-673_as |
| AD-63819 | GUGAAGAUGCUGCAGCGGAUA | 155 | XM_005264298.1_687-707_s | UAUCCGCUGCAGCAUCUUCACCU | 205 | XM_005264298.1_685-707_as |
| AD-63825 | GAAGAUGCUGCAGCGGAUAGA | 156 | XM_005264298.1_689-709_s | UCUAUCCGCUGCAGCAUCUUCAC | 206 | XM_005264298.1_687-709_as |
| AD-63837 | UGGCUACGGAGACGUGGUGUU | 157 | XM_005264298.1_797-817_s | AACACCACGUCUCCGUAGCCAAA | 207 | XM_005264298.1_195-817_as |
| AD-63853 | GGCUACGGAGACGUGGUGUUU | 158 | XM_005264298.1_798-818_s | AAACACCACGUCUCCGUAGCCAA | 208 | XM_005264298.1_796-818_as |
| AD-63854 | GCUACGGAGACGUGGUGUUUA | 159 | XM_ 005264298.1_799-819_G21A_s | UAAACACCACGUCUCCGUAGCCA | 209 | XM_ 005264298.1_797-819_G21A_ as |
| AD-63859 | GGAGACGUGGUGUUUGUCAGA | 160 | XM_ 005264298.1_804-824_C21A_s | UCUGACAAACACCACGUCUCCGU | 210 | XM_ 005264298.1_802-824_C21A_ as |
| AD-63820 | ACGUGGUGUUUGUCAGCAAAU | 161 | XM_ 005264298.1_808-828_G21U_s | AUUUGCUGACAAACACCACGUCU | 211 | XM_ 005264298.1_806-828_G21U _as |
| AD-63851 | GUGGUGUUUGUCAGCAAAGAU | 162 | XM_005264298.1_810-830_s | AUCUUUGCUGACAAACACCACGU | 212 | XM_005264298.1_808-830_as |
| AD-63873 | GGUGUUUGUCAGCAAAGAUGU | 163 | XM_005264298.1_812-832_s | ACAUCUUUGCUGACAAACACCAC | 213 | XM 005264298.1_810-832_as |
| AD-63886 | GUGUUUGUCAGCAAAGAUGUA | 164 | XM_ 005264298.1_813-833_G21A_s | UACAUCUUUGCUGACAAACACCA | 214 | XM_ 005264298.1_811-833_G21A_ as |
| AD-63892 | UGUUUUGUCAGCAAAGAUGUGA | 165 | XM_ 005264298.1_814-834-G21A_s | UCACAUCUUUGCUGACAAACACC | 215 | XM_ 005264298.1_812-834_G21A_ as |
| AD-63901 | GUUUGUCAGCAAAGAUGUGGA | 166 | XM_ 005264298.1_815-835_C21A_s | UCCACAUCUUUGCUGACAAACAC | 216 | XM 005264298.1_813-835_C21A_ as |
| AD-63885 | UUGUCAGCAAAGAUGUGGCCA | 167 | XM_005264298.1_817-837_s | UGGCCACAUCUUUGCUGACAAAC | 217 | XM_005264298.1_815-837_as |
| AD-63861 | UGUCAGCAAAGAUGUGGCCAA | 168 | XM_005264298.1_818-838_s | UUGGCCACAUCUUUGCUGACAAA | 218 | XM_005264298.1_816-838_as |
| AD-63842 | UGUGGCCAAGCACUUGGGGUU | 169 | XM_005264298.1_830-850_s | AACCCCAAGUGCUUGGCCACAUC | 219 | XM_005264298.1_828-850_as |
| AD-63849 | AGCACUUGGGGUUCCAGUCAA | 170 | XM_ 005264298.1_838-858_G21A_s | UUGACUGGAACCCCAAGUGCUUG | 220 | XM 005264298.1_836-858_G21A_ as |
| AD-63844 | GGGCUUGUAUGGUCGUGUGAA | 171 | XM_ 005264298.1_875-895_G21A_s | UUCACACGACCAUACAAGCCCCU | 221 | XM_ 005264298.1_873-895_G21A_ as |
| AD-63832 | UUGUAUGGUCGUGUGAGGAAA | 172 | XM_005264298.1_879-899_s | UUUCCUCACACGACCAUACAAGC | 222 | XM_005264298.1_877-899_as |
| AD-63827 | GGUCGUGUGAGGAAAGGGGCU | 173 | XM_005264298.1_885-90_s | AGCCCCUUUCCUCACACGACCAU | 223 | XM_005264298.1_883-905_as |
| AD-63858 | GAGGAAAGGGGCUGUGCUUGU | 174 | XM_005264298.1_893-913_s | ACAAGCACAGCCCCUUUCCUCAC | 224 | XM_005264298.1_891-913_as |

**Table 7. KHK Modified Sequences**

| duplexName | senseOligoName | Sense strand | SEQ ID NO: | antisOligoName | Antisense strand | SEQ ID NO: | Species |
|---|---|---|---|---|---|---|---|
| AD-63824 | A-127677 | AfsusCfaAfuGfuGfGfUfgGfaCfaAfaUfa AfL96 | 225 | A-127678 | usUfsaUfuUfgUfcCfaccAfcAfuUfgAfu sgsa | 275 | Mm |
| AD-63829 | A-127663 | GfsgsUfgGfaCfaAfAfUfaCfcCfaGfaGfg AfL96 | 226 | A-127664 | usCfscUfcUfgGfgUfauuUfgUfcCfaCf csasc | 276 | Mm |
| AD-63855 | A-127673 | CfsusUfuGfaGfaAfGfGfuCfgAfuCfuGfa AfL96 | 227 | A-127674 | usUfscAfgAfuCfgAfccuUfcUfcAfaAfg susc | 277 | Mm |
| AD-63835 | A-127665 | CfscsCfgGfuUfcAfAfGfuGfgAfuCfcAfc AfL96 | 228 | A-127666 | usGfsuGfgAfuCfcAfcuuGfaAfcCfgGf gsusc | 278 | Mm |
| AD-63845 | A-127669 | UfsgsUfgGfcCfaAfGfCfaCfcUfgGfgGfu UfL96 | 229 | A-127670 | asAfscCfcCfaGfgUfgcuUfcGfcCfaCf asusc | 279 | Mm |
| AD-63823 | A-127661 | UfsusGfcAfgGfgGfUfUfuGfaUfgGfcAfu UfL96 | 230 | A-127662 | asAfsuGfcCfaUfcAfaacCfcCfuGfcAf asgsc | 280 | Mm |
| AD-63863 | A-127587 | GfsasAfgAfgAfaGfCfAfgAfuCfcUfgUfg AfL96 | 231 | A-127588 | usCfsaCfaGfgAfuCfugcUfuCfuCfuUf cscsa | 281 | Hs |
| AD-63881 | A-127593 | AfsasGfaGfaAfgCfAfGfaUfcCfuGfuGfc AfL96 | 232 | A-127594 | usGfscAfcAfgGfaUfcugCfuUfcUfcUf uscsc | 282 | Hs |
| AD-63862 | A-127571 | GfsasGfaAfgCfaGfAfUfcCfuGfuGfcGfu AfL96 | 233 | A-127572 | usAfscGfcAfcAfgGfaucUfgCfuUfcUfc susu | 283 | Hs |
| AD-63887 | A-127595 | AfsgsAfaGfcAfgAfUfCfcUfgUfgCfgUfg AfL96 | 234 | A-127596 | usCfsaCfgCfaCfaGfgauCfuGfcUfuCf uscsu | 284 | Hs |
| AD-63902 | A-127585 | GfsasAfgCfaGfaUfCfCfuGfuGfcGfuGfg AfL96 | 235 | A-127586 | usCfscAfcGfcAfcAfggaUfcUfgCfuUfc susc | 285 | Hs |
| AD-63896 | A-127567 | GfscsAfgAfuCfcUfGfUfgCfgUfgGfgGfc UfL96 | 236 | A-127568 | asGfscCfcCfaCfgCfacaGfgAfuCfuGf csusu | 286 | Hs |
| AD-63843 | A-127637 | GfsasUfcCfuGfuGfCfGfuGfgGfgCfuAfg UfL96 | 237 | A-127638 | asCfsuAfgCfcCfcAfcgcAfcAfgGfaUfc susg | 287 | Hs |
| AD-63857 | A-127627 | GfsgsUfgGfaCfaAfGfUfaCfcCfuAfaGfg AfL96 | 238 | A-127628 | usCfscUfuAfgGfgUfacuUfgUfcCfaCf csasg | 288 | Hs |
| AD-63836 | A-127603 | GfscsCfuGfcCfaGfAfUfgUfgUfcUfgCfu AfL96 | 239 | A-127604 | usAfsgCfaGfaCfaCfaucUfgGfcAfgGf csusc | 289 | Hs |
| AD-63834 | A-127649 | UfsusUfgAfgAfaGfGfUfuGfaUfcUfgAfc AfL96 | 240 | A-127650 | usGfsuCfaGfaUfcAfaccUfuCfuCfaAf asgsu | 290 | Hs |
| AD-63839 | A-127651 | GfsusUfgAfuCfuGfAfCfcCfaGfuUfcAfa AfL96 | 241 | A-127652 | usUfsuGfaAfcUfgGfgucAfgAfuCfaAf cscsu | 291 | Hs |
| AD-63821 | A-127629 | AfsusCfuGfaCfcCfAfGfuUfcAfaGfuGfg AfL96 | 242 | A-127630 | usCfscAfcUfuGfaAfcugGfgUfcAfgAf uscsa | 292 | Hs |
| AD-63847 | A-127623 | CfscsAfgUfuCfaAfGfUfgGfaUfcCfaCfa UfL96 | 243 | A-127624 | asUfsgUfgGfaUfcCfacuUfgAfaCfuGf gsgsu | 293 | Hs |
| AD-63846 | A-127607 | CfsasGfuUfcAfaGfUfGfgAfuCfcAfcAfu UfL96 | 244 | A-127608 | asAfsuGfuGfgAfuCfcacUfuGfaAfcUf gsgsg | 294 | Hs |
| AD-63826 | A-127615 | AfsgsUfuCfaAfgUfGfGfaUfcCfaCfaUfu AfL96 | 245 | A-127616 | usAfsaUfgUfgGfaUfccaCfuUfgAfaCf usgsg | 295 | Hs |
| AD-63841 | A-127605 | GfsusUfcAfaGfuGfGfAfuCfcAfcAfuUfg AfL96 | 246 | A-127606 | usCfsaAfuGfuGfgAfuccAfcUfuGfaAf csusg | 296 | Hs |
| AD-63856 | A-127611 | UfsusCfaAfgUfgGfAfUfcCfaCfaUfuGfa AfL96 | 247 | A-127612 | usUfscAfaUfgUfgGfaucCfaCfuUfgAf ascsu | 297 | Hs |
| AD-63868 | A-127573 | UfscsAfaGfuGfgAfUfCfcAfcAfuUfgAfgA fL96 | 248 | A-127574 | usCfsuCfaAfuGfuGfgauCfcAfcUfuGf asasc | 298 | Hs |
| AD-63869 | A-127589 | CfsasAfgUfgGfaUfCfCfaCfaUfuGfaGfg AfL96 | 249 | A-127590 | usCfscUfcAfaUfgUfggaUfcCfaCfuUf gsasa | 299 | Hs |
| AD-63880 | A-127577 | AfsasGfuGfgAfuCfCfAfcAfuUfgAfgGfg AfL96 | 250 | A-127578 | usCfscCfuCfaAfuGfuggAfuCfcAfcUf usgsa | 300 | Hs |
| AD-63875 | A-127591 | AfsssUfgGfaUfcCfAfCfaUfuGfaGfgGfc AfL96 | 251 | A-127592 | usGfscCfcUfcAfaUfgugGfaUfcCfaCf ususg | 301 | Hs |
| AD-63874 | A-127575 | GfsusGfgAfuCfcAfCfAfuUfgAfgGfgCfc AfL96 | 252 | A-127576 | usGfsgCfcCfuCfaAfuguGfgAfuCfcAf csusu | 302 | Hs |
| AD-63897 | A-127583 | UfsgsGfaUfcCfaCfAfUfuGfaGfgGfcCfg AfL96 | 253 | A-127584 | usCfsgGfcCfcUfcAfaugUfgGfaUfcCf ascsu | 303 | Hs |
| AD-63879 | A-127561 | GfsasUfcCfaCfaU fUfGfaGfgGfcCfgGfaAfL96 | 254 | A-127562 | usUfscCfgGfcCfcUfcaaUfgUfgGfaUf cscsa | 304 | Hs |
| AD-63819 | A-127597 | GfsusGfaAfgAfuGfCfUfgCfaGfcGfgAfu AfL96 | 255 | A-127598 | usAfsuCfcGfcUfgCfagcAfuCfuUfcAfc scsu | 305 | Hs |
| AD-63825 | A-127599 | GfsasAfgAfuGfcUfCfCfaGfcGfgAfuAfg AfL96 | 256 | A-127600 | usCfsuAfuCfcGfcUfgcaGfcAfuCfuUf csasc | 306 | Hs |
| AD-63837 | A-127619 | UfsgsGfcUfaCfgGfAfGfaCfgUfgGfuGfu UfL96 | 257 | A-127620 | asAfscAfcCfaCfgUfcucCfgUfaGfcCf asasa | 307 | Hs |
| AD-63853 | A-127641 | GfsgsCfuAfcGfgAfGfAfcGfuGfgUfgUfu UfL96 | 258 | A-127642 | asAfsaCfaCfcAfcGfucuCfcGfuAfgCf csasa | 308 | Hs |
| AD-63854 | A-127657 | GfscsUfaCfgGfaGfAfCfgUfgGfuGfuUfu AfL96 | 259 | A-127658 | usAfsaAfcAfcCfaCfgucUfcCfgUfaGfc scsa | 309 | Hs |
| AD-63859 | A-127659 | GfsgsAfgAfcGfuGfGfUfgUfuUfgUfcAfg AfL96 | 260 | A-127660 | usCfsuGfaCfaAfaCfaccAfcGfuCfuCf csgsu | 310 | Hs |
| AD-63820 | A-127613 | AfscsGfuGfgUfgUfUfUfgUfcAfgCfaAfa UfL96 | 261 | A-127614 | asUfsuUfgCfuGfaCfaaaCfaCfcAfcGf uscsu | 311 | Hs |
| AD-63851 | A-127609 | GfsusGfgUfgUfuUfGfUfcAfgCfaAfaGfa UfL96 | 262 | A-127610 | asUfscUfuUfgCfuGfacaAfaCfaCfcAf csgsu | 312 | Hs |
| AD-63873 | A-127559 | GfsgsUfgUfuUfgUfCfAfgCfaAfaGfgUfg UfL96 | 263 | A-127560 | asCfsaUfcUfuUfgCfugaCfaAfaCfaCf csasc | 313 | Hs |
| AD-63886 | A-127579 | GfsusGfuUfuGfuCfAfGfcAfaAfgAfuGfu AfL96 | 264 | A-127580 | usAfscAfuCfuUfuGfcugAfcAfaAfcAfc scsa | 314 | Hs |
| AD-63892 | A-127581 | UfsgsUfuUfgUfcAfGfCfaAfaGfaUfgUfg AfL96 | 265 | A-127582 | usCfsaCfaUfcU fuUfgcuGfaCfaAfaCfascsc | 315 | Hs |
| AD-63901 | A-127569 | GfsusUfuGfuCfaGfCfAfaAfgAfuGfuGfg AfL96 | 266 | A-127570 | usCfscAfcAfuCfuUfugcUfgAfcAfaAfc sasc | 316 | Hs |
| AD-63885 | A-127563 | UfsusGfuCfaGfcAfAfAfgAfuGfuGfgCfc AfL96 | 267 | A-127564 | usGfsgCfcAfcAfuCfuuuGfcUfgAfcAf asasc | 317 | Hs |
| AD-63861 | A-127555 | UfsgsUfcAfgCfaAfAfGfaUfgUgGfcCfaA fL96 | 268 | A-127556 | usUfsgGfcCfaCfaUfcuuUfgCfuGfaCf asasa | 318 | Hs |
| AD-63842 | A-127621 | UfsgsUfgGfcCfaAfGfCfaCfuUfgGfgGfu UfL96 | 269 | A-127622 | asAfscCfcCfaAfgUfgcuUfgGfcCfaCf asusc | 319 | Hs |
| AD-63849 | A-127655 | AfsgsCfaCfuUfgGfGfGfuUfcCfaGfuCfa AfL96 | 270 | A-127656 | usUfsgAfcUfgGfaAfcccCfaAfgUfgCf ususg | 320 | Hs |
| AD-63844 | A-127653 | GfsgsGfcUfuGfuAfUfGfgUfcGfuGfuGfa AfL96 | 271 | A-127654 | usUfscAfcAfcGfaCfcauAfcAfaGfcCfc scsu | 321 | Hs |
| AD-63832 | A-127617 | UfsusGfuAfuGfgUfCfGfuGfuGfaGfgAfa AfL96 | 272 | A-127618 | usUfsuCfcUfcAfcAfcgaCfcAfuAfcAfa sgsc | 322 | Hs |
| AD-63827 | A-127631 | GfsgsUfcGfuGfuGfAfGfgAfaAfgGfgGfc UfL96 | 273 | A-127632 | asGfscCfcCfuUfuCfcucAfcAfcGfaCfc sasu | 323 | Hs |
| AD-63858 | A-127643 | GfsasGfgAfaAfgGfGfGfcUfgUfgCfuUfg UfL96 | 274 | A-127644 | asCfsaAfgCfaCfaGfcccCfuUfuCfcUf csasc | 324 | Hs |

**Table 8. KHK Single Dose Screen in Hep3b**

| DuplexID | 10nM_AVG | 0.1nM_AVG | 10nM_STDEV | 0.1nM_STDEV |
|---|---|---|---|---|
| AD-63819 | 82.0 | 82.1 | 24.1 | 10.0 |
| AD-63820 | 11.2 | 45.6 | N/A | 3.6 |
| AD-63821 | 68.2 | 102.4 | 19.8 | 10.5 |
| AD-63823 | 134.3 | 84.4 | 22.3 | 14.0 |
| AD-63824 | 94.1 | 98.0 | 4.1 | 4.3 |
| AD-63825 | 70.4 | 77.7 | 6.5 | 5.3 |
| AD-63826 | 45.3 | 89.7 | 5.5 | 7.5 |
| AD-63827 | 51.7 | 130.0 | 21.2 | 82.8 |
| AD-63829 | 89.6 | 78.2 | 1.3 | 1.5 |
| AD-63832 | 17.4 | 80.5 | 1.4 | 19.5 |
| AD-63834 | 11.0 | 72.7 | 0.8 | 20.7 |
| AD-63835 | 17.2 | 103.5 | 8.1 | 76.4 |
| AD-63836 | 37.7 | 63.8 | 2.8 | 23.5 |
| AD-63837 | 87.3 | 66.8 | 33.0 | 5.2 |
| AD-63839 | N/A | 34.0 | N/A | 2.8 |
| AD-63841 | 46.4 | 64.4 | 0.7 | 7.9 |
| AD-63842 | 72.6 | 76.9 | 22.7 | 13.9 |
| AD-63843 | 48.6 | 108.0 | 9.0 | 35.8 |
| AD-63844 | 68.2 | 80.1 | 16.5 | 12.9 |
| AD-63845 | 54.4 | 68.1 | 43.3 | 4.0 |
| AD-63846 | 66.0 | 88.9 | 18.8 | 2.2 |
| AD-63847 | 43.7 | 58.1 | 17.5 | 12.7 |
| AD-63849 | 31.0 | 85.4 | 4.4 | 30.3 |
| AD-63851 | 28.9 | 29.5 | 4.1 | 21.4 |
| AD-63853 | 39.9 | 49.3 | 0.0 | 11.7 |
| AD-63854 | 7.1 | 53.3 | 2.6 | 10.1 |
| AD-63855 | 15.0 | 32.6 | 14.5 | 7.3 |
| AD-63856 | 66.8 | 96.9 | 3.3 | 15.1 |
| AD-63857 | 64.9 | 101.4 | 7.3 | 3.5 |
| AD-63858 | 67.5 | 89.9 | 5.6 | 18.8 |
| AD-63859 | 44.8 | 91.0 | 1.1 | 10.2 |
| AD-63861 | 78.2 | 89.1 | 0.0 | 8.3 |
| AD-63862 | 48.0 | 92.9 | 14.8 | 14.5 |
| AD-63863 | 39.3 | 65.7 | 17.9 | 13.1 |
| AD-63868 | 47.4 | 106.8 | 4.4 | 13.1 |
| AD-63869 | 20.7 | 68.0 | 3.8 | 28.2 |
| AD-63873 | 22.6 | 102.8 | 8.0 | 31.7 |
| AD-63874 | 64.7 | 107.6 | 2.9 | 6.3 |
| AD-63875 | 85.2 | 108.4 | 30.6 | 60.3 |
| AD-63879 | 66.1 | 81.1 | 3.6 | 7.9 |
| AD-63880 | 86.7 | 85.9 | 19.4 | 3.8 |
| AD-63881 | 60.8 | 63.1 | 4.5 | 3.1 |
| AD-63885 | 75.1 | 66.3 | 12.4 | 11.6 |
| AD-63886 | 27.5 | 53.3 | 5.1 | 7.8 |
| AD-63887 | 40.0 | 70.5 | 8.4 | 0.7 |
| AD-63892 | 19.9 | 82.7 | 1.5 | 5.7 |
| AD-63896 | 88.6 | 86.6 | 1.7 | 15.6 |
| AD-63897 | 96.7 | 91.4 | 4.3 | 4.9 |
| AD-63901 | 48.1 | 97.6 | 4.5 | 2.9 |
| AD-63902 | 43.1 | 99.2 | 8.6 | 9.7 |

**Table 9. KHK Dose Response Screen in Hep3b**

| Duplex ID | IC50 (nM) |
|---|---|
| AD-63851 | 0.027 |
| AD-63820 | 0.010 |
| AD-63853 | 0.067 |
| AD-63839 | 0.015 |
| AD-63854 | 0.075 |
| AD-63855 | 0.017853 |
| AD-63886 | 0.082879 |

## Claims

1. A double stranded RNAi agent for inhibiting expression of human ketohexokinase (KHK), wherein said double stranded RNAi agent comprises a sense strand and an antisense strand forming a double stranded region, wherein the sense and antisense strands comprise sequences selected from the group consisting of SEQ ID NOs: 161 and 211, SEQ ID NOs: 162 and 212, SEQ ID NOs: 163 and 213, SEQ ID NOs: 164 and 214; and SEQ ID NOs: 165 and 215,
wherein said double stranded RNAi agent comprises at least one modified nucleotide, and
wherein at least one strand is conjugated to a ligand.

2. The double stranded RNAi agent of claim 1, wherein said modified nucleotide is selected from the group consisting of a 2'-O-methyl modified nucleotide, a nucleotide comprising a 5'-phosphorothioate group, a deoxy-nucleotide, a 3'-terminal deoxy-thymine (dT) nucleotide, a 2'-fluoro modified nucleotide, a 2'-deoxy-modified nucleotide, a terminal nucleotide linked to a cholesteryl derivative or a dodecanoic acid bisdecylamide group, a 2'-deoxy-2'-fluoro modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxyl-modified nucleotide, a 2'-methoxyethyl modified nucleotide, a 2'-O-alkyl-modified nucleotide, a morpholino nucleotide, a phosphoramidate, a non-natural base comprising nucleotide, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate, and a nucleotide comprising a 5'-phosphate mimic.

3. The double stranded RNAi agent of claim 1,
wherein substantially all of the nucleotides of said sense strand and substantially all of the nucleotides of said antisense strand are modified nucleotides, and
wherein said sense strand is conjugated to the ligand attached at the 3'-terminus,
wherein preferably all of the nucleotides of said sense strand and all of the nucleotides of said antisense strand are modified nucleotides, and
wherein preferably said modified nucleotide is selected from the group consisting of a 2'-O-methyl modified nucleotide, a nucleotide comprising a 5'-phosphorothioate group, a deoxy-nucleotide, a 3'-terminal deoxy-thymine (dT) nucleotide, a 2'-fluoro modified nucleotide, a 2'-deoxy-modified nucleotide, a terminal nucleotide linked to a cholesteryl derivative or a dodecanoic acid bisdecylamide group, a 2'-deoxy-2'-fluoro modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxly-modified nucleotide, a 2'-methoxyethyl modified nucleotide, a 2'-O-alkyl-modified nucleotide, a morpholino nucleotide, a phosphoramidate, a non-natural base comprising nucleotide, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate, and a nucleotide comprising a 5'-phosphate mimic.

4. The double stranded RNAi agent of claim 1 to 3, wherein the ligand is
(a) one or more GalNAc derivatives attached through a bivalent or trivalent branched linker;
(b) 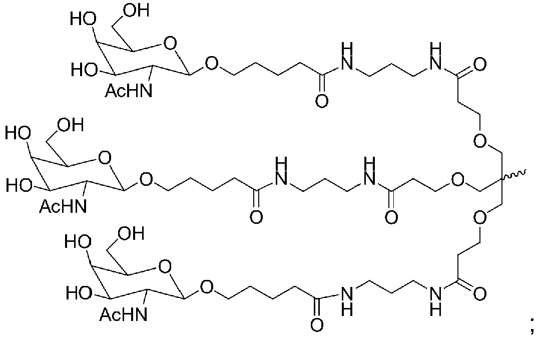 or
(c) attached to the 3' end of the sense strand.

5. The double stranded RNAi agent of claim 4(c), wherein the RNAi agent is conjugated to the ligand as shown in the following schematic 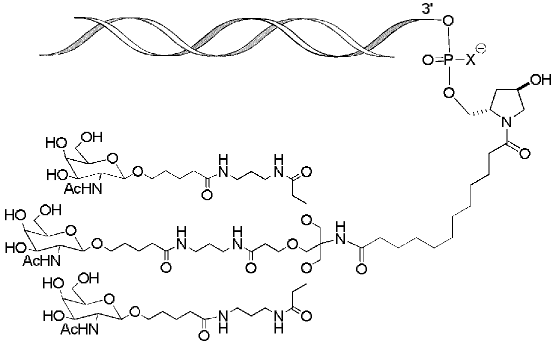 wherein X is O or S.

6. The double stranded RNAi agent of any one of claims 1 or 3, wherein said RNAi agent further comprises at least one phosphorothioate or methylphosphonate internucleotide linkage.

7. The double stranded RNAi agent of claim 6, wherein
(a) the phosphorothioate or methylphosphonate internucleotide linkage is at the 3'-terminus of one strand, wherein preferably said strand is the antisense strand;
(b) the phosphorothioate or methylphosphonate internucleotide linkage is at the both the 5'- and 3'-terminus of one strand; or
(c) said RNAi agent comprises 6-8 phosphorothioate internucleotide linkages, wherein preferably the antisense strand comprises two phosphorothioate internucleotide linkages at the 5'-terminus and two phosphorothioate internucleotide linkages at the 3'-terminus, and the sense strand comprises at least two phosphorothioate internucleotide linkages at either the 5'-terminus or the 3'-terminus.

8. The double stranded RNAi agent of claim 1, wherein said double stranded RNAi agent consists of a:
sense strand consisting of AfscsGfuGfgUfgUfUfUfgUfcAfgCfaAfaUfL96 and an antisense strand consisting of asUfsuUfgCfuGfaCfaaaCfaCfcAfcGfuscsu;
sense strand consisting of GfsusGfgUfgUfuUfGfUfcAfgCfaAfaGfaUfL96 and an antisense strand consisting of asUfscUfuUfgCfuGfacaAfaCfaCfcAfcsgsu;
sense strand consisting of GfsgsUfgUfuUfgUfCfAfgCfaAfaGfaUfgUfL96 and an antisense strand consisting of asCfsaUfcUfuUfgCfugaCfaAfaCfaCfcsasc;
sense strand consisting of GfsusGfuUfuGfuCfAfGfcAfaAfgAfuGfuAfL96 and an antisense strand consisting of usAfscAfuCfuUfuGfcugAfcAfaAfcAfcscsa; or
sense strand consisting of UfsgsUfuUfgUfcAfGfCfaAfaGfaUfgUfgAfL96 and an antisense strand consisting of usCfsaCfaUfcUfuUfgcuGfaCfaAfaCfascsc;
wherein a, g, c, and u are 2'-O-methyl (2'-OMe) modified A, G, C, and U nucleotides, respectively; Af, Gf, Cf and Uf are 2'fluoro A, G, C and U modified nucleotides, respectively; s is a phosphorothioate linkage and L96 is N-[tris(GalNAc-alkyl)-amidodecanoyl)]-4-hydroxyprolinol Hyp-(GalNAc-alkyl)3

9. A pharmaceutical composition comprising the double stranded RNAi agent of any one of claims 1 to 8.

10. A method of inhibiting ketohexokinase (KHK) expression in a cell, the method comprising:
(a) contacting the cell with the double stranded RNAi agent of any one of claims 1 to 8 or the pharmaceutical composition of claim 12; and
(b) maintaining the cell produced in step (a) for a time sufficient to obtain degradation of the mRNA transcript of a KHK gene, thereby inhibiting expression of the KHK gene in the cell;
wherein any method of treatment of the human or animal body by therapy is excluded.

11. The method of claim 10, wherein the KHK expression is inhibited by at least about 30%, about 40%, about 50%, about 60%, about 70%, about 80%, about 90%, about 95%, about 98% or about 100%.

12. The double stranded RNAi agent of any one of claims 1 to 8 or the pharmaceutical composition of claim 9 for use in a method of treating a subject having a ketohexokinase (KHK)-associated disorder, wherein preferably the subject is a human.

13. The double stranded RNAi for use of claim 12, wherein the ketohexokinase-associated disease is selected from the group consisting liver disease, dyslipidemia, disorders of glycemic control, cardiovascular disease, kidney disease, metabolic syndrome, adipocyte dysfunction, visceral adipose deposition, obesity, hyperuricemia, gout, eating disorders, and excessive sugar craving, wherein preferably
(a) the liver disease is fatty liver and/or steatohepatitis;
(b) the dyslipidemia is selected from the group consisting of hyperlipidemia, high LDL cholesterol, low HDL cholesterol, hypertriglyceridemia, and postprandial hypertriglyceridemia;
(c) the disorder of glycemic control is insulin resistance and/or diabetes;
(d) the cardiovascular disease is hypertension and/or endothelial cell dysfunction; or
(e) the kidney disease is selected from the group consisting of acute kidney disorder, tubular dysfunction, and proinflammatory changes to the proximal tubules.

14. The double stranded RNAi for use of claim 12, wherein the double stranded RNAi agent is to be administered at a dose of about 0.01 mg/kg to about 10 mg/kg or about 1 mg/kg to about 10 mg/kg.

## Patentansprüche

1. Doppelsträngiges RNAi-Agens zur Inhibition der Expression von menschlicher Ketohexokinase (KHK), wobei das doppelsträngige RNAi-Agens einen Sinnstrang und einen Antisinnstrang umfasst, die eine doppelsträngige Region bilden, wobei der Sinn- und der Antisinnstrang Sequenzen umfassen, die aus der Gruppe bestehend aus den SEQ ID Nrn. 161 und 211, SEQ ID Nrn. 162 und 212, SEQ ID Nrn. 163 und 213, SEQ ID Nrn. 164 und 214; und SEQ ID Nrn. 165 und 215 ausgewählt sind,
wobei das doppelsträngiges RNAi-Agens mindestens ein modifiziertes Nukleotid umfasst, und
wobei mindestens ein Strang mit einem Liganden verknüpft ist.

2. Doppelsträngiges RNAi-Agens nach Anspruch 1, wobei das modifizierte Nukleotid vorzugsweise aus der Gruppe bestehend aus einem 2'-O-Methyl modifiziertem Nukleotid, einem Nukleotid umfassend eine 5'-Phosphorothioat-Gruppe, einem Deoxy-Nukleotid, einem 3'-terminalen Deoxy-Thymin (dT) Nukleotid, einem 2'-Fluor modifiziertem Nukleotid, einem 2'-Deoxy modifiziertem Nukleotid, einem terminalen Nukleotid, das mit einem Cholesteryl-Derivat oder einer Dodecansäure-Bisdecylamid-Gruppe verknüpft ist, einem 2'-Deoxy-2'-Fluor modifiziertem Nukleotid, einem versperrten ("locked") Nukleotid, einem nicht-versperrten ("unlocked") Nukleotid, einem konformatorisch beschränkten Nukleotid, einem eingeschränkten Ethyl Nukleotid, einem abasischen Nukleotid, einem 2'-Amino modifizierten Nukleotid, einem 2'-O-Allyl modifizierten Nukleotid, einem 2'-C-Alkyl modifizierten Nukleotid, einem 2'-Hydroxyl modifizierten Nukleotid, einem 2'-Methoxyethyl modifizierten Nukleotid, einem 2'-O-Alkyl modifizierten Nukleotid, einem Morpholino-Nukleotid, einem Phosphoramidat, einem eine nicht natürliche Base umfassenden Nukleotid, einem Tetrahydropyran modifiziertem Nukleotid, einem 1,5-Anhydrohexitol modifizierten Nukleotid, einem Cyclohexenyl modifizierten Nukleotid, einem Nukleotid umfassend eine Phosphorothioat-Gruppe, einem Nukleotid umfassend eine Methylphosphonat-Gruppe, einem Nukleotid umfassend ein 5'-Phosphat und einem Nukleotid umfassend ein 5'-Phosphat-Mimetik ausgewählt ist.

3. Das doppelsträngige RNAi-Agens nach den Anspruch 1,
wobei im Wesentlichen alle Nukleotide des Sinnstrangs und im Wesentlichen alle Nukleotide des Antisinnstrangs modifizierte Nukleotide sind und
wobei der Sinnstrang am 3'-Terminus mit einem Liganden verknüpft ist,
wobei vorzugsweise alle Nukleotide des Sinnstrangs und alle Nukleotide des Antisinnstrangs modifizierte Nukleotide sind, und
wobei das modifizierte Nukleotid vorzugsweise aus der Gruppe bestehend aus einem 2'-O-Methyl modifizierten Nukleotid, einem Nukleotid umfassend eine 5'-Phosphorothioat-Gruppe, einem Deoxy-Nukleotid, einem 3'-terminalen Deoxy-Thymin (dT) Nukleotid, einem 2'-Fluor modifizierten Nukleotid, einem 2'-Deoxy modifizierten Nukleotid, einem terminalen Nukleotid, das mit einem Cholesteryl-Derivat oder einer Dodecansäure-Bisdecylamid-Gruppe verknüpft ist, einem 2'-Deoxy-2'-Fluor modifizierte Nukleotid, einem versperrten ("locked") Nukleotid, einem nicht-versperrten ("unlocked") Nukleotid, einem konformatorisch beschränkten Nukleotid, einem eingeschränkten Ethyl-Nukleotid, einem abasischen Nukleotid, einem 2'-Amino modifizierten Nukleotid, einem 2'-O-Allyl modifizierten Nukleotid, einem 2'-C-Alkyl modifizierten Nukleotid, einem 2'-Hydroxyl modifizierten Nukleotid, einem 2'-Methoxyethyl modifizierten Nukleotid, einem 2'-O-Alkyl modifizierten Nukleotid, einem Morpholino-Nukleotid, einem Phosphoramidat, einem eine nicht natürliche Base umfassenden Nukleotid, einem Tetrahydropyran modifizierten Nukleotid, einem 1,5-Anhydrohexitol modifizierten Nukleotid, einem Cyclohexenyl modifizierten Nukleotid, einem Nukleotid umfassend eine Phosphorothioat-Gruppe, einem Nukleotid umfassend eine Methylphosphonat-Gruppe, einem Nukleotid umfassend ein 5'-Phosphat und einem Nukleotid umfassend ein 5'-Phosphat-Mimetik ausgewählt ist.

4. Doppelsträngiges RNAi-Agens nach Anspruch 1 bis 3, wobei der Ligand
(a) ein oder mehrere über einen bivalenten der trivalenten verzweigten Linker verknüpfte(s) GaINAc-Derivat(e) ist/sind;
(b) 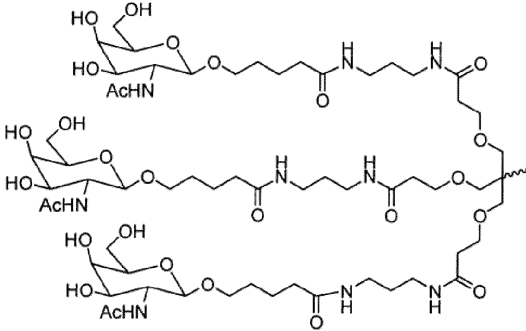 ist; oder
(c) mit dem 3'-Ende des Sinnstrangs verknüpft ist.

5. Doppelsträngiges RNAi-Agens nach Anspruch 4(c), wobei das RNAi-Agens mit dem Liganden wie in dem folgenden Schema gezeigt verknüpft ist 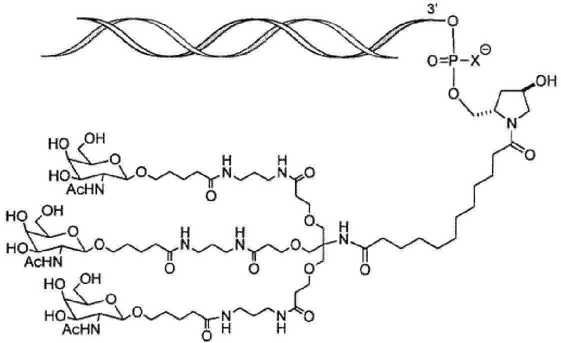 wobei X O oder S ist.

6. Doppelsträngiges RNAi-Agens nach Anspruch 1 oder 3, wobei das RNAi-Agens weiterhin mindestes eine Phosphorothioat- oder Methylphosphonat-Internukleotid-Verknüpfung aufweist.

7. Doppelsträngiges RNAi-Agens nach Anspruch 6, wobei
(a) die Phosphorothioat- oder Methylphosphonat-Internukleotid-Verknüpfung am 3'-Terminus eines Strangs ist, wobei dieser Strang bevorzugt der Antisinnstrang ist,
(b) die Phosphorothioat- oder Methylphosphonat-Internukleotid-Verknüpfung sowohl am 5'- als auch am 3'-Terminus eines Strangs ist; oder
(c) das RNAi-Agens 6-8 Phosphorothioat-Internukleotid-Verknüpfungen umfasst, wobei bevorzugt der Antisinnstrang zwei Phosphorothioat-Internukleotid-Verknüpfungen am 5'-Terminus und zwei Phosphorothioat-Internukleotid-Verknüpfungen am 3'-Terminus und der Sinnstrang mindestens zwei Phosphorothioat-Internukleotid-Verknüpfungen entweder am 5'-Terminus oder am 3'-Terminus umfasst.

8. Doppelsträngiges RNAi-Agens nach Anspruch 1, wobei das RNAi-Agens aus einem:
Sinnstrang bestehend aus AfscsGfuGfgUfgUfUfUfgUfcAfgCfaAfaUfL96 und einem Antisinnstrang bestehend aus asUfsuUfgCfuGfaCfaaaCfaCfcAfcGfuscsu;
Sinnstrang bestehend aus GfsusGfgUfgUfuUfGfUfcAfgCfaAfaGfaUfL96 und einem Antisinnstrang bestehend aus asUfscUfuUfgCfuGfacaAfaCfaCfcAfcsgsu;
Sinnstrang bestehend aus GfsgsUfgUfuUfgUfCfAfgCfaAfaGfaUfgUfL96 und einem Antisinnstrang bestehend aus asCfsaUfcUfuUfgCfugaCfaAfaCfaCfcsasc;
Sinnstrang bestehend aus GfsusGfuUfuGfuCfAfGfcAfaAfgAfuGfuAfL96 und einem Antisinnstrang bestehend aus usAfscAfuCfuUfuGfcugAfcAfaAfcAfcscsa besteht; oder
einem Sinnstrang bestehend aus UfsgsUfuUfgUfcAfGfCfaAfaGfaUfgUfgAfL96 und einem Antisinnstrang bestehend aus usCfsaCfaUfcUfuUfgcuGfaCfaAfaCfascsc
wobei a, g, c, und u 2'-O-Methyl (2'-OMe) jeweils modifizierte A, G, C und U Nukleotide sind; Af, Gf, Cf und Uf jeweils 2'Fluor A, G, C und U modifizierte Nukleotide sind; s eine Phosphorothioat-Verknüpfung ist und L96 N-[tris(GalNAc-alkyl)-amidodecanoyl]-4-hydroxyprolinol Hyp-(GalNAc-alkyl)3 ist.

9. Pharmazeutische Zusammensetzung, umfassend das doppelsträngige RNAi-Agens nach einem der Ansprüche 1 bis 8.

10. Verfahren zur Inhibition der Ketohexokinase (KHK) Expression in einer Zelle, wobei das Verfahren umfasst:
(a) Inkontaktbringen der Zelle mit dem doppelsträngigen RNAi-Agens nach einem der Ansprüche 1 bis 8 oder der pharmazeutischen Zusammensetzung nach Anspruch 12; und
(b) Erhalten der in (a) hergestellten Zelle für eine Zeit, die ausreicht, um die Degradierung des mRNA-Transkripts eines KHK-Gens zu erreichen, wodurch die Expression des KHK-Gens in der Zelle inhibiert wird, wobei jegliches Verfahren zur Behandlung des menschlichen oder tierischen Körpers mittels Therapie ausgenommen ist.

11. Verfahren nach Anspruch 10, wobei die KHK-Expression mindestens um etwa 30%, etwa 40%, etwa 50%, etwa 60%, etwa 70%, etwa 80%, etwa 90%, etwa 95%, etwa 98% oder etwa 100% inhibiert ist.

12. Doppelsträngiges RNAi-Agens nach einem der Ansprüche 1 bis 8 oder pharmazeutische Zusammensetzung nach Anspruch 9 zur Verwendung in einem Verfahren zur Behandlung eines Individuums, das eine Ketohexokinase (KHK)-assoziierte Krankheit hat, wobei das Individuum bevorzugt ein Mensch ist.

13. Doppelsträngiges RNAi-Agens zur Verwendung nach Anspruch 12, wobei die Ketohexokinase-assoziierte Krankheit aus der Gruppe ausgewählt ist bestehend aus Leberkrankheit, Dyslipidämie, Erkrankungen der glykämischen Kontrolle, kardiovaskuläre Krankheit, Nierenkrankheit, metabolisches Syndrom, Adipozytendysfunktion, viskerale adipöse Deposition, Fettsucht, Hyperurikämie, Gicht, Essstörungen und exzessives Verlagen nach Zucker, wobei bevorzugt
(a) die Leberkrankheit Fettleber und/oder Steatohepatitis ist;
(b) die Dyslipidämie aus der Gruppe ausgewählt ist bestehend aus Hyperlipidämie, Cholesterin mit hohem LDL, Cholesterin mit niedrigem HDL, Hypertriglyceridämie und postprandialer Hypertriglyceridämie;
(c) die Erkrankung der glykämischen Kontrolle Insulinresistenz and/oder Diabetes ist;
(d) die kardiovaskuläre Krankheit Hypertension und/oder eine Dysfunktion endothelialer Zellen ist; oder
(e) die Nierenkrankheit aus der Gruppe ausgewählt ist bestehend aus akuter Nierenkrankheit, tubulärer Dysfunktion und proinflammatorischen Veränderungen des proximalen Tubulus.

14. Doppelsträngiges RNAi-Agens zur Verwendung nach Anspruch 15, wobei das doppelsträngige RNAi-Agens in einer Dosis von etwa 0,01 mg/kg bis etwa 10 mg/kg oder etwa 1 mg/kg bis etwa 10 mg/kg zu verabreichen ist.

## Revendications

1. Agent ARNi double brin pour inhiber l'expression de la cétohexokinase (KHK) humaine, lequel agent ARNi double brin comprend un brin sens et un brin antisens formant une région double brin, dans lequel les brins sens et antisens comprennent des séquences sélectionnées parmi le groupe consistant en SEQ ID NOs : 161 et 211, SEQ ID NOs : 162 et 212, SEQ ID NOs : 163 et 213, SEQ ID NOs : 164 et 214, et SEQ ID NOs : 165 et 215,
dans lequel ledit agent ARNi double brin comprend au moins un nucléotide modifié, et
dans lequel au moins un brin est conjugué à un ligand.

2. Agent ARNi double brin selon la revendication 1, dans lequel ledit nucléotide modifié est choisi dans le groupe constitué par un nucléotide modifié par 2'-O-méthyle, un nucléotide comprenant un groupe 5'-phosphorothioate, un désoxynucléotide, un nucléotide avec une désoxythymine (dT) 3'-terminale, un nucléotide modifié par 2'-O-méthyle, un nucléotide modifié par 2'-fluoro, un nucléotide modifié par 2'-désoxy, un nucléotide terminal lié à un dérivé de cholestéryle ou à un groupe bisdécylamide d'acide dodécanoïque, un nucléotide modifié par 2'-désoxy-2'-fluoro, un nucléotide bloqué, un nucléotide débloqué, un nucléotide à conformation restreinte, un éthyl-nucléotide contraint, un nucléotide abasique, un nucléotide modifié par 2'-amino, un nucléotide modifié par 2'-O-allyle, un nucléotide modifié par 2'-C-alkyle, un nucléotide modifié par 2'-hydroxyle, un nucléotide modifié par 2'-méthoxyéthyle, un nucléotide modifié par 2'-O-alkyle, un morpholino-nucléotide, un phosphoramidate, un nucléotide comprenant une base non naturelle, un nucléotide modifié par du tétrahydropyrane, un nucléotide modifié par du 1,5-anhydrohexitol, un nucléotide modifié par cyclohexényle, un nucléotide comprenant un groupe phosphorothioate, un nucléotide comprenant un groupe méthylphosphonate, un nucléotide comprenant un 5'-phosphate, et un nucléotide comprenant un mime de 5'-phosphate.

3. Agent ARNi double brin selon la revendication 1,
dans lequel pratiquement tous les nucléotides dudit brin sens et pratiquement tous les nucléotides dudit brin antisens sont des nucléotides modifiés, et
dans lequel ledit brin sens est conjugué au ligand rattaché à l'extrémité 3',
dans lequel de préférence tous les nucléotides dudit brin sens et tous les nucléotides dudit brin antisens sont des nucléotides modifiés, et
dans lequel de préférence ledit nucléotide modifié est choisi dans le groupe constitué par un nucléotide modifié par 2'-O-méthyle, un nucléotide comprenant un groupe 5'-phosphorothioate, un désoxy-nucléotide, un nucléotide avec une désoxythymine (dT) 3'-terminale, un nucléotide modifié par 2'-O-méthyle, un nucléotide modifié par 2'-fluoro, un nucléotide modifié par 2'-désoxy, un nucléotide terminal lié à un dérivé de cholestéryle ou un groupe bisdécylamide d'acide dodécanoïque, un nucléotide modifié par 2'-désoxy-2'-fluoro, un nucléotide bloqué, un nucléotide débloqué, un nucléotide à conformation restreinte, un éthyl-nucléotide contraint, un nucléotide abasique, un nucléotide modifié par 2'-amino, un nucléotide modifié par 2'-O-allyle, un nucléotide modifié par 2'-C-alkyle, un nucléotide modifié par 2'-hydroxyle, un nucléotide modifié par 2'-méthoxyéthyle, un nucléotide modifié par 2'-O-alkyle, un morpholino-nucléotide, un phosphoramidate, un nucléotide comprenant une base non naturelle, un nucléotide modifié par du tétrahydropyrane, un nucléotide modifié par du 1,5-anhydrohexitol, un nucléotide modifié par cyclohexényle, un nucléotide comprenant un groupe phosphorothioate, un nucléotide comprenant un groupe méthylphosphonate, un nucléotide comprenant un 5'-phosphate, et un nucléotide comprenant un mime de 5'-phosphate.

4. Agent ARNi double brin selon la revendication 1 à 3, dans lequel le ligand est
(a) un ou plusieurs dérivés de GalNAc rattachés par l'intermédiaire d'un bras de liaison ramifié divalent ou trivalent ;
(b) 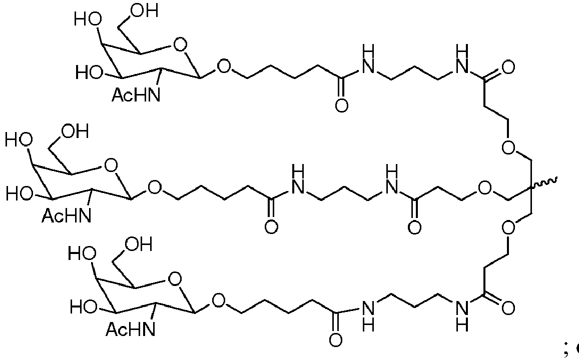 ou
(c) rattaché à l'extrémité 3' du brin sens.

5. Agent ARNi double brin selon la revendication 4(c), lequel agent ARNi est conjugué au ligand comme indiqué dans le schéma suivant : 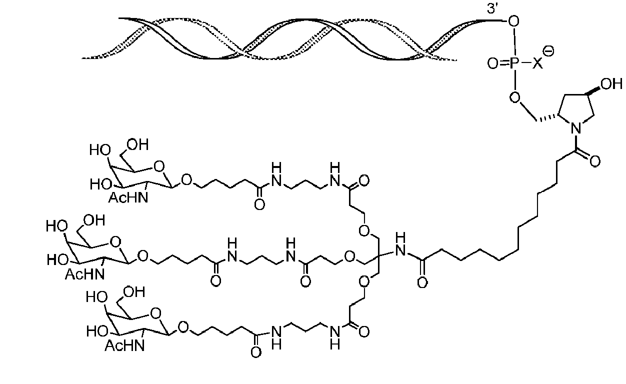 dans lequel X est O ou S.

6. Agent ARNi double brin selon l'une quelconque des revendications 1 ou 3, lequel agent ARNi comprend en outre au moins une liaison internucléotidique phosphorothioate ou méthylphosphonate.

7. Agent ARNi double brin selon la revendication 6, dans lequel
(a) la liaison internucléotidique phosphorothioate ou méthylphosphonate est à l'extrémité 3' d'un brin, de préférence ledit brin est le brin antisens ;
(b) la liaison internucléotidique phosphorothioate ou méthylphosphonate est aux deux extrémités 5' et 3' d'un brin ; ou
(c) ledit agent ARNi comprend 6 à 8 liaisons internucléotidiques phosphorothioate, de préférence le brin antisens comprend deux liaisons internucléotidiques phosphorothioate à l'extrémité 5' et deux liaisons internucléotidiques phosphorothioate à l'extrémité 3', et le brin sens comprend au moins deux liaisons internucléotidiques phosphorothioate soit à l'extrémité 5' soit à l'extrémité 3'.

8. Agent ARNi double brin selon la revendication 1, lequel agent ARNi double brin consiste en :
un brin sens consistant en AfscsGfuGfgUfgUfUfUfgUfcAfgCfaAfaUfl96 et un brin antisens consistant en asUfsuUfgCfuGfaCfaaaCfaCfcAfcGfuscsu ;
un brin sens consistant en GfsusGfgUfgUfuUfGfUfcAfgCfaAfaGfaUfL96 et un brin antisens consistant en asUfscUfuUfgCfuGfacaAfaCfaCfcAfcsgsu ;
un brin sens consistant en GfsgsUfgUfuUfgUfCfafgCfaAfaGfaUfgUfl96 et un brin antisens consistant en asCfsaUfcUfuUfgCfugaCfaAfaCfaCfcsasc ;
un brin sens consistant en GfsusGfuUfuGfuCfAfGfcAfaAfgAfuGfuAfL96 et un brin antisens consistant en usAfscAfuCfuUfuGfcugAfcAfaAfcAfcscsa ; ou
un brin sens consistant en UfsgsUfuUfgUfcAfGfCfaAfaGfaUfgUfgAfL96 et un brin antisens consistant en usCfsaCfaUfcUfuUfgcuGfaCfaAfaCfascsc ;
dans lequel a, g, c et u sont des nucléotides A, G, C et U modifiés par 2'-O-méthyle (2'-OMe), respectivement ; Af, Gf, Cf et Uf sont des nucléotides A, G, C et U modifiés par 2'-fluoro, respectivement ; s est une liaison phosphorothioate et L96 est un N-[tris(GalNAc-alkyl)amidodécanoyl)]-4-hydroxyprolinol Hyp-(GalNAc-alkyl)3.

9. Composition pharmaceutique comprenant l'agent ARNi double brin de l'une quelconque des revendications 1 à 8.

10. Méthode d'inhibition de l'expression de cétohexokinase (KHK) dans une cellule, la méthode comprenant :
(a) la mise en contact de la cellule avec l'agent ARNi double brin de l'une quelconque des revendications 1 à 8 ou la composition pharmaceutique de la revendication 12 ; et
(b) le maintien de la cellule produite dans l'étape (a) pendant un temps suffisant pour que soit obtenue une dégradation du produit de transcription d'ARNm d'un gène de KHK, inhibant ainsi l'expression du gène de KHK dans la cellule ;
à l'exclusion d'une quelconque méthode de traitement thérapeutique du corps humain ou animal.

11. Méthode selon la revendication 10, dans laquelle l'expression de KHK est inhibée d'au moins environ 30 %, environ 40 %, environ 50 %, environ 60 %, environ 70 %, environ 80 %, environ 90 %, environ 95 %, environ 98 % ou environ 100 %.

12. Agent ARNi double brin selon l'une quelconque des revendications 1 à 8 ou composition pharmaceutique selon la revendication 9 pour une utilisation dans une méthode de traitement d'un sujet ayant un trouble associé à la cétohexokinase (KHK), dans lequel de préférence le sujet est un humain.

13. ARNi double brin pour une utilisation selon la revendication 12, dans lequel la maladie associée à la cétohexokinase est choisie dans le groupe constitué par une maladie du foie, une dyslipidémie, des troubles du contrôle de la glycémie, une maladie cardiovasculaire, une maladie rénale, un syndrome métabolique, un dysfonctionnement des adipocytes, un dépôt adipeux viscéral, une obésité, une hyperuricémie, la goutte, des troubles de l'alimentation, et une envie de sucre excessive, dans lequel de préférence
(a) la maladie du foie est une maladie du foie gras et/ou une stéatose hépatique ;
(b) la dyslipidémie est choisie dans le groupe constitué par une hyperlipidémie, un cholestérol LDL élevé, un cholestérol HDL bas, une hypertriglycéridémie, et une hypertriglycéridémie postprandiale ;
(c) le trouble du contrôle de la glycémie est une résistance à l'insuline et/ou un diabète ;
(d) la maladie cardiovasculaire est une hypertension et/ou un dysfonctionnement des cellules endothéliales ; ou
(e) la maladie rénale est choisie dans le groupe constitué par un trouble rénal aigu, un dysfonctionnement tubulaire, et des changements pro-inflammatoires des tubules proximales.

14. ARNi double brin pour une utilisation selon la revendication 12, dans lequel l'agent ARNi double brin est destiné à être administré à une dose d'environ 0,01 mg/kg à environ 10 mg/kg ou d'environ 1 mg/kg à environ 10 mg/kg.
